(19) Europäisches Patentamt
European Patent Office
Office européen des brevets

(11) Publication number: 0 079 683
B1

(12) # EUROPEAN PATENT SPECIFICATION

(45) Date of publication of patent specification: 06.05.87

(21) Application number: 82305498.6

(22) Date of filing: 15.10.82

(51) Int. Cl.$^4$: C 07 D 405/12, C 07 D 409/12, C 07 D 491/04, A 01 N 47/36 // C07D307/79, C07D239/46, C07D333/54, C07D335/06, (C07D491/04, 307:00, 239:00)

(54) **Herbicidal sulfonamides.**

(30) Priority: 16.10.81 US 312183
27.08.82 US 410993
11.08.82 US 406191

(43) Date of publication of application:
25.05.83 Bulletin 83/21

(45) Publication of the grant of the patent:
06.05.87 Bulletin 87/19

(84) Designated Contracting States:
AT BE CH DE FR GB IT LI LU NL SE

(56) References cited:
EP-A-0 015 683
EP-A-0 099 339
US-A-4 127 405

(73) Proprietor: E.I. DU PONT DE NEMOURS AND COMPANY
1007 Market Street
Wilmington Delaware 19898 (US)

(72) Inventor: Rorer, Morris Padgett
64 Lower Valley Lane
Newark Delaware 19711 (US)
Inventor: Pasteris, Robert James
305 Plymouth Road Fairfax
Wilmington Delaware 19803 (US)

(74) Representative: Hildyard, Edward Martin et al
Frank B. Dehn & Co. Imperial House
15-19 Kingsway
London WC2B 6UZ (GB)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 079 683 B1

Courier Press, Leamington Spa, England.

**Description**

Background of the Invention

This invention relates to novel benzofuran, benzothiophene, 1-benzopyran, 1-benzothiopyran, 1-benzoxepin, and 1-benzothiepin sulfonamides and their agriculturally suitable salts which are useful as plant growth regulants and herbicides. The invention also includes intermediates useful for the synthesis of these compounds.

Netherlands Patent 121,788, published September 15, 1966, discloses the preparation of compounds of the following Formula and their use as general or selective herbicides:

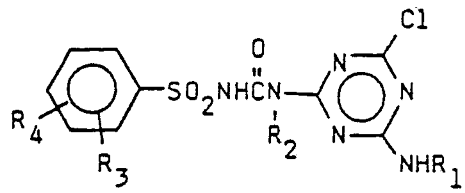

(i)

wherein

$R_1$ and $R_2$ may independently be alkyl of 1—4 carbon atoms; and

$R_3$ and $R_4$ may independently be hydrogen, chlorine or alkyl of 1—4 carbon atoms.

U.S. Patent 3,637,366 discloses compounds having the formula:

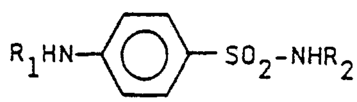

wherein

$R_1$ is hydrogen or lower saturated aliphatic acyl and

$R_2$ is hydrogen, 2-pyrimidinyl, pyridyl, amidino, acetyl or carbamoyl.

The disclosed compounds are said to provide control of crabgrass, cress, endive, clover and *Poa annua*.

French Patent No. 1,468,747 discloses the following *para*-substituted phenylsulfonamides as being useful as antidiabetic agents:

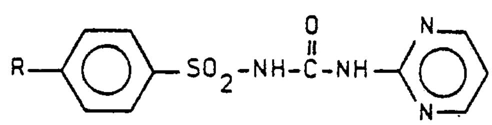

wherein

R = H, halogen, $CF_3$ or alkyl.

Logemann et al. Chem. Ab., *53*, 18052 g (1959), disclose a number of sulfonamides, including uracil derivatives and those having the formula:

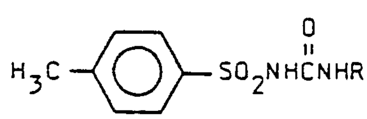

wherein

R is butyl, phenyl or 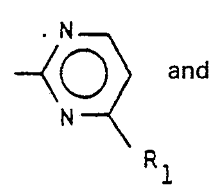 and

$R_1$ is hydrogen or methyl.

When tested for hypoglycemic effect in rats (oral doses of 25 mg/100 g), the compounds in which R is butyl and phenyl were most potent. The others were of low potency or inactive.

Wojciechowski, J. Acta. Polon. Pharm. *19*, p. 121—5 (1962) [Chem. Ab., *59* 1633 e] describes the synthesis of N-[(2,6-dimethoxypyrimidin-4-yl)aminocarbonyl]-4-methylbenzenesulfonamide;

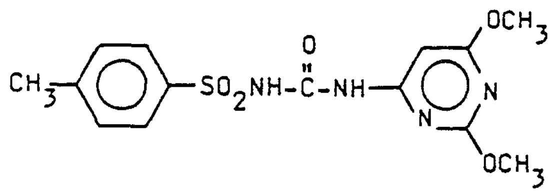

U.S. Patent 4,127,405 teaches compounds which are useful for controlling weeds in wheat having the formula

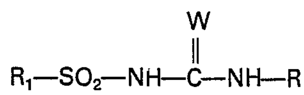

wherein

R is 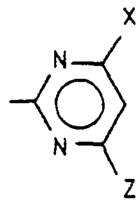 or 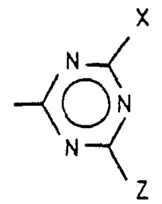 ;

$R_1$ is 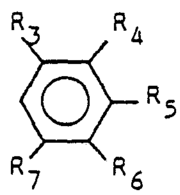 , 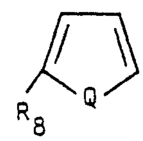 ,

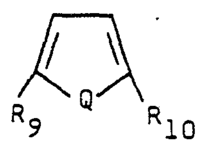 or 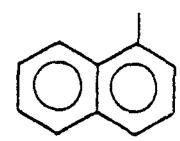 ;

$R_3$ and $R_6$ are independently hydrogen, fluorine, chlorine, bromine, iodine, alkyl of 1—4 carbon atoms, alkoxy of 1—4 carbon atoms, nitro, trifluoromethyl, cyano, $CH_3S(O)_n$— or $CH_3CH_2S(O)_n$—;

$R_4$ is hydrogen, fluorine, chlorine, bromine or methyl;

$R_5$ is hydrogen, fluorine, chlorine, bromine, methyl or methoxy;

$R_7$ is hydrogen, fluorine, chlorine, bromine, alkyl of 1—2 carbon atoms or alkoxy of 1—2 carbon atom;

$R_8$ is hydrogen, methyl, chlorine or bromine;

$R_9$ and $R_{10}$ are independently hydrogen, methyl, chlorine or bromine;

W and Q are independently oxygen or sulfur;

n is 0, 1 or 2;

X is hydrogen, chlorine, bromine, methyl, ethyl, alkoxy of 1—3 carbon atoms, trifluoromethyl, $CH_3S$— or $CH_3OCH_2$—; and

Z is methyl or methoxy;

or their agriculturally suitable salts; provided that:

(a) when $R_5$ is other than hydrogen, at least one of $R_3$, $R_4$, $R_6$ and $R_7$ is other than hydrogen and at least two of $R_3$, $R_4$, $R_6$ and $R_7$ must be hydrogen;

(b) when $R_5$ is hydrogen and all of $R_3$, $R_4$, $R_6$ and $R_7$ are other than hydrogen, then all of $R_3$, $R_4$, $R_6$ and $R_7$ must be either chlorine or methyl; and

(c) when $R_3$ and $R_7$ are both hydrogen, at least one of $R_4$, $R_5$ or $R_6$ must be hydrogen.

In addition, EP—A—35893 teaches *o*-alkylsulfonylbenzenesulfonylureas which are useful as herbicides.

IN EP—A—45196 there is a disclosure of herbicidal benzo[b]thiophene- and benzofuransulfonylureas in which the sulfonylureido group is bonded to the heterocyclic ring.

Our EP—A—15683 also discloses *o*-substituted benzenesulfonylureas useful as herbicides.

The presence of undesired vegetation causes substantial damage to useful crops, especially agricultural products that satisfy man's basic food and fiber needs, such as cotton, rice, corn, wheat, and

the like. The current population explosion and concomitant world food and fiber shortage demand improvements in the efficiency of producing these crops. Prevention or minimizing the loss of a portion of such valuable crops by killing, or inhibiting the growth of undesired vegetation is one way of improving this efficiency. A wide variety of materials useful for killing, or inhibiting (controlling) the growth of undesired vegetation is available; such materials are commonly referred to as herbicides. The need still exists however, for more effective herbicides.

Summary of the Invention

This invention relates to compounds of Formula I, agricultural compositions containing them and their method-of-use as general or selective pre-emergent or post-emergent herbicides or plant growth regulants.

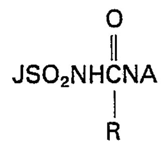

I

wherein

J is

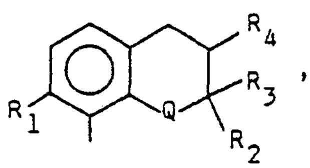 ,

J1

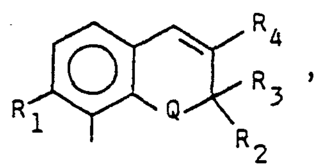 ,

J2

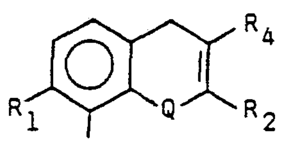 ,

J3

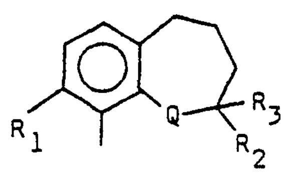 ,

J4

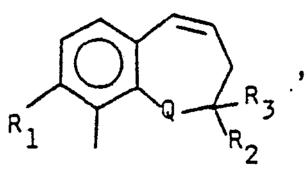 ,

J5

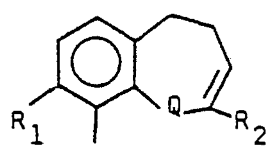

J6

or 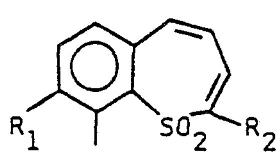 ;

J7

and

Q is O, S or $SO_2$;

R is H or $CH_3$;

$R_1$ is H, $CH_3$, $OCH_3$, Cl, Br, $CO_2R_5$, $SO_2R_6$, $OSO_2R_7$ or $SO_2NR_8R_9$;

$R_2$ and $R_3$ are independently H or $C_1$—$C_3$ alkyl;
$R_4$ is H or $CH_3$;
$R_5$ is $C_1$—$C_3$ alkyl, $CH_2CH{=}CH_2$, $CH_2CH_2OCH_3$ or $CH_2CH_2Cl$;
$R_6$ is $C_1$—$C_3$ alkyl;
$R_7$ is $C_1$—$C_3$ alkyl or $CF_3$;
$R_8$ and $R_9$ are independently $C_1$—$C_2$ alkyl;
A is

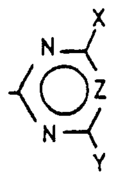 , 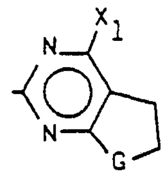 ,

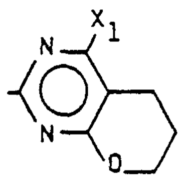 or 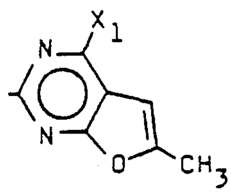 ;

G is O or $CH_2$;
X is $CH_3$, $OCH_3$ or Cl;
$X_1$ is $CH_3$, $OCH_3$ or $OC_2H_5$;
Y is $CH_3$, $OCH_3$, $OC_2H_5$, $CH_2OCH_3$, $C_2H_5$, $NH_2$, $NHCH_3$ or $N(CH_3)_2$;
Z is CH or N; and

their agriculturally suitable salts provided that

1) when X is Cl, then Z is CH and Y is $OCH_3$, $NH_2$, $NHCH_3$, $N(CH_3)_2$ or $OC_2H_5$;
2) the total number of carbon atoms in $R_2$ and $R_3$ are less than or equal to 3;
3) In Structure $J_2$ when $R_2$ and $R_3$ is other than H, then $R_4$ is H;
4) In Structure $J_3$ when $R_2$ is other than H, then $R_4$ is H;
5) when Q is S, then $R_1$ is not $SO_2NR_8R_9$;
6) In Structures $J_3$ and $J_6$, Q may not be 0;
7) In Formula $J_7$, $R_1$ is not $CH_3$;
8) when J is $J_1$ wherein Q is O and $R_1$, $R_2$, $R_3$ and $R_4$ are H and A is

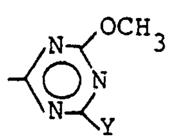 , then

Y is $OC_2H_5$, $CH_2OCH_3$, $C_2H_5$, $NH_2$, or $NHCH_3$; and
9) when J is $J_1$ wherein Q is O and $R_1$, $R_2$, $R_3$ and $R_4$ are H and A is

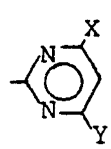 ,

wherein X is $CH_3$ or $OCH_3$, then Y is $OC_2H_5$, $CH_2OCH_3$, $C_2H_5$, $NH_2$, $NHCH_3$ or $N(CH_3)_2$.

Preferred for reasons of their higher herbicidal activity, greater plant growth regulant activity, or more favorable ease of synthesis are:

1) Compounds of Formula I where J is $J_1$
2) Compounds of Preferred 1 where R is H
3) Compounds of Preferred 2 where $R_1$ is H
4) Compounds of Preferred 3 where

A is 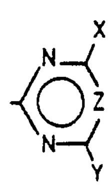 and X is $OCH_3$ or Cl

5) Compounds of Preferred 4 where Y is $CH_3$ or $OCH_3$
6) Compounds of Formula I where J is $J_2$
7) Compounds of Preferred 6 where R is H
8) Compounds of Preferred 7 where $R_1$ is H
9) Compounds of Preferred 8 where

A is 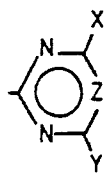 and X is $OCH_3$ or Cl

10) Compounds of Preferred 9 where Y is $CH_3$ or $OCH_3$
11) Compounds of Formula I where J is $J_3$
12) Compounds of Preferred 11 where R is H
13) Compounds of Preferred 12 where $R_1$ is H
14) Compounds of Preferred 13 where

A is 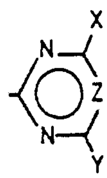 and X is $OCH_3$ or Cl

15) Compounds of Preferred 14 where Y is $CH_3$ or $OCH_3$
16) Compounds of Formula I where J is $J_4$
17) Compounds of Preferred 16 where R is H
18) Compounds of Preferred 17 where $R_1$ is H
19) Compounds of Preferred 18 where

A is 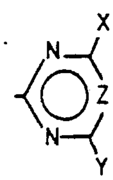 and X is $OCH_3$ or Cl

20) Compounds of Preferred 19 where Y is $CH_3$ or $OCH_3$
21) Compounds of Formula I where J is $J_4$
22) Compounds of Preferred 21 where R is H
23) Compounds of Preferred 22 where $R_1$ is H
24) Compounds of Preferred 23 where

A is 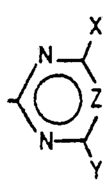 and X is $OCH_3$ or Cl

25) Compounds of Preferred 24 where Y is $CH_3$ or $OCH_3$
26) Compounds of Formula I where J is $J_6$
27) Compounds of Preferred 26 where R is H
28) Compounds of Preferred 27 where $R_1$ is H
29) Compounds of Preferred 28 where

A is 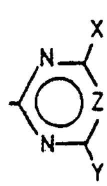 and X is $OCH_3$ or Cl

30) Compounds of Preferred 29 where Y is $CH_3$ or $OCH_3$
31) Compounds of Formula I where J is $J_7$
32) Compounds of Preferred 31 where R is H
33) Compounds of Preferred 32 where $R_1$ is H
34) Compounds of Preferred 33 where

A is 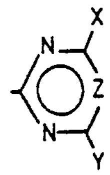 and X is $OCH_3$ or Cl

35) Compounds of Preferred 34 where Y is $CH_3$ or $OCH_3$

Specifically preferred for reasons of their highest herbicidal activity, greatest plant growth regulant activity and/or ease of synthesis are:

N-[(4-methoxy-6-methylpyrimidin-2-yl)aminocarbonyl]-3,4-dihydro-2H-1-benzothiopyran-8-sulfonamide, 1,1-dioxide;

N-[(4,6-dimethoxypyrimidin-2-yl)aminocarbonyl]-3,4-dihydro-2H-1-benzothiopyran-8-sulfonamide, 1,1-dioxide; and

N-[(4-methoxy-6-methyl-1,3,5-triazin-2-yl)aminocarbonyl]-3,4-dihydro-2H-1-benzothiopyran-8-sulfonamide, 1,1-dioxide.

This invention also relates to compounds of Formula I', suitable agricultural compositions containing them, and their use as pre-emergence and/or post-emergence herbicides.

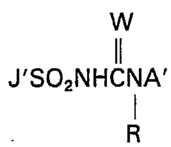

I'

wherein J' is

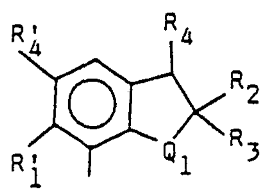

J'1

,

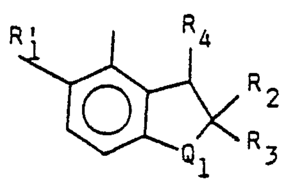

J'2

,

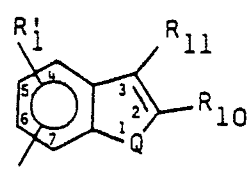

J'3

and

Q is O, S or $SO_2$;
$Q_1$ is O, S, SO or $SO_2$;
R is H or $CH_3$;
$R_1'$ is H, $CH_3$, $OCH_3$, Cl, Br, $NO_2$, $CO_2R_5$, $SO_2R_6$, $OSO_2R_7$ or $SO_2NR_8R_9$;
$R_2$ is H or $C_1$—$C_3$ alkyl;
$R_3$ is H or $CH_3$;
$R_4$ is H or $CH_3$;
$R_4'$ is H, Cl, $CH_3$, $CF_3$, $OCH_3$ or Br;
$R_5$ is $C_1$—$C_3$ alkyl, $CH_2CH{=}CH_2$, $CH_2CH_2OCH_3$ or $CH_2CH_2Cl$;
$R_6$ is $C_1$—$C_3$ alkyl;
$R_7$ is $C_1$—$C_3$ alkyl or $CF_3$;
$R_8$ and $R_9$ are independently $C_1$—$C_2$ alkyl;
$R_{10}$ is H, Cl, Br or $C_1$—$C_3$ alkyl;
$R_{11}$ is H, $CH_3$, Cl or Br;
W is O or S;

A′ is

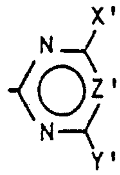 , 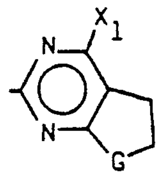 , 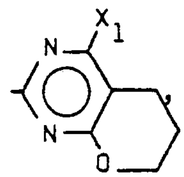 .

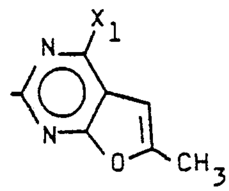 or 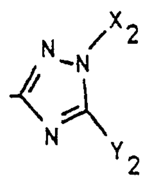 ;

G is O or $CH_2$;
X′ is H, $CH_3$, $OCH_3$ or Cl;
$X_1$ is $CH_3$, $OCH_3$ or $OC_2H_5$;
$X_2$ is $CH_3$, $C_2H_5$ or $CH_2CF_3$;
Y′ is $CH_3$, $OCH_3$, $OC_2H_5$, $CH_2OCH_3$, $NH_2$, $NHCH_2$, $N(CH_3)_2$, $CH(OCH_3)_2$,

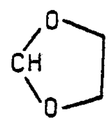 ,

$C_2H_5$, $CF_3$, $CH_2{=}CHCH_2O$, $SCH_3$, $HC{\equiv}CCH_2O$ or $CF_3CH_2O$;
$Y_2$ is $C_2H_5$, $CH_3$, $OCH_3$, $OC_2H_5$, $SCH_3$ or $SC_2H_5$; and
Z′ is CH, N, $CCH_3$, $CC_2H_5$, CCl or CBr; and their agriculturally suitable salts provided that

1) In Structures $J_1'$ and $J_2'$ when $Q_1$ is O and $R_1'$ is other than H, Cl, Br or $NO_2$, then at least one of $R_2$ and $R_3$ is alkyl, and when $Q_1$ is S, then $R_1'$ cannot be $NO_2$;
2) In Structure $J_3'$, when $R_1'$ is $NO_2$ or $SO_2NR_8R_9$, then $R_{10}$ is $C_1$—$C_3$ alkyl and $R_{11}$ is $CH_3$;
3) when X′ is Cl, then Z′ is CH and Y′ is $OCH_3$, $NH_2$, $NHCH_3$ or $N(CH_3)_2$;
4) when $Q_1$ is SO, then W is 0; and
5) when $R_4'$ is other than H, then $R_1'$ is H.

Preferred for reasons of their higher herbicidal activity or more favorable ease of synthesis are:
1) Compounds of the generic scope of Formula I′ where J′ is $J_1'$ and $R_4$ is H.
2) Compounds of Preferred 1 where W is O and R is H.
3) Compounds of Preferred 2 where $R_1'$ is H, Cl, $CH_3$, $OCH_3$, $CO_2R_5$ or $SO_2R_6$.
4) Compounds of Preferred 3 where $R_4'$ and $R_1$are H and $R_2$ and $R_3$ are independently H or $CH_3$.
5) Compounds of Preferred 4 where

A′ is 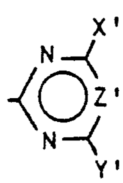 ; Z′ is CH or N; and

X′ is $CH_3$, $OCH_3$ or Cl.
6) Compounds of Preferred 5 where Y′ is $CH_3$, $OCH_3$, $CH_2OCH_3$ or $N(CH_3)_2$.
7) Compounds of the generic scope of Formula I where J′ is $J_2$′.
8) Compounds of Preferred 7 where W is O and R is H.
9) Compounds of Preferred 8 where $R_1'$ is H, Cl, $CH_3$, $OCH_3$, $CO_2R_5$ or $SO_2R_6$.
10) Compounds of Preferred 9 where $R_1$′ is H and $R_2$ and $R_3$ are independently H or $CH_3$.
11) Compounds of Preferred 10 where

A′ is 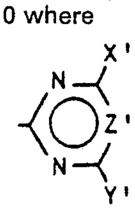 ; Z′ is CH or N; and

X′ is $CH_3$, $OCH_3$ or Cl.

12) Compounds of Preferred 11 where Y′ is $CH_3$, $OCH_3$, $CH_2OCH_3$ or $N(CH_3)_2$.

13) Compounds of the generic scope of Formula I′ where J′ is $J_3'$.

14) Compounds of Preferred 13 where W is O and R is H.

15) Compounds of Preferred 14 where $R_{11}$ is H, and $R_1'$ is H, Cl, $CH_3$, $OCH_3$, $CO_2R_5$ or $SO_2R_6$ and where $SO_2NHC(W)NRA'$ is bonded to the 7-position.

16) Compounds of Preferred 15 where $R_1'$ is H and $R_{10}$ is H or $CH_3$.

17) Compounds of Preferred 16 where

A′ is 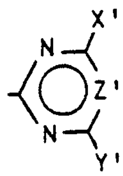 ; Z′ is CH or N; and X′ is $CH_3$, $OCH_3$ or Cl.

18) Compounds of Preferred 17 where Y′ is $CH_3$, $OCH_3$, $CH_2OCH_3$ or $N(CH_3)_2$.

Specifically preferred for reasons of their highest herbicidal activity, greatest plant growth regulant activity and/or ease of synthesis are:

N-[(4,6-dimethylpyrimidin-2-yl)aminocarbonyl]-2,3-dihydro-2-methyl-7-benzothiophenesulfonamide, 1,1-dioxide;

N-[(4-methoxy-6-methylpyrimidin-2-yl)aminocarbonyl]-2,3-dihydro-2-methyl-7-benzothiophenesulfonamide, 1,1-dioxide;

N-[(4,6-dimethoxypyrimidin-2-yl)aminocarbonyl]-2,3-dihydro-2-methyl-7-benzothiophenesulfonamide, 1,1-dioxide;

N-[(4,6-dimethyl-1,3,5-triazin-2-yl)aminocarbonyl]-2,3-dihydro-2-methyl-7-benzothiophenesulfonamide, 1,1-dioxide;

N-[(4-methoxy-6-methyl-1,3,5-triazin-2-yl)aminocarbonyl]-2,3-dihydro-2-methyl-7-benzothiophenesulfonamide, 1,1-dioxide;

N-[(4,6-dimethoxy-1,3,5-triazin-2-yl)aminocarbonyl]-2,3-dihydro-2-methyl-7-benzothiophenesulfonamide, 1,1-dioxide;

2,3-dihydro-N-[(4-methoxy-6-methylpyrimidin-2-yl)aminocarbonyl]-2-methylbenzofuran-7-sulfonamide;

N-[(4,6-dimethoxypyrimidin-2-yl)aminocarbonyl]-2,3-dihydro-2-methylbenzofuran-7-sulfonamide;

N-[(4,6-dimethoxypyrimidin-2-yl)aminocarbonyl]-2,3-dihydrobenzo[b]thiophene-7-sulfonamide, 1,1-dioxide; and

2,3-dihydro-N-[(4-methoxy-6-methylpyrimidin-2-yl)aminocarbonyl]benzo[b]thiophene-7-sulfonamide, 1,1-dioxide.

Detailed Description of the Invention

*Synthesis*

The compounds of Formulae (I) and (I′) can be prepared by methods described below in Equations 1, 2 and 3.

As shown in Equation 1a, compounds of Formula (I) can be prepared by reacting a sulfonylcarbamate of Formula (II) with an appropriate amine of Formula (III). J, R and A are as previously defined.

Equation 1a

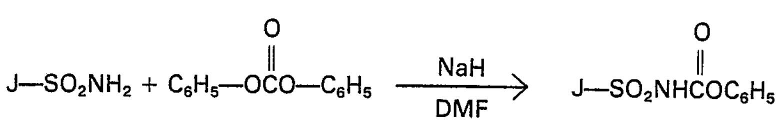


IV II

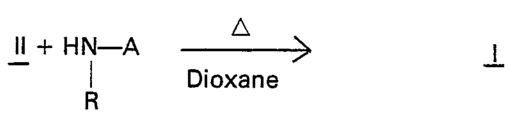


III

Similarly, as shown in Equation 1b, compounds of Formula (I′) (where W=O) can be prepared by

reacting a sulfonylcarbamate of Formula (II') with an appropriate amine of Formula (III'). J', R and A' are as previously defined.

Equation 1b

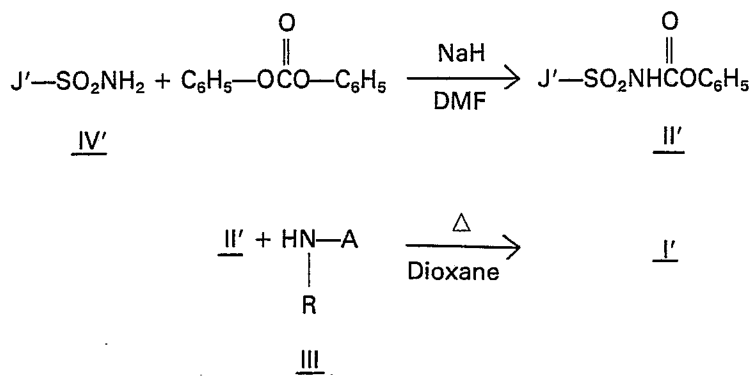

IV'
II'
III
I'

The reactions are carried out at 50—100°C in a solvent such as dioxane for 1/2 to 24 hours as taught in EP—A—0044807. The required carbamates (II) or (II') are prepared by reacting the corresponding sulfonamides (IV) or (IV'), respectively, with diphenylcarbonate in the presence of a strong base.

Alternatively, as shown in Equation 2a, compounds (I), where $R_1$ is other than $CO_2R_5$, can be prepared by reacting sulfonamides of Formula (IV) with an appropriate methylcarbamate of Formula (V) in the presence of an equimolar amount of trimethylaluminum.

Equation 2a

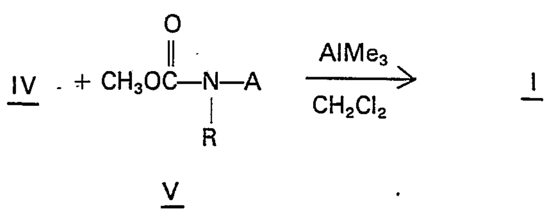

IV
V
I

Similarly, as shown in Equation 2b, compounds I', where $R_1'$ is other than $CO_2R_5$ and W=O, can be prepared.

Equation 2b

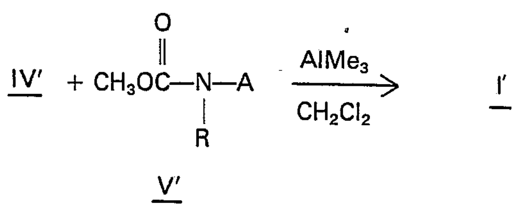

IV'
V'
I'

The reactions of Equations 2a and 2b are carried out at 25° to 40°C in a solvent such as methylene chloride for 10 to 96 hours under an inert atmosphere.

The reaction of equations 2a and 2b is best run in methylene chloride at about 25° to 40°C for 10 to 96 hours under a nitrogen atmosphere. The products can be isolated by addition of an aqueous acetic acid solution followed by extraction of the products into methylene chloride, or by filtration of a product of low solubility. The products can be purified by trituration with solvents such as 1-chlorobutane, ethyl acetate or ethyl ether or by column chromatography on silica gel.

The compounds of Formula (I) where J is $J_1$, $J_4$ and $J_7$ for all values of Q and $J_2$, $J_3$, $J_4$ and $J_6$ when Q is $SO_2$ can be prepared by reacting sulfonylisocyanates of Formula (VI) with an appropriate amine of Formula (III) as shown in Equation 3a.

Equation 3a

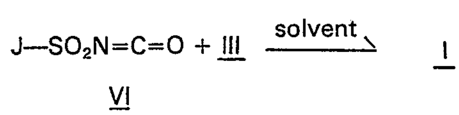

VI
III
I

Similarly, compounds of Formula (I′) can be prepared as shown in Equation 3b.

Equation 3b

$$\underset{\underline{VI'}}{J'\text{—}SO_2N{=}C{=}W} + \underline{III'} \xrightarrow{\text{solvent}} \underline{I'}$$

The reactions of Equations 3a and 3b are carried out at 25° to 40°C in an inert aprotic solvent such as methylene chloride for 0.5 to 24 hours as taught in U.S. Patent 4,127,405.

The intermediate sulfonylisocyanates (VI) or (VI′) (W=O), can be prepared from the appropriate sulfonamides (IV) or (IV′), respectively, by the reaction with phosgene, in the presence *n*-butylisocyanate and a tertiary amine catalyst, at reflux in a solvent such as xylene by the method of U.S. Patent 4,238,621.

Alternatively, the sulfonylisocyanates can be prepared from the sulfonamides by a two step procedure involving (a) reacting the sulfonamides with *n*-butylisocyanate in the presence of a base such as $K_2CO_3$ at reflux in an inert solvent such as 2-butanone forming a *n*-butylsulfonylurea; and (b) reacting this compound with phosgene and a tertiary amine catalyst at reflux in xylene solvent. The method is similar to a procedure taught by Ulrich and Sayigh, *Newer Methods of Preparative Organic Chemistry,* Vol. VI, p. 223—241, Academic Press, New York and London, W. Foerst Ed.

The intermediate sulfonylisothiocyanates of Formula VI′ (W=S) in Equation 3b can be prepared from sulfonamides by methods taught in H. Hartke, *Arch. Pharm., 229,* 174 (1966). The method requires (a) reacting an appropriate sulfonamide with an equivalent amount of carbon disulfide and two equivalents of potassium hydroxide in dimethylformamide at room temperature for 1—8 hours to form the dipotassium salt of the sulfonyliminodithiocarbonate; (b) diluting the suspension with ethyl acetate, ethyl ether or similar aprotic solvent to cause the salt to precipitate, (c) reacting the isolated, dried salt with phosgene in an inert solvent such as xylenes, benzene, carbon tetrachloride or methylene chloride at about room temperature for 1—3 hours; and (d) isolating the sulfonylisothiocyanate, which is usually soluble in the solvent, by filtering off the insoluble potassium chloride and concentrating the filtrate. In place of phosgene, a chloroformic ester (e.g., methyl chloroformate), phosphorus pentachloride, sulfuryl chloride or thionyl chloride may be used.

The sulfonylisothiocyanates VI′ (W=S) may tend to be unstable and dimerize readily, however, the dimers can be used in the same manner as the parent isothiocyanates for the purposes of this invention.

The sulfonamides of Formula IV and IV′ in Equations 1, 2 and 3 are important intermediates for the preparation of the compounds of this invention. The synthesis of the required sulfonamide intermediates are described in Equations 4, 5, 6 and 7.

As shown in Equation 4a, sulfonamides of Formula IV, where J is $J_1$, $J_2$, $J_4$, $J_5$ and $J_7$ for all Q, can be prepared from the corresponding sulfonyl chlorides of Formula VII.

Equation 4a

$$\underset{\underline{VIII}}{J\text{—}NH_2} \xrightarrow[]{\substack{1)\ HONO/HCl \\ 2)\ SO_2/CuCl_2/HOAc}} \underset{\underline{VII}}{J\text{—}SO_2Cl}$$

$$\xrightarrow[\text{or } NH_3]{NH_4OH} \underline{IV}$$

Similarly, as shown in Equation 4b, sulfonamides of Formula IV′ can be prepared from the corresponding sulfonyl chlorides of Formula VII′.

Equation 4b

$$\underset{\underline{VIII'}}{J\text{—}NH_2} \xrightarrow[]{\substack{1)\ HONO/HCl \\ 2)\ SO_2/CuCl_2/HOAc}} \underset{\underline{VII'}}{J'\text{—}SO_2Cl}$$

$$\xrightarrow[\text{or } NH_3]{NH_4OH} \underline{IV'}$$

The preparation of sulfonamides from ammonium hydroxide and sulfonyl chlorides is widely reported in the literature, e.g., Crossley et al., *J. Am. Chem. Soc., 60,* 2223 (1938) and Pailer, *Monatsh., 92,* 677 (1961). Preparation utilizing the reaction of sulfonyl chlorides with an excess of anhydrous ammonia at 0°C in ethyl ether or chlorobutane is also known.

The sulfonyl chlorides VII or VII′ can be prepared from the appropriate amine VIII or VIII′, respectively, by diazotization with sodium nitrite in HCl, followed by reaction of the diazonium salt with sulfur dioxide and cupric chloride in acetic acid analogous to the teachings of Yale and Sowinski, *J. Org. Chem., 25,* 1824 (1960).

Alternatively, sulfonyl chlorides of Formula VII and VII′ can be prepared by a modification of the above procedure whereby the diazotization reaction is carried out in dilute sulfuric acid (20—50%). at 0° to 10°C for 0.2 to 1 hour. The resulting diazonium salt is reacted with sulfur dioxide, HCl and cupric chloride in a cosolvent mixture consisting of acetic acid-water (1:1) and an immiscible, inert solvent such as 1-chlorobutane or methylene chloride at 0°—40°C for 1 to 24 hours. The mode of addition is not critical; it is however, often convenienbt to add the diazonium salt to the suspension containing the sulfur dioxide. The sulfonyl chlorides are isolated by addition of water, separation of the organic phase, washing the organic phase with saturated aqueous $NaHCO_3$ and water and evaporation of the solvent under reduced pressure at less than about 50°C.

Sulfonamides of Formula IVa, where n is 1 or 2, are prepared from their corresponding saturated analogs IVb as shown in Equation 5. $R_1$, $R_2$, and $R_4$ are as previously defined. When n is 2, $R_4$ is H.

Equation 5

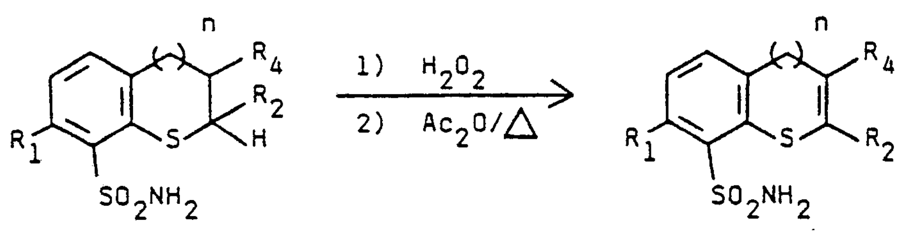


IVb IVa

The reaction is carried out by contacting the saturated sulfide with one equivalent of a suitable oxidizing agent such as hydrogen peroxide in a solvent such as acetic acid to produce the corresponding sulfoxide. The sulfoxide is then heated with acetic anhydride with or without a cosolvent at 50° to 140°C for 2 to 24 hours. This is similar to the method taught by Parham and Koncos, *J. Amer. Chem. Soc., 83,* 4034 (1961) for the preparation of 4H-1-benzothiopyran.

Sulfonamides of Formula IVc where n is 0 or 1 can also be prepared from the corresponding saturated analogs IVd by the two step sequence shown in Equation 6. $R_1$, $R_2$, $R_3$, $R_4$ and Q are as previously described except that $R_1$ is not $CH_3$. When n is 1, $R_4$ is H.

Equation 6

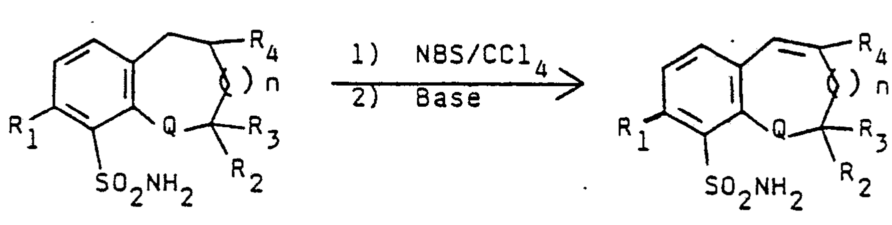


IVd IVc

The first step involves benzylic bromination by N-bromosuccinimide in refluxing carbon tetrachloride to give a monobromo derivative. The monobromide is dehydrobrominated in the second step by contacting with a suitable base such as DABCO or ethoxide in an inert solvent such as benzene. This method has been used to prepare 2H-1-benzopyran from dihydrobenzopyran (Clemo and Ghatge, *J. Chem. Soc.,* 4347 (1955)).

Applying the above procedure a second time to the compounds IVc where n is 1, Q is $SO_2$ and $R_3$ is H will provide sulfonamides IV where J is $J_7$, as taught by Traynelis and Love, *J. Org. Chem., 26,* 2728 (1961).

Sulfonamides of Formula IV′a, where J′ is $J_1'$ and Q is S and $R_1'$ and $R_4'$ are H, can also be prepared from *N-t*-butylbenzenesulfonamide as shown in Equation 7.

Equation 7

(a)

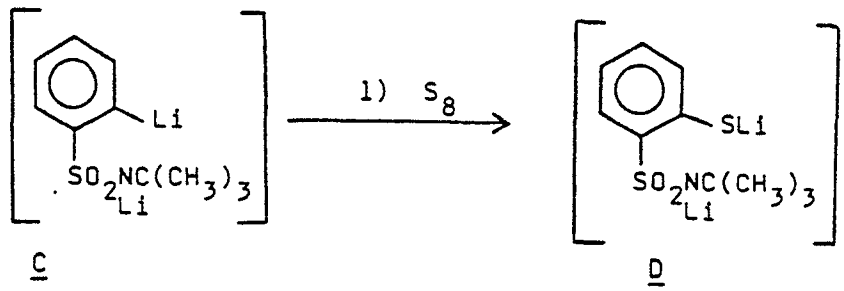


C D

[ D ]

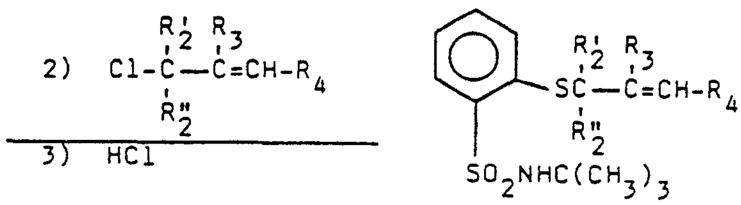


E

b)

E

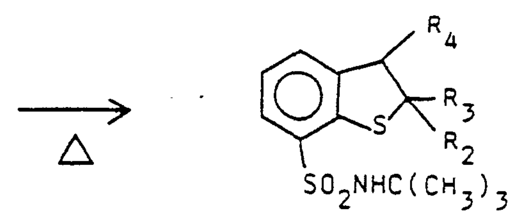

F

c)

F

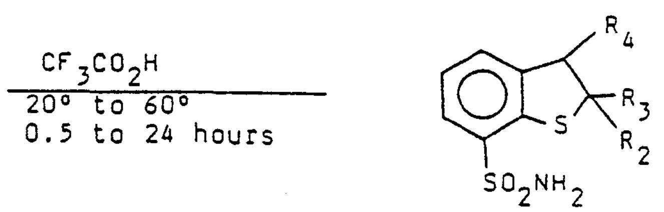


(VI'a)

wherein

$R_2$ is $C_1$—$C_3$ alkyl;

$R_3$ and $R_4$ are as previously defined;

$R_2'$ is H or $CH_3$; and

$R_2''$ is H, $CH_3$ or $C_2H_5$; when $R_2''$ is $C_2H_5$, $R_2'$ is H.

The reactions of Equation 7(a) are run *in-situ* as follows: The dilithium salt *C* is prepared by reacting *N*-*t*-butylbenzenesulfonamide with two equivalents of *n*-butyl lithium at 0° to 30°C in an inert solvent such as tetrahydrofuran for one to five hours, according to the teachings of Lombardino, *J. Org. Chem., 36,* 1843 (1971). The *N*-*t*-butyl-2-propenylthiobenzenesulfonamide of Formula *E* is then prepared by (1) contacting this mixture containing *C* with elemental sulfur at 0° and stirring at ambient temperature for one to five hours to form lithium thiolate *D*; (2) contacting this mixture with an appropriate allyl halide at 0° and stirring at ambient temperature for 0.5 to 24 hours to form *E;* and (3) isolating *E* by addition of dilute hydrochloric

acid to this mixture to decompose any salts present, followed by separation and concentration of the organic phase. The reaction of organolithium reagents with sulfur to form lithium thiolates which may be alkylated *in-situ* is known in the art, e.g. Gschwend et al., "Organic Reactions", *26,* Chapter 1, p. 83 (1979) and references cited therein.

In Reaction 7(b) above, the *N-t*-butyl-2,3-dihydro-7-benzo[b]thiophenesulfonamides of Formula *F* are prepared by heating *E* either neat or in an inert solvent such as quinoline or N,N-dimethylaniline at 150—300°C for 0.25 to 2 hours to cause cyclization. Compound *F* is isolated by addition of an inert solvent such as ether or methylene chloride, washing well with dilute hydrochloric acid and water, followed by separation and concentration of the organic phase. Compound F can be purified by column chromatography and recrystallization procedures.

And in Reaction 7(c), the *t*-butylsulfonamides *F* are dealkylated to form sulfonamides VI'a by reacting *F* with excess trifluoroacetic acid at 20° to 40°C for about 10 to 30 hours. Compounds VI'a are isolated and purified by concentration of the reaction mixture, addition of methylene chloride to the residue, washing the suspension with dilute aqueous $NaHCO_3$ and concentration of the organic phase. Alternatively, *t*-butylsulfonamides *F* may be converted to sulfonamides VI'a by heating in methanol containing at least an equimolar quantity of hydrochloric acid, followed by concentration of the reaction mixture and precipitation of the product with ether.

Finally, sulfonamides of Formula IV where Q is $SO_2$ can be prepared from the appropriate sulfonamides IV where Q is S by a variety of standard literature procedures with *m*-chloroperbenzoic acid (Johnson et. al., *Tetrahedron 25,* 5649 (1969), or with aqueous hydrogen peroxide in acetic acid (Bordwell et. al., *J. Amer. Chem. Soc., 77,* 1141 (1955)).

The amines of Formula VIII in Equation 4a, where J is $J_1$, $J_2$, $J_4$, $J_5$ and $J_7$ for all Q, can be prepared by reduction of the corresponding nitro compounds of Formula IX, as shown in Equation 8.

Equation 8

$$\underset{\text{IX}}{J\text{—}NO_2} \xrightarrow{\text{Reduction}} \text{VIII}$$

The reduction of nitro compounds to amines can be carried out by any of several known methods as described in *Preparative Organic Chemistry,* 4 Ed., p. 557—563, John Wiley and Sons, New York and London, G. Hilgetag and A. Martini Ed.

Alternatively, the amines of Formula VIIIa can be prepared by a thioClaisen rearrangement of an appropriate allyl phenylthio ether X as outlined in Equation 9. $R_1$, $R_2$, $R_3$ and $R_4$ are as previously defined.

Equation 9

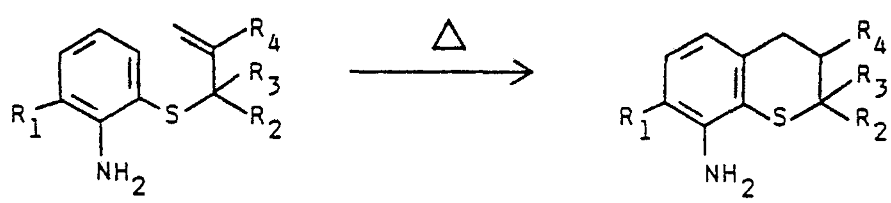


X VIIIa

The rearrangements are carried out at 180° to 250°C for 0.5 to 24 hours either neat or in the presence of a high boiling amine solvent such as quinoline or diethylaniline as taught by S. J. Rhoads and N. R. Raulins, *Organic Reactions,* Vol. 22, p. 1—252, John Wiley and Sons, New York and London, W. G. Dauben, Editor in Chief. The 6-ring fused product VIIIa is accompanied by a 5-ring fused isomer (see Equation 23) and the desired amine VIIIa can be separated by either fractional crystallization, fractional distillation or by a chromatographic method. The starting allylphenylthio ethers X can be prepared from the appropriate aminothiophenols by methods known to one skilled in the art.

The preparation of the nitro compounds of Formula IX in Equation 8 where J is either $J_1$ and $J_4$ or $J_2$ and $J_5$ is $R_1$ is H, $CH_3$, $OCH_3$, Cl or Br is outlined in Equation 10. The two routes utilize a common nitro ketone intermediate XI; $R_2$, $R_3$, $R_4$ and Q are as previously defined and n is 0 or 1; when n is 1, $R_4$ is H.

Equation 10

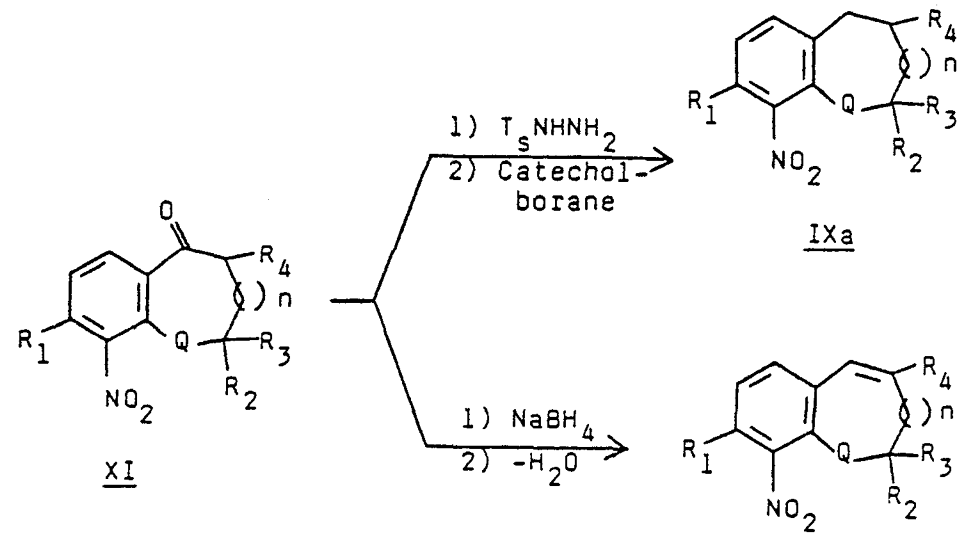


XI IXa IXb

The saturated nitro compounds of Formula IXa are prepared by selectively reducing the tosylhydrazone derivative of nitro ketone XI with catecholborane in a solvent such as chloroform for 2 to 24 hours at ambient temperatures according to the teachings of Kabalka and Chandler, *Synthetic Commun., 9,* 275 (1979).

Alternatively, ketone XI can be reduced directly to give the corresponding alcohol which is then dehydrated by one of a variety of known methods such as $P_2O_5$ by one skilled in the art.

The nitro compounds of Formula IX is Equation 8 where J is $J_1$ and $J_4$ and $R_1$ is $CO_2R_5$, $SO_2R_6$, $OSO_2R_7$ or $SO_2NR_8R_9$ are prepared from the corresponding compounds IXc (from Equation 10, compounds IXa where $R_1$ is Cl) as shown in Equation 11. $R_2$, $R_3$ and $R_4$ are as previously defined, Q is O or S and n is 0 or 1; when n is 1, and $R_4$ is H.

Equation 11

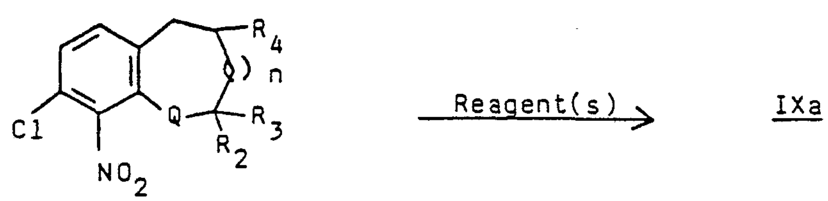


IXc

As shown in Equation 11, the chloronitro compounds IXc are contacted with a reagent or reagents, to be discussed below, to form compounds IXa containing the desired $R_1$ group.

The nitro compounds of Formula IXa where $R_1$ is $SO_2R_6$ are prepared in one step by reacting IXc with an appropriate sulfinic acid salt in a solvent such as dimethylformamide at 25° to 80°C for 1 to 24 hours. Such displacements are reviewed in *Orgânic Chemistry of Sulfur*, p. 536—539, Plenum Press, New York and London, 1977, S. Oae, Ed. Alternatively, IXc can be reacted with an appropriate thiol in the presence of a suitable base such as sodium hydride or potassium hydroxide under the conditions described above to give an intermediate sulfide of Formula IXa where $R_1$ is $SR_6$. The sulfide is then oxidized to the desired compounds IXa where $R_1$ is $SO_2R_8$ and Q is O or $SO_2$ by methods known to one skilled in the art.

The nitro compounds of Formula IXa where $R_1$ is $SO_2NR_8R_9$ and Q is O or $SO_2$ are prepared in two steps starting from an intermediate sulfide (IXa where $R_1$ is $SR_6$) prepared as described above. The sulfide is reacted with molecular chlorine in aqueous acetic acid at 10° to 50°C for 0.5 to 4 hours to give the corresponding sulfonyl chloride IXa where $R_1$ is $SO_2Cl$ as taught by Langler, *Can. J. Chem., 54,* 498 (1976) and Langler and Grossert, *Can. J. Chem., 55,* 407 (1977). The sulfonyl chloride is then contacted with an excess of an appropriate dialkyl amine in an inert solvent such as tetrahydrofuran by methods known to one skilled in the art.

The nitro compounds of Formula IXa where $R_1$ is $OSO_2R_7$ can be prepared in two steps by first heating IXc with one equivalent of a strong base such as sodium or potassium hydroxide in dimethylformamide at 25° to 80°C for 1 to 8 hours to form an intermediate compound IXa where $R_1$ is OH. This intermediate is reacted with an appropriate sulfonyl chloride $R_8SO_2Cl$ or the appropriate anhydride in the presence of a base such as triethyl amine in an inert solvent such as methylene chloride at 0° to 40°C for 1 to 24 hours.

The nitro compounds of Formula IXa where $R_1$ is $CO_2R_5$ can be prepared in three steps by first reacting IXc with one equivalent of potassium cyanide in dimethylformamide at 25° to 80°C for 1 to 8 hours to form an intermediate compound IXa where $R_1$ is CN. This intermediate can be hydrolized by heating in a strong acid such as HCl in a solvent such as acetic acid to form a carboxylic acid which can then be converted to its corresponding esters by methods known to one skilled in the art.

The nitro compounds of Formula IX in Equation 8 where J is $J_2$ and $J_5$ (Formula IXb in Equation 10) and $R_1$ is $CO_2R_5$, $SO_2R_6$, $OSO_2R_7$ or $SO_2NR_8R_9$ are prepared from the corresponding saturated analogs IXa by the bromination/dehydrobromination sequence described earlier for sulfonamides IVc in Equation 6. This procedure can also be used to prepare compounds of Formula IXb where $R_1$ is H, $OCH_3$, Cl or Br. When this procedure is applied a second time to compounds IXb where n is 1, Q is $SO_2$ and $R_3$ and $R_4$ are H, the nitro compounds of Formula IX where J is $J_7$ are obtained.

Finally, the nitro compounds of Formula IX in Equation 8 where Q is $SO_2$ can be prepared from the corresponding nitro compound IX where Q is S by methods discussed earlier for sulfonamides IV.

Several of the nitro compounds of Formula IX are known, for example 8-nitro-3,4-dihydro-2H-1-benzopyran (Bowie and Chai, *Aust. J. Chem., 30,* 675 (1977)), and 8-nitro-2H-1-benzopyran (Box and McCaw, *Rev. Latinoam. Quim, 10,* 118, (1979)).

The nitro ketones of Formula XI in Equation 10 where $R_1$ is H, $CH_3$, $OCH_3$, Cl or Br are prepared by cyclization of the appropriate carboxylic acid XII as shown in Equation 12. $R_2$, $R_3$ and $R_4$ are as previously defined, Q is O or S and n is 0 or 1, when n is 1, $R_4$ is H.

Equation 12

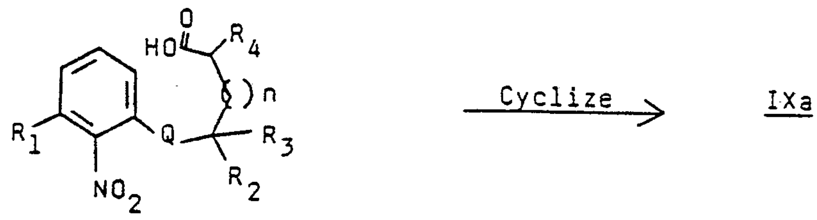


XII

The reaction is carried out by heating the acid with a suitable condensing agent such as polyphosphoric acid, hydrofluoric acid, sulfuric acid or stannic chloride or the acid may be converted to its chloride and heated with a typical Friedel-Crafts reagent such as aluminum chloride or stannic chloride. For a comprehensive review of this and related reactions, see "Friedel-Crafts and Related Reactions" Vols. 1—4, Interscience, New York and London, G. A. Olah, Ed.

In cases where $R_2$ does not equal $R_3$, and $R_4$ is other than H, isomeric mixtures may result. These mixtures can be used in subsequent reactions or separated by fractional crystallization or chromatographic methods.

The starting nitro carboxylic acids XII can be prepared by reacting an appropriate nitrophenol or nitrothiophenol with the appropriate β-bromopropionate or γ-bromobutyrate in the presence of base by methods known to one skilled in the art. The preparation of benzopyrans, benzothiopyrans, benzoxepin and benzothiepin from substituted phenols and thiophenols are widely reported in the literature. For general reviews see "Chromans and Tocopherols", *The Chemistry of Heterocyclic Compounds,* Vol. 36, John Wiley and Sons, New York, 1981, G. P. Ellis, Ed.; "Chromenes, Chromanones and Chromones", *The Chemistry of Heterocyclic Compounds,* Vol. 31, John Wiley and Sons, New York, 1977, G. P. Ellis, Ed.; "Seven Membered Heterocyclic Compounds Containing Oxygen and Sulfur", *The Chemistry of Heterocylic Compounds,* Vol. 26, John Wiley and Sons, New York, 1972, A. Rosowsky, Ed.; "Polycyclic Five- and Six-Membered Compounds Containing One O or S Atom", *Heterocylic Compounds,* Vol. 2, John Wiley and Sons, New York, 1951, R. C. Elderfield, Ed. For a most recent review on the Synthesis and Reactions of 4-Chromanones, see: Kabbe and Widdig, *Angew Chem. Int. Ed. Engl., 21,* 247 (1982).

The amines of Formula (VIII') in Equation 4b above are also important starting materials for the preparation of the compounds of this invention, which can be prepared by the following general methods.

Several of the starting 4- and 7-amino-2,3-dihydrobenzofurans of Formula (VIII') in Equation 4b are known. For instance, 4-amino-2,3-dihydro-2,2-dimethylbenzofuran may be prepared by the procedure of Cruickshank et al., *J. Med. Chem., 13,* 1110 (1970); 7-amino-2,3-dihydro-2,2-dimethylbenzofuran by the procedure of Netherlands 6,602,601; 7-amino-2,3-dihydro-2-methylbenzofuran by the procedure of

BE—A—744,858; 4-amino-2,3-dihydrobenzofuran by the procedure of U.S. 3,963,717; and 7-amino-2,3-dihydrobenzofuran by the procedure of U.S. 3,963,717.

The 7-amino-2,3-dihydrobenzofurans of Formula (VIII') in Equation 4b, where $R_1'$ is H, $CH_3$, $OCH_3$, Cl or Br can be prepared by a procedure analogous to that taught in NL—A—6,602,601. This procedure is illustrated in Equation 13 below.

Equation 13

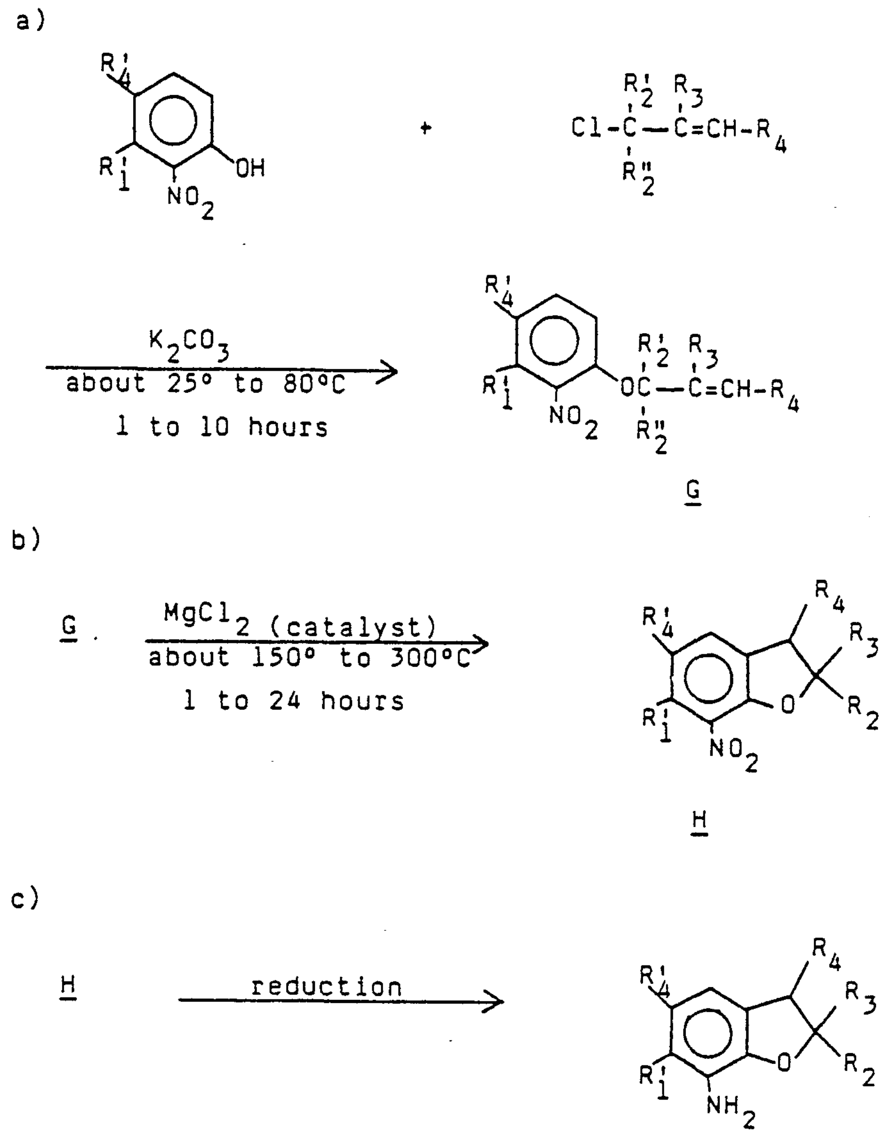


(VIII'a)

wherein

$R_1'$ is H, $CH_3$, $OCH_3$, Cl or Br;

$R_2$ is $C_1$—$C_3$ alkyl;

$R_3$, $R_4$ and $R_4'$ are as previously defined;

$R_2'$ is H or $CH_3$; and

$R_2''$ is H, $CH_3$ or $C_2H_5$; when $R_2''$ is $C_2H_5$; $R_2'$ is H.

As shown in Equation 13, the preparation requires three steps: (1) reacting a 3- or 4-(substituted)-2-nitrophenol with an appropriately substituted allyl chloride to form intermediate *G*; (2) heating this compound at elevated temperatures 150° to 300°C with a catalyst to cause cyclization to form *H*; and (3) reducing this compound to form VIII'a. The first reaction is run in a warm protic solvent such as ethanol in the presence of a weak base such as $K_2CO_3$. The cyclization step is normally run neat at elevated temperatures, in the range of 150 to 300°C for 1 to 24 hours. A Friedel-Crafts catalyst, such as magnesium

chloride, is ordinarily used to promote the cyclization reaction and increase product yields. Intermediate *H* may be purifed by recrystallization or chromatography procedures. The reduction step can be carried out by any of several known methods for reducing nitro groups to amino groups. For example, intermediate *H* can be catalytically reduced with 5% palladium-on-charcoal in ethanol solvent at about 25° to 45°C and at 1 to 3 atmospheres of pressure. Alternatively, *H* can be heated with stannous chloride in concentrated hydrochloric acid at about 25° to 80°C for 0.5 to 3 hours to form VIII'a.

Alternatively, the Claisen rearrangement-cyclization reaction in Equation 13b above can be run stepwise as follows: (a) phenyl allyl ether *G* can be rearranged at about 150° to 200°C for 0.5 to 10 hours either neat or in the presence of a suitable high boling solvent such as diethylaniline to yield the corresponding 4- or 5-(substituted)-6-nitro-2-allylphenol, according to the teachings in S. J. Rhoads and N. R. Raulins, *Organic Reactions,* Vol. 22, p. 1—253, John Wiley and Sons, New York and London, W. G. Dauben, Ed.; and (b) this compound may be cyclized to dihydrobenzofuran *H* by methods widely reported in the literature for analogous type reactions. For instance, heating the rearranged product with pyrimidine·HCl ($R_4$ and $R_5$ are not $OCH_3$), or with acidic reagents such as hydrogen bromide in acetic acid, or with a Friedel-Crafts catalyst such as magnesium chloride can yield *H* according to teachings of Claisen and Tietze, *Ann, 449,* 81 (1926), and *442,* 235 (1925); Arnold and Mc Cool, *J. Am. Chem. Soc., 64,* 1315 (1942); J. Entel et al., *ibid., 73,* 2365 (1951); P. Cruickshank et al., *J. Med. Chem., 13,* 1110 (1970); and Q. Bartz et al., *J. Am. Chem. Soc., 57,* 371 (1935).

The 7-amino-2,3-dihydrobenzofurans of Formula (VIII') in Equation 4b, where $R_1'$ is $OSO_2R_7$, $SO_2R_6$ or $CO_2R_5$, can be prepared as shown in Equation 14 below.

Equation 14

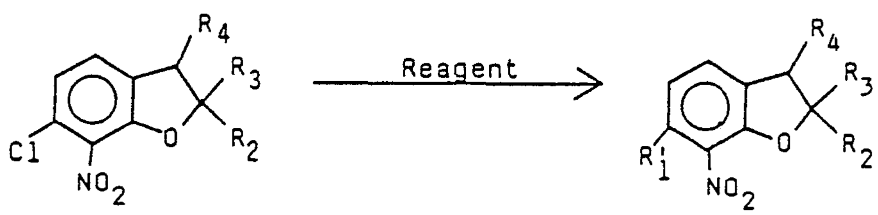


(XIII) I

I

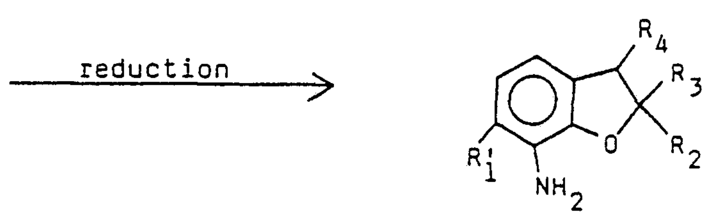


(VIII'b)

wherein

$R_2$ is $C_1$—$C_3$ alkyl;

$R_3$ and $R_4$ are as originally defined; and

$R_1'$ is $OSO_2R_7$, $SO_2R_6$ or $CO_2R_5$.

According to Equation 14, a 6-chloro-7-nitro-2,3-dihydrobenzofuran of Formula (XIII) is reacted with reagents as discussed below to form intermediate *I* containing the desired $R_1'$ group. The nitro compound *I* is then reduced to form VIII'b. These reactions can be run by obvious methods by one skilled in the art.

Intermediate (*I*), where $R_1'$ is $OSO_2CH_3$ for example, is prepared in two steps; (1) heating XIII with one equivalent of a strong base such as sodium or potassium hydroxide in dimethylformamide at 50° to 80°C, for 1 to 8 hours, to form (*I*), where $R_1'$ is OH; and (2) reacting the resulting phenol with methanesulfonyl chloride, in the presence of a base such as triethylamine, in an inert solvent such as tetrahydrofuran, at 25° to 70°C, for 1 to 24 hours, to form (*I*) where $R_1'$ is $OSO_2CH_3$. Intermediate (*I*), where $R_1'$ is $SO_2CH_3$ for example, is also prepared in two steps: (1) heating XIII with one equivalent of sodium methylmercaptide in dimethylformamide at 25° to 80°, for 1 to 8 hours, to form (*I*), where $R_1'$ is $SCH_3$; and (2) oxidizing the resulting compound with 30% hydrogen peroxide in acetic acid solvent, at 0 to 60°, for 1 to 8 hours, to form (*I*), where $R_1'$ is $SO_2CH_3$. Intermediate (*I*), where $R_1'$ is $CO_2CH_3$ for example, is prepared in three steps: (1) reacting XIII with one equivalent of potassium cyanide in dimethylformamide at 50° to 80°C, for 1 to 24 hours, to form (*I*) where $R_1'$ is CN; (2) hydrolyzing the cyano group to a carboxylic acid by any of several methods known in the art; for example, (*I*) where $R_1'$ is CN can be refluxed with concentrated hydrochloric acid in acetic acid solvent to form (*I*) where $R_1'$ is $CO_2H$; and (3) converting the carboxylic acid to a carbonyl chloride which is

then reacted with methanol to form (*I*) where $R_1'$ is $CO_2CH_3$. The latter reactions can be run by known methods by one skilled in the art.

In the final step of Equation 14, (*I*) can be reduced to VIII'b by methods previously described in Equation 13. The starting 6-chloro-7-nitro-2,3-dihydrobenzofurans of Formula (XIII) are also prepared by methods described above in Equation 13.

Similarly, the 4-amino-2,3-dihydrobenzofurans of Formula (VIII') in Equation 4b, where $R_1'$ is $SO_2R_6$, $OSO_2R_7$ or $CO_2R_5$, $R_2$ is $C_1$—$C_3$ alkyl, and $R_3$ and $R_4$ are as originally defined, can be prepared by procedures described in Equation 14. Thus, by starting with an appropriate 5-chloro-4-nitro-2,3-dihydrobenzofuran, and proceeding with the reactions described above in Equation 14, one skilled in the art can prepare the subject 4-amino-2,3-dihydrobenzofurans of Formula (VIII'). The starting 5-chloro-4-nitro-2,3-dihydrobenzofurans are described below in Equation 16.

The 7-amino-2,3-dihydrobenzofurans of Formula (VIII') in Equation 4b, where $R_1'$ is $NO_2$ or $SO_2NR_8R_9$, can be prepared as shown in Equation 15.

Equation 15

a) 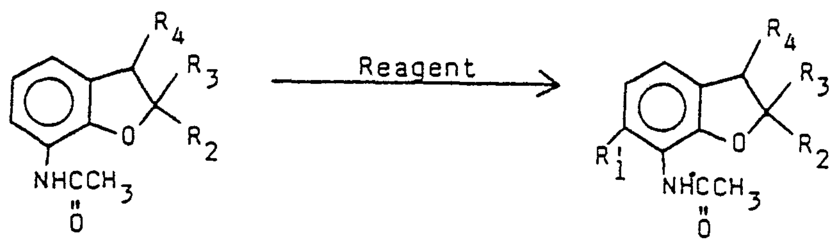


(XIV) (J)

b) J 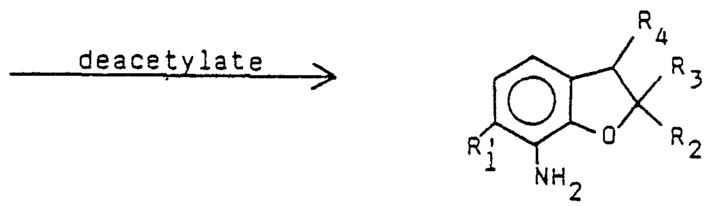


(VIII'c)

wherein

$R_2$, $R_3$ and $R_4$ are as originally defined; and

$R_1'$ is $NO_2$ or $SO_2NR_8R_9$.

According to Equation 15, a 7-acetamido-2,3-dihydrobenzofuran XIV is reacted with reagents as described below to form intermediate *J*. Then *J* is deacetylated to form VIII'c.

Intermediate *J*, where $R_1'$ is $NO_2$, is prepared in two steps: (1) nitration of XIV with nitric acid in acetic acid solvent, at about 10° to 25°C, for 0.5 to 5 hours, to form a mixture containing in part *J* where $R_1'$ is $NO_2$; and (2) separation of *J* from the mixture by recrystallization or chromatographic procedures.

Intermediate *J*, where $R_1'$ is $SO_2NR_8R_9$, is prepared in three steps: (1) chlorosulfonating XIV with chlorosulfonic acid to form a mixture of products containing in part *J*, where $R_1'$ is $SO_2Cl$; (2) reacting the resulting mixture with an appropriate dialkylamine in an inert solvent such as tetrahydrofuran, at about 10 to 60°C, for 0.5 to 10 hours to form in part *J* where $R_1'$ is $SO_2NR_8R_9$; and (3) separating out the desired intermediate *J* by recrystallization or chromatographic procedures. The chlorosulfonation step can be run by reacting XIV with excess chlorosulfonic acid, i.e., about a three equivalent excess, at 25° to 70°C, in an inert solvent such as chloroform, for 0.5 to 8 hours.

Deacetylation of *J* to form VIII'c, can be carried out by saponification or acid hydrolysis procedures. Thus, refluxing *J* in 90% ethanol with one equivalent of sodium hydroxide, for 0.25 to 3 hours, can provide VIII'c. Alternatively, heating *J*, at 50 to 80°C, with hydrochloric acid in acetic acid for 0.5 to 3 hours can also provide VIII'c. The starting compound XIV can be prepared by known methods, i.e., by heating, at 50 to 100°C, an appropriate 7-amino-2,3-dihydrobenzofuran in acetic anhydride, with a catalytic amount of sulfuric acid, for 1 to 10 hours.

Similarly, the 4-amino-2,3-dihydrobenzufurans of Formula (VIII') in Equation 4b, where $R_1'$ is $NO_2$ or $SO_2NR_8R_9$, can be prepared by procedures described in Equation 15 above. Thus, by starting with an appropriate 4-acetamido-2,3-dihydrobenzofuran, and proceeding with the appropriate reactions described above in Equation 15, the following compounds can be prepared: 4-amino-2,3-dihydro-5-nitrobenzofurans and 4-amino-5-(N,N-dialkylsulfonamido)-2,3-dihydrobenzofurans of Formula (VIII'), where $R_2$ to $R_4$ are as defined above. The starting 4-acetamido-2,3-dihydrobenzofurans can be prepared from 4-amino-2,3-dihydrobenzofurans by methods also described in Equation 15.

The 4-amino-2,3-dihydrobenzofurans of Formula (VIII') in Equation 4b, where $R_1'$ is H, $OCH_3$, Cl, Br, $CO_2R_5$ or $OSO_2R_7$, can be prepared as illustrated below in Equation 16.

Equation 16

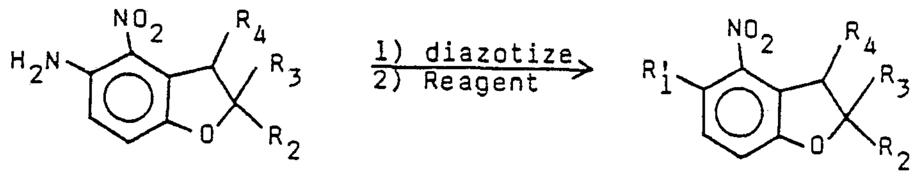


(XV) (K)

K

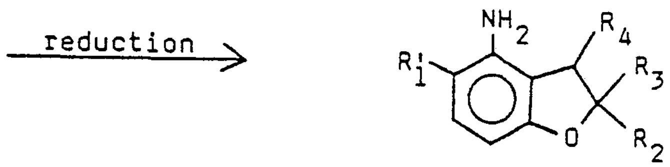


(VIII'd)

wherein

$R_2$ is $C_1$—$C_3$ alkyl;

$R_3$ and $R_4$ are as originally defined; and

$R_1'$ is H, $OCH_3$, Cl, Br, $CO_2R_5$ or $OSO_2R_7$.

According to Equation 16, a 5-amino-2,3-dihydro-4-nitrobenzofuran of Formula (XV) is diazotized, then reacted with appropriate reagents to form intermediate *K*. Reduction of *K* can then provide VIII'd. The diazonium salt can be prepared with sodium nitrite in dilute sulfuric (20 to 50%) at about 0° to 10°C by methods known in the art, e.g., Arnold and McCool, *J. Am. Chem. Soc., 64,* 1315 (1942). Intermediate *K*, where $R_1'$ is as defined above, can be prepared from the diazonium salt by methods also known in the art.

Thus, intermediate *K*, where $R_1'$ is Cl or Br, can be prepared by heating the diazonium salt with cuprous chloride and hydrochloric acid or with cuprous bromide and hydrobromic acid, according to the Sandmeyer reaction, e.g., Powers, *J. Med. Chem., 19,* 57 (1976). Intermediate *K*, where $R_1'$ is $OCH_3$, can be prepared in two steps: (1) intermediate *K*, where $R_1'$ is OH, is prepared by refluxing the diazonium salt with copper sulfate solution (50%), a procedure well-known in the art for preparing phenols from diazonium salts, e.g., Arnold and McCool, *J. Am. Chem. Soc., 64,* 1315 (1942); and (2) the phenol can then be methylated by a variety of known methods to give *K* where $R_1'$ is $OCH_3$. For instance, heating the phenol with dimethylsulfate at about 25° to 60°C in water in the presence of an equivalent of NaOH, gives *K* where $R_1'$ is $OCH_3$.

Intermediate *K*, where $R_1'$ is $OSO_2R_7$, can be prepared by reacting the phenol described above with an appropriate alkylsulfonyl chloride and a base such as triethylamine in an inert solvent such as tetrahydrofuran at a temperature of about 0° to 60°C.

Intermediate *K*, where $R_1'$ is $CO_2R_5$, is prepared in the following manner: (1) intermediate *K*, where $R_1'$ is CN, is prepared by the Sandmeyer reaction. Thus, the diazonium salt is heated with cuprous cyanide according to methods well known in the art, e.g., Hansch and Schmidhalter, *J. Org. Chem., 20,* 1056 (1955); (2) intermediate *K*, where $R_1'$ is $CO_2H$, is then prepared by refluxing the cyano compound, with hydrochloric acid in acetic acid solvent for 1 to 10 hours; and (3) intermediate *K*, where $R_1'$ is $CO_2R_5$, is prepared by converting the carboxylic acid to a carbonyl chloride which is then reacted with appropriate alcohols to form *K*, where $R_1'$ is $CO_2R_5$. Reaction (3), takes place according to methods known in the art.

Intermediate *K*, where $R_1'$ is H, is prepared by reacting the diazonium salt with 50% hypophosphorous acid at 0 to 20°C for 1 to 24 hours, according to a procedure of Bordwell and Stange, *J. Am. Chem. Soc., 77,* 5939 (1955). The final step in Equation 16, the reduction of *K* to form VIII'd, is carried out by the methods described previously in Equation 14.

The starting compounds of Formula (XV) in Equation 16 can be prepared according to the disclosure of Arnold and McCool, *J. Am. Chem. Soc., 64,* 1315 (1942), the disclosure of which is herein incorporated by reference. Arnold and McCool teach the preparation of 5-amino-2,3-dihydro-2-methyl-4-nitrobenzofuran by a multistep procedure starting with the reaction of 4-hydroxyacetophenone with allylbromide. By reacting 4-hydroxyacetophenone with other appropriately substituted allylbromides, or allyl chlorides, and using the multistep reactions and conditions taught by Arnold and McCool, one skilled in the art can prepare the compounds of Formula (XV) in Equation 16.

The 4-amino-2,3-dihydrobenzofurans of Formula (VIII') in Equation 4b, where $R_1'$ is $CH_3$, H, Cl or Br, can be prepared by a procedure analogous to that taught by Cruickshank, *J. Med. Chem., 13,* 1110 (1970). This procedure is illustrated in Equation 17 below.

Equation 17

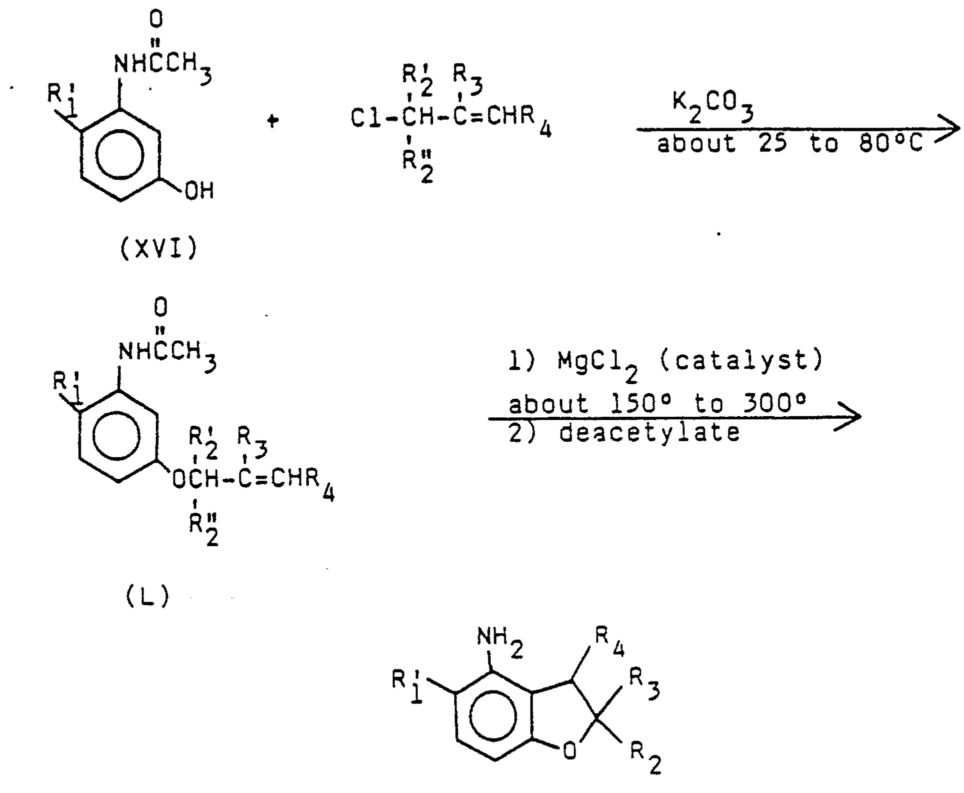


(XVI)

(L)

(VIII'e)

wherein

$R_2$ is $C_1$—$C_3$ alkyl;

$R_3$ and $R_4$ are as originally defined;

$R_1'$ is H, $CH_3$, Cl or Br; and

$R_2'$ is H or $CH_3$, $R_2''$ is H, $CH_3$ or $C_2H_5$; when $R_2''$ is $C_2H_5$, $R_2'$ is H.

According to Equation 17, the preparation requires three steps: (1) reacting a 2-(substituted)-5-hydroxybenzacetamide of Formula (XVI) with an appropriately substitued allyl chloride and potassium carbonate at about 25° to 80°C in an inert solvent such as acetone to form intermediate *L*; (2) heating this compound at elevated temperatures, about 150° to 300°C with a suitable Friedel-Crafts catalyst such as magnesium chloride to cause cyclization to form a 5-(substituted)-4-acetamido-2,3-dihydrobenzofuran; and (3) deacetylating this compound in the usual manner to form VIII'e. These reactions can be run using methods described previously in Equations 13 and 15. Thus, by starting with an appropriate 2-(substituted)-5-hydroxybenzacetamide of Formula (XVI), and using the reactions and conditions described in steps (a) and (b) of Equation 13 and step (b) of Equation 15, one skilled in the art can prepare the compounds of Formula (VIII'e).

The 4- and 7-aminobenzofurans of Formula (VIII') in Equation 4b, where $R_1'$ is H and $R_{10}$ and $R_{11}$ are H or $CH_3$, can be prepared by known methods. Such methods are exemplified by Pene et al., *Bull. Soc. Chim. France,* 586 (1966); Rodighiero et al., *Gazz. Chim. Ital., 91,* 90 (1961); Royer et al., *Bull. Soc. Chim. France,* 1026 (1970); Kawase, *Chem, Ind. (London),* 687 (1970); Belgium 744,859; Kawase et al., *Bull. Chem. Soc. Japan, 44,* 749 (1971); Fr. Demande 2,338,041; U.S. 3,577,441; and U.S. 3,452,033.

The 7-aminobenzofurans of Formula (VIII') in Equation 4b, where $R_1'$ is H, $CH_3$, $OCH_3$, Cl, Br, $CO_2R_5$, $SO_2R_6$, or $OSO_2R_7$ can be prepared as shown in Equation 18 below.

Equation 18

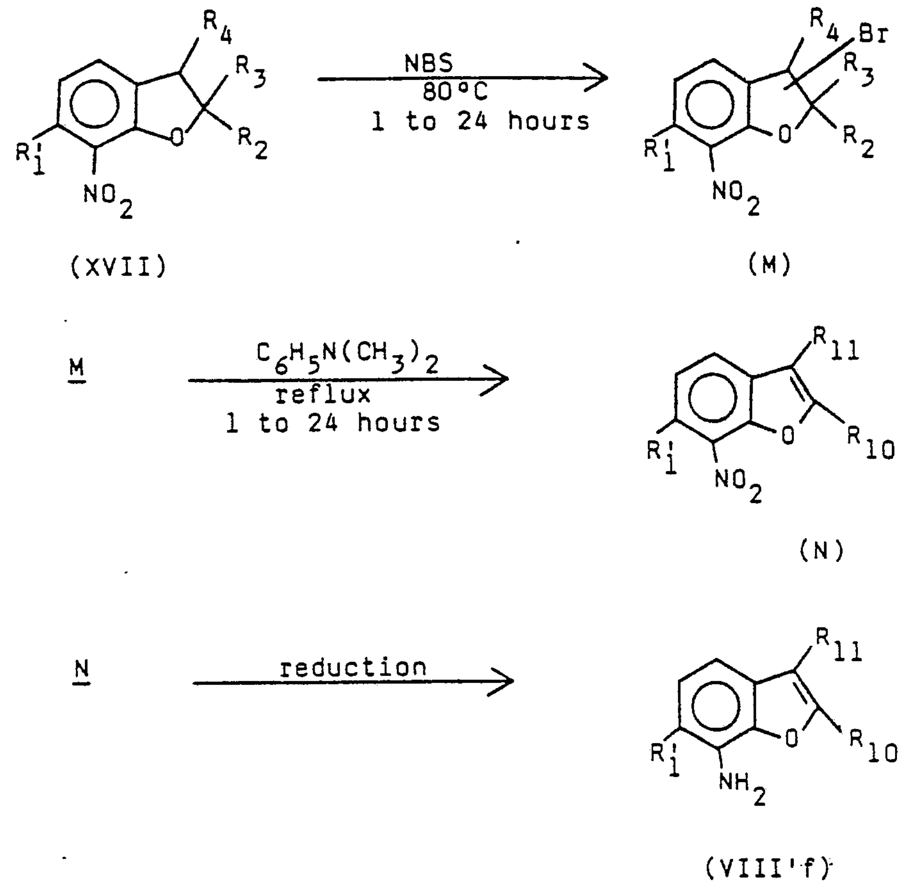


(XVII) (M) (N) (VIII'f)

wherein

$R_2$, $R_3$ and $R_4$ are as originally defined, except at least one of $R_2$ or $R_3$ must be H;

$R_1'$ is H, $OCH_3$, Cl, Br, $CO_2R_5$, $SO_2R_6$ or $OSO_2R_7$;

$R_{11}$ is H or $CH_3$; and

$R_{10}$ is H or $C_1$—$C_3$ alkyl.

As shown in Equation 18, the 7-aminobenzofurans of Formula (VIII'f) can be prepared from corresponding 7-nitro-2,3-dihydrobenzofurans of Formula (XVII), where at least one of $R_2$ or $R_3$ is H. The procedure involves a dehydrogenation reaction followed by a reduction reaction. The dehydrogenation reaction may be carried out using a procedure of Geisman, *J. Am. Chem. Soc., 72,* 4326 (1950) and Hurd, *J. Am. Chem. Soc., 80,* 4711 (1958).

Thus, compound XVII is dehydrogenated by a two-step sequence: (1) 2,3-dihydrobenzofuran (XVII) is heated at 60° to 80°C for 1 to 24 hours with N-bromosuccinimide (NBS) and benzoyl peroxide catalyst in an inert organic solvent such as benzene or carbon tetrachloride thereby brominating the non-aromatic portion of the molecule to form intermediate *M*; (2) this intermediate is heated with excess N,N-dimethylaniline, $[C_6H_5N(CH_3)_2]$, either neta or in an inert aprotic solvent such as toluene for 1 to 24 hours, to cause dehydrobromination to form 7-nitrobenzofuran *N*. Reduction of *N* to form 7-aminobenzofuran VIII'f can be carried out by any of several methods known in the art for reducing nitrobenzofurans. For instance, catalytic reduction of *N* with Raney Nickel catalyst in an inert solvent such as ethanol at about 25° to 70°C and 1 to 3 atmospheres of hydrogen can provide VIII'f, according to a procedure of Belgium 744,858. Many of the starting compounds XVII are described above in Equations 13 and 14.

Similarly, the 4-aminobenzofurans of Formula (VIII') in Equation 4b, where $R_1'$ is H, $OCH_3$, Cl, Br,m $CO_2R_5$, $OSO_2R_7$ or $SO_2R_6$ and $R_{10}$ is H or $C_1$—$C_3$ alkyl and $R_{11}$ is H or $CH_3$, can be prepared by procedures described in Equation 18. Thus, by starting with an appropriate 5-(substituted)-2,3-dihdyro-4-nitrobenzofuran, and proceeding with the reactions described in Equation 18, one skilled in the art can prepare the subject 4-amino-benzofurans of Formula (VIII'). The preparation of many of the starting 5-(substituted)-2,3-dihydro-4-nitrobenzofurans is described above, e.g. Equations 14 and 16.

The 7-aminobenzofurans of Formula (VIII') in Equation 4b, where $R_1'$ is H, $CH_3$, Cl, Br, $OCH_3$ or $SO_2R_6$, can be prepared as shown in Equation 19 below.

Equation 19

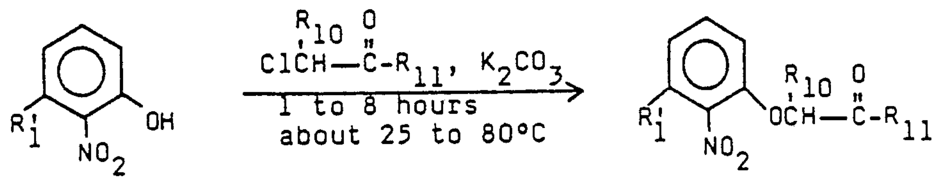


(XVIII) (O)

O

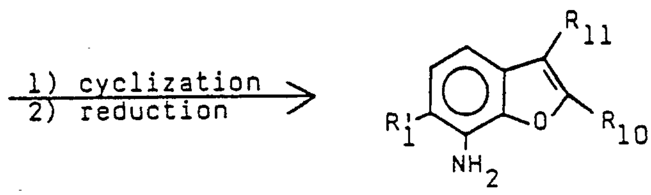


(VIII'g)

wherein

$R_1'$ is H, $CH_3$, Cl, Br, $OCH_3$ or $SO_2R_6$;

$R_{10}$ is H or $C_1$—$C_3$ alkyl; and

$R_{11}$ is $CH_3$.

According to Equation 19, the preparation requires three steps (1) reacting a 3-(substituted)-2-nitrophenol with an α-chloroketone such as chloropropanone for 1 to 8 hours at 30 to 80°C in the presence of a base such as $K_2CO_3$ in an inert solvent such as acetone to form intermediate *O*; (2) cyclizing *O* to form a 7-nitrobenzofuran; and (3) reducing this compound to form a 7-aminobenzofuran of Formula (VIII'g). The cyclization step is carried out in polyphosphoric acid at a temperature of about 100°C for 0.5 to 24 hours. Other methods are known in the art for preparing benzofurans from phenoxypropanones e.g., Pene, *Bull. Soc. Chim. France,* 586 (1966); Kawase, *Chem. Ind. (London),* 687 (1970); and Kawase et al., *Bull. Chem. Soc. Japan, 44,* 749 (1971). The reduction step can be carried out by the methods described in Equation 18 above. Compound VIII'g, where $R_1'$ is $SO_2R_6$, is prepared by (a) oxidizing the intermediate 7-nitrobenzofuran, where $R_1'$ is $SR_6$, with 30% peracetic acid in acetic acid at 0° to 60°C to form an intermediate 7-nitrobenzofuran, where $R_1'$ is $SO_2R_6$, and (b) reducing this compound to form VIII'g where $R_1'$ is $SO_2R_6$, by methods described in Equation 18 above.

The 7-aminobenzofurans of Formula (VIII') in Equation 4b, where $R_1'$ is $NO_2$ or $SO_2NR_8R_9$ and $R_{10}$ is $C_1$—$C_3$ alkyl and $R_{11}$ is $CH_3$, can be prepared as shown in Equation 20.

Equation 20

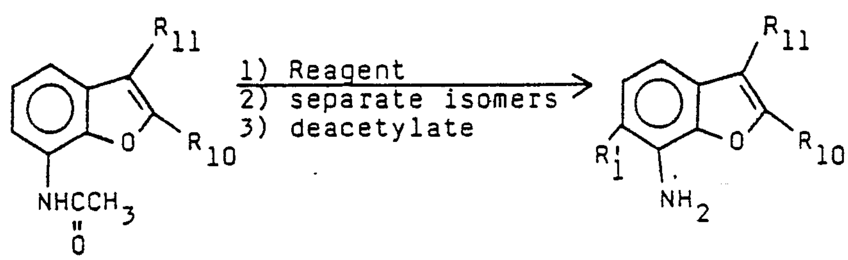


(XIX) (VIII'h)

The reactions of Equation 20 can be carried out by procedures described previously in Equation 15. Thus, by nitrating or chlorosulfonating an appropriate 7-acetamidobenzofuran XIX according to procedures described in Equation 15, one skilled in the art can prepare the subject compounds VIII'h.

Similarly, the 4-aminobenzofurans of Formula (VIII') in Equation 4b, where $R_1'$ is $NO_2$ or $SO_2NR_8R_9$, $R_{10}$ is $C_1$—$C_3$ alkyl and $R_{11}$ is $CH_3$, can be prepared by procedures described in Equation 20 above. Thus, by starting with an appropriate 4-acetamidobenzofuran, and carrying out the reactions described in Equation 20, one skilled in the art can prepare the subject 4-aminobenzofurans of Formula (VIII').

The 4-aminobenzofurans of Formula (VIII') in Equation 4b, wherein $R_1'$ is $CH_3$, Cl, Br or H, can be prepared by a procedure analogous to that taught by Kawase et al., *Bull. Chem. Soc. Jap., 749,* 44 (1971). This procedure is illustrated in Equation 21 below.

Equation 21

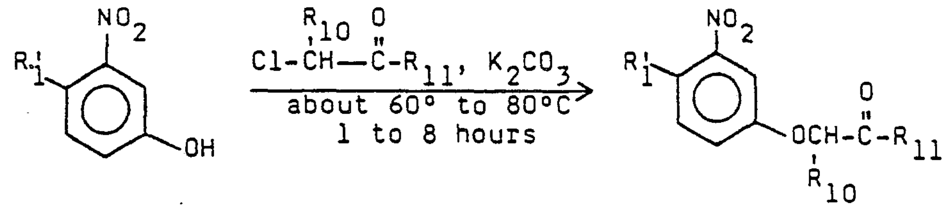


(XX) (P)

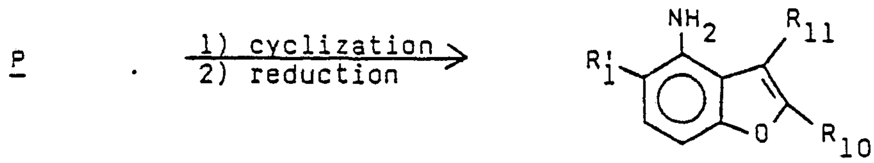


(VIII'i)

wherein

$R_1'$ is H, Cl, Br or $CH_3$;

$R_{11}$ is $CH_3$; and

$R_{10}$ is H or $C_1$—$C_3$ alkyl.

According to Equation 21, the procedure requires three steps: (1) refluxing an appropriately substituted 4-substituted-3-nitrophenol with an α-chloroketone such as chloropropanone for 1 to 8 hours in the presence of a base, e.g., $K_2CO_3$, in a solvent, e.g., acetone, to form intermediate *P*; (2) cyclizing *P* to form a 4-nitrobenzofuran; and (3) reducing this compound to form a 4-aminobenzofuran of Formula (VIII'i). The cyclization step can be carried out in polyphosphoric acid at about 80 to 120°C for 0.5 to 24 hours. The reduction step can be carried out by methods described in Equation 18 above.

The 4- and 7-amino-2,3-dihydrobenzo[b]thiophenes of Formaul (VIII') in Equation 4b, where $R_4'$ and $R_1'$ are H can be prepared by the Bucherer reaction, as shown in Equation 22 below.

Equation 22

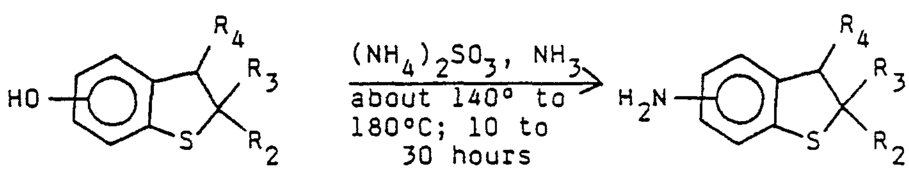


(XXI) (VIII'j)

wherein

$R_2$, $R_3$ and $R_4$ are as originally defined.

The reaction of Equation 22 can be carried out using conditions reported in the literature for Bucherer reactions, e.g., Boswell et al., *J. Heterocyclic Chem., 5,* 69 (1968). An appropriate 4- or 7-hydroxy-2,3-dihydrobenzo[b]thiophene of Formula (XXI) is heated with concentrated ammonium hydroxide, sulfur dioxide and water in an autoclave at about 140° to 180°C for 10 to 30 hours to provide VIII'j. The starting compounds XXI can be prepared by known methods. Several such methods are exemplified by DE—A—2,252,335; Kilsheimer, *J. Agr. Food Chem., 17,* 91 (1969); U.S. 4.032,649; and DE—A—2,534,857.

The 7-amino-2,3-dihydrobenzo[b]thiophenes of FOrmula (VIII') in Equation 4b, where $R_1'$ is H, $CH_3$, $OCH_3$, Cl or Br, can be prepared as shown in Equation 23 below.

Equation 23

a)

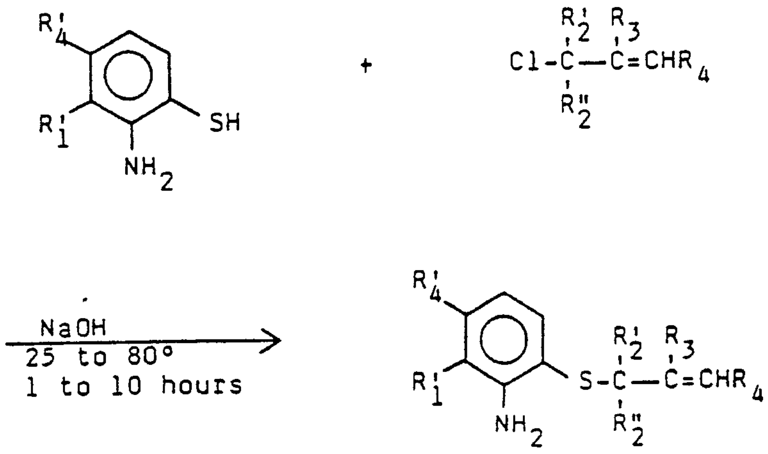


Q

b)

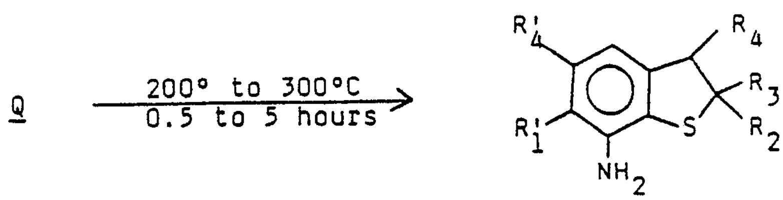


(VIII'k)

wherein

$R_2$ is $C_1$—$C_3$ alkyl;

$R_3$, $R_4$ and $R_4'$ are as originally defined;

$R_1'$ is H, $CH_3$, $OCH_3$, Cl or Br; and

$R_2'$ is H or $CH_3$; $R_2''$ is H, $CH_3$ or $C_2H_5$; when $R_2''$ is $C_2H_5$, $R_2'$ is H.

According to Equation 23, the preparation requires two steps: In Step 23(a) a 3- or 4-(substituted)-2-aminothiphenol is reacted with an appropriately substituted allyl chloride, or allyl bromide, to form intermediate *Q*. The reaction is run in a warm protic solvent such as ethanol in the presence of an equimolar amount of a base such as sodium hydroxide for 1 to 10 hours. After dilution with water the product *Q* is isolated by extraction with methylene chloride and concentration of the organic phase. Compound *Q* may be further purified by chromatography procedures.

And in step 23(b), *Q* is heated neat at 200° to 300°C for about 0.5 to 5 hours to cause cyclization to form (VIII'k). The 5-ring fused product VIII'k is accompanied by a 6-ring isomer (see Equation 9). The desired product VIII'k, can be separated and purified by high resolution distillation or chromatography procedures by one skilled in the art.

The 7-amino-2,3-dihydrobenzo[b]thiophenes of Formula (VIII') in Equation 4b, where $R_1'$ is H, $CH_3$, $OCH_3$, Cl or Br, can be prepared by a procedure analogous to that taught in Singerman, U.S. 4,032,649, the disclosure of which is herein incorporated by reference. The relevant portion of the procedure is illustrated in Equation 24 below.

Equation 24

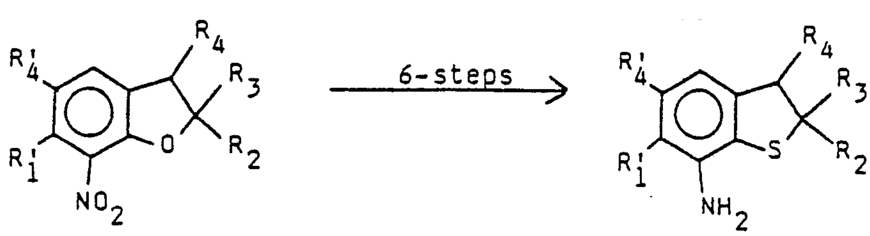


(XXII) (VIII'1)

wherein

$R_2$ is $C_1$—$C_3$ alkyl;
$R_3$ and $R_4'$ are as originally defined;
$R_4$ is H; and
$R_1'$ is H, $CH_3$, $OCH_3$, Cl or Br.

U.S. 4,032,649 teaches the preparation of 7-amino-2,3-dihydro-2,2-dimethylbenzo[b]thiophene by a six-step reaction sequence starting with 7-nitro-2,3-dihydro-2,2-dimethylbenzofuran. By starting with an appropriate 5- or 6-(substituted)-7-nitro-2,3-dihydrobenzofuran of Formula (XXII), and following the six reactions described in U.S. 4,032,649, one skilled in the art can prepare the 5- or 6-(substituted)-7-amino-2,3-dihydrobenzo[b]thiophenes of Formula (VIII'l). The starting compound XXII can be prepared by methods described heretofore in Equation 13.

The six-step reaction sequence of Equation 24 involves (a) reacting XXII with potassium *tert*-butoxide in dimethylsulfoxide at 0° to 60°C for 0.5 to 10 hours to form a 6-nitro-4 or 5-(substituted)-2-vinylphenol, (b) reacting this compound with sodium hydride and dimethylthiocarbamoyl chloride in tetrahydrofuran solvent at 0° to 60°C for 1 to 10 hours to form a O-[6-nitro-4 or 5-(substituted)-2-vinyl]-N,N-dimethylthiocarbamate, (c) heating this compound under nitrogen at 150° to 200°C for 0.5 to 5 hours to form a S-[6-nitro-4 or 5-(substituted)-2-vinyl]-N,N-dimethylthiocarbamate, (d) reducing this compound with iron powder in acetic acid at 25° to 90°C for 0.5 to 3 hours to form a S-[6-amino-4 or 5-(substituted)-2-vinyl]-N,N-dimethylthiocarbamate, (e) reacting this compound with potassium hydroxide in water-methanol solvent at 40° to 80°C for 1 to 24 hours to form a 6-amino-4 or 5-(substituted)-2-vinylthiophenol, and (f) cyclizing this compound by heating under nitrogen at 150° to 250°C for 0.5 to 6 hours to form VIII'l in Equation 24.

The 7-amino-2,3-dihydrobenzo[b]thiophenes of Formula (VIII') in Equation 4b, where $R_1'$ is $CO_2R_5$, $OSO_2R_7$ or $SO_2R_6$, can be prepared as shown in Equation 25 below.

Equation 25

a)

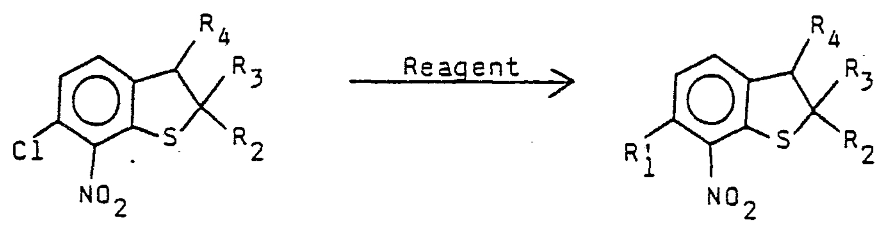


(R) (S)

b)

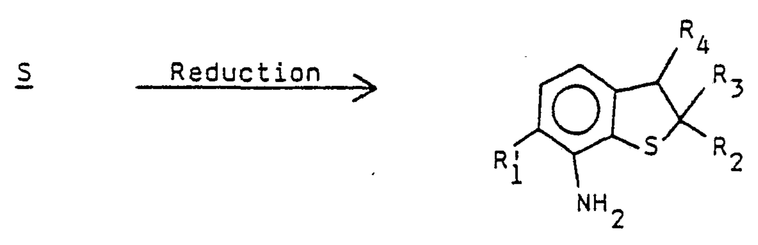


(VIII'm)

wherein

$R_2$ is $C_1$—$C_3$ alkyl;
$R_3$ is as originally defined;
$R_4$ is H; and
$R_1'$ is $CO_2R_5$, $OSO_2R_7$ or $SO_2R_6$.

According to Equation 25, a 6-chloro(or bromo-)-7-nitro-2,3-dihydrobenzo[b]thiophene *R* is reacted with reagents described below to form intermediate *S* containing the desired $R_1'$ group. The nitro-compound *S* is then reduced for form VIII'm. These reactions can be run by obvious methods.

Thus, compound *S*, where $R_1'$ is $OSO_2R_7$ or $CO_2R_5$, can be prepared by one skilled in the art using procedures similar to those described above in Equation 14 for preparing *I*, where $R_1'$ is $OSO_2R_7$ or $CO_2R_5$. Compound *S*, where $R_1'$ is $SO_2R_6$, is prepared by reacting *R* with an appropriate sulfinate salt, i.e., $KSO_2R_6$, in an inert solvent such as dimethylformamide at about 40° to 100°C for 1 to 8 hours. Nitro compond *S* is reduced to amine VIII'm with iron powder and acetic acid in ethanol solvent at about 50° to 80°C for 1 to 5

hours. The starting compound *R* may be prepared by analogous procedures described above in Equation 24 and in U.S. 4,032,649 by one skilled in the art. Thus, an appropriate S-[6-nitro-5-chloro(or bromo)-2-vinyl]-N,N-dimethylthiocarbamate may be reacted with potassium hydroxide to form the corresponding 6-nitro-5-chloro(or bromo)-2-vinylthiophenol. This compound may then be heated at elevated temperatures, i.e., 150° to 250°C, to cause cyclization to form a mixture containing *R* and the corresponding 7-chloro(or bromo)-8-nitrothiochroman. Compound *R* may be isolated and purified by high resolution distillation or chromatography.

The 7-amino-2,3-dihydrobenzo[b]thiophenes of Formula (VIII') in Equation 4b, where $R_1'$ is $CH_3$, H, Cl, Br, $OCH_3$, $CO_2R_5$, or $OSO_2R_7$, can be prepared as shown in Equation 26 below.

Equation 26

a)

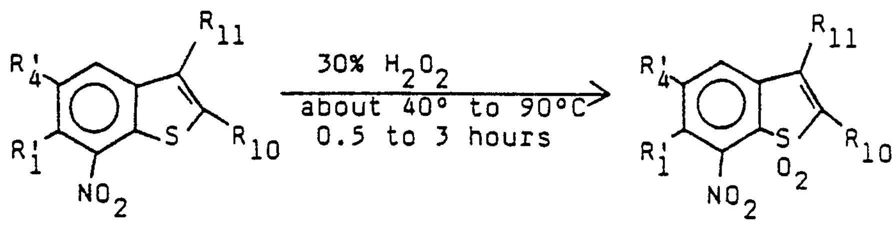


(XXIII) (T)

b)

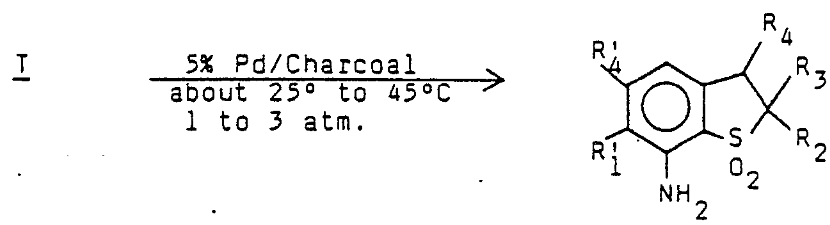


(U)

c)

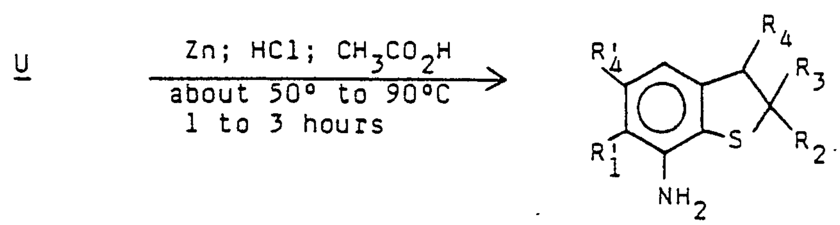


(VIII'n)

wherein

$R_2$ and $R_4'$ are H;

$R_3$ and $R_4$ are as originally defined;

$R_1'$ is H, $CH_3$, Cl, Br, $OCH_3$, $CO_2R_5$ or $OSO_2R_7$;

$R_{10}$ is H or $C_1$—$C_3$ alkyl; and

$R_{11}$ is H or $CH_3$.

The stepwise reduction of benzo[b]thiophenes to form 2,3-dihydrobenzo[b]thiphenes is known in the art, e.g. Bordwell and Stange, *J. Am. Chem. Soc.*, 77, 5939 (1955) and Bordwell and McKellin, ibid., *73,* 2251 (1951). The reactions of Equation 26 can be carried out using procedures disclosed in the cited references. In the first step, a 7-nitrobenzo[b]thiophene of Formula (XXIII), is oxidized with 30% hydrogen peroxide in acetic acid at about 50° to 115°C for 0.5 to 2 hours to form benzo[b]thiophene-1,1-dioxide *T*. Intermediate *T* is catalytically hydrogenated with 5% palladium-on-charcoal at 1 to 3 atmospheres of pressure, at 25° to 45°C, in ethanol solvent to form 2,3-dihydrobenzo[b]thiophene-1,1-dioxide *U*. In the last step, *U* is refluxed

with zinc and concentrated hydrochloric acid in acetic acid solvent for 1 to 3 hours to produce VIII'n. Many of the starting 7-nitrobenzo[b]thiophenes of Formula (XXIII) are described hereinafter.

The 4-amino-2,3-dihydobenzo[b]thiophenes of Formula (VIII') in Equation 4b, where $R_1'$ is H, $CH_3$, Cl, Br, $OCH_3$, $CO_2R_5$, $OSO_2R_7$ or $SO_2R_6$, can also be prepared using procedures described in Equations 25 and 26 above. Thus, by starting with an appropriate 5-chloro-4-nitro-2,3-dihydrobenzo[b]thiophene, and carrying out the appropriate reactions described in Equation 25 above, one skilled in the art can prepare the subject compounds, where $R_1'$ is $SO_2R_6$. Similarly, by starting with an appropriate 5-(substituted)-4-nirobenzo[b]thiophene, and carrying out the appropriate reactions described in Equation 26, the subject compounds can be prepared, where $R_1'$ is H, $CH_3$, $OCH_3$, Cl, Br, $CO_2R_5$ or $OSO_2R_7$. The required starting 5-(substituted)-4-nitribenzo[b]thiophenes are described hereinafter.

The 4- and 7-amino-2,3-dihydrobenzo[b]thiophens of Formula (VIII') in Equation 4b, where $R_1'$ is $SO_2NR_8R_9$, can be prepared by using methods described previously in Equation 15. By starting with an appropriate 4- or 7-acetamido-2,3-dihdyrobenzo[b]thiophene, an carrying out the chlorosulfonation reactions described in Equation 15, one skilled in the art can prepare the subject 4- and 7-amino-2,3-dihydrobenzo[b]thiophenes of Formula (VIII'). The starting acetamides can be prepared by known methods, i.e., refluxing a 4- or 7-amino-2,3-dihydrobenzo[b]thiophene in acetic anhydride in the presence of sulfuric acid as catalyst. The latter amino compounds can be prepared by the Bucherer reaction, as described above in Equation 22.

The 4- and 7-aminobenzo[b]thiophenes of Formula (VIII') in Equation 4b, where $R_1'$ is H, $R_{10}$ is H or $C_1$—$C_3$ alkyl and $R_{11}$ is H or $CH_3$, can also be prepared by the Bucherer reaction. By starting with an appropriate 4- or 7-hydroxybenzo[b]thiophene, and carrying out the reaction described in Equation 22, one skilled in the art can prepare the subject 4- and 7-aminobenzo[b]thiophenes of Formula (VIII'). Many of the starting 4- and 7-hydroxybenzo[b]thiophenes are known in the literature.

The 4-amino-benzo[b]thiophenes of Formula (VIII') in Equation 4b, where $R_1'$ is H, $OCH_3$, Cl, Br, $CO_2R_5$, or $OSO_2R_7$, can be prepared as shown in Equation 27 below.

Equation 27

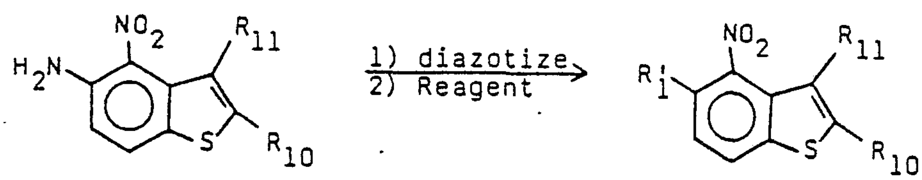


(XXIV) (V)

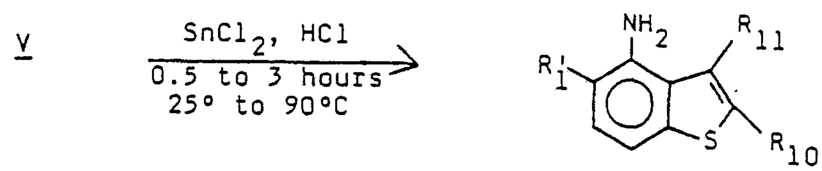


(VIII'o)

wherein

$R_1'$ is H, $OCH_3$, Cl, Br, $CO_2R_5$ or $OSO_2R_7$;
$R_{10}$ is H or $C_1$—$C_3$ alkyl; and
$R_{11}$ is H or $CH_3$.

According to Equation 27, a 5-amino-4-nitrobenzo[b]thiophene of FOrmula (XXIV) is diazotized, then the diazonium salt is reacted with appropriate reagents to form intermediate *V*. Reduction of *V* then provides VIII'o. The diazonium salt can be prepared with sodium nitrite in dilute sulfuric acid (20 to 50%) at 0° to 10°C, a method well-known in the art for diazotizing aminobenzo[b]thiophenes, e.g., Bordwell and Stange, *J. Am. Chem. Soc.*, *77*, 5939 (1955). Intermediate *V*, where $R_1'$ is as defined above, can be prepared from the diazonium salt using methods described heretofore in Equation 16 by one skilled in the art. The reduction step to form VIII'o from *V* can be carried out by any of several methods known in the art for reducing nitrobenzo[b]thiophenes to aminobenzo[b]thiophenes, e.g., Bordwell and Albisetti, *J. Am. Chem. Soc.*, *70*, 1955 (1948); and Martin-Smith and Reid, *J. Chem. Soc.*, 938, (1960). Thus, refluxing *V* with stannous chloride and hydrochloric acid in acetic acid solvent for 0.5 to 3 hours can provide VII'o. The starting 5-amino-4-nitrobenzo[b]thiophens of Formula (XXIV) can be prepared by one skilled in the art according to the teachings of Bordwell and Standge, *J. Am. Chem. Soc.*, *77*, 5939 (1955); Fries et al., *Ann*, *527*, 83 (1936); and G. Karimov et al., *Dokl. Akad. Nauk. Tadzh. SSR*, *13*, 41 (1970). Chem. Abstr. *75*:5605j.

The 7-aminobenzo[b]thiophenes of Formula (VIII') in Equation 4b, where $R_1'$ is H, $CH_3$, $OCH_3$, Cl or Br can be prepared as shown in Equation 28.

Equation 28

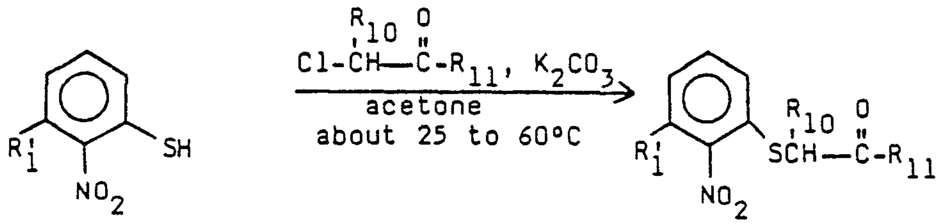


(XXV) (W)

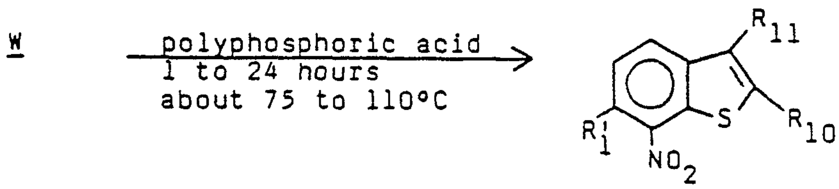


(X)

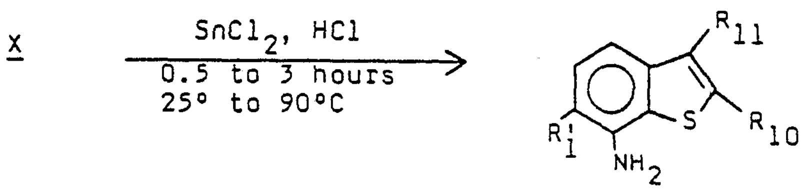


(VIII'p)

wherein

$R_1'$ is H, $CH_3$, $OCH_3$, Cl or Br;

$R_{10}$ is H or $C_1$—$C_3$ alkyl; and

$R_{11}$ is $CH_3$.

The procedure of Equation 28 involves three reaction steps: (1) refluxing an appropriate 3-(substituted)-2-nitrothiophenol with an α-chloroketone such as chloropropanone in the presence of a base such as $K_2CO_3$ in acetone solvent for 1 to 10 hours to form intermediate *W*; cyclizing *W* in polyphosphoric acid at about 100° for 1 to 24 hours to form a 6-(substituted)-7-nitrobenzo[b]thiophene *X*; and reducing *X* to form VIII'p. The reduction can be carried out by procedures described above in Equation 27. The cyclization of thiophenoxypropanones to form benzo[b]thiophenes is known in the art, e.g., Karimov et al., *Dokl. Akad. Nauk, Tadzh, USSR, 13,* 41 (1970), Chem. Abstr. *75*:5605j; and Yasuo, *Nippon Kagaku Zasshi 88,* 758 (1967), Chem. Abstr. *69*:59018g.

Also, by substituting chloroacetaldehyde diethyl acetal or 2-chloropropionaldehyde diethyl acetal for the α-chloroketone in Equation 28 above, and carrying out the procedure described therein, one skilled in the art can prepare 7-aminobenzo[b]thiophenes VIII'p, where $R_1'$ is as defined above, $R_{10}$ is H or $CH_3$ and $R_{11}$ is H.

The 4- and 7-aminobenzo[b]thiophenes of Formula (VIII') in Equation 4b, where $R_1'$ is $SO_2R_6$, $CO_2R_5$ or $OSO_2R_7$, $R_{10}$ is H or $C_1$—$C_3$ alkyl and $R_{11}$ is H or $CH_3$, can be prepared by methods analogous to those described previously in Equation 14. Thus, by starting with an appropriate 6-chloro-7-nitrobenzo[b]thiophene or 5-chloro-4-nitrobenzo[b]thiophene, and carrying out the appropriate reactions described in Equation 14, one skilled in the art can prepare the subject compounds of Formula (VIII'). The starting 5-chloro-4-nitrobenzo[b]thiophenes and 6-chloro-7-nitrobenzo[b]thiophenes are described above in Equations 27 and 28, respectively.

The 4- and 7-aminobenzo[b]thiophenes of Formula (VIII') in Equation 4b, where $R_1'$ is $NO_2$ or $SO_2NR_8R_9$, $R_{10}$ is $C_1$—$C_3$ alkyl and $R_{11}$ is $CH_3$, can be prepared by methods described heretofore in Equation 15. THus, be starting with an appropriate 4- or 7-acetamidobenzo[b]thiophene, and carrying out the nitration procedures described in Equation 15, one skilled in the art can prepare the subject 4- or 7-aminobenzo[b]-thiophene of Formula (VIII'), where $R_1'$ is $NO_2$. Similarly, by carrying out the chlorosulfonation reactions on an appropriate 4- or 7-acetamidobenzo[b]thiophene, one can prepare the subject 4- or 7-aminobenzo[b]-thiophene of Formula (VIII'), where $R_1'$ is $SO_2NR_8R_9$. The nitration of acetamidobenzo[b]thiophenes is

known in the art, e.g., Bordwell and Stange, *J. Am. Chem. Soc., 77,* 5939 (1955). The chlorosulfonation of benzo[b]thiophenes is also known, e.g., Pailer, *Monatsh 92,* 677 (1961).

The 4- and 7-aminobenzo[b]thiophene-1,1-dioxides of Formula (VIII') in Equation 4b ($Q=SO_2$), where $R_1'$ is other than $CO_2R_5$ or $OSO_2R_7$, can be prepared from corresponding 4- and 7-aminobenzo[b]thiophenes. The preparation involves three steps: (1) acetylating the amine with acetic anhydride to form a 4- or 7-acetamidobenzo[b]thiophene; (2) oxidizing this compound with 30% hydrogen peroxide to form a 4- or 7-acetamidobezo[b]thiophene-1,1-dixoide; and (3) deacetylating this compound to form a 4- or 7-aminobenzo[b]thiophene-1,1-dioxide of Formula (VIII'). The acetylation step can be carried out in refluxing acetic anhydride with sulfuric acid catalyst by obvious methods. The oxidation step can be carried out in acetic acid solvent at 50° to 115°C for 0.5 to about 3 hours, according to the teachings of Bordwell and Stange, *J. Am. Chem. Soc., 77,* 5939 (1955). The deacetylation step can be carried out by hydrolysis with hydrochloric acid or saponification with sodium hydroxide by methods described heretofore in Equation 15.

Alternatively, certain 4- and 7-aminobenzo[b]thiophene-1,1-dioxides of Formula (VIII') described below can be prepared from corresponding 4- and 7-nitrobenzo[b]thiophenes. This involves two steps: (1) oxidizing the nitro compound with 30% hydrogen peroxide by methods described above to form a 4- or 7-nitrobenzo[b]thiophene-1,1-dioxide; and (2) reducing this nitro compound to form a 4- or 7-aminobenzo[b]-thiophene-1,1-dioxide of Formula (VIII'). The reduction step can be carried out with warm stannous chloride and hydrochloric acid in ethanol solvent according to the teachings of Bordwell and Albisetti, *J. Am. Chem. Soc., 70,* 1955 (1948) and Fries et al., *Ann. 527* 83 (1936). The starting 4- and 7-nitrobenzo[b]thiophenes have been described heretofore. By this method the 4- and 7-aminobenzo[b]thiophene-1,1-dioxides of Formula (VIII') can be prepared, where $R_1'$ is H, Cl, Br, $OCH_3$, $SO_2R_6$, $CO_2R_5$ or $OSO_2R_7$.

The 4- and 7-amino-2,3-dihydrobenzo[b]thiophene-1,1-dioxides of Formula (VIII') in Equation 4b ($Q_1 = SO_2$), where $R_1'$ is other than $CO_2R_5$, $OSO_2R_7$ or $NO_2$, can be prepared from corresponding 4- and 7-aminobenzo[b]thiophenes. The preparation involves four steps: (1) acetylating the amine with acetic anhydride to form a 4- or 7-acetamidobenzo[b]thiophene; (2) oxidizing this compound with 30% hydogen peroxide to form a 4- or 7-acetamidobenzo[b]thiophene-1,1-dioxide; (3) reducing this compound catalytically with 5% palladium-on-charcoal to form a 4- or 7-acetamido-2,3-dihhydro-benzo[b]thiophene-1,1-dioxide; and (4) deacetylating this compound to form a 4- or 7-amino-2,3-dihydrobenzo[b]thiophene-1,1-dioxide of Formula (VIII'). The acetylation, oxidation and deactylation steps can be carried out by methods described above for preparing 4- and 7-aminobenzo[b]thiophene-1,1-dioxides. The reduction step is best carried out at 25° to 40°C at 1 to 3 atmospheres of pressure in an inert solvent such as ethanol. The catalytic reduction of benzo[b]thiophene-1,1-dioxides to form 2,3-dihydrobenzo[b]thiophene-1,1-dioxides is known in the art, e.g., Bordwell and Stange, *J. Am. Chem. Soc., 77,* 5939 (1955) and Bordwell and McKellin, ibid, *73,* 2251 (1951).

Also, certain 4- and 7-amino-2,3-dihydrobenzo[b]thiophene-1,1-dioxides of Formula (VIII') described below can be prepared from corresponding 4- and 7-nitrobenzo[b]thiophenes. By starting with an appropriate 5-(substituted)-4-nitrobenzo[b]thiophene or 6-(substituted)-7-nitrobenzo[b]thiophene, and carrying out the reactions heretofore described in Equations 26a and 26b, one skilled in the art can prepare 4- and 7-amino-2,3-dihydrobenzo[b]thiophene-1,1-dioxides of Formula (VIII'), where $R_1'$ is H, Cl, Br, $CH_3$, $OCH_3$, $SO_2R_6$, $CO_2R_5$ or $OSO_2R_7$. The starting 5-(substituted)-4-nitrobenzo[b]thiophenes and 6-(substituted)-7-nitrobenzo[b]thiophenes have been described heretofore.

In addition, the 4- and 7-amino-2,3-dihydrobenzo[b]thiophene-1,1-dioxides of Formula (VIII'), where $R_1'$ is other than $CO_2R_5$ or $OSO_2R_7$, can be prepared from corresponding 4- and 7-amino-2,3-dihydrobenzo[b]-thiophenes. The preparation involves three steps: (1) acetyltating the amine with acetic anhydride to form a 4- or 7-acetamido-2,3-dihydrobenzo[b]thiophene; (2) oxidizing this compound with 30% hydrogen peroxide in acetic acid at 15° to 80°C for 0.5 to about 5 hours to form a 4- or 7-acetamido-2,3-dihydrobenzo[b]thiophene-1,1-dioxide; and (3) deacetylating this compound to form the subject compounds of Formula (VIII'). The acetylation and deacetylation reactions can be run by methods described above for preparing 4- and 7-aminobenzo[b]thiophene-1,1-dioxides from corresponding amines.

Similarly, the 4- and 7-amino-2,3-dihydrobenzo[b]thiophene-1-oxides of Formula (VIII') in Equation 4b ($Q_1$=SO), where $R_1'$ is other than $CO_2R_5$ or $OSO_2R_7$, can be prepared from corresponding 4- and 7-amino-2,3-dihydrobenzo[b]thiophenes. The preparation involves three steps: (1) acetylating the amine with acetic anhydride to form a 4- or 7-acetamido-2,3-dihydrobenzo[b]thiophene; (2) oxidizing this compound with one mole equivalent of *m*-chloroperbenzoic acid to form a 4- or 7-acetamido-2,3-dihydrobenzo[b]-thiophene-1-oxide) and (3) deacetylating this compound to form the subject compound of Formula (VIII'). The oxidation step can be run in an inert solvent such as methylene chloride at about 0° to 10°C for 1 to 16 hours, according to the teachings of Johnson and McCants, Jr., *J. Am. Chem. Soc., 87,* 1109 (1965). The acetylation and deacetylation steps can be run by methods described above.

The 4- and 7-amino-benzofurans, -benzo[b]thiophenes and -benzo[b]thiophene-1,1-dioxides of Formula (VIII') in Equation 4b, where $R_{10}$ or $R_{11}$ is Cl or Br, can be prepared by halogenation reactions by obvious methods. Thus, the compounds are prepared by (a) heating an appropriate 4- or 7-acetamidobenzofuran, -benzo[b]thiophene or -benzo[b]thiophene-1,1-dioxide with chlorine or bromine at about 10° to 60°C for 0.5 to 5 hours in an inert solvent such as chloroform or acetic acid, followed by (b) deacetylating the resulting halogenated product by one of the methods described previously in Equation 15.

It will also be apparent that the amines of Formulae III and III′ in Equations 1 and 3 are important intermediates for preparing the compounds of this invention. The following discussion refers to the amines III′, however, it should be noted that all amines III are encompassed in the discussion of amines III′.

The pyrimidines and triazines of Formula (III′a) to (III′d) below are either known or can be prepared by obvious methods by one skilled in the art. For instance, the synthesis of pyrimidines and triazines of the general formula III′a has been reviewed in "The Chemistry of Heterocyclic Compounds", a series published by Interscience Publishers, Inc., New York and London, 2-Aminopyrimidines are described by D. J. Brown in "The Pyrimidines", Vo. 16 of this series. 2-Amino-1,3,5-triazines are reviewed by E. M. Smolin and L. Rapaport in "*s*-Triazines and Derivatives", Vol. 13 of the same series. The synthesis of triazines is also described by F. C. Schaefer, U.S. 3,154,547 and by K. R. Huffman and F. C. Schaefer, *J. Org. Chem., 28,* 1812 (1963). The synthesis of the bicyclic amines III′c and III′d are described in our EP—A—0015683 and that of III′b in our EP—A—0046677.

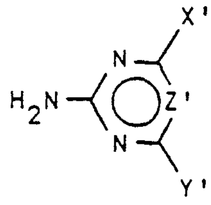

(III'a)

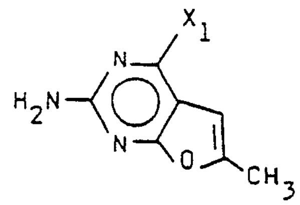

(III'b)

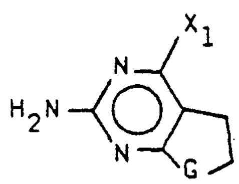

(III'c)

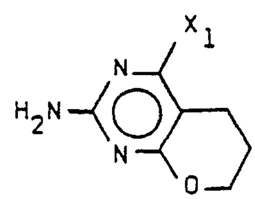

(III'd)

wherein

G, X′, $X_1$, Y′ and Z′ are as originally defined except Y′ is not $CH(OCH_3)_2$ or 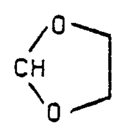 .

Pyrimidines below of Formula (III′e), where Y′ is $CH(OC_2H_5)_2$, are described by W. Braker et al., *J. Am. Chem. Soc., 69*, 3072 (1947), the disclosure of which is herein incorporated by reference. Using tehcniques taught by Braker, or suitable modifications that would be obvious to one skilled in the art, the pyrimidines III′e can be prepared.

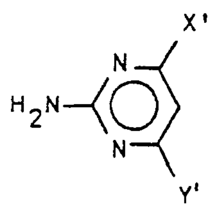

(III'e)

wherein

X′ is $CH_3$, $OCH_3$ or Cl; and

Y′ is $CH(OCH_3)_2$ or 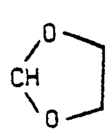 .

Triazines of Formula (III′f) may be prepared according to the methods outlines in Equations 29 and 30.

Equation 29

a)

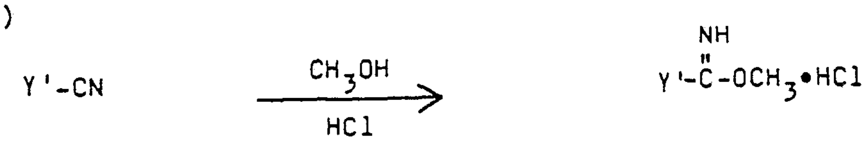


(XXVI) (XXVII)•HCl

b)

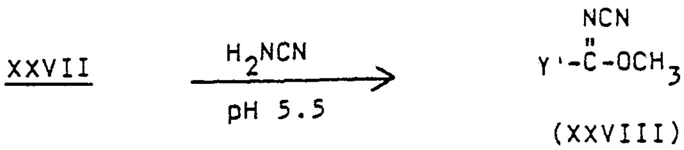


(XXVIII)

c)

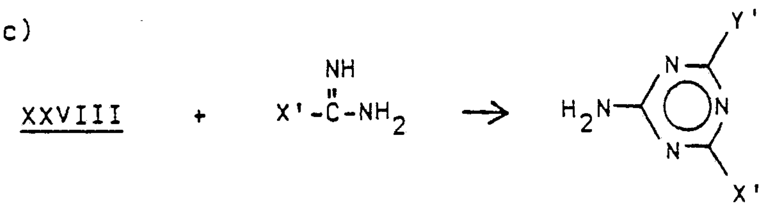

(III'f)

wherein

X' is $CH_3$ or $OCH_3$; and

Y' is $CH(OCH_3)_2$ or 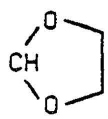 .

Equation 30

a)

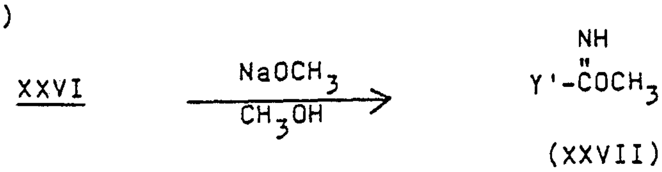


(XXVII)

b)

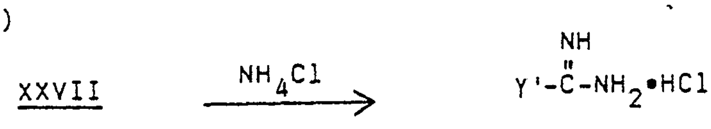


(XXIX)

c)

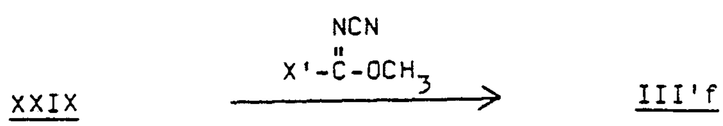

wherein

X' and Y' are as defined in Equation 29.

The reaction of Equation 29a is carried out according to the teachings of J. M. McElvain and R. L. Clarke, *J. Amer. Chem. Soc., 69,* 2657 (1947), in which the preparation of ethyl diethoxyiminoacetate is described. The intermediate N-cyanoimidate of Formula (XXVIII) may be prepared according to the teaching of D. Lwowski in *Synthesis, 1971,* 263, by reacting XXVII with cyanamide at pH 5.5, and this may be condensed according to reaction 29c with either acetamidine or O-methyl isourea in an alcoholic solvent at 25 to 80°C to provide the appropriate triazines. Alternatively, the reaction of Equation 30a, described for substituted acetonitriles by F. C. Schaefer and G. A. Peters in *J. Org. Chem., 26,* 412 (1961), may be used to convert nitrile of Formula XXVI to the correspondong iminoester. The free base may be carried on through reactions 30b and 30c, or, alternatively, converted to the amidinium hydrochloride salt (XXIX) as described in the aforementioned reference, and condensed with either methyl N-cyanoacetimidate or with dimethyl N-cyano imidocarbonate in the presence of one equivalent of sodium methoxide to provide the triazines of Formula (III'f).

Cyclic acetals of Formula (IIi'h) may also be prepared from compounds of Formula (III'g) according to Equation 31 by acetal exchange.

Equation 31

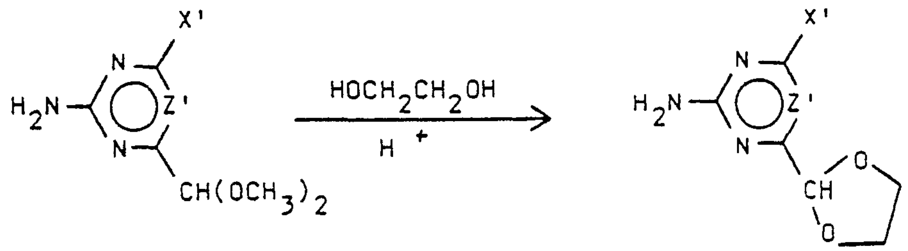


(III'g) (III'h)

wherein

X' is $CH_3$ or $OCH_3$; and

Z' is CH or N.

The reaction of Equation 31 is carried out by heating the acyclic acetal in an inert solvent in the presence of one equivalent ethylene glycol and slightly more than one equivalent or a strong acid, such as *p*-toluenesulfonic acid with removal of the methanol or ethanol formed in the reaction by distillation. The product is isolated by treatment with aqueous base, and extraction with an organic solvent, and purified by crystallization or column chromatography.

Preparations of 3-amino-1,2,4-triazoles of Formula (III') in Equations 1b and 3b are known in the art and 1,2,4-triazoles are reviewed in *The Chemistry of Heterocyclic Compounds* "Triazolews 1,2,4" (John Wiley and Sons, New York, 1981). Commonly used starting materials containing nitrogen are N-aminoguanidine, hydrazine, alkylhydrazines, cyanamide, ethyl cyanoacetidate, dimethyl cyanodithioimidocarbonate, dimethyl cyanoimidocarbonate, ethoxymethylenecyanamide, and acylhydrazines. Some literature synthesis are illustrated below. Using these techniques or suitable modifications that would be apparent to one skilled in the art, the 3-amino-1,2,4-traizole intermediates can be readily prepared.

Heating equimolar amounts of ethyl propionimidate hydrochloride and N-aminoguanidine nitrate in pyridine gives 3-amino-5-ethyltriazole; *German Patent* 1,073,499 (1960); *Berichte, 96,* 1064 (1963).

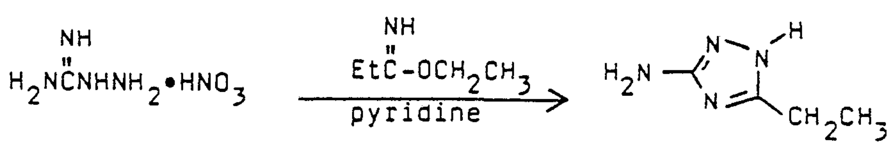


Condensation of hydrazine with ethyl N-cyanoacetimidate yields 3-amino-5-methyltriazole; *Journal of Organic Chemistry, 28,* 1816 (1963).

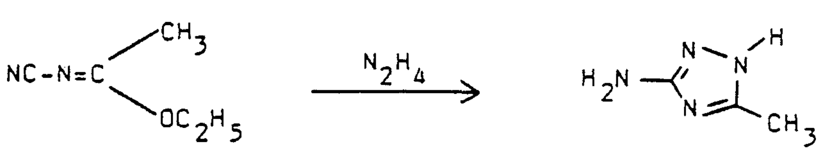


U.S. Patent 2,835,581 (1958) teaches the preparation of 3-amino-5-(hydroxymethyl)triazole from N-amino-guanidine and glycolic acid and *British Patent* 736,568 (1955) describes the synthesis of 3-amino-5-mercaptotriazole.

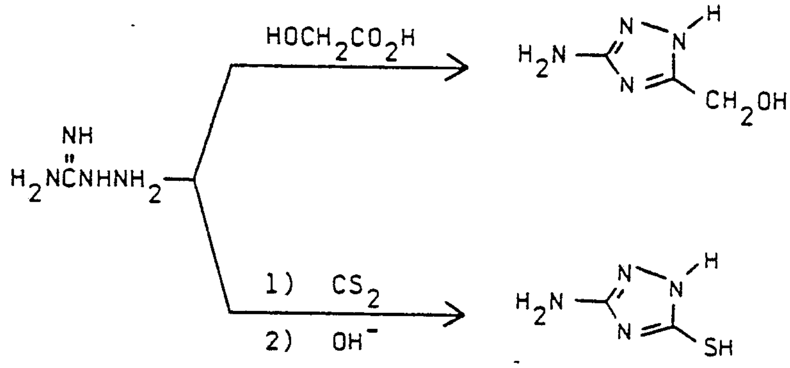


Condensing hydrazine with dimethyl cyanodithioimidocarbonate in acetonitrile gives 3-amino-5-methylthio-1,2,4-triazole while reaction of hydrazine with dimethyl N-cyanoimidocarbonate produces 3-amino-5-methoxy-1,2,4-triazole; *Journal of Organic Chemistry, 39*, 1522 (1974).

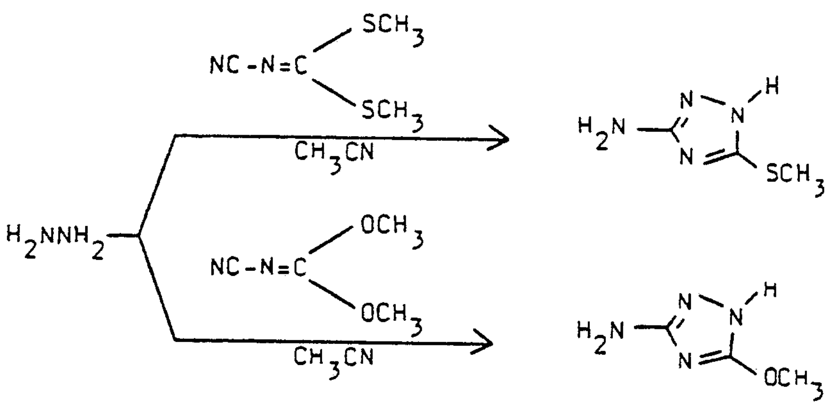


Reaction of substituted hydrazines with N-cyanothioimidocarbonates (prepared according to the procedure given in D. M. Wieland, Ph.D. Thesis, 1971. pp. 123—124) yields disubstituted aminotriazoles as shown below.

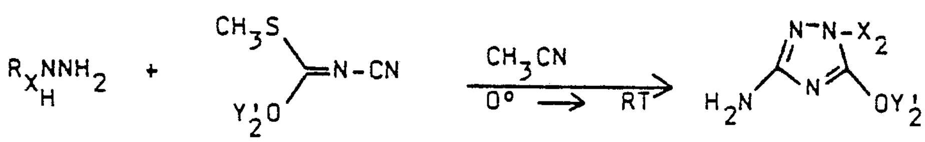


($Y'_2$ = $CH_3$ or $C_2H_5$)

Many of the aminoheterocyclic intermediates of Formula (III') where R is methyl may be prepared by a two-step procedure as described for III'i in Equation 32.

Equation 32

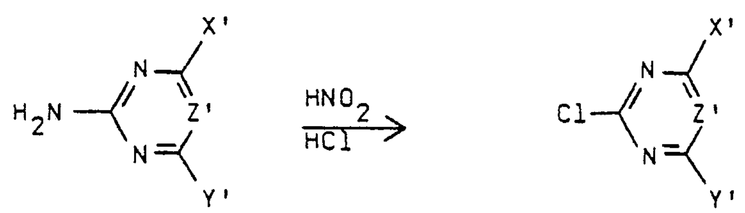


(XXX) (XXXI)

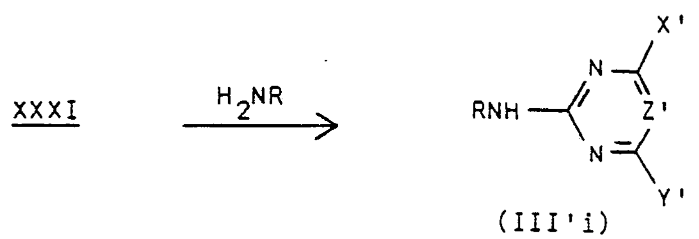


(III'i)

wherein

X', Y' and Z' are as originally defined and R is $CH_3$.

A solution of the amine (XXX) in concentrated hydrochloric acid is treated with sodium nitrite solution and the chloro compound (XXXI) is isolated in the usual manner by filtration of the acidic solution. A representative procedure is described by Bee and Rose in *J. Chem. Soc. C,* 2031 (1966), for the case in which Z' = CH, and X' = Y' = $OCH_3$. Displacement of the chlorine of (XXXI) may be accomplished by heating with an excess of methylamine in water to obtain the methylamino heterocycle (III'i).

Equation 33 below illustrates the preparation of the required methyl pyrimidinyl carbamates and methyl triazinyl carbamates of Formula (V') in Equations 2b. By obvious modiffications, other methyl carbamates of Formulae (V) and (V') may be prepared by this method.

Equation 33

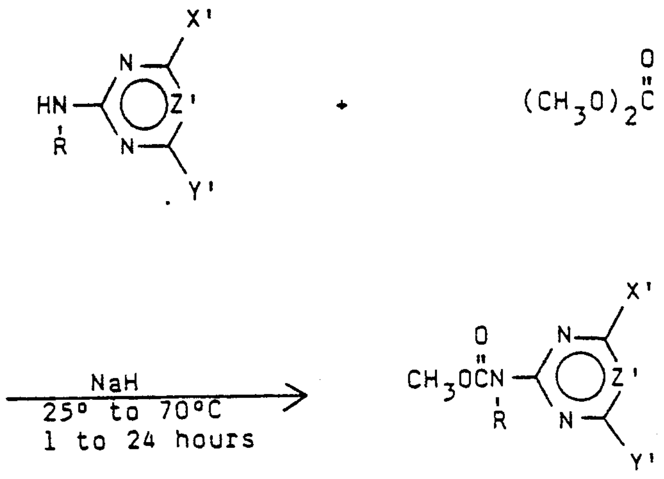


(V'a)

wherein

X', Y', Z' and R are as originally defined.

According to Equation 33, a heterocyclic amine is reacted with two equivalents of sodium hydride and excess dimethyl carbonate to form V'a. The reaction is run in an inert solvent such as tetrahydrofuran at 25°C to reflux for 1 to 24 hours . The product is isolated by (a) adding about two equivalents of concentrated hydrochloric acid under nitrogen at 0° to 30°C; (b) filtering; and (c) separating out the organic phase, then drying (sodium sulfate and/or magnesium sulfate) and concentrating to dryness *in vacuo*. The product V'a may be purified further by recrystallization or chromatography procedures.

Agriculturally suitable salts of compounds of Formulae I and I' are also useful herbicides and can be prepared by a number of ways known to the art. For example, metal salts can be made by treating compounds of Formulae I or I' with a solution of alkali or alkaline earth metal salt having a sufficiently basic anion (e.g., hydroxide, alkoxide, carbonate, or hydride). Quaternary amine salts can be made by similar techniques.

Salts of compounds of Formulae I and I' can also be prepared by exchange of one cation to another. Cationic exchange can be effected by direct treatment of an aqueous solution of a salt of a compound of Formulae I or I' (e.g., alkali metal or quaternary amine salt) with a solution containing the cation to be exchanged. This method is most effective when the desired salt containing the exchanged cation is insoluble in water, e.g. a copper salt, and can be separated by filtration.

Exchange may also be effected by passing an aqueous solution of a salt of a compound of Formulae I or I' (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water soluble, e.g., a potassium, sodium or calcium salt.

Acid addition salts, useful in this invention, can be obtained by reacting a compound of Formulae I or I' with a suitable acid, e.g. *p*-toluenesulfonic acid or trichloroacetic acid.

In the following examples all parts are by weight and temperature in °C unless otherwise indicated.

Example 1

2,3-Dihydro-2,2-dimethyl-7-benzofuransulfonyl chloride

A diazonium salt was prepared by adding 13.8 g of sodium nitrite to a suspension of 32.6 g of 7-amino-2,3-dihydro-2,2-dimethylbenzofuran and 40 ml of concentrated sulfuric acid in 200 ml water cooled 0° to

5°C. After stirring for about 0.4 hour at 0° to 5°C, the diazonium salt suspension was poured in one portion into a mixture consisting of 170 ml of acetic acid, 40 ml of concentrated hydrochloric acid, 17 g of cupric chloride dihydrate and 30 ml of sulfur dioxide and cooled at 10°C by an ice-water bath. The mixture was stirred about 1 hour at 15° to 25°C. then 400 ml of 1-chlorobutane and 200 ml of water was added and the mixture was stirred and heated at 35°C for 5 hours. After cooling to room temperature, the organic layer was separated, washed with saturated aqueous $NaHCO_3$ and water, and dried over sodium sulfate for 0.5 hour. The solvent was evaporated under reduced pressure at less than 45°C to give 26 g of crude 2,3-dihydro-2,2-dimethyl-7-benzofuransulfonyl chloride as an oil.

Example 2

2,3-Dihydro-2,2-dimethyl-7-benzofuransulfonamide

A solution of 26 g of 2,3-dihydro-2,2-dimethyl-7-benzofuransulfonyl chloride prepared in Example 1, in 130 ml of tetrahydrofuran, was cooled in an ice-water bath while about 30 ml of concentrated aqueous ammonium hydroxide was added portionwise at 10° to 30°C. The resulting suspension was stirred at room temperature for 3 hours, then the solvent was evaporated under reduced pressure. The residue was stirred in 150 ml of water for 0.5 hour, then filterd. The crude, wet solid was dissolved in chloroform and dried over sodium sulfate. The solvent was evaporated under reduced pressure to give a dry solid. The solid was washed once with about 100 ml of hot toluene to give 20 g of 2,3-dihydro-2,2-dimethyl-7-benzofuransulfonamide, m.p. 163—165°C.

| | | | |
|---|---|---|---|
| Anal. Calcd. for $C_{10}H_{13}NO_3S$: | C, 52.8; | H, 5.8; | N, 6.2; |
| Found: | C, 52.5; | H, 5.7; | N, 6.1. |

Example 3

N-(Butylaminocarbonyl)-2,3-dihydro-2,2-dimethyl-7-benzofuransulfonamide

A solution of 19 g of 2,3-dihydro-2,2-dimethyl-7-benzofuransulfonamide prepared in Example 2 and 9.9 g of *n*-butyl isocyanate in 200 ml of 2-butanone was refluxed with 11.5 g of anhydrous potassium carbonate for 7 hours. The resulting mixture was concentrated to dryness *in vacuo*. The residue was taken up in 400 ml of water and extracted once with 100 ml of ethyl ether. The aqueous layer was acidified with 2N HCl and the resulting mixture was filtered and suction dried. The still slightly wet solid was washed once with 100 ml of hot acetonitrile, then suction dried an additional 8 hours to give 23 g of N-(butylaminocarbonyl)-2,3-dihydro-2,2-dimethyl-7-benzofuransulfonamide, m.p. 200—203°C.

| | | | |
|---|---|---|---|
| Anal. Calcd. for $C_{15}H_{22}N_2O_4S$: | C, 55.2; | H, 6.8; | N, 8.6; |
| Found: | C, 54.8; | H, 6.6; | N, 8.5. |

Example 4

2,3-Dihydro-2,2-dimethyl-7-benzofuransulfonyl isocyanate

A suspension of 22 g of the N-(*n*-butylaminocarbonyl)-2,3-dihydro-2,2-dimethyl-7-benzofuransulfonamide prepared in Example 3, in 125 ml of xylene containing 0.3 g of DABCO was heated at 130—135°C while 5.3 ml of phosgene was added portionwise at a rate to maintain a reflux temperature of 130—135°C. The mixture was refluxed for an additional 1.5 hours, cooled under nitrogen, and concentrated to dryness *in vacuo*. A sample of the crude oily product displayed a characteristic sulfonyl isocyanate band in the IR at 2200 $cm^{-1}$.

Example 5

N-[(4,6-Dimethylpyrimidin-2-yl)aminocarbonyl]-2,3-dihydro-2,2-dimethyl-7-benzofuransulfonamide

To a solution of 1.2 g of 2-amino-4,6-dimethylpyrimidine in 25 ml of tetrahydrofuran was added 2.5 g of 2,3-dihydro-2,2-dimethyl-7-benzofuranylsulfonyl isocyanate prepared above. After a slight exotherm the solution was stirred at room temperature for 4 hours. The solution was concentrated *in vacuo* to give a viscous oil that precipitated a solid from 10 ml of acetonitrile. The solid was recrystallized from acetonitrile to give 2 g of N - [(4,6 - dimethylpyrimidin - 2 - yl)aminocarbonyl] - 2,3 - dihydro - 2,2 - dimethyl - 7 - benzofuransulfonamide, m.p. 203—206°C. The IR spectrum showed a carbonyl absorption at 1680 $cm^{-1}$ indicative of a sulfonylurea.

| | | | |
|---|---|---|---|
| Anal. Calcd. for $C_{17}H_{20}N_4O_4S$: | C, 54.2; | H, 5.4; | N, 14.9; |
| Found: | C, 54.3; | H, 5.4; | N, 15.2. |

Example 6

Methyl (4-methoxy-6-methylpyrimidin-2-yl)carbamate

To a suspension of 50 g of 2-amino-4-methoxy-6-methylpyrimidine in 1000 ml of tetrahydrofuran was added portionwise, under a nitrogen atmosphere, 42.8 g of 50% sodium hydride while cooling the reaction flask in an ice-water bath. After stirring one hour at 25°C, 58.5 g of dimethylcarbonate was added dropwise at 5° to 25°C. The suspension was stirred about 16 hours at ambient temperature, then 80 ml of concentrated hydrochloric acid was added dropwise while maintaining a reaction temperature of 20° to 25°C with external ice-bath cooling. The suspension was stirred 0.5 hour, filtered and then concentrated *in vacuo*. The residue was recrystallized from hexane to yield 54 g of the title compound, m.p. 89—92.5°C.

Example 7

7-Amino-2,3-dihydro-2-methylbenzo[b]thiophene

(2-Aminophenyl)allylsulfide (80 g) was heated at 250°—280°C for 3 hours, cooled, then subjected to spinning band distillation through a 20 cm column using a 5:1 reflux ratio. The fraction distilling at 80°—82° at 0.25 mm of mercury was collected (18.0 g) and shown by NMR spectrum analysis to be the title compound in approximatley 90% purity. (See also Example 21).

NMR ($CDCl_3$)δ: 7.0—6.3 (m, 3H, ArH); 3.9 (m, 1H, CH); 3.5 (broad, 2H, $NH_2$); 3.5—2.6 (m, 2H, $CH_2$); and 1.35 (d, 3H, $CH_3$).

Example 8

7-Acetamido-2,3-dihydro-2-methylbenzo(b)thiophene

To a solution of 18.0 g of 7-amino-2,3-dihydro-2-methylbenzo[b]thiophene (Example 7) in 100 ml of 1-chlorobutane was added a solution of 13.0 ml of acetic anhydride in 20 ml of 1-chlorobutane. After the exothermic reaction subsided, the mixture was refluxed for 0.3 hour, cooled in an ice-bath and filtered. The isolated solid was washed with 1-chlorobutane to yield 15.2 g of the title compound; m.p. 125—127°C.

NMR ($CDCl_3$)δ: 7.8—6.7 (m, 4H, ArH + NH); 4.0 (m, 1H, CH); 3.6—2.8 (m, 2H, $CH_2$); 2.2 (s, 3H, $CH_3$); and 1.4 (d, 3H, $CH_3$).

Example 9

7-Acetamido-2,3-dihydro-2-methylbenzo[b]thiophene-1,1-dioxide

To a solution of 24.7 g of 7-acetamido-2,3-dihydro-2-methylbenzo[b]thiophene (Example 8) in 100 ml of glacial acetic acid was added dropwise 60 ml of a 30% aqueous solution of hydrogen peroxide. The reaction temperature rose to 65°C during the addition. The mixture was heated at 65—75°C for one hour, cooled to 25°C, diluted with water and extracted with methylene chloride. The extract was washed with water saturated with sodium bisulfite, dried over magnesium sulfate and concentrated *in vacuo*. The oily residue was crystallized from 1-chlorobutane to yield 14.3 g of the title compound as light yellow crystals; m.p. 115—117°C.

NMR ($CDCl_3$)δ: 8.4—7.0 (m, 4H, ArH + NH); 3.8—2.7 (m, 3H, $CH_2$ + CH); 2.2 (s, 3H, $CH_3$); and 1.5 (d, 3H, $CH_3$).

Example 10

2,3-Dihydro-2-methyl-7-benzo[b]thiophenesulfonamide-1,1-dioxide

A. A solution of 18.6 g of 7-acetamido-2,3-dihydro-2-methylbenzo[b]thiophene-1,1-dioxide (Example 9) in 100 ml of concentrated hydrochloric acid was refluxed for one hour to yield a suspension containing the hydrochloride salt of 7-amino-2,3-dihydro-2-methylbenzo[b]thiophene-1,1-dioxide.

B. This suspension was diluted with 25 ml of glacial acetic acid, cooled to −5°C and treated with a solution of 6.4 g of sodium nitrite in 10 ml of water such that the temperature did not rise above 5°C. The mixture was stirred for 0.5 hour at 0°C, then added in one portion to a suspension cooled at −7°C and containing 50 ml of concentrated hydrochloric acid, 50 ml of glacial acetic acid, 2.0 g cupric chloride dihydrate and 10 ml of liquified sulfur dioxide. The mixture was stirred at 20°C for two hours then diluted with excess water to yield a precipitate. The mixture was filtered and the solid residue was washed with water to yield 2-methyl-2,3-dihydro-7-benzo[b]thiophenesulfonyl chloride 1,1-dioxide as a crude solid.

C. This solid was dissolved in methylene chloride and contacted with 7 ml of liquified ammonia at −10°C. The mixture was stirred at 20°C for 16 hours, then filtered and the solids were washed with water and ether. The wet, solid residue was suspended in benzene and refluxed under a Dean-Stark trap until no more water distilled from the suspension. The suspension was cooled and filtered to yield 11.4 g of the title compound; m.p. 167—169°C.

NMR ($CDCl_3$ + DMSO)δ: 8.1—7.5 (m, 3H, Ar); 7.1 (broad, 2H, $NH_2$); 3.8—2.5 (m, 3H, $CH_2$ + CH); and 1.5 (d, 3H, $CH_3$).

IR (nujol): 3300, 3200 $cm^{-1}$ ($NH_2$).

Example 11

N-[(4-Methoxy-6-methylpyrimidin-2-yl)aminocarbonyl)-2,3-dihydro-2-methyl-7-benzo[b]thiophenesulfonamide-1,1-dioxide

A suspension of 1.15 g 2,3-dihydro-2-methyl-7-benzo[b]thiophenesulfonamide-1,1-dioxide (Example 10) in 20 ml of dry methylene chloride was purged with nitrogen. To the slurry was added carefully 3.0 ml of a 20% toluene solution of trimethyl aluminum (Aldrich Chemicals) while cooling the flask at 10 to 30°C. After stirring for 0.2 hour, 0.9 g of methyl (4-methoxy-6-methylpyrimidin-2-yl)carbamate (Example 6) was added in one portion, and the suspension was refluxed under nitrogen atmosphere for 24 hours. The suspension was cooled in an ice-water bath while 20 ml of 1N hydrochloric acid was slowly added. After several minutes of stirring, the organic layer was separated, washed with water followed by water saturated with sodium chloride (brine), then dried over magnesium sulfate and concentrated *in vacuo*. The residue was triturated with chloroform and methylene chloride to yield 0.3 g of the title compound; m.p. 224—226°C.

NMR ($CDCl_3$ + DMSO)δ: 10.3 (broad, 1H, NH); 8.2—7.6 (m, 3H, Ar); 6.3 (s, 1H, CH); 4.0 (s, 3H, $OCH_3$); 3.7—2.8 (m, 3H, $CH_2$ + CH); 2.5 (s, 3H, $CH_3$); and 1.5 (d, 3H, $CH_3$).

IR (nujol): 1700 $cm^{-1}$ (c=o).

## Example 12

N-*t*-Butyl-2-(2-methyl-2-propenylthio)benzenesulfonamide

To an ice-cooled solution of 42.6 g of N-*t*-butylbenzenesulfonamide in 800 ml of dry tetrahydrofuran under a nitrogen atmosphere was added dropwise 262 ml of a 1.6M solution of *n*-butyl lithium in hexane. The mixture was stirred for 2 hours at 20°C during which time a precipitate formed. The suspension was cooled to 0°C and 6.4 g of elemental sulfur was added in one portion. The mixture was warmed to 20°C and stirred for one hour, then cooled at 0°C while 20.5 ml of methallyl chloride was added slowly. The mixture was stirred at 20°C for 16 hours, then 100 ml of 10% hydrochloric acid was added. After stirring several minutes, the organic layer was separated, washed with water and brine, dried over magnesium sulfate and concentrated *in vacuo.* The residue was slurried in 20% ether in hexane for several minutes and filtered to yield 47 g of the title compound; m.p. 104—107°C.

NMR ($CDCl_3$)δ: 8.2—7.2 (m, 4H, Ar); 5.6 (broad, 1H, NH); 4.9 (broad, 2H, vinyl); 3.7 (s, 2H, $CH_2$); 1.9 (m, 3H, $CH_3$); and 1.2 (s, 9H, *t*-butyl).

## Example 13

N-*t*-Butyl-2,3-dihydro-2,2-dimethyl-7-benzo[b]thiophenesulfonamide

A solution of 37 g of N-*t*-butyl-2-(2-methyl-2-propenylthio)benzenesulfonamide (Example 12) in 40 ml of quinoline was heated at 220°C for one hour, then cooled to 25°C and diluted with ether. The ether suspension was washed well with 10% hydrochloric acid followed by water and brine, then dried over magnesium sulfate and concentrated *in vacuo.* The oily residue was chromatographed on 300 g of silica gel, packed and eluted with 20% ether in hexane, to give a major band which was concentrated *in vacuo* to yield a solid. The solid was slurried in 20% ether in hexane and filtered to yield 14.2 g of the title compound; m.p. 103—105°C.

NMR ($CDCl_3$)δ: 7.9—7.0 (m, 3H, Ar); 5.0 (broad, 1H, NH); 3.15 (s, 2H, $CH_2$); 1.6 (m, 6H, $CH_3$); and 1.2 (s, 9H, *t*-butyl).

IR (nujol): 3200 $cm^{-1}$ (NH).

## Example 14

2,3-Dihydro-2,2-dimethyl-7-benzo[b]thiophenesulfonamide

A solution of 17.2 g of N-*t*-butyl-2,3-dihydro-2,2-dimethyl-7-benzo[b]thiophenesulfonamide (Example 13) in 100 ml of trifluoroacetic acid was stirred at 20°C for 16 hours. The solution was concentrated *in vacuo,* 100 ml of fresh trifluoroacetic acid was added and the mixture stirred at 20°C for four more hours. After concentrating the solution *in vacuo,* the residue was dissolved in methylene chloride and the solution was washed with saturated aqueous sodium bicarbonate followed by brine, then dried over magnesium sulfate and concentrated *in vacuo.* The solid residue was slurried in 50% ether in hexane for several minutes then filtered to yield 8.4 g of the title compound; m.p. 102—104°C.

NMR ($CDCl_3$)δ: 8.9—7.0 (m, 3H, Ar); 5.1 (broad, 2H, $NH_2$); 3.2 (s, 2H, $CH_2$); and 1.6 (s, 6H, $CH_3$).

IR (nujol): 3300 $cm^{-1}$ (NH).

## Example 15

2,3-Dihydro-2,2-dimethyl-7-benzo[b]thiophenesulfonylisocyanate

To a refluxing solution of 8.6 g of 2,3-dihydro-2,2-dimethyl-7-benzo[b]thiophenesulfonamide (Example 14), 4.0 ml of *n*-butylisocyanate and 0.1 g of DABCO in 100 ml of xylenes was added 3.0 ml of phosgene at such a rate that the reaction temperature did not drop below 135°C. The mixture was refluxed for 1.5 hours, then the excess phosgene was purged with a stream of dry nitrogen. The mixture was cooled, filtered and concentrated *in vacuo* to yield the title compound as an oil. The crude yield was assumed to be near quantitative. IR (neat) 2220 $cm^{-1}$ (NCO).

The oil was diluted to a volume of 88 ml with dry acetonitrile for use as a stock solution in subsequent reactions.

## Example 16

N-[(4,6-Dimethoxypyrimidin-2-yl)aminocarbonyl]-2,3-dihydro-2,2-dimethyl-7-benzo[b]thiophenesulfonamide

To 22 ml of the sulfonylisocyanate stock solution prepared in the previous Example (15) was added 0.78 g of 2-amino-4,6-dimethoxypyrimidine in one portion. The mixture was heated to reflux and stirred for 16 hours at 20°C during which time a precipitate formed. The suspension was filtered and the solid washed with ether to yield 1.4 g of the title compound; m.p. 190—192°C.

NMR ($CDCl_3$)δ: 12.7 (broad, 1H, NH); 8.0—7.0 (m, 4H, Ar + NH); 5.8 (s, 6H, CH); 4.0 (s, 6H, $OCH_3$); 3.15 (s, 2H, $CH_2$); and 1.5 (s, 6H, $CH_3$).

IR (nujol): 1700 $cm^{-1}$ (c=o).

Example 17

7-Nitrobenzo[b]thiophene 1,1-dioxide

To a solution of 24.8 g of 7-nitrobenzo[b]thiophene in 200 ml of glacial acetic acid pre-heated to 100°C was added 90 ml of a 30% aqueous solution of hydrogen peroxide at such a rate that the temperature remained between 100° to 105°C. The solution was refluxed for one hour, then 200 ml of water was added and the mixture was cooled in an ice-bath and filtered. The solid residue was washed sequentially with water, ethanol and ether to yield 20g of the title compound; m.p. 195—197°C.

NMR ($CDCl_3$ + DMSO)δ: 8.5 (m, 1H, Ar); 8.0 (m, 2H, Ar); 7.6 (m, 1H, Ar); and 7.2 (m, 1H, Ar).

IR (nujol): 1540 and 1300 $cm^{-1}$.

Example 18

7-Amino-2,3-dihydrobenzo[b]thiophene 1,1-dioxide

7-Nitrobenzo[b]thiophene-1,1-dioxide (19 g, Example 17) was hydrogenated in 200 ml of ethyl acetate over 1.0 g of 10% palladium-on-charcoal catalyst at 500 pounds-per-square inch of pressure and 100°C until no more hydrogen gas was absorbed. The mixture was filtered through celite, and the filtrate was concentrated *in vacuo.* The solid residue was recrystallized from 1-chlorobutane to yield 13.2 of the title compound; m.p. 117—119°C.

NMR ($CDCl_3$ + DMSO)δ: 7.3—6.7 (m, 3H, Ar); 5.1 (broad, 2H, $NH_2$); and 3.4 (m, 4H, $CH_2$).

IR (nujol): 3400, 3300, 1630 and 1590 $cm^{-1}$.

Example 19

2,3-Dihydro-7-benzo[b]thiophenesulfonamide 1,1-dioxide

A. A diazonium salt was prepared by adding a solution of 5.3 g of sodium nitrite in 10 ml of water to a suspension containing 12.8 g of 7-amino-2,3-dihydrobenzo[b]thiophene-1,1-dioxide (Example 18), 25 ml of glacial acetic acid and 75 ml of concentrated hydrochloric acid, cooled at −5° to 5°C during the addition. After stirring for 0.5 hour at 0°C, the diazonium salt suspension was added dropwise to a mixture consisting of 75 ml of concentrated hydrochloric acid, 50 ml of glacial acetic acid, 1.0 g of cupric chloride dihydrate and 8.0 ml of liquified sulfur dioxide, cooled at −5°C during the addition. The mixture was stirred at 0°C for one hour and at 20°C for two hours, then diluted with excess water and stirred to yield a precipitate. The mixture was filtered and the solid residue was washed with water to yield crude 2,3-dihydro-7-benzo[b]thiophene-sulfonyl chloride-1,1-dioxide.

B. The above sulfonyl chloride was dissolved in methylene chloride and dried over magnesium sulfate. The dried solution was contacted with 5.0 ml of liquified ammonia at −7°C, then stirred at 20°C for 18 hours. The suspension was concentrated *in vacuo,* and the residue was slurried in 100 ml of 10% hydrochloric acid, then filtered. The solid residue was washed with water and ether to yield 11.5 g of the title compound; m.p. 215—217°C.

NMR ($CDCl_3$ + DMSO)δ: 8.1—7.6 (m, 3H, Ar); 7.0 (broad, 2H, $NH_2$); and 3.8—3.3 (m, 4H, $CH_2$).

IR (nujol): 3300, 3200 and 1350 $cm^{-1}$.

Example 20

N-[(4-Methoxy-6-methylpyrimidin-2-yl)aminocarbonyl-2,3-dihydro-7-benzo[b]thiophene 1,1-dioxide

A slurry of 1.0 g of 2,3-dihydro-7-benzo[b]thiophenesulfonamide 1,1-dioxide (Example 19) in 20 ml of dry methylene chloride was treated with 2.5 ml of a 20% solution of trimethylaluminum in toluene (Aldrich Chemicals) under a blanket of dry nitrogen. After stirring at 20°C for 0.25 hour, 1.0 g of methyl (4-methoxy-6-methylpyrimidin-2-yl)carbamate (Example 6) was added in one portion and the resulting solution was stirred at 20°C for 60 hours followed by refluxing for 24 hours. The mixture was cooled in an ice-bath, then treated with 10 ml of 10% hydrochloric acid and filtered. The solid residue was heated in tetrahydrofuran and filtered hot to yield 0.45 g of the title compound; m.p. 227—229°C.

NMR ($CDCl_3$ + DMSO)δ: 13.7 (broad, 1H, NH); 10.1 (broad, 1H, NH); 8.2—7.7 (m, 3H, Ar); 6.3 (s, 1H, CH); 4.0 (s, 3H, $OCH_3$); 3.5 (m, 4H, $CH_2$, $CH_2$); and 2.4 (s, 3H, $CH_3$).

IR (nujol): 3200 (NH), 1710 (c=o) $cm^{-1}$.

Example 21

8-Amino-2H-1-benzothiopyran

(2-Aminophenyl)allylsulfide (55 g) was heated at 250°C for 1 hour, cooled, and then the product was distilled through a 20 cm jacketed spinning band column using a 5:1 reflux ratio. The fraction distilling at 88—90°C at 0.25 mmHg was collected (13.3 g) and shown to be approximately 90% pure 8-aminobenzothiopyran by NMR analysis. (See also Example 7).

NMR ($CDCl_3$)δ: 7.0—6.3 (m, 3H, arom); 3.7 (br, 2H, $NH_2$); 3.0 (m, 2H, $CH_2$); 2.7 (m, 2H, $CH_2$); and 2.0 (m, 2H, $CH_2$).

IR (neat) 3350, 3300 and 1600 $cm^{-1}$.

Example 22

8-Acetamido-2H-1-benzothiopyran

To a solution of 21.1 g of 8-amino-2H-1-benzothiopyran in 200 ml of 1-chlorobutane was added a

solution of 13.3 ml of acetic anhydride in 50 ml of 1-chlorobutane. The temperature of the mixture rose during the addition and the mixture was refluxed for an additional 30 minutes. The solution was cooled in ice and filtered to give 18.0 g of the desired acetamide as colorless needles, m.p. 133°—135°C. The mother liquor was concentrated to ~1/5 volume to give a second crop of 4.1 g, m.p. 133°—135°C.

NMR ($CDCl_3$)δ: 8.0—6.8 (m, 4H, arom + NH); 3.0 (m, 2H, $CH_2$); 2.8 (m, 2H, $CH_2$); and 2.1 (s and m, 5H, $CH_3$ and $CH_2$).

IR (nujol) 3100 and 1630 $cm^{-1}$.

Example 23

8-Acetamido-2H-1-benzothiopyran, 1,1-dioxide

To a solution of 21.0 g of 8-acetamido-2H-1-benzothiopyran in 100 ml of glacial acetic acid was added 60 ml of 30% $H_2O_2$ in water. The addition was accompanied by an exotherm to 70°C and the temperature was held at 70°C for an additional hour. The solution was cooled, diluted with water and extracted with methylene chloride. The extract was washed with water, saturated sodium bisulfite and brine, dried over $MgSO_4$ and concentrated to give 23 g of an orange oil. The oil was dissolved in 50 ml of hot 1-chlorobutane and allowed to cool to give 18.0 g of the desired dioxide as orangish-white crystals, m.p. 98—100°C.

NMR ($CDCl_3$)δ: 9.2 (br, 1H, NH); 8.3 (d, J=8Hz, 1H, arom); 7.4 (t, J=8Hz, 1H, arom); 7.0 (d, J=8Hz, 1H, arom); 3.4 (m, 2H, $CH_2$); 3.0 (m, 2H, $CH_2$); 2.5 (m, 2H, $CH_2$); and 2.2 (s, 3H, $CH_3$).

IR (nujol) 3300 and 1690 $cm^{-1}$.

Example 24

8-Amino-2H-1-benzothiopyran, 1,1-dioxide Hydrochloride

A solution of 17.5 g of 8-acetamido-2H-1-benzothiopyran, 1,1-dioxide in 100 ml of concentrated HCl was heated at 80—95°C for 1 hour, cooled in ice and filtered to give a white solid which was washed with ether and air dried to give 14.7 g of the free amine as its hydrochloride salt, m.p. 205—209°C.

NMR (TFA)δ: 7.9—7.5 (m, 3H, arom); 3.7 (m, 2H, $CH_2$); 3.2 (m, 2H, $CH_2$); and 2.7 (m, 2H, $CH_2$).

IR (nujol) 3200—2300 $cm^{-1}$.

Example 25

2H-1-Benzothiopyran-8-sulfonamide, 1,1-dioxide

To a suspension of 14.0 g of 8-amino-2H-1-benzothiopyran, 1,1-dioxide hydrochloride cooled to −5°C in 25 ml of concentrated HCl and 25 ml of glacial acetic acid was added a solution of 4.55 g of sodium nitrite in 10 ml of water at such a rate that the reaction temperature did not rise above 5°C. The mixture was stirred at 0°C for 30 minutes then added dropwise to a solution of 50 ml of concentrated HCl, 50 ml of glacial acetic acid, 2.0 g of $CuCl_2 \cdot 2H_2O$ and 8 ml of liquified $SO_2$ cooled to −5°C. The mixture was stirred at 0°C for 1 hour and at 20°C for 2 hours during which time gas evolution was noted. The mixture was diluted with 400 ml of water and the resulting solid was filtered, washed with water and air dried. The solid was suspended in 250 ml of methylene chloride and contacted with 4 ml of liquified ammonia at −5°C. The suspension was stirred at room temperature for 65 hours, concentrated to a white powder, suspended in 100 ml of 1*N* HCl and filtered to give the sulfonamide as a white solid, 14.5 g, m.p. 214—216°C (wet). 13.5 g of the wet sulfonamide was suspended in xylene and refluxed under a dean-stark trap to remove water. Cooling and filtration gave 11.5 g of the sulfonamide as a white solid, m.p. 239—241 g.

NMR ($CDCl_3$ + DMSO)δ: 8.2 (m, 1H, arom); 7.7—7.5 (m, 2H, arom); 7.1 (br, 2H, $SO_2NH_2$); 3.7—3.1 (m, 4H, $CH_2$'s); and 2.5 (m, $CH_2$ + DMSO solvent peak).

IR (nujol) 3300, 3200, 1330 and 1170 $cm^{-1}$.

Example 26

2H-1-Benzothiopyran-8-sulfonylisocyanate, 1,1-dioxide

A solution of 8.0 g of 2H-1-benzothiopyran-8-sulfonamide, 1,1-dioxide and 6.0 ml of *n*-butylisocyanate in 100 ml of 2-butanone was refluxed in the presence of 6.0 g of $K_2CO_3$ for 16 hours. The mixture was cooled, poured into 500 g of ice-water, acidified with 10% HCl and extracted with methylene chloride. The extract was dried over $MgSO_4$ and concentrated to give 10.2 g of the crude *n*-butylurea as an amber oil which solidified on standing. The crude urea was dissolved in 200 ml of dry xylenes containing 0.2 g of DABCO and 4.0 ml of *n*-butylisocyanate and heated to 138°C. The mixture was contacted with 4.0 ml of phosgene at such a rate that the temperature remained between 132 to 138°C. The solution was refluxed for an additional hour, cooled to 25°C, filtered under nitrogen and concentrated to give the crude sulfonylisocyanate as an oil. IR (neat) 2230 $cm^{-1}$. The crude sulfonylisocyanate was diluted to 120 ml with dry methylene chloride to give a stock solution for use in subsequent reactions.

Example 27

N-[(4-Methoxy-6-methylpyrimidin-2-yl)aminocarbonyl]-2H-1-benzothiopyran-8-sulfonamide, 1,1-dioxide

0.35 g of 2-amino-4-methoxy-6-methylpyrimidine was added to 20 ml of the sulfonylisocyanate stock solution (ca. 1.3 g of the sulfonylisocyanate) prepared in Example 26 and the mixture stirred for 16 hours at 25°C. The solution was triturated with 1-chlorobutane and the crystals which formed were filtered to give 0.13 g of the desired urea, m.p. 217—220°C.

NMR ($CDCl_3$ + DMSO)δ: 13.4 (br, 1H, NH); 9.8 (br, 1H, NH); 8.4 (m, 1H, arom); 7.8—7.5 (m, 2H, arom); 6.3 (s, 1H, CH); 4.0 (s, 3H, $OCH_3$); 3.5—3.0 (m, $CH_2$'s); 2.6—2.3 (m, $CH_2$ + DMSO solvent peak); and 2.4 (s, $CH_3$).

IR (nujol) 1700 $cm^{-1}$.

Using the techniques described in Equations 1—33 and Examples 4, 15 and 26, or simple modifications thereof, the following compounds in Table I can be made by one skilled in the art.

TABLE I

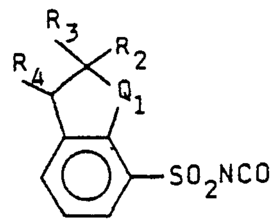

| $R_2$ | $R_3$ | $R_4$ | $Q_1$ | IR($cm^{-1}$) |
|---|---|---|---|---|
| H | H | H | O | |
| $CH_3$ | H | H | O | |
| $CH_3$ | $CH_3$ | H | O | 2200 |
| $C_2H_5$ | H | H | O | |
| $CH_3$ | H | $CH_3$ | O | |
| $C_2H_5$ | H | $CH_3$ | O | |
| H | H | H | S | |
| $CH_3$ | H | H | S | |
| $CH_3$ | $CH_3$ | H | S | 2220 |
| $C_2H_5$ | H | H | S | |
| $CH_3$ | H | $CH_3$ | S | |
| H | H | H | $SO_2$ | |
| $CH_3$ | H | H | $SO_2$ | |
| $CH_3$ | $CH_3$ | H | $SO_2$ | |
| $C_2H_5$ | H | H | $SO_2$ | |
| $CH_3$ | H | $CH_3$ | $SO_2$ | |
| $n$-$C_3H_7$ | H | H | O | |

Using the techniques described in Equations 1—33 and Examples 5, 11, 16, 20 and 27 or simple modifications thereof, the following compounds in Tables II—IXd can be made by one skilled in the art.

TABLE II

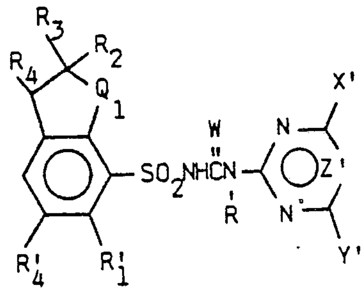

| $R_2$ | $R_3$ | $R_4$ | $R_4'$ | $R_1'$ | R | W | $Q_1$ | X' | Y' | Z' | m.p.(°C) |
|---|---|---|---|---|---|---|---|---|---|---|---|
| $CH_3$ | H | H | H | H | H | O | O | $OCH_3$ | $OCH_3$ | CH | 182—186° |
| $CH_3$ | H | H | H | H | H | O | O | $OCH_3$ | $CH_3$ | CH | 195—198° |
| $CH_3$ | H | H | H | H | H | O | O | $CH_3$ | $CH_3$ | CH | 178—182° |
| $CH_3$ | H | H | H | H | H | O | O | $OCH_3$ | $OCH_3$ | N | |
| $CH_3$ | H | H | H | H | H | O | O | $OCH_3$ | $CH_3$ | N | |
| $CH_3$ | H | H | H | H | H | O | O | $CH_3$ | $CH_3$ | N | |
| $CH_3$ | H | H | H | H | H | O | O | Cl | $OCH_3$ | CH | |
| $CH_3$ | $CH_3$ | H | H | H | H | O | O | $OCH_3$ | $OCH_3$ | CH | 198—200° |
| $CH_3$ | $CH_3$ | H | H | H | H | O | O | $OCH_3$ | $CH_3$ | CH | 170—172° |
| $CH_3$ | $CH_3$ | H | H | H | H | O | O | $CH_3$ | $CH_3$ | CH | 203—206° |
| $CH_3$ | $CH_3$ | H | H | H | H | O | O | $OCH_3$ | $OCH_3$ | N | 164—168° |
| $CH_3$ | $CH_3$ | H | H | H | H | O | O | $OCH_3$ | $CH_3$ | N | 162—165° |
| $CH_3$ | $CH_3$ | H | H | H | H | O | O | $CH_3$ | $CH_3$ | N | 172—176° |
| $CH_3$ | $CH_3$ | H | H | H | H | O | O | Cl | $OCH_3$ | CH | |
| $C_2H_5$ | H | H | H | H | H | O | O | $OCH_3$ | $OCH_3$ | CH | |
| $C_2H_5$ | H | H | H | H | H | O | O | $OCH_3$ | $CH_3$ | CH | |
| $C_2H_5$ | H | H | H | H | H | O | O | $CH_3$ | $CH_3$ | CH | |
| $C_2H_5$ | H | H | H | H | H | O | O | $OCH_3$ | $OCH_3$ | N | |
| $C_2H_5$ | H | H | H | H | H | O | O | $OCH_3$ | $CH_3$ | N | |
| $C_2H_5$ | H | H | H | H | H | O | O | $CH_3$ | $CH_3$ | N | |
| $C_2H_5$ | H | H | H | H | H | O | O | Cl | $OCH_3$ | CH | |
| H | H | H | H | H | H | O | O | $OCH_3$ | $OCH_3$ | CH | |
| H | H | H | H | H | H | O | O | $CH_3$ | $OCH_3$ | CH | |
| H | H | H | H | H | H | O | O | $CH_3$ | $CH_3$ | CH | |
| H | H | H | H | H | H | O | O | $OCH_3$ | $OCH_3$ | N | |

TABLE II (continued)

| $R_2$ | $R_3$ | $R_4$ | $R_4'$ | $R_1'$ | R | W | $Q_1$ | X' | Y' | Z' | m.p.(°C) |
|---|---|---|---|---|---|---|---|---|---|---|---|
| H | H | H | H | H | H | O | O | $OCH_3$ | $CH_3$ | N | |
| H | H | H | H | H | H | O | O | $CH_3$ | $CH_3$ | N | |
| H | H | H | H | H | H | O | O | Cl | $OCH_3$ | CH | |
| $CH_3$ | H | $CH_3$ | H | H | H | O | O | $OCH_3$ | $OCH_3$ | CH | |
| $CH_3$ | H | $CH_3$ | H | H | H | O | O | $OCH_3$ | $CH_3$ | CH | |
| $CH_3$ | H | $CH_3$ | H | H | H | O | O | $CH_3$ | $CH_3$ | CH | |
| $CH_3$ | H | $CH_3$ | H | H | H | O | O | $OCH_3$ | $OCH_3$ | N | |
| $CH_3$ | H | $CH_3$ | H | H | H | O | O | $OCH_3$ | $CH_3$ | N | |
| $CH_3$ | H | $CH_3$ | H | H | H | O | O | $CH_3$ | $CH_3$ | N | |
| $CH_3$ | H | $CH_3$ | H | H | H | O | O | Cl | $OCH_3$ | CH | |
| $n$-$C_3H_7$ | H | H | H | H | H | O | O | $OCH_3$ | $OCH_3$ | CH | |
| $n$-$C_3H_7$ | H | H | H | H | H | O | O | $OCH_3$ | $CH_3$ | CH | |
| $n$-$C_3H_7$ | H | H | H | H | H | O | O | $CH_3$ | $CH_3$ | CH | |
| $n$-$C_3H_7$ | H | H | H | H | H | O | O | $OCH_3$ | $OCH_3$ | N | |
| $n$-$C_3H_7$ | H | H | H | H | H | O | O | $OCH_3$ | $CH_3$ | N | |
| $n$-$C_3H_7$ | H | H | H | H | H | O | O | Cl | $OCH_3$ | CH | |
| $CH_3$ | H | H | H | H | $CH_3$ | O | O | $OCH_3$ | $OCH_3$ | N | |
| $CH_3$ | H | H | H | H | $CH_3$ | O | O | $OCH_3$ | $OCH_3$ | CH | |
| $CH_3$ | $CH_3$ | H | H | H | $CH_3$ | O | O | $OCH_3$ | $OCH_3$ | N | 151—152° |
| $CH_3$ | H | H | H | H | H | O | S | $OCH_3$ | $OCH_3$ | CH | 177—179° |
| $CH_3$ | H | H | H | H | H | O | S | $OCH_3$ | $CH_3$ | CH | 220—223° |
| $CH_3$ | H | H | H | H | H | O | S | $CH_3$ | $CH_3$ | CH | |
| $CH_3$ | H | H | H | H | H | O | S | $OCH_3$ | $OCH_3$ | N | 167—169° |
| $CH_3$ | H | H | H | H | H | O | S | $OCH_3$ | $CH_3$ | N | 145—147° |
| $CH_3$ | H | H | H | H | H | O | S | $CH_3$ | $CH_3$ | N | |
| $CH_3$ | H | H | H | H | H | O | S | Cl | $OCH_3$ | CH | |
| $CH_3$ | $CH_3$ | H | H | H | H | O | S | $OCH_3$ | $OCH_3$ | CH | 190—192° |
| $CH_3$ | $CH_3$ | H | H | H | H | O | S | $OCH_3$ | $CH_3$ | CH | 210—212° |
| $CH_3$ | $CH_3$ | H | H | H | H | O | S | $CH_3$ | $CH_3$ | CH | |
| $CH_3$ | $CH_3$ | H | H | H | H | O | S | $OCH_3$ | $OCH_3$ | N | 146—149° |
| $CH_3$ | $CH_3$ | H | H | H | H | O | S | $OCH_3$ | $CH_3$ | N | 183—185° |

TABLE II (continued)

| $R_2$ | $R_3$ | $R_4$ | $R_4'$ | $R_1'$ | R | W | $Q_1$ | X' | Y' | Z' | m.p.(°C) |
|---|---|---|---|---|---|---|---|---|---|---|---|
| $CH_3$ | $CH_3$ | H | H | H | H | O | S | Cl | $OCH_3$ | CH | |
| $C_2H_5$ | H | H | H | H | H | O | S | $OCH_3$ | $OCH_3$ | CH | |
| $C_2H_5$ | H | H | H | H | H | O | S | $OCH_3$ | $CH_3$ | CH | |
| $C_2H_5$ | H | H | H | H | H | O | S | $CH_3$ | $CH_3$ | CH | |
| $C_2H_5$ | H | H | H | H | H | O | S | $OCH_3$ | $OCH_3$ | N | |
| $C_2H_5$ | H | H | H | H | H | O | S | $OCH_3$ | $CH_3$ | N | |
| $C_2H_5$ | H | H | H | H | H | O | S | Cl | $OCH_3$ | CH | |
| *n*-$C_3H_7$ | H | H | H | H | H | O | S | $OCH_3$ | $OCH_3$ | CH | |
| *n*-$C_3H_7$ | H | H | H | H | H | O | S | $OCH_3$ | $CH_3$ | N | |
| $CH_3$ | H | $CH_3$ | H | H | H | O | S | $OCH_3$ | $OCH_3$ | CH | |
| $CH_3$ | H | $CH_3$ | H | H | H | O | S | $OCH_3$ | $CH_3$ | CH | |
| H | H | H | H | H | H | O | S | $OCH_3$ | $OCH_3$ | CH | |
| H | H | H | H | H | H | O | S | $OCH_3$ | $CH_3$ | CH | |
| H | H | H | H | H | H | O | S | $CH_3$ | $CH_3$ | CH | |
| H | H | H | H | H | H | O | S | $OCH_3$ | $OCH_3$ | N | |
| H | H | H | H | H | H | O | S | $OCH_3$ | $CH_3$ | N | |
| H | H | H | H | H | H | O | S | Cl | $OCH_3$ | CH | |
| $CH_3$ | H | H | H | H | $CH_3$ | O | S | $OCH_3$ | $OCH_3$ | N | |
| $CH_3$ | H | H | H | H | $CH_3$ | O | S | $OCH_3$ | $OCH_3$ | CH | |
| $CH_3$ | $CH_3$ | H | H | H | $CH_3$ | O | S | $OCH_3$ | $OCH_3$ | N | |
| $CH_3$ | H | H | H | H | H | O | $SO_2$ | $OCH_3$ | $OCH_3$ | CH | 234—236° |
| $CH_3$ | H | H | H | H | H | O | $SO_2$ | $CH_3$ | $OCH_3$ | CH | 238—240° |
| $CH_3$ | H | H | H | H | H | O | $SO_2$ | $CH_3$ | $CH_3$ | CH | 214—226° |
| $CH_3$ | H | H | H | H | H | O | $SO_2$ | $OCH_3$ | $OCH_3$ | N | 160—170° |
| $CH_3$ | H | H | H | H | H | O | $SO_2$ | $OCH_3$ | $CH_3$ | N | 214—216° |
| $CH_3$ | H | H | H | H | H | O | $SO_2$ | $CH_3$ | $CH_3$ | N | |
| $CH_3$ | H | H | H | H | H | O | $SO_2$ | Cl | $OCH_3$ | CH | 223—226° |
| $CH_3$ | $CH_3$ | H | H | H | H | O | $SO_2$ | $OCH_3$ | $OCH_3$ | CH | 236—238° |
| $CH_3$ | $CH_3$ | H | H | H | H | O | $SO_2$ | $OCH_3$ | $CH_3$ | CH | 228—229° |

TABLE II (continued)

| $R_2$ | $R_3$ | $R_4$ | $R_4'$ | $R_1'$ | R | W | $Q_1$ | X' | Y' | Z' | m.p.(°C) |
|---|---|---|---|---|---|---|---|---|---|---|---|
| $CH_3$ | $CH_3$ | H | H | H | H | O | $SO_2$ | $CH_3$ | $CH_3$ | CH | 228—230° |
| $CH_3$ | $CH_3$ | H | H | H | H | O | $SO_2$ | $OCH_3$ | $OCH_3$ | N | 214—216° |
| $CH_3$ | $CH_3$ | H | H | H | H | O | $SO_2$ | $CH_3$ | $OCH_3$ | N | 205—207° |
| $CH_3$ | $CH_3$ | H | H | H | H | O | $SO_2$ | $CH_3$ | $CH_3$ | N | 208—211° |
| $CH_3$ | $CH_3$ | H | H | H | H | O | $SO_2$ | Cl | $OCH_3$ | CH | |
| $C_2H_5$ | H | H | H | H | H | O | $SO_2$ | $OCH_3$ | $OCH_3$ | CH | |
| $C_2H_5$ | H | H | H | H | H | O | $SO_2$ | $OCH_3$ | $CH_3$ | CH | |
| $C_2H_5$ | H | H | H | H | H | O | $SO_2$ | $CH_3$ | $CH_3$ | CH | |
| $C_2H_5$ | H | H | H | H | H | O | $SO_2$ | $OCH_3$ | $OCH_3$ | N | |
| $C_2H_5$ | H | H | H | H | H | O | $SO_2$ | $OCH_3$ | $CH_3$ | N | |
| $C_2H_5$ | H | H | H | H | H | O | $SO_2$ | Cl | $OCH_3$ | CH | |
| $n$-$C_3H_7$ | H | H | H | H | H | O | $SO_2$ | $OCH_3$ | $OCH_3$ | CH | |
| $n$-$C_3H_7$ | H | H | H | H | H | O | $SO_2$ | $OCH_3$ | $CH_3$ | N | |
| H | H | H | H | H | H | O | $SO_2$ | $OCH_3$ | $OCH_3$ | CH | >300° |
| H | H | H | H | H | H | O | $SO_2$ | $CH_3$ | $OCH_3$ | CH | 227—229° |
| H | H | H | H | H | H | O | $SO_2$ | $CH_3$ | $CH_3$ | CH | 230—232° |
| H | H | H | H | H | H | O | $SO_2$ | $OCH_3$ | $OCH_3$ | N | 208—210° |
| H | H | H | H | H | H | O | $SO_2$ | $OCH_3$ | $CH_3$ | N | 210—213° |
| H | H | H | H | H | H | O | $SO_2$ | Cl | $OCH_3$ | CH | |
| H | H | $CH_3$ | H | H | H | O | $SO_2$ | $OCH_3$ | $OCH_3$ | CH | 232—235° |
| H | H | $CH_3$ | H | H | H | O | $SO_2$ | $OCH_3$ | $CH_3$ | CH | 221—223° |
| H | H | $CH_3$ | H | H | H | O | $SO_2$ | $CH_3$ | $CH_3$ | CH | 199—203° |
| H | H | $CH_3$ | H | H | H | O | $SO_2$ | $OCH_3$ | $OCH_3$ | N | 200—204° |
| H | H | $CH_3$ | H | H | H | O | $SO_2$ | $OCH_3$ | $CH_3$ | N | 211—213° |
| $CH_3$ | H | $CH_3$ | H | H | H | O | $SO_2$ | $OCH_3$ | $OCH_3$ | CH | |
| $CH_3$ | H | $CH_3$ | H | H | H | O | $SO_2$ | $OCH_3$ | $CH_3$ | CH | |
| $CH_3$ | H | $CH_3$ | H | H | H | O | $SO_2$ | $CH_3$ | $CH_3$ | CH | |
| $CH_3$ | H | $CH_3$ | H | H | H | O | $SO_2$ | $OCH_3$ | $CH_3$ | N | |
| $CH_3$ | H | H | H | H | $CH_3$ | O | $SO_2$ | $OCH_3$ | $OCH_3$ | CH | 208—211° |
| $CH_3$ | H | H | H | H | $CH_3$ | O | $SO_2$ | $OCH_3$ | $OCH_3$ | N | 160—163° |
| $CH_3$ | $CH_3$ | H | H | H | $CH_3$ | O | $SO_2$ | $OCH_3$ | $OCH_3$ | N | |

TABLE II (continued)

| $R_2$ | $R_3$ | $R_4$ | $R_4'$ | $R_1'$ | R | W | $Q_1$ | X' | Y' | Z' | m.p. (°C) |
|---|---|---|---|---|---|---|---|---|---|---|---|
| $CH_3$ | H | H | H | H | H | O | SO | $OCH_3$ | $OCH_3$ | CH | |
| $CH_3$ | H | H | H | H | H | O | SO | $OCH_3$ | $CH_3$ | N | |
| $CH_3$ | $CH_3$ | H | Br | H | H | O | O | $CH_3$ | $OCH_3$ | N | |
| $CH_3$ | H | H | Cl | H | H | O | O | $OCH_3$ | $OCH_3$ | CH | |
| $CH_3$ | H | H | $CH_3$ | H | H | O | O | $OCH_3$ | $OCH_3$ | CH | |
| $CH_3$ | H | H | $CF_3$ | H | H | O | O | $OCH_3$ | $OCH_3$ | CH | |
| $CH_3$ | H | H | $OCH_3$ | H | H | O | O | $OCH_3$ | $CH_3$ | CH | |
| $CH_3$ | H | H | $CH_3$ | H | H | O | S | $OCH_3$ | $OCH_3$ | CH | |
| $CH_3$ | H | H | $CH_3$ | H | H | O | $SO_2$ | $OCH_3$ | $OCH_3$ | CH | |
| $CH_3$ | H | H | H | H | H | S | O | $OCH_3$ | $OCH_3$ | CH | |
| $CH_3$ | $CH_3$ | H | H | H | H | S | O | $OCH_3$ | $CH_3$ | N | |
| $CH_3$ | H | H | H | H | H | S | S | $OCH_3$ | $OCH_3$ | CH | |
| $CH_3$ | H | H | H | H | H | S | S | $CH_3$ | $OCH_3$ | N | |
| $CH_3$ | H | H | H | H | H | S | $SO_2$ | $CH_3$ | $OCH_3$ | N | |
| $CH_3$ | H | H | H | H | H | S | $SO_2$ | $OCH_3$ | $OCH_3$ | CH | |
| $CH_3$ | H | H | H | $CH_3$ | H | O | $SO_2$ | $OCH_3$ | $OCH_3$ | CH | |
| $CH_3$ | H | H | H | $OCH_3$ | H | O | O | $OCH_3$ | $OCH_3$ | CH | |
| $CH_3$ | $CH_3$ | H | H | Cl | H | O | S | $OCH_3$ | $CH_3$ | CH | |
| $CH_3$ | H | H | H | Br | H | O | O | $OCH_3$ | $CH_3$ | N | |
| $CH_3$ | H | H | H | $NO_2$ | H | O | O | $OCH_3$ | $OCH_3$ | CH | |
| $CH_3$ | H | H | H | $CO_2CH_3$ | H | O | O | $CH_3$ | $OCH_3$ | N | |
| $CH_3$ | H | H | H | $CO_2C_2H_5$ | H | O | $SO_2$ | $OCH_3$ | $OCH_3$ | CH | |
| $CH_3$ | H | H | H | $CO_2CH_2CH_2CH_3$ | H | O | O | $OCH_3$ | $OCH_3$ | CH | |
| $CH_3$ | $CH_3$ | H | H | $CO_2CH(CH_3)_2$ | H | O | S | $OCH_3$ | $OCH_3$ | CH | |
| $CH_3$ | H | H | H | $CO_2CH_2CH{=}CH_2$ | H | O | O | $OCH_3$ | $OCH_3$ | CH | |
| $CH_3$ | H | H | H | $CO_2CH_2CH_2OCH_3$ | H | O | O | $OCH_3$ | $OCH_3$ | CH | |
| $CH_3$ | H | H | H | $CO_2CH_2CH_2Cl$ | H | O | O | $OCH_3$ | $OCH_3$ | CH | |
| $CH_3$ | $CH_3$ | H | H | $SO_2CH_3$ | H | O | O | $CH_3$ | $OCH_3$ | N | |
| $CH_3$ | $CH_3$ | H | H | $SO_2C_2H_5$ | H | O | O | $OCH_3$ | $CH_3$ | CH | |
| $CH_3$ | H | H | H | $SO_2CH_2CH_2CH_3$ | H | O | O | $OCH_3$ | $OCH_3$ | CH | |
| $CH_3$ | H | H | H | $SO_2CH(CH_3)_2$ | H | O | O | $OCH_3$ | $OCH_3$ | CH | |

TABLE II (continued)

| $R_2$ | $R_3$ | $R_4$ | $R_4'$ | $R_1'$ | R | W | $Q_1$ | X' | Y' | Z' | m.p.(°C) |
|---|---|---|---|---|---|---|---|---|---|---|---|
| $CH_3$ | H | H | H | $OSO_2CH_3$ | H | O | $SO_2$ | $OCH_3$ | $CH_3$ | CH | |
| $CH_3$ | H | H | H | $OSO_2C_2H_5$ | H | O | O | $OCH_3$ | $OCH_3$ | CH | |
| $CH_3$ | H | H | H | $OSO_2CH_2CH_2CH_3$ | H | O | O | $OCH_3$ | $OCH_3$ | CH | |
| $CH_3$ | H | H | H | $OSO_2CH(CH_3)_2$ | H | O | S | $CH_3$ | $OCH_3$ | CH | |
| $CH_3$ | $CH_3$ | H | H | $OSO_2CF_3$ | H | O | O | $CH_3$ | $OCH_3$ | N | |
| $CH_3$ | H | H | H | $SO_2N(CH_3)_2$ | H | O | O | $CH_3$ | $CH_3$ | CH | |
| $CH_3$ | H | H | H | $SO_2N(CH_3)C_2H_5$ | H | O | O | $OCH_3$ | $OCH_3$ | CH | |
| $CH_3$ | H | H | H | H | H | O | O | $CH_3$ | $CH_3$ | CCl | |
| $CH_3$ | H | H | H | H | H | O | S | H | $CH_3$ | CCl | |
| $CH_3$ | H | H | H | H | H | O | O | H | $CH_3$ | CBr | |
| $CH_3$ | $CH_3$ | H | H | H | H | O | S | H | $OCH_3$ | CCl | |
| $CH_3$ | $CH_3$ | H | H | H | H | O | O | H | $OCH_3$ | CBr | |
| $CH_3$ | $CH_3$ | H | H | H | H | O | $SO_2$ | $CH_3$ | $CH_3$ | CBr | |
| $CH_3$ | H | H | H | H | H | O | O | H | $CH_3$ | $CCH_3$ | |
| $CH_3$ | H | H | H | H | H | O | O | $OCH_3$ | $CH_3$ | $CC_2H_5$ | |
| $CH_3$ | $CH_3$ | H | H | H | H | O | S | $OCH_3$ | $OC_2H_5$ | N | |
| $CH_3$ | H | H | H | H | H | O | O | $OCH_3$ | $OC_2H_5$ | CH | |
| $CH_3$ | H | H | H | H | H | O | $SO_2$ | $OCH_3$ | $CH_2OCH_3$ | N | |
| $CH_3$ | H | H | H | H | H | O | O | $CH_3$ | $CH_2OCH_3$ | CH | |
| $CH_3$ | $CH_3$ | H | H | H | H | O | O | $OCH_3$ | $OCH_2CF_3$ | N | |
| $CH_3$ | $CH_3$ | H | H | H | H | O | S | $CH_3$ | $OCH_2CF_3$ | CH | |

TABLE II (continued)

| $R_2$ | $R_3$ | $R_4$ | $R_4'$ | $R_1'$ | R | W | $Q_1$ | X' | Y' | Z' | m.p.(°C) |
|---|---|---|---|---|---|---|---|---|---|---|---|
| $CH_3$ | H | H | H | H | H | O | O | $OCH_3$ | $N(CH_3)_2$ | N | |
| $CH_3$ | H | H | H | H | H | O | O | $OCH_3$ | $N(CH_3)_2$ | CH | |
| $CH_3$ | H | H | H | H | H | O | O | $OCH_3$ | $NHCH_3$ | N | |
| $CH_3$ | H | H | H | H | H | O | O | $CH_3$ | $C_2H_5$ | CH | |
| $CH_3$ | H | H | H | H | H | O | O | $CH_3$ | $OC_2H_5$ | N | |
| $CH_3$ | H | H | H | H | H | O | $SO_2$ | $CH_3$ | $OC_2H_5$ | CH | |
| $CH_3$ | $CH_3$ | H | H | H | H | O | O | $CH_3$ | $OCH_2CF_3$ | N | |
| $CH_3$ | $CH_3$ | H | H | H | H | O | O | $CH_3$ | $OCH_2CH{=}CH_2$ | N | |
| $CH_3$ | H | H | H | H | H | O | O | $CH_3$ | $OCH_2C{\equiv}CH$ | N | |
| $CH_3$ | H | H | H | H | H | O | O | Cl | $NH_2$ | CH | |
| $CH_3$ | H | H | H | H | H | O | O | Cl | $NHCH_3$ | CH | |
| $CH_3$ | H | H | H | H | H | O | O | Cl | $N(CH_3)_2$ | CH | |
| $CH_3$ | $CH_3$ | H | H | H | H | O | S | $OCH_3$ | $CH(OCH_3)_2$ | CH | |
| $CH_3$ | H | H | H | H | H | O | O | $CH_3$ | $CH(OCH_3)_2$ | N | |
| $CH_3$ | H | H | H | H | H | O | O | $CH_3$ | $CH(OCH_3)_2$ | CH | |
| $CH_3$ | H | H | H | H | H | O | O | $OCH_3$ | $NH_2$ | CH | |
| $CH_3$ | H | H | H | H | H | O | $SO_2$ | $CH_3$ | $CF_3$ | CH | |
| $CH_3$ | H | H | H | H | H | O | O | $OCH_3$ | $NHCH_3$ | CH | |
| $CH_3$ | H | H | H | H | H | O | O | $CH_3$ | 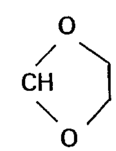 | CH | |

TABLE II (continued)

| $R_2$ | $R_3$ | $R_4$ | $R_4'$ | $R_1'$ | R | W | $Q_1$ | X′ | Y′ | Z′ | m.p.(°C) |
|---|---|---|---|---|---|---|---|---|---|---|---|
| $CH_3$ | H | H | H | H | H | O | $SO_2$ | $OCH_3$ | $CH(OCH_3)_2$ | CH | 111—113° |
| $CH(CH_3)_2$ | H | H | H | H | H | O | O | $OCH_3$ | $OCH_3$ | CH | |
| H | H | $CH_3$ | H | H | H | O | $SO_2$ | $CH_3$ | $CH_3$ | N | 203—206° |
| H | H | H | H | H | H | O | $SO_2$ | $CH_3$ | $CH_3$ | N | |
| $CH_3$ | $CH_3$ | H | H | H | H | O | S | $CH_3$ | $CH_3$ | N | |
| $CH_3$ | H | H | H | H | H | O | O | $OCH_3$ | $CH(OCH_3)_2$ | CH | |
| $CH_3$ | H | H | H | H | H | O | O | $OCH_3$ | 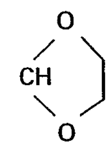 | CH | |
| $CH_3$ | $CH_3$ | H | H | H | H | O | O | $OCH_3$ | 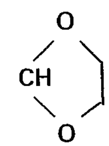 | CH | |
| $CH_3$ | $CH_3$ | H | H | H | H | O | O | $CH_3$ | 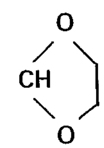 | CH | |
| $CH_3$ | H | H | H | H | H | O | O | $OCH_3$ | $SCH_3$ | N | |
| $CH_3$ | $CH_3$ | H | H | H | H | O | O | $OCH_3$ | $SCH_3$ | N | |
| $CH_3$ | H | H | H | H | H | O | O | $OCH_3$ | $SCH_3$ | CH | |
| $CH_3$ | $CH_3$ | H | H | H | H | O | $SO_2$ | $OCH_3$ | $SCH_3$ | N | |
| $CH_3$ | $CH_3$ | H | H | H | H | O | O | $OCH_3$ | $SCH_3$ | CH | |
| $CH_3$ | H | H | H | H | H | O | O | $CH_3$ | $SCH_3$ | CH | |

TABLE II (continued)

| $R_2$ | $R_3$ | $R_4$ | $R_4'$ | $R_1'$ | R | W | $Q_1$ | X' | Y' | Z' | m.p.(°C) |
|---|---|---|---|---|---|---|---|---|---|---|---|
| $CH_3$ | H | H | H | H | $CH_3$ | O | $SO_2$ | $CH_3$ | $OCH_3$ | CH | 220—222° |
| $CH_3$ | H | H | H | H | $CH_3$ | O | $SO_2$ | $CH_3$ | $OCH_3$ | N | 175—180° |
| $CH_3$ | H | H | H | H | H | O | $SO_2$ | $CH_3$ | $CH_2OCH_3$ | CH | 203—206° |
| $CH_3$ | H | H | H | H | H | O | $SO_2$ | $CH_3$ | $OC_2H_5$ | N | 178—180° |
| $CH_3$ | H | H | H | H | H | O | $SO_2$ | $CH_3$ | $OCH_2CF_3$ | N | 168—173° |
| $CH_3$ | H | H | H | H | H | O | $SO_2$ | $OCH_3$ | $N(CH_3)_2$ | N | 180—183° |

TABLE IIa

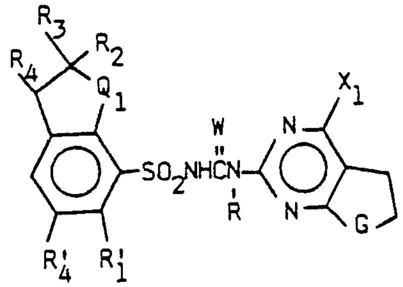

| $R_2$ | $R_3$ | $R_4$ | $R_4'$ | $R_1'$ | R | W | $Q_1$ | G | $X_1$ | m.p. (°C) |
|---|---|---|---|---|---|---|---|---|---|---|
| $CH_3$ | $CH_3$ | H | H | H | H | O | O | O | $CH_3$ | 207—108° |
| $CH_3$ | $CH_3$ | H | H | H | H | O | O | O | $OCH_3$ | |
| $CH_3$ | $CH_3$ | H | H | H | H | O | O | O | $OC_2H_5$ | |
| $CH_3$ | H | H | H | H | H | O | O | O | $CH_3$ | |
| $CH_3$ | H | H | H | H | H | O | O | O | $OCH_3$ | |
| $C_2H_5$ | H | H | H | H | H | O | O | O | $CH_3$ | |
| $C_2H_5$ | H | H | H | H | H | O | O | O | $OCH_3$ | |
| $n$-$C_3H_7$ | H | H | H | H | H | O | O | O | $CH_3$ | |
| $n$-$C_3H_7$ | H | H | H | H | H | O | O | O | $OCH_3$ | |
| H | H | H | H | H | H | O | O | O | $CH_3$ | |
| H | H | H | H | H | H | O | O | O | $OCH_3$ | |
| $CH_3$ | H | $CH_3$ | H | H | H | O | O | O | $CH_3$ | |
| $CH_3$ | H | $CH_3$ | H | H | H | O | O | O | $OCH_3$ | |
| $CH_3$ | H | H | H | H | H | O | O | $CH_2$ | $OCH_3$ | |
| $CH_3$ | H | H | H | H | H | O | O | $CH_2$ | $CH_3$ | |
| $CH_3$ | $CH_3$ | H | H | H | H | O | O | $CH_3$ | $OC_2H_5$ | |
| $CH_3$ | $CH_3$ | H | H | H | H | O | O | $CH_2$ | $OCH_3$ | |
| $CH_3$ | $CH_3$ | H | H | H | H | O | O | $CH_2$ | $CH_3$ | |
| $CH_3$ | H | H | H | H | $CH_3$ | O | O | O | $OCH_3$ | |
| $CH_3$ | H | H | H | H | H | O | S | O | $CH_3$ | |
| $CH_3$ | $CH_3$ | H | H | H | H | O | S | O | $CH_3$ | |
| $CH_3$ | H | H | H | H | H | O | S | $CH_2$ | $CH_3$ | |
| $CH_3$ | $CH_3$ | H | H | H | H | O | S | $CH_2$ | $OCH_3$ | |

TABLE IIa (continued)

| $R_2$ | $R_3$ | $R_4$ | $R_4'$ | $R_1'$ | R | W | $Q_1$ | G | $X_1$ | m.p. (°C) |
|---|---|---|---|---|---|---|---|---|---|---|
| $CH_3$ | H | H | H | H | H | O | $SO_2$ | O | $CH_3$ | 208—210° |
| $CH_3$ | $CH_3$ | H | H | H | H | O | $SO_2$ | O | $CH_3$ | |
| $CH_3$ | H | H | H | H | H | O | $SO_2$ | O | $OCH_3$ | |
| $CH_3$ | $CH_3$ | H | H | H | H | O | $SO_2$ | O | $OCH_3$ | |
| $CH_3$ | H | H | H | H | H | O | S | O | $OCH_3$ | |
| $CH_3$ | $CH_3$ | H | H | H | H | O | S | O | $OCH_3$ | |
| $CH_3$ | H | H | H | H | H | O | $SO_2$ | $CH_2$ | $CH_3$ | |
| $CH_3$ | $CH_3$ | H | H | H | H | O | $SO_2$ | $CH_2$ | $OCH_3$ | |
| $C_2H_5$ | H | H | H | H | H | O | $SO_2$ | O | $CH_3$ | |
| $CH_3$ | $CH_3$ | H | H | H | H | S | O | O | $CH_3$ | |
| $CH_3$ | H | H | H | H | H | S | O | O | $CH_3$ | |
| $CH_3$ | $CH_3$ | H | H | H | H | S | $SO_2$ | O | $CH_3$ | |
| $CH_3$ | H | H | H | H | H | S | S | O | $CH_3$ | |
| H | H | H | H | H | H | O | $SO_2$ | O | $CH_3$ | |
| H | H | H | H | H | H | O | $SO_2$ | O | $OCH_3$ | |
| H | H | H | H | H | H | O | $SO_2$ | $CH_2$ | $OCH_3$ | |

TABLE IIb

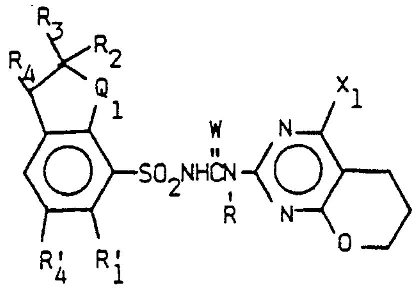

| $R_2$ | $R_3$ | $R_4$ | $R_4'$ | $R_1'$ | R | W | $Q_1$ | $X_1$ | m.p. (°C) |
|---|---|---|---|---|---|---|---|---|---|
| $CH_3$ | $CH_3$ | H | H | H | H | O | O | $CH_3$ | |
| $CH_3$ | H | H | H | H | H | O | O | $CH_3$ | |
| $CH_3$ | $CH_3$ | H | H | H | H | O | O | $OCH_3$ | |
| $CH_3$ | H | H | H | H | H | O | O | $OCH_3$ | |
| $CH_3$ | H | H | H | H | H | O | S | $OCH_3$ | |
| $CH_3$ | H | H | H | H | H | O | $SO_2$ | $OCH_3$ | |
| $CH_3$ | $CH_3$ | H | H | H | H | O | O | $OC_2H_5$ | |
| $CH_3$ | H | H | H | H | H | O | O | $OC_2H_5$ | |
| $CH_3$ | $CH_3$ | H | H | H | H | O | S | $OCH_3$ | |
| $CH_3$ | $CH_3$ | H | H | H | H | O | $SO_2$ | $OCH_3$ | |
| $CH_3$ | H | H | H | H | H | O | S | $CH_3$ | |
| $CH_3$ | $CH_3$ | H | H | H | H | O | S | $CH_3$ | |
| $CH_3$ | H | H | H | H | H | O | $SO_2$ | $CH_3$ | |
| $CH_3$ | $CH_3$ | H | H | H | H | O | $SO_2$ | $CH_3$ | |
| H | H | H | H | H | H | O | $SO_2$ | $OCH_3$ | |
| H | H | H | H | H | H | O | $SO_2$ | $CH_3$ | |
| H | H | H | H | H | H | O | O | $CH_3$ | |
| H | H | H | H | H | H | O | O | $OCH_3$ | |
| $CH_3$ | H | $CH_3$ | H | H | H | O | O | $CH_3$ | |

TABLE IIc

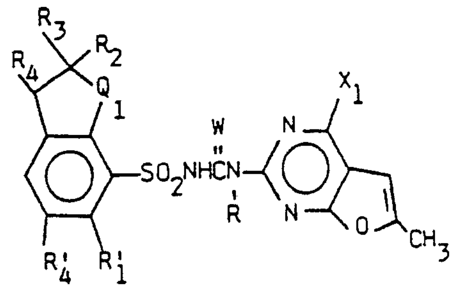

| $R_2$ | $R_3$ | $R_4$ | $R_4'$ | $R_1'$ | R | W | $Q_1$ | $X_1$ | m.p. (°C) |
|---|---|---|---|---|---|---|---|---|---|
| $CH_3$ | $CH_3$ | H | H | H | H | O | O | $CH_3$ | |
| $CH_3$ | H | H | H | H | H | O | O | $CH_3$ | |
| $CH_3$ | H | $CH_3$ | H | H | H | O | O | $CH_3$ | |
| $CH_3$ | H | H | H | H | H | O | $SO_2$ | $CH_3$ | 219—221° |
| H | H | H | H | H | H | O | $SO_2$ | $CH_3$ | |
| $CH_3$ | $CH_3$ | H | H | Cl | H | O | O | $CH_3$ | |
| $CH_3$ | $CH_3$ | H | H | H | H | O | O | $OCH_3$ | |
| $CH_3$ | H | H | H | H | H | O | O | $OCH_3$ | |
| $CH_3$ | H | H | H | H | H | O | $SO_2$ | $OCH_3$ | |
| $CH_3$ | $CH_3$ | H | H | H | H | O | $SO_2$ | $OCH_3$ | |
| $CH_3$ | $CH_3$ | H | H | H | H | O | O | $OC_2H_5$ | |
| $CH_3$ | H | H | H | H | H | O | O | $OC_2H_5$ | |
| $CH_3$ | $CH_3$ | H | H | H | H | O | $SO_2$ | $CH_3$ | |
| H | H | H | H | H | H | O | $SO_2$ | $OCH_3$ | |
| $CH_3$ | H | H | H | H | H | O | S | $CH_3$ | |
| $CH_3$ | $CH_3$ | H | H | H | H | O | S | $OCH_3$ | |
| $CH_3$ | H | $CH_3$ | H | H | H | O | O | $OCH_3$ | |
| $CH_3$ | H | H | H | H | H | S | O | $CH_3$ | |

TABLE IId

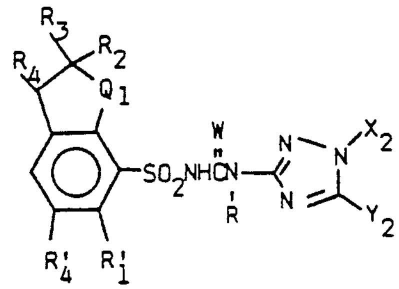

| $R_2$ | $R_3$ | $R_4$ | $R_4'$ | $R_1'$ | R | W | $Q_1$ | $X_2$ | $Y_2$ | m.p. (°C) |
|---|---|---|---|---|---|---|---|---|---|---|
| $CH_3$ | $CH_3$ | H | H | H | H | O | O | $CH_3$ | $OCH_3$ | |
| $CH_3$ | H | H | H | H | H | O | O | $CH_3$ | $OCH_3$ | |
| $CH_3$ | H | $CH_3$ | H | H | H | O | O | $CH_3$ | $OCH_3$ | |
| H | H | H | H | H | H | O | O | $CH_3$ | $OCH_3$ | |
| $C_2H_5$ | H | H | H | H | H | O | O | $CH_3$ | $OCH_3$ | |
| $n$-$C_3H_7$ | H | H | H | H | H | O | O | $CH_3$ | $OCH_3$ | |
| $CH_3$ | $CH_3$ | H | H | H | H | O | O | $CH_3$ | $SCH_3$ | |
| $CH_3$ | H | H | H | H | H | O | O | $CH_3$ | $SCH_3$ | |
| $CH_3$ | H | $CH_3$ | H | H | H | O | O | $CH_3$ | $SCH_3$ | |
| H | H | H | H | H | H | O | O | $CH_3$ | $SCH_3$ | |
| $C_2H_5$ | H | H | H | H | H | O | O | $CH_3$ | $SCH_3$ | |
| $n$-$C_3H_7$ | H | H | H | H | H | O | O | $CH_3$ | $SCH_3$ | |
| $CH_3$ | H | H | H | H | H | O | O | $CH_3$ | $OC_2H_5$ | |
| $CH_3$ | $CH_3$ | H | H | H | H | O | O | $CH_3$ | $OC_2H_5$ | |
| $CH_3$ | H | H | H | H | H | O | O | $CH_3$ | $SC_2H_5$ | |
| $CH_3$ | $CH_3$ | H | H | H | H | O | O | $CH_3$ | $SC_2H_5$ | |
| $CH_3$ | H | H | H | H | H | O | O | $CH_3$ | $C_2H_5$ | |
| $CH_3$ | $CH_3$ | H | H | H | H | O | O | $CH_3$ | $C_2H_5$ | |
| $CH_3$ | H | H | H | H | H | O | O | $CH_3$ | $CH_3$ | |
| $CH_3$ | $CH_3$ | H | H | H | H | O | O | $CH_3$ | $CH_3$ | |
| $CH_3$ | H | H | H | H | H | O | O | $C_2H_5$ | $OCH_3$ | |
| $CH_3$ | $CH_3$ | H | H | H | H | O | O | $C_2H_5$ | $SCH_3$ | |

TABLE IId (continued)

| $R_2$ | $R_3$ | $R_4$ | $R_4'$ | $R_1'$ | R | W | $Q_1$ | $X_2$ | $Y_2$ | m.p. (°C) |
|---|---|---|---|---|---|---|---|---|---|---|
| $CH_3$ | H | H | H | H | H | O | O | $CH_2CF_3$ | $OCH_3$ | |
| $CH_3$ | H | H | H | H | H | O | O | $CH_2CF_3$ | $SCH_3$ | |
| $CH_3$ | H | H | H | H | H | S | O | $CH_3$ | $OCH_3$ | |
| $CH_3$ | $CH_3$ | H | H | H | H | S | O | $CH_3$ | $OCH_3$ | |
| $CH_3$ | $CH_3$ | H | H | H | H | O | $SO_2$ | $CH_3$ | $OCH_3$ | |
| $CH_3$ | H | H | H | H | H | O | $SO_2$ | $CH_3$ | $OCH_3$ | 204—206° |
| $CH_3$ | H | $CH_3$ | H | H | H | O | $SO_2$ | $CH_3$ | $OCH_3$ | |
| H | H | H | H | H | H | O | $SO_2$ | $CH_3$ | $OCH_3$ | |
| $C_2H_5$ | H | H | H | H | H | O | $SO_2$ | $CH_3$ | $OCH_3$ | |
| *n*-$C_3H_7$ | H | H | H | H | H | O | $SO_2$ | $CH_3$ | $OCH_3$ | |
| $CH_3$ | $CH_3$ | H | H | H | H | O | $SO_2$ | $CH_3$ | $SCH_3$ | |
| $CH_3$ | H | H | H | H | H | O | $SO_2$ | $CH_3$ | $SCH_3$ | |
| $CH_3$ | H | $CH_3$ | H | H | H | O | $SO_2$ | $CH_3$ | $SCH_3$ | |
| H | H | H | H | H | H | O | $SO_2$ | $CH_3$ | $SCH_3$ | |
| $C_2H_5$ | H | H | H | H | H | O | $SO_2$ | $CH_3$ | $SCH_3$ | |
| *n*-$C_3H_7$ | H | H | H | H | H | O | $SO_2$ | $CH_3$ | $SCH_3$ | |
| $CH_3$ | $CH_3$ | H | H | H | H | O | S | $CH_3$ | $OCH_3$ | |
| $CH_3$ | H | H | H | H | H | O | S | $CH_3$ | $OCH_3$ | |
| H | H | H | H | H | H | O | S | $CH_3$ | $OCH_3$ | |
| $C_2H_5$ | H | H | H | H | H | O | S | $CH_3$ | $OCH_3$ | |
| $CH_3$ | $CH_3$ | H | H | H | H | O | S | $CH_3$ | $SCH_3$ | |
| $CH_3$ | H | H | H | H | H | O | S | $CH_3$ | $SCH_3$ | |
| H | H | H | H | H | H | O | S | $CH_3$ | $SCH_3$ | |
| $C_2H_5$ | H | H | H | H | H | O | S | $CH_3$ | $SCH_3$ | |
| $CH_3$ | H | $CH_3$ | H | H | H | O | S | $CH_3$ | $OCH_3$ | |
| $CH_3$ | H | H | H | H | H | O | $SO_2$ | $C_2H_5$ | $OCH_3$ | |
| $CH_3$ | $CH_3$ | H | H | H | H | O | S | $C_2H_5$ | $SCH_3$ | |
| $CH_3$ | $CH_3$ | H | H | H | H | O | $SO_2$ | $CH_2CF_3$ | $OCH_3$ | |
| $CH_3$ | H | H | H | H | H | O | S | $CH_2CF_3$ | $SCH_3$ | |
| $CH_3$ | $CH_3$ | H | H | H | H | O | $SO_2$ | $CH_3$ | $OC_2H_5$ | |
| $CH_3$ | $CH_3$ | H | H | H | H | O | S | $CH_3$ | $SC_2H_5$ | |

TABLE IId (continued)

| $R_2$ | $R_3$ | $R_4$ | $R_4'$ | $R_1'$ | R | W | $Q_1$ | $X_2$ | $Y_2$ | m.p. (°C) |
|---|---|---|---|---|---|---|---|---|---|---|
| $CH_3$ | H | H | H | H | H | O | $SO_2$ | $CH_3$ | $C_2H_5$ | |
| $CH_3$ | H | H | H | H | H | O | S | $CH_3$ | $CH_3$ | |
| $CH_3$ | $CH_3$ | H | H | H | H | S | S | $CH_3$ | $OCH_3$ | |
| $CH_3$ | $CH_3$ | H | H | H | $CH_3$ | O | O | $CH_3$ | $OCH_3$ | |
| $CH_3$ | H | H | H | H | H | O | SO | $CH_3$ | $OCH_3$ | |
| $CH_3$ | H | H | $CH_3$ | H | H | O | O | $CH_3$ | $OCH_3$ | |
| $CH_3$ | H | H | H | Cl | H | O | O | $CH_3$ | $OCH_3$ | |

TABLE III

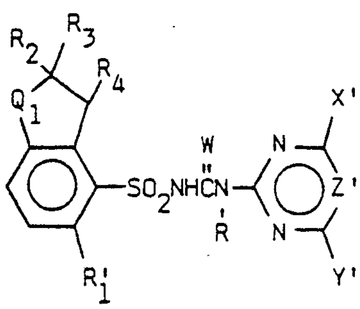

| $R_2$ | $R_3$ | $R_4$ | $R_1'$ | R | W | $Q_1$ | X' | Y' | Z | m.p. (°C) |
|---|---|---|---|---|---|---|---|---|---|---|
| H | H | H | H | H | O | O | $OCH_3$ | $OCH_3$ | CH | 177—180° |
| H | H | H | H | H | O | O | $OCH_3$ | $CH_3$ | N | |
| H | H | H | H | H | O | S | $OCH_3$ | $OCH_3$ | CH | |
| H | H | H | H | H | O | S | $OCH_3$ | $CH_3$ | N | |
| H | H | H | H | H | O | $SO_2$ | $OCH_3$ | $OCH_3$ | CH | |
| H | H | H | H | H | O | $SO_2$ | $OCH_3$ | $CH_3$ | N | |
| $CH_3$ | H | $CH_3$ | H | H | O | $SO_2$ | $OCH_3$ | $OCH_3$ | CH | |
| $CH_3$ | H | $CH_3$ | H | H | O | $SO_2$ | $OCH_3$ | $CH_3$ | CH | |
| H | H | H | H | H | O | O | $OCH_3$ | $OCH_3$ | CH | |
| H | H | H | H | $CH_3$ | O | O | $OCH_3$ | $OCH_3$ | CH | |
| H | H | H | H | H | O | SO | $OCH_3$ | $OCH_3$ | CH | |
| H | H | H | H | H | O | O | Cl | $OCH_3$ | CH | |
| H | H | H | H | H | O | O | $OCH_3$ | $CH_2OCH_3$ | CH | |
| H | H | H | H | H | O | O | $OCH_3$ | $N(CH_3)_2$ | CH | |

TABLE IIIa

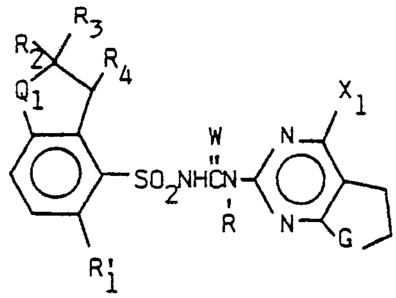

| $R_2$ | $R_3$ | $R_4$ | $R_1'$ | R | W | $Q_1$ | G | $X_1$ | m.p. (°C) |
|---|---|---|---|---|---|---|---|---|---|
| H | H | H | H | H | O | O | O | $CH_3$ | |
| H | H | H | H | H | O | O | O | $OCH_3$ | |
| H | H | H | H | H | O | O | O | $OC_2H_5$ | |
| H | H | H | H | H | O | O | $CH_2$ | $CH_3$ | |
| H | H | H | H | H | O | O | $CH_2$ | $OCH_3$ | |
| H | H | H | H | H | O | O | $CH_2$ | $OC_2H_5$ | |
| H | H | H | H | H | O | S | O | $CH_3$ | |
| H | H | H | H | H | O | $SO_2$ | O | $CH_3$ | |
| H | H | H | H | H | O | S | O | $OCH_3$ | |
| H | H | H | H | H | O | $SO_2$ | O | $OCH_3$ | |
| H | H | H | H | H | O | S | $CH_2$ | $OCH_3$ | |
| H | H | H | H | H | O | $SO_2$ | $CH_2$ | $OCH_3$ | |
| $CH_3$ | H | $CH_3$ | H | H | O | $SO_2$ | O | $OCH_3$ | |
| $CH_3$ | H | $CH_3$ | H | H | O | $SO_2$ | O | $CH_3$ | |

TABLE IIIb

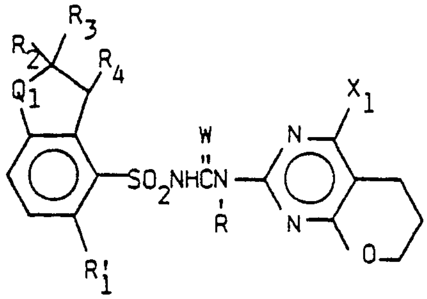

| $R_2$ | $R_3$ | $R_4$ | $R_1'$ | R | $Q_1$ | $X_1$ | W | m.p. (°C) |
|---|---|---|---|---|---|---|---|---|
| H | H | H | H | H | O | $CH_3$ | O | |
| H | H | H | H | H | $SO_2$ | $CH_3$ | O | |
| H | H | H | H | H | O | $OCH_3$ | O | |
| $CH_3$ | H | $CH_3$ | H | H | S | $OCH_3$ | O | |
| H | H | $CH_3$ | H | H | $SO_2$ | $OCH_3$ | O | |
| $CH_3$ | H | $CH_3$ | Cl | H | O | $OCH_3$ | O | |
| H | H | H | H | H | O | $OC_2H_5$ | O | |
| H | H | H | H | H | S | $CH_3$ | O | |
| H | H | H | H | H | SO | $CH_3$ | O | |
| $CH_3$ | H | $CH_3$ | H | H | O | $CH_3$ | O | |
| H | H | H | H | H | O | $CH_3$ | S | |

TABLE IIIc

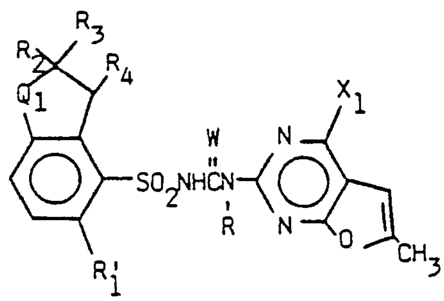

| $R_2$ | $R_3$ | $R_4$ | $R_1'$ | R | $Q_1$ | $X_1$ | W | m.p. (°C) |
|---|---|---|---|---|---|---|---|---|
| $CH_3$ | H | $CH_3$ | H | H | O | $CH_3$ | H | |
| H | H | $CH_3$ | H | H | $SO_2$ | $CH_3$ | H | |
| H | H | $CH_3$ | $CH_3$ | H | $SO_2$ | $CH_3$ | H | |
| H | H | $CH_3$ | Cl | H | $SO_2$ | $CH_3$ | H | |
| H | H | H | H | H | O | $OCH_3$ | H | |
| H | H | H | H | H | O | $OC_2H_5$ | H | |
| H | H | H | H | H | O | $CH_3$ | O | |
| H | H | H | H | H | S | $CH_3$ | O | |
| H | H | H | H | H | $SO_2$ | $CH_3$ | O | |
| H | H | H | H | H | SO | $CH_3$ | O | |
| H | H | H | H | H | O | $CH_3$ | S | |
| $CH_3$ | H | $CH_3$ | H | H | O | $OCH_3$ | O | |

TABLE IIId

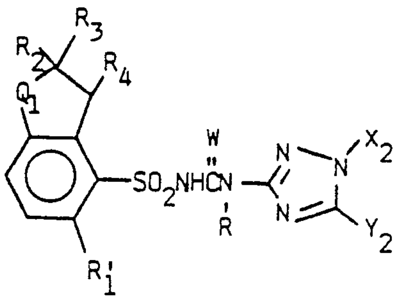

| $R_2$ | $R_3$ | $R_4$ | $R_1'$ | R | W | $Q_1$ | $X_2$ | $Y_2$ | m.p. (°C) |
|---|---|---|---|---|---|---|---|---|---|
| H | H | H | H | H | O | O | $CH_3$ | $OCH_3$ | |
| H | H | H | H | H | O | O | $CH_3$ | $SCH_3$ | |
| H | H | H | H | H | O | S | $CH_3$ | $OCH_3$ | |
| H | H | H | H | H | O | $SO_2$ | $CH_3$ | $OCH_3$ | |
| H | H | H | H | H | O | SO | $CH_3$ | $OCH_3$ | |
| H | H | H | H | H | S | O | $CH_3$ | $OCH_3$ | |
| $CH_3$ | H | $CH_3$ | H | H | O | O | $CH_3$ | $OCH_3$ | |
| $CH_3$ | H | $CH_3$ | H | H | O | O | $CH_3$ | $SCH_3$ | |
| H | H | $CH_3$ | Cl | H | O | $SO_2$ | $CH_3$ | $OCH_3$ | |

TABLE IV

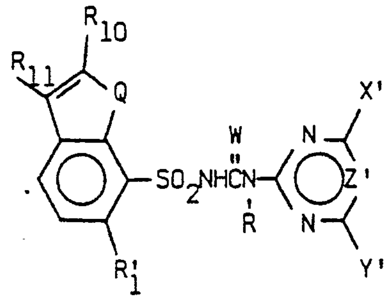

| $R_1'$ | $R_{10}$ | $R_{11}$ | R | W | Q | X' | Y' | Z' | m.p. (°C) |
|---|---|---|---|---|---|---|---|---|---|
| H | H | H | H | O | O | $OCH_3$ | $OCH_3$ | CH | |
| H | H | H | H | O | O | $OCH_3$ | $CH_3$ | N | |
| H | $CH_3$ | H | H | O | O | $OCH_3$ | $OCH_3$ | CH | 188—191° |
| H | $CH_3$ | H | H | O | O | $OCH_3$ | $CH_3$ | CH | 208—211° |
| H | $CH_3$ | H | H | O | O | $CH_3$ | $CH_3$ | CH | 198—202° |
| H | $CH_3$ | H | H | O | O | $OCH_3$ | $OCH_3$ | N | 172—176° |
| H | $CH_3$ | H | H | O | O | $OCH_3$ | $CH_3$ | N | 178—181° |
| H | $CH_3$ | $CH_3$ | H | O | O | $OCH_3$ | $OCH_3$ | CH | |
| H | $CH_3$ | $CH_3$ | H | O | O | $OCH_3$ | $CH_3$ | CH | |
| H | $CH_3$ | $CH_3$ | H | O | O | $CH_3$ | $CH_3$ | CH | |
| H | $CH_3$ | $CH_3$ | H | O | O | $OCH_3$ | $OCH_3$ | N | |
| H | $CH_3$ | $CH_3$ | H | O | O | $OCH_3$ | $CH_3$ | N | |
| H | H | $CH_3$ | H | O | O | $OCH_3$ | $OCH_3$ | CH | |
| H | H | $CH_3$ | H | O | O | $OCH_3$ | $CH_3$ | CH | |
| H | H | $CH_3$ | H | O | O | $OCH_3$ | $CH_3$ | N | |
| H | $CH_3$ | $CH_3$ | H | O | S | $OCH_3$ | $OCH_3$ | CH | |
| H | $CH_3$ | $CH_3$ | H | O | S | $OCH_3$ | $CH_3$ | CH | |
| H | $CH_3$ | $CH_3$ | H | O | S | $CH_3$ | $CH_3$ | CH | |
| H | $CH_3$ | $CH_3$ | H | O | S | $OCH_3$ | $OCH_3$ | N | |
| H | $CH_3$ | $CH_3$ | H | O | S | $OCH_3$ | $CH_3$ | N | |
| H | H | $CH_3$ | H | O | S | $OCH_3$ | $OCH_3$ | CH | 216—218° |
| H | H | $CH_3$ | H | O | S | $OCH_3$ | $CH_3$ | CH | 218—220° |
| H | H | $CH_3$ | H | O | S | $CH_3$ | $CH_3$ | CH | |
| H | $CH_3$ | H | H | O | O | $CH_3$ | $CH_3$ | N | 201—204° |

TABLE IV (continued)

| $R_1'$ | $R_{10}$ | $R_{11}$ | R | W | Q | X' | Y' | Z' | m.p. (°C) |
|---|---|---|---|---|---|---|---|---|---|
| H | H | $CH_3$ | H | O | S | $OCH_3$ | $OCH_3$ | N | |
| H | H | $CH_3$ | H | O | S | $OCH_3$ | $CH_3$ | N | |
| H | H | H | H | O | S | $OCH_3$ | $OCH_3$ | CH | 204—206° |
| H | H | H | H | O | S | $OCH_3$ | $CH_3$ | CH | 195—197° |
| H | H | H | H | O | S | $CH_3$ | $CH_3$ | CH | |
| H | H | H | H | O | S | $OCH_3$ | $OCH_3$ | N | |
| H | H | H | H | O | S | $OCH_3$ | $CH_3$ | N | |
| H | H | H | H | O | S | $CH_3$ | $CH_3$ | N | |
| H | $CH_3$ | $CH_3$ | H | O | $SO_2$ | $OCH_3$ | $OCH_3$ | CH | 236—239° |
| H | $CH_3$ | $CH_3$ | H | O | $SO_2$ | $OCH_3$ | $CH_3$ | CH | 237—239° |
| H | $CH_3$ | $CH_3$ | H | O | $SO_2$ | $CH_3$ | $CH_3$ | CH | 249—252° |
| H | $CH_3$ | $CH_3$ | H | O | $SO_2$ | $OCH_3$ | $OCH_3$ | N | 226—230° |
| H | H | $CH_3$ | H | O | $SO_2$ | $OCH_3$ | $CH_3$ | N | |
| H | H | H | H | O | $SO_2$ | $OCH_3$ | $OCH_3$ | CH | |
| H | H | H | H | O | $SO_2$ | $OCH_3$ | $CH_3$ | CH | |
| H | H | H | H | O | $SO_2$ | $OCH_3$ | $OCH_3$ | N | |
| H | H | H | H | O | $SO_2$ | $OCH_3$ | $CH_3$ | N | |
| H | H | $CH_3$ | H | O | $SO_2$ | $OCH_3$ | $OCH_3$ | CH | |
| H | H | $CH_3$ | H | O | $SO_2$ | $OCH_3$ | $CH_3$ | CH | |
| H | H | $CH_3$ | H | O | $SO_2$ | $OCH_3$ | $OCH_3$ | N | |
| H | H | $CH_3$ | H | O | $SO_2$ | $CH_3$ | $CH_3$ | CH | |
| H | $CH_3$ | H | $CH_3$ | O | O | $OCH_3$ | $OCH_3$ | CH | |
| H | $CH_3$ | H | $CH_3$ | O | O | $OCH_3$ | $OCH_3$ | N | |
| H | $CH_3$ | $CH_3$ | H | O | O | H | $CH_3$ | $CCH_3$ | |
| H | $CH_3$ | $CH_3$ | H | O | O | $OCH_3$ | $CH_3$ | $CC_2H_5$ | |
| H | H | H | H | O | S | $CH_3$ | $CH_3$ | CCl | |
| H | $CH_3$ | $CH_3$ | H | O | O | H | $CH_3$ | CCl | |
| H | H | H | H | O | S | H | $CH_3$ | CBr | |
| H | $CH_3$ | $CH_3$ | H | O | O | H | $OCH_3$ | CCl | |
| H | H | $CH_3$ | H | O | S | $CH_3$ | $CH_3$ | CBr | |
| H | $CH_3$ | H | H | O | O | $CH_3$ | $NH_2$ | CH | |
| H | H | $CH_3$ | H | O | $SO_2$ | $CH_3$ | $CH_3$ | CH | |

TABLE IV (continued)

| $R_1'$ | $R_{10}$ | $R_{11}$ | R | W | Q | X' | Y' | Z' | m.p. (°C) |
|---|---|---|---|---|---|---|---|---|---|
| H | $CH_3$ | $CH_3$ | H | O | $SO_2$ | $CH_3$ | $CH_3$ | N | 233—236° |
| H | $CH_3$ | H | H | O | O | $CH_3$ | $NHCH_3$ | CH | |
| H | $CH_3$ | $CH_3$ | H | O | O | $CH_3$ | $N(CH_3)_2$ | CH | |
| H | $CH_3$ | H | H | O | O | H | $OCH_3$ | CBr | |
| H | H | H | H | O | S | $CH_3$ | $OC_2H_5$ | CH | |
| H | H | $CH_3$ | H | O | S | $CH_3$ | $OC_2H_5$ | N | |
| H | $CH_3$ | H | H | O | O | $CH_3$ | $CH_2OCH_3$ | CH | |
| H | $CH_3$ | $CH_3$ | H | O | O | Cl | $OCH_3$ | CH | |
| $CH_3$ | $CH_3$ | $CH_3$ | H | O | $SO_2$ | $OCH_3$ | $OCH_3$ | CH | |
| $OCH_3$ | $CH_3$ | $CH_3$ | H | O | S | $OCH_3$ | $CH_3$ | CH | |
| Cl | $CH_3$ | H | H | O | O | $OCH_3$ | $CH_3$ | N | |
| Br | $CH_3$ | $CH_3$ | H | O | S | $OCH_3$ | $OCH_3$ | CH | |
| $NO_2$ | $CH_3$ | $CH_3$ | H | O | O | $OCH_3$ | $OCH_3$ | CH | |
| $CO_2CH_3$ | $CH_3$ | $CH_3$ | H | O | O | $OCH_3$ | $CH_3$ | CH | |
| $CO_2CH(CH_3)_2$ | $CH_3$ | $CH_3$ | H | O | S | $OCH_3$ | $CH_3$ | N | |
| $CO_2CH_2CH_2CH_3$ | $CH_3$ | $CH_3$ | H | O | $SO_2$ | $OCH_3$ | $CH_3$ | N | |
| $CO_2CH_2CH{=}CH_2$ | $CH_3$ | $CH_3$ | H | O | O | $CH_3$ | $CH_3$ | CH | |
| $CO_2CH_2CH_2OCH_3$ | $CH_3$ | $CH_3$ | H | O | O | $CH_3$ | $CH_3$ | CH | |
| $CO_2CH_2CH_2Cl$ | $CH_3$ | $CH_3$ | $CH_3$ | O | O | $CH_3$ | $CH_3$ | CH | |
| $SO_2CH_3$ | $CH_3$ | $CH_3$ | H | O | O | $OCH_3$ | $OCH_3$ | CH | |
| $SO_2CH(CH_3)_2$ | $CH_3$ | $CH_3$ | H | O | O | $OCH_3$ | $CH_3$ | N | |
| $SO_2CH_2CH_2CH_3$ | $CH_3$ | $CH_3$ | H | O | O | $OCH_3$ | $OCH_3$ | CH | |
| $OSO_2CH_3$ | $CH_3$ | $CH_3$ | H | O | O | $OCH_3$ | $CH_3$ | CH | |
| $OSO_2CH(CH_3)_2$ | $CH_3$ | $CH_3$ | H | O | O | $OCH_3$ | $CH_3$ | N | |
| $OSO_2CH_2CH_2CH_3$ | $CH_3$ | $CH_3$ | H | O | O | $OCH_3$ | $OCH_3$ | CH | |
| $OSO_2CF_3$ | $CH_3$ | $CH_3$ | H | O | $SO_2$ | $OCH_3$ | $CH_3$ | CH | |
| $SO_2N(CH_3)_2$ | $CH_3$ | $CH_3$ | H | O | S | $CH_3$ | $OCH_3$ | CH | |
| $SO_2N(CH_3)CH_2CH_3$ | $CH_3$ | $CH_3$ | H | O | O | $CH_3$ | $OCH_3$ | N | |
| $CO_2C_2H_5$ | $CH_3$ | $CH_3$ | H | O | O | $CH_3$ | $OCH_3$ | CH | |
| $SO_2C_2H_5$ | $CH_3$ | $CH_3$ | H | O | O | $OCH_3$ | $OCH_3$ | CH | |
| $OSO_2C_2H_5$ | $CH_3$ | $CH_3$ | H | O | O | $CH_3$ | $OCH_3$ | CH | |

TABLE IV (continued)

| $R_1'$ | $R_{10}$ | $R_{11}$ | R | W | Q | X' | Y' | Z' | m.p. (°C) |
|---|---|---|---|---|---|---|---|---|---|
| H | H | H | H | O | O | $OCH_3$ | $CH_3$ | CH | |
| H | H | H | H | O | O | $CH_3$ | $CH_3$ | CH | |
| H | H | H | H | O | O | $OCH_3$ | $OCH_3$ | N | |
| H | $C_2H_5$ | H | H | O | O | $OCH_3$ | $OCH_3$ | CH | |
| H | $n$-$C_3H_7$ | H | H | O | O | $OCH_3$ | $OCH_3$ | CH | |
| H | $CH(CH_3)_2$ | H | H | O | O | $OCH_3$ | $OCH_3$ | CH | |
| H | Cl | $CH_3$ | H | O | O | $OCH_3$ | $OCH_3$ | CH | |
| H | Br | $CH_3$ | H | O | O | $OCH_3$ | $OCH_3$ | CH | |
| H | $CH_3$ | Cl | H | O | O | $OCH_3$ | $OCH_3$ | CH | |
| H | $CH_3$ | Br | H | O | O | $OCH_3$ | $OCH_3$ | CH | |
| H | $CH_3$ | H | H | O | S | $OCH_3$ | $OCH_3$ | CH | |
| H | $CH_3$ | H | H | O | S | $CH_3$ | $OCH_3$ | CH | |
| H | $CH_3$ | H | H | O | S | $CH_3$ | $CH_3$ | CH | |
| H | $CH_3$ | H | H | O | S | $OCH_3$ | $CH_3$ | N | |
| H | $CH_3$ | H | H | O | S | $OCH_3$ | $OCH_3$ | N | |
| H | H | H | H | O | $SO_2$ | $CH_3$ | $CH_3$ | CH | |
| H | $CH_3$ | H | H | O | $SO_2$ | $OCH_3$ | $OCH_3$ | CH | |
| H | $CH_3$ | H | H | O | $SO_2$ | $OCH_3$ | $CH_3$ | CH | |
| H | $CH_3$ | H | H | O | $SO_2$ | $CH_3$ | $CH_3$ | CH | |
| H | $CH_3$ | H | H | O | $SO_2$ | $OCH_3$ | $OCH_3$ | N | |
| H | $CH_3$ | H | H | O | $SO_2$ | $CH_3$ | $OCH_3$ | N | |
| H | $CH_3$ | $CH_3$ | H | O | $SO_2$ | $OCH_3$ | $CH_3$ | N | |
| H | $CH_3$ | H | H | O | O | Cl | $OCH_3$ | CH | |
| H | H | H | H | O | $SO_2$ | Cl | $OCH_3$ | CH | |
| H | $CH_3$ | $CH_3$ | H | O | $SO_2$ | $OCH_3$ | $CH(OCH_3)_2$ | N | |
| H | $CH_3$ | H | H | O | O | $CH_3$ | $C_2H_5$ | CH | |
| H | $CH_3$ | H | H | O | O | $CH_3$ | $CF_3$ | CH | |
| H | H | H | H | O | S | $CH_3$ | $OCH_2CH=CH_2$ | N | |
| H | $CH_3$ | H | H | O | O | $CH_3$ | $OCH_2C\equiv CH$ | N | |
| H | $CH_3$ | H | H | O | O | $OCH_3$ | $OCH_2CF_3$ | N | |

TABLE IV (continued)

| $R_1'$ | $R_{10}$ | $R_{11}$ | R | W | Q | X′ | Y′ | Z′ | m.p. (°C) |
|---|---|---|---|---|---|---|---|---|---|
| H | $CH_3$ | H | H | O | O | $CH_3$ | 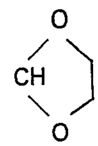 | CH | |
| H | H | H | H | O | S | Cl | $NH_2$ | CH | |
| H | $CH_3$ | H | H | O | O | Cl | $NHCH_3$ | CH | |
| H | $CH_3$ | $CH_3$ | H | O | $SO_2$ | Cl | $N(CH_3)_2$ | CH | |
| H | $CH_3$ | H | H | S | O | $OCH_3$ | $OCH_3$ | CH | |
| H | H | H | H | S | S | $OCH_3$ | $OCH_3$ | CH | |
| H | H | H | H | S | $SO_2$ | $OCH_3$ | $OCH_3$ | CH | |
| 4-, 5- or 6- Cl | H | $CH_3$ | H | O | S | $CH_3$ | $CH_3$ | CH | 218—220° |
| 4-, 5- or 6- Cl | H | $CH_3$ | H | O | S | $CH_3$ | $OCH_3$ | CH | 231—233° |
| 4-, 5- or 6- Cl | H | $CH_3$ | H | O | S | $OCH_3$ | $OCH_3$ | CH | 218—221° |
| H | $CH_3$ | H | H | O | O | $OCH_3$ | $SCH_3$ | CH | |
| H | $CH_3$ | H | H | O | O | $OCH_3$ | $SCH_3$ | N | |
| H | H | H | H | O | S | $OCH_3$ | $SCH_3$ | CH | |
| H | $CH_3$ | H | H | O | O | $CH_3$ | $SCH_3$ | CH | |

TABLE IVa

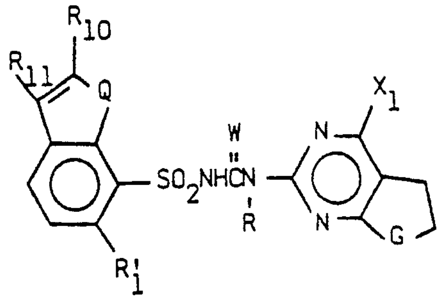

| $R_1'$ | $R_{10}$ | $R_{11}$ | R | W | Q | G | $X_1$ | m.p.(°C) |
|---|---|---|---|---|---|---|---|---|
| H | $CH_3$ | H | H | O | O | O | $CH_3$ | |
| H | $CH_3$ | H | H | O | O | O | $OCH_3$ | |
| H | $CH_3$ | H | H | O | O | O | $OC_2H_5$ | |
| H | H | H | H | O | O | O | $CH_3$ | |
| H | $CH_3$ | $CH_3$ | H | O | O | O | $CH_3$ | |
| H | $CH_3$ | H | H | O | O | $CH_2$ | $OCH_3$ | |
| H | $CH_3$ | H | H | O | O | O | $CH_2$ | |
| H | $CH_3$ | H | H | O | O | $CH_2$ | $OC_2H_5$ | |
| H | H | H | H | O | S | O | $CH_3$ | |
| H | H | H | H | O | $SO_2$ | O | $CH_3$ | |
| H | $CH_3$ | $CH_3$ | H | O | $SO_2$ | O | $CH_3$ | |
| H | $CH_3$ | $CH_3$ | H | O | S | O | $CH_3$ | |
| H | $CH_3$ | H | H | S | O | O | $CH_3$ | |
| Cl | $CH_3$ | H | H | O | O | O | $CH_3$ | |
| H | $CH_3$ | H | $CH_3$ | O | O | O | $CH_3$ | |

TABLE IVb

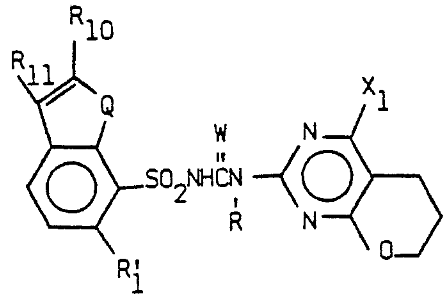

| $R_1'$ | $R_{10}$ | $R_{11}$ | R | W | Q | $X_1$ | m.p.(°C) |
|---|---|---|---|---|---|---|---|
| H | $CH_3$ | H | H | O | O | $CH_3$ | |
| H | $CH_3$ | H | H | O | O | $OCH_3$ | |
| H | $CH_3$ | H | H | O | O | $OC_2H_5$ | |
| H | $CH_3$ | $CH_3$ | H | O | O | $CH_3$ | |
| H | H | H | H | O | O | $CH_3$ | |
| H | $CH_3$ | H | H | S | O | $CH_3$ | |
| H | $CH_3$ | H | $CH_3$ | O | O | $CH_3$ | |
| H | $CH_3$ | $CH_3$ | H | O | $SO_2$ | $CH_3$ | |
| H | H | H | H | O | S | $CH_3$ | |
| H | H | H | H | O | S | $OCH_3$ | |
| H | H | H | H | O | $SO_2$ | $OCH_3$ | |
| Cl | $CH_3$ | H | H | O | O | $CH_3$ | |

TABLE IVc

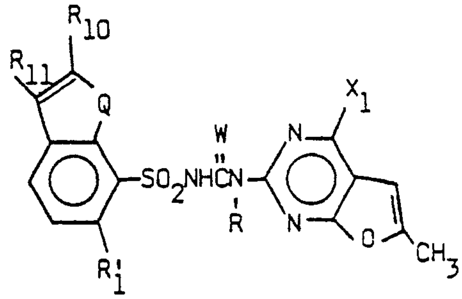

| $R_1'$ | $R_{10}$ | $R_{11}$ | R | W | Q | $X_1$ | m.p.(°C) |
|---|---|---|---|---|---|---|---|
| H | $CH_3$ | $CH_3$ | H | O | O | $CH_3$ | |
| H | $CH_3$ | H | H | O | O | $CH_3$ | |
| H | H | H | H | O | O | $CH_3$ | |
| H | $CH_3$ | $CH_3$ | H | O | $SO_2$ | $CH_3$ | |
| H | H | H | H | O | S | $CH_3$ | |
| H | $CH_3$ | H | H | O | $SO_2$ | $CH_3$ | |
| H | $CH_3$ | H | H | O | S | $CH_3$ | |
| H | $CH_3$ | $CH_3$ | H | O | O | $OCH_3$ | |
| H | $CH_3$ | H | H | O | O | $OCH_3$ | |
| H | $CH_3$ | $CH_3$ | H | O | S | $OCH_3$ | |
| H | $CH_3$ | $CH_3$ | H | O | $SO_2$ | $OCH_3$ | |
| H | H | H | H | O | S | $OCH_3$ | |
| H | $CH_3$ | H | H | O | S | $OCH_3$ | |
| H | $CH_3$ | H | H | O | O | $OC_2H_5$ | |
| H | $CH_3$ | H | $CH_3$ | O | O | $CH_3$ | |
| Cl | $CH_3$ | H | H | O | O | $CH_3$ | |
| H | $CH_3$ | H | H | S | O | $CH_3$ | |

TABLE IVd

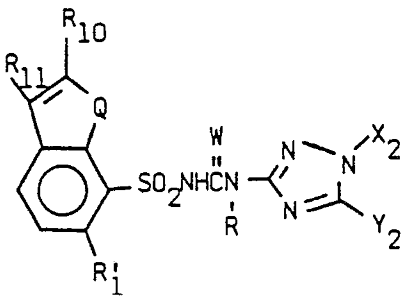

| $R_1'$ | $R_{10}$ | $R_{11}$ | R | W | Q | $X_2$ | $Y_2$ | m.p.(°C) |
|---|---|---|---|---|---|---|---|---|
| H | $CH_3$ | H | H | O | O | $CH_3$ | $OCH_3$ | |
| H | $CH_3$ | $CH_3$ | H | O | O | $CH_3$ | $OCH_3$ | |
| H | H | H | H | O | O | $CH_3$ | $OCH_3$ | |
| H | $CH_3$ | H | H | O | O | $CH_3$ | $SCH_3$ | |
| H | $CH_3$ | $CH_3$ | H | O | O | $CH_3$ | $SCH_3$ | |
| H | H | H | H | O | O | $CH_3$ | $SCH_3$ | |
| H | H | H | H | O | S | $CH_3$ | $OCH_3$ | |
| H | H | H | H | O | S | $CH_3$ | $SCH_3$ | |
| H | $CH_3$ | $CH_3$ | H | O | $SO_2$ | $CH_3$ | $OCH_3$ | |
| H | $CH_3$ | $CH_3$ | H | O | $SO_2$ | $CH_3$ | $SCH_3$ | |
| H | $CH_3$ | H | $CH_3$ | O | O | $CH_3$ | $OCH_3$ | |
| Cl | $CH_3$ | H | H | O | O | $CH_3$ | $OCH_3$ | |
| H | $CH_3$ | H | H | S | O | $CH_3$ | $OCH_3$ | |
| H | H | H | H | S | S | $CH_3$ | $OCH_3$ | |
| H | $CH_3$ | $CH_3$ | H | S | $SO_2$ | $CH_3$ | $OCH_3$ | |
| H | H | H | H | O | S | $C_2H_5$ | $OCH_3$ | |
| H | $CH_3$ | H | H | O | O | $CH_2CF_3$ | $OCH_3$ | |
| H | $CH_3$ | $CH_3$ | H | O | $SO_2$ | $CH_3$ | $OC_2H_5$ | |
| H | $CH_3$ | H | H | O | O | $CH_3$ | $SC_2H_5$ | |
| H | $CH_3$ | H | H | O | O | $CH_3$ | $C_2H_5$ | |
| H | $CH_3$ | H | H | O | O | $CH_3$ | $CH_3$ | |

TABLE V

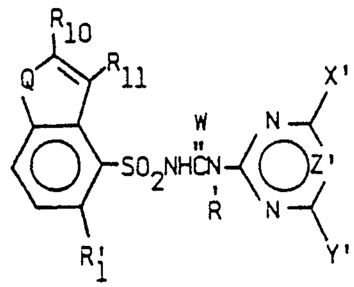

| $R_1'$ | $R_{10}$ | $R_{11}$ | R | W | Q | X' | Y' | Z' | m.p.(°C) |
|---|---|---|---|---|---|---|---|---|---|
| H | H | H | H | O | O | $OCH_3$ | $OCH_3$ | CH | |
| H | H | H | H | O | O | $OCH_3$ | $CH_3$ | N | |
| H | H | $CH_3$ | H | O | O | $OCH_3$ | $OCH_3$ | CH | |
| H | H | $CH_3$ | H | O | O | $OCH_3$ | $CH_3$ | N | |
| H | H | H | H | S | O | $OCH_3$ | $OCH_3$ | CH | |
| H | H | $CH_3$ | H | O | S | $OCH_3$ | $OCH_3$ | CH | |
| H | H | $CH_3$ | H | O | $SO_2$ | $OCH_3$ | $OCH_3$ | CH | |
| H | H | $CH_3$ | H | O | $SO_2$ | $OCH_3$ | $CH_3$ | N | |
| H | $CH_3$ | $CH_3$ | H | O | O | $OCH_3$ | $OCH_3$ | CH | |

TABLE Va

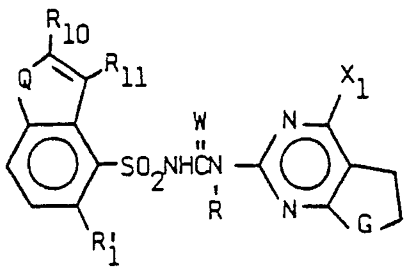

| $R_1'$ | $R_{10}$ | $R_{11}$ | R | W | Q | G | $X_1$ | m.p.(°C) |
|---|---|---|---|---|---|---|---|---|
| H | H | $CH_3$ | H | O | O | O | $CH_3$ | |
| H | $CH_3$ | $CH_3$ | H | O | O | O | $OCH_3$ | |
| H | H | $CH_3$ | H | O | O | O | $OC_2H_5$ | |
| H | H | H | H | O | S | O | $CH_3$ | |
| H | H | $CH_3$ | H | O | $SO_2$ | O | $CH_3$ | |
| H | H | $CH_3$ | H | O | O | $CH_2$ | $CH_3$ | |
| H | H | H | H | O | S | $CH_2$ | $OCH_3$ | |
| H | H | H | H | O | $SO_2$ | $CH_2$ | $OC_2H_5$ | |
| H | H | $CH_3$ | H | S | O | O | $CH_3$ | |
| Cl | H | $CH_3$ | H | O | O | O | $CH_3$ | |

TABLE Vb

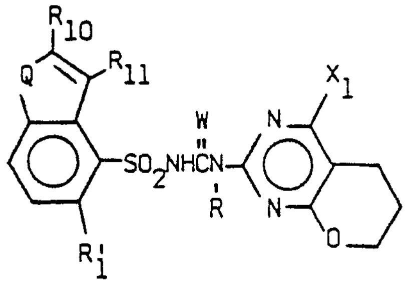

| $R_1'$ | $R_{10}$ | $R_{11}$ | R | W | Q | $X_1$ | m.p.(°C) |
|---|---|---|---|---|---|---|---|
| H | H | $CH_3$ | H | O | O | $CH_3$ | |
| H | H | $CH_3$ | H | O | S | $CH_3$ | |
| Cl | H | $CH_3$ | H | O | O | $CH_3$ | |
| H | H | $CH_3$ | H | O | $SO_2$ | $CH_3$ | |
| H | $CH_3$ | $CH_3$ | H | O | O | $OCH_3$ | |
| H | H | $CH_3$ | H | O | S | $OCH_3$ | |
| H | H | $CH_3$ | $CH_3$ | O | S | $OCH_3$ | |
| H | H | $CH_3$ | H | O | S | $OCH_3$ | |
| H | H | H | H | O | O | $OCH_3$ | |
| H | H | $CH_3$ | H | O | O | $OC_2H_5$ | |

TABLE Vc

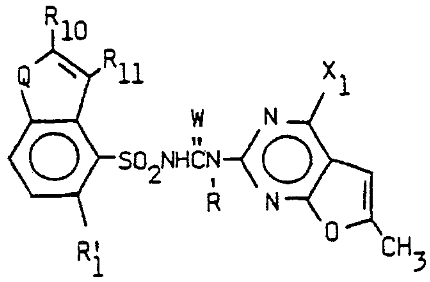

| $R_1'$ | $R_{10}$ | $R_{11}$ | R | W | Q | $X_1$ | m.p.(°C) |
|---|---|---|---|---|---|---|---|
| H | $CH_3$ | $CH_3$ | H | O | O | $CH_3$ | |
| H | H | $CH_3$ | H | O | S | $CH_3$ | |
| H | H | $CH_3$ | H | O | $SO_2$ | $CH_3$ | |
| H | H | $CH_3$ | H | O | O | $OCH_3$ | |
| H | H | $CH_3$ | H | O | S | $OCH_3$ | |
| H | $CH_3$ | $CH_3$ | H | O | $SO_2$ | $OCH_3$ | |
| H | H | $CH_3$ | H | O | $SO_2$ | $OCH_3$ | |
| H | H | H | H | O | O | $OC_2H_5$ | |
| H | H | H | H | S | O | $CH_3$ | |
| H | H | $CH_3$ | H | S | $SO_2$ | $CH_3$ | |
| Cl | H | $CH_3$ | H | O | O | $CH_3$ | |

TABLE Vd

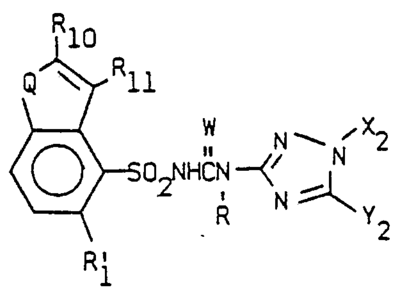

| $R_1'$ | $R_{10}$ | $R_{11}$ | R | W | Q | $X_2$ | $Y_2$ | m.p.(°C) |
|---|---|---|---|---|---|---|---|---|
| H | H | $CH_3$ | H | O | O | $CH_3$ | $OCH_3$ | |
| H | H | $CH_3$ | H | O | O | $CH_3$ | $SCH_3$ | |
| H | $CH_3$ | $CH_3$ | H | O | O | $CH_3$ | $OCH_3$ | |
| H | H | $CH_3$ | H | S | O | $CH_3$ | $OCH_3$ | |
| H | $CH_3$ | $CH_3$ | H | O | S | $CH_3$ | $OCH_3$ | |
| H | H | H | H | O | $SO_2$ | $CH_3$ | $OCH_3$ | |
| Cl | H | $CH_3$ | H | O | O | $CH_3$ | $OCH_3$ | |

TABLE VIa

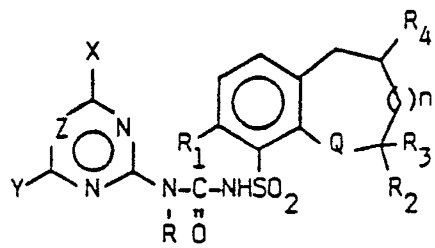

| n | Q | R | $R_1$ | $R_2$ | $R_3$ | $R_4$ | X | Y | Z | m.p.(°) |
|---|---|---|---|---|---|---|---|---|---|---|
| O | O | H | H | H | H | H | $CH_3$ | $CH_3$ | N | |
| O | S | H | H | H | H | H | $CH_3$ | $CH_3$ | CH | |
| O | S | H | H | H | H | H | $CH_3$ | $OCH_3$ | CH | |
| O | S | H | H | H | H | H | $OCH_3$ | $OCH_3$ | CH | |
| O | S | H | H | H | H | H | $CH_3$ | $CH_3$ | N | |
| O | S | H | H | H | H | H | $CH_3$ | $OCH_3$ | N | |
| O | S | H | H | H | H | H | $OCH_3$ | $OCH_3$ | N | |
| O | $SO_2$ | H | H | H | H | H | $CH_3$ | $CH_3$ | CH | 214—216° |
| O | $SO_2$ | H | H | H | H | H | $CH_3$ | $OCH_3$ | CH | 217—220° |
| O | $SO_2$ | H | H | H | H | H | $OCH_3$ | $OCH_3$ | CH | 208—216° |
| O | $SO_2$ | H | H | H | H | H | $CH_3$ | $CH_3$ | N | |
| O | $SO_2$ | H | H | H | H | H | $CH_3$ | $OCH_3$ | N | 214—218° |
| O | $SO_2$ | H | H | H | H | H | $OCH_3$ | $OCH_3$ | N | |
| O | O | H | H | $CH_3$ | H | H | $CH_3$ | $CH_3$ | CH | |
| O | O | H | H | $CH_3$ | H | H | $CH_3$ | $OCH_3$ | CH | |
| O | O | H | H | $CH_3$ | H | H | $OCH_3$ | $OCH_3$ | CH | |
| O | O | H | H | $CH_3$ | H | H | $CH_3$ | $CH_3$ | N | |
| O | O | H | H | $CH_3$ | H | H | $CH_3$ | $OCH_3$ | N | |
| O | O | H | H | $CH_3$ | H | H | $OCH_3$ | $OCH_3$ | N | |
| O | S | H | H | $CH_3$ | H | H | $CH_3$ | $CH_3$ | CH | |
| O | S | H | H | $CH_3$ | H | H | $CH_3$ | $OCH_3$ | CH | |
| O | S | H | H | $CH_3$ | H | H | $OCH_3$ | $OCH_3$ | CH | |
| O | S | H | H | $CH_3$ | H | H | $CH_3$ | $CH_3$ | N | |
| O | S | H | H | $CH_3$ | H | H | $CH_3$ | $OCH_3$ | N | |
| O | S | H | H | $CH_3$ | H | H | $OCH_3$ | $OCH_3$ | N | |
| O | $SO_2$ | H | H | $CH_3$ | H | H | $CH_3$ | $CH_3$ | CH | |

TABLE VIa (continued)

| n | Q | R | $R_1$ | $R_2$ | $R_3$ | $R_4$ | X | Y | Z | m.p.(°) |
|---|---|---|---|---|---|---|---|---|---|---|
| O | $SO_2$ | H | H | $CH_3$ | H | H | $CH_3$ | $OCH_3$ | CH | |
| O | $SO_2$ | H | H | $CH_3$ | H | H | $OCH_3$ | $OCH_3$ | CH | |
| O | $SO_2$ | H | H | $CH_3$ | H | H | $CH_3$ | $CH_3$ | N | |
| O | $SO_2$ | H | H | $CH_3$ | H | H | $CH_3$ | $OCH_3$ | N | |
| O | $SO_2$ | H | H | $CH_3$ | H | H | $OCH_3$ | $OCH_3$ | N | |
| O | O | H | H | $CH_3$ | $CH_3$ | H | $CH_3$ | $CH_3$ | CH | |
| O | O | H | H | $CH_3$ | $CH_3$ | H | $CH_3$ | $OCH_3$ | CH | |
| O | O | H | H | $CH_3$ | $CH_3$ | H | $OCH_3$ | $OCH_3$ | CH | |
| O | O | H | H | $CH_3$ | $CH_3$ | H | $CH_3$ | $CH_3$ | N | |
| O | O | H | H | $CH_3$ | $CH_3$ | H | $CH_3$ | $OCH_3$ | N | |
| O | O | H | H | $CH_3$ | $CH_3$ | H | $OCH_3$ | $OCH_3$ | N | |
| O | S | H | H | $CH_3$ | $CH_3$ | H | $CH_3$ | $CH_3$ | CH | |
| O | S | H | H | $CH_3$ | $CH_3$ | H | $CH_3$ | $OCH_3$ | CH | |
| O | S | H | H | $CH_3$ | $CH_3$ | H | $OCH_3$ | $OCH_3$ | CH | |
| O | S | H | H | $CH_3$ | $CH_3$ | H | $CH_3$ | $CH_3$ | N | |
| O | S | H | H | $CH_3$ | $CH_3$ | H | $CH_3$ | $OCH_3$ | N | |
| O | S | H | H | $CH_3$ | $CH_3$ | H | $OCH_3$ | $OCH_3$ | N | |
| O | $SO_2$ | H | H | $CH_3$ | $CH_3$ | H | $CH_3$ | $CH_3$ | CH | |
| O | $SO_2$ | H | H | $CH_3$ | $CH_3$ | H | $CH_3$ | $OCH_3$ | CH | |
| O | $SO_2$ | H | H | $CH_3$ | $CH_3$ | H | $OCH_3$ | $OCH_3$ | CH | |
| O | $SO_2$ | H | H | $CH_3$ | $CH_3$ | H | $CH_3$ | $CH_3$ | N | |
| O | $SO_2$ | H | H | $CH_3$ | $CH_3$ | H | $CH_3$ | $OCH_3$ | N | |
| O | $SO_2$ | H | H | $CH_3$ | $CH_3$ | H | $OCH_3$ | $OCH_3$ | N | |
| 1 | O | H | H | H | H | H | $CH_3$ | $CH_3$ | CH | |
| 1 | O | H | H | H | H | H | $CH_3$ | $OCH_3$ | CH | |
| 1 | O | H | H | H | H | H | $OCH_3$ | $OCH_3$ | CH | |
| 1 | O | H | H | H | H | H | $CH_3$ | $CH_3$ | N | |
| 1 | O | H | H | H | H | H | $CH_3$ | $OCH_3$ | N | |
| 1 | O | H | H | H | H | H | $OCH_3$ | $OCH_3$ | N | |
| 1 | S | H | H | H | H | H | $CH_3$ | $CH_3$ | CH | |

TABLE VIa (continued)

| n | Q | R | $R_1$ | $R_2$ | $R_3$ | $R_4$ | X | Y | Z | m.p.(°) |
|---|---|---|---|---|---|---|---|---|---|---|
| 1 | S | H | H | H | H | H | $CH_3$ | $OCH_3$ | CH | |
| 1 | S | H | H | H | H | H | $OCH_3$ | $OCH_3$ | CH | |
| 1 | S | H | H | H | H | H | $CH_3$ | $CH_3$ | N | |
| 1 | S | H | H | H | H | H | $CH_3$ | $OCH_3$ | N | |
| 1 | S | H | H | H | H | H | $OCH_3$ | $OCH_3$ | N | |
| 1 | $SO_2$ | H | H | H | H | H | $CH_3$ | $CH_3$ | CH | |
| 1 | $SO_2$ | H | H | H | H | H | $CH_3$ | $OCH_3$ | CH | |
| 1 | $SO_2$ | H | H | H | H | H | $OCH_3$ | $OCH_3$ | CH | |
| 1 | $SO_2$ | H | H | H | H | H | $CH_3$ | $CH_3$ | N | |
| 1 | $SO_2$ | H | H | H | H | H | $CH_3$ | $OCH_3$ | N | |
| 1 | $SO_2$ | H | H | H | H | H | $OCH_3$ | $OCH_3$ | N | |
| 1 | O | H | H | $CH_3$ | H | H | $CH_3$ | $CH_3$ | CH | |
| 1 | O | H | H | $CH_3$ | H | H | $CH_3$ | $OCH_3$ | CH | |
| 1 | O | H | H | $CH_3$ | H | H | $OCH_3$ | $OCH_3$ | CH | |
| 1 | O | H | H | $CH_3$ | H | H | $CH_3$ | $CH_3$ | N | |
| 1 | O | H | H | $CH_3$ | H | H | $CH_3$ | $OCH_3$ | N | |
| 1 | O | H | H | $CH_3$ | H | H | $OCH_3$ | $OCH_3$ | N | |
| 1 | S | H | H | $CH_3$ | H | H | $CH_3$ | $CH_3$ | CH | |
| 1 | S | H | H | $CH_3$ | H | H | $CH_3$ | $OCH_3$ | CH | |
| 1 | S | H | H | $CH_3$ | H | H | $OCH_3$ | $OCH_3$ | CH | |
| 1 | S | H | H | $CH_3$ | H | H | $CH_3$ | $CH_3$ | N | |
| 1 | S | H | H | $CH_3$ | H | H | $CH_3$ | $OCH_3$ | N | |
| 1 | S | H | H | $CH_3$ | H | H | $OCH_3$ | $OCH_3$ | N | |
| 1 | $SO_2$ | H | H | $CH_3$ | H | H | $CH_3$ | $CH_3$ | CH | |
| 1 | $SO_2$ | H | H | $CH_3$ | H | H | $CH_3$ | $OCH_3$ | CH | |
| 1 | $SO_2$ | H | H | $CH_3$ | H | H | $OCH_3$ | $OCH_3$ | CH | |
| 1 | $SO_2$ | H | H | $CH_3$ | H | H | $CH_3$ | $CH_3$ | N | |
| 1 | $SO_2$ | H | H | $CH_3$ | H | H | $CH_3$ | $OCH_3$ | N | |
| 1 | $SO_2$ | H | H | $CH_3$ | H | H | $OCH_3$ | $OCH_3$ | N | |
| 1 | O | H | H | $CH_3$ | $CH_3$ | H | $CH_3$ | $CH_3$ | CH | |
| 1 | O | H | H | $CH_3$ | $CH_3$ | H | $CH_3$ | $OCH_3$ | CH | |

TABLE VIa (continued)

| n | Q | R | $R_1$ | $R_2$ | $R_3$ | $R_4$ | X | Y | Z | m.p.(°) |
|---|---|---|---|---|---|---|---|---|---|---|
| 1 | O | H | H | $CH_3$ | $CH_3$ | H | $OCH_3$ | $OCH_3$ | CH | |
| 1 | O | H | H | $CH_3$ | $CH_3$ | H | $CH_3$ | $CH_3$ | N | |
| 1 | O | H | H | $CH_3$ | $CH_3$ | H | $CH_3$ | $OCH_3$ | N | |
| 1 | O | H | H | $CH_3$ | $CH_3$ | H | $OCH_3$ | $OCH_3$ | N | |
| 1 | S | H | H | $CH_3$ | $CH_3$ | H | $CH_3$ | $CH_3$ | CH | |
| 1 | S | H | H | $CH_3$ | $CH_3$ | H | $CH_3$ | $OCH_3$ | CH | |
| 1 | S | H | H | $CH_3$ | $CH_3$ | H | $OCH_3$ | $OCH_3$ | CH | |
| 1 | S | H | H | $CH_3$ | $CH_3$ | H | $CH_3$ | $CH_3$ | N | |
| 1 | S | H | H | $CH_3$ | $CH_3$ | H | $CH_3$ | $OCH_3$ | N | |
| 1 | S | H | H | $CH_3$ | $CH_3$ | H | $OCH_3$ | $OCH_3$ | N | |
| 1 | $SO_2$ | H | H | $CH_3$ | $CH_3$ | H | $CH_3$ | $CH_3$ | CH | |
| 1 | $SO_2$ | H | H | $CH_3$ | $CH_3$ | H | $CH_3$ | $OCH_3$ | CH | |
| 1 | $SO_2$ | H | H | $CH_3$ | $CH_3$ | H | $OCH_3$ | $OCH_3$ | CH | |
| 1 | $SO_2$ | H | H | $CH_3$ | $CH_3$ | H | $CH_3$ | $CH_3$ | N | |
| 1 | $SO_2$ | H | H | $CH_3$ | $CH_3$ | H | $CH_3$ | $OCH_3$ | N | |
| 1 | $SO_2$ | H | H | $CH_3$ | $CH_3$ | H | $OCH_3$ | $OCH_3$ | N | |
| O | $SO_2$ | $CH_3$ | H | $CH_3$ | H | H | $OCH_3$ | $OCH_3$ | N | |
| O | $SO_2$ | H | H | H | H | $CH_3$ | $CH_3$ | $CH_3$ | CH | |
| O | $SO_2$ | H | H | H | H | $CH_3$ | $CH_3$ | $OCH_3$ | CH | 243—245° |
| O | $SO_2$ | H | H | H | H | $CH_3$ | $OCH_3$ | $OCH_3$ | CH | 244—245° |
| O | $SO_2$ | H | H | H | H | $CH_3$ | $CH_3$ | $CH_3$ | N | |
| O | $SO_2$ | H | H | H | H | $CH_3$ | $CH_3$ | $OCH_3$ | N | 206—209° |
| O | $SO_2$ | H | H | H | H | $CH_3$ | $OCH_3$ | $OCH_3$ | N | 228—230° |
| O | O | H | $OCH_3$ | $CH_3$ | $CH_3$ | H | $CH_3$ | $CH_3$ | CH | |
| O | O | H | $OCH_3$. | $CH_3$ | $CH_3$ | H | $CH_3$ | $OCH_3$ | CH | |
| O | O | H | $OCH_3$ | $CH_3$ | $CH_3$ | H | $OCH_3$ | $OCH_3$ | CH | |
| O | O | H | $OCH_3$ | $CH_3$ | $CH_3$ | H | $CH_3$ | $CH_3$ | N | |
| O | O | H | $OCH_3$ | $CH_3$ | $CH_3$ | H | $CH_3$ | $OCH_3$ | N | |
| O | o | H | $OCH_3$ | $CH_3$ | $CH_3$ | H | $OCH_3$ | $OCH_3$ | N | |
| O | O | H | $CH_3$ | H | H | H | $OCH_3$ | $OCH_3$ | CH | |

TABLE VIa (continued)

| n | Q | R | $R_1$ | $R_2$ | $R_3$ | $R_4$ | X | Y | Z | m.p.(°) |
|---|---|---|---|---|---|---|---|---|---|---|
| O | S | H | Cl | $CH_3$ | H | H | $OCH_3$ | $OCH_3$ | CH | |
| O | $SO_2$ | H | Br | H | $CH_3$ | H | $OCH_3$ | $OCH_3$ | CH | |
| O | O | H | $CO_2CH_3$ | $CH_3$ | H | H | $CH_3$ | $OCH_3$ | CH | |
| O | S | H | $CO_2CH_2CH_3$ | H | $CH_3$ | H | $OCH_3$ | $OCH_3$ | CH | |
| O | $SO_2$ | H | $CO_2CH_2CH_2CH_3$ | $CH_3$ | H | H | $CH_3$ | $OCH_3$ | N | |
| O | O | H | $CO_2CH(CH_3)_2$ | H | $CH_3$ | H | $OCH_3$ | $OCH_3$ | N | |
| O | S | H | $CO_2CH_2CH{=}CH_2$ | $CH_3$ | H | H | $CH_3$ | $OCH_3$ | CH | |
| O | $SO_2$ | H | $CO_2CH_2CH_2OCH_3$ | H | $CH_3$ | H | $OCH_3$ | $OCH_3$ | CH | |
| O | O | H | $CO_2CH_2CH_2Cl$ | $CH_3$ | H | H | $CH_3$ | $OCH_3$ | N | |
| O | S | H | $SO_2CH_3$ | H | $CH_3$ | H | $OCH_3$ | $OCH_3$ | N | |
| O | $SO_2$ | H | $SO_2CH_2CH_2CH_3$ | $CH_3$ | H | H | $CH_3$ | $OCH_3$ | CH | |
| O | O | H | $SO_2CH(CH_3)_2$ | H | $CH_3$ | H | $OCH_3$ | $OCH_3$ | CH | |
| O | S | H | $OSO_2CH_3$ | $CH_3$ | H | H | $CH_3$ | $OCH_3$ | N | |
| O | $SO_2$ | H | $OSO_2CF_3$ | H | $CH_3$ | H | $OCH_3$ | $OCH_3$ | N | |
| O | O | H | $OSO_2CH(CH_3)_2$ | H | $CH_3$ | H | $CH_3$ | $OCH_3$ | CH | |
| O | $SO_2$ | H | $SO_2N(CH_3)_2$ | $CH_3$ | H | H | $OCH_3$ | $OCH_3$ | CH | |
| O | $SO_2$ | H | $SO_2N(CH_2CH_3)_2$ | H | $CH_3$ | H | $CH_3$ | $OCH_3$ | N | |
| O | O | H | $SO_2N(CH_3)CH_2CH_3$ | $CH_3$ | H | H | $OCH_3$ | $OCH_3$ | N | |
| O | S | H | H | $CH_3$ | $CH_2CH_3$ | H | $CH_3$ | $OCH_3$ | CH | |
| O | $SO_2$ | H | H | H | $CH_2CH_2CH_3$ | H | $OCH_3$ | $OCH_3$ | CH | |
| O | O | H | H | H | $CH(CH_3)_2$ | H | $CH_3$ | $OCH_3$ | N | |
| O | S | H | H | $CH_2CH_3$ | H | H | $OCH_3$ | $OCH_3$ | N | |
| O | $SO_2$ | H | H | $CH_2CH_2CH_3$ | H | H | $CH_3$ | $OCH_3$ | CH | |
| O | O | H | H | $CH(CH_3)_2$ | H | H | $OCH_3$ | $OCH_3$ | CH | |
| O | S | H | H | H | $CH_3$ | H | Cl | $OCH_3$ | CH | |
| O | $SO_2$ | H | H | $CH_3$ | H | H | $CH_3$ | $NH_2$ | CH | |
| O | O | H | H | H | $CH_3$ | H | $CH_3$ | $NHCH_3$ | CH | |
| O | S | H | H | $CH_3$ | H | H | $CH_3$ | $N(CH_3)_2$ | CH | |
| O | $SO_2$ | H | H | H | $CH_3$ | H | $CH_3$ | $OC_2H_5$ | CH | |
| O | O | H | H | $CH_3$ | H | H | $CH_3$ | $OCH_2CH_3$ | N | |
| O | S | H | H | H | $CH_3$ | H | $OCH_3$ | $CH_2OCH_3$ | CH | |

TABLE VIb

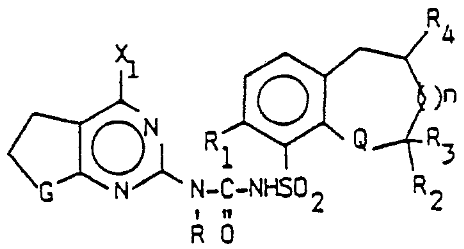

| n | Q | R | $R_1$ | $R_2$ | $R_3$ | $R_4$ | $X_1$ | G | m.p.(°) |
|---|---|---|---|---|---|---|---|---|---|
| O | $SO_2$ | H | H | H | H | H | $CH_3$ | $CH_2$ | |
| O | $SO_2$ | H | H | H | H | H | $OCH_3$ | $CH_2$ | |
| O | $SO_2$ | H | H | H | H | H | $OCH_2CH_3$ | $CH_2$ | |
| O | $SO_2$ | H | H | H | H | H | $CH_3$ | O | |
| O | $SO_2$ | H | H | H | H | H | $OCH_2$ | O | |
| O | $SO_2$ | H | H | H | H | H | $OCH_2CH_3$ | O | |
| O | $SO_2$ | H | H | H | H | $CH_2$ | $OCH_3$ | O | |
| O | O | H | H | H | $CH_3$ | H | $OCH_3$ | O | |
| O | S | H | H | $CH_3$ | H | H | $OCH_3$ | O | |
| O | $SO_2$ | H | H | H | $CH_3$ | H | $OCH_3$ | O | |
| O | S | H | $CH_3$ | $CH_3$ | H | H | $OCH_3$ | O | |
| O | $SO_2$ | H | $OCH_3$ | $CH_3$ | H | H | $OCH_3$ | O | |
| O | S | H | Cl | $CH_3$ | H | H | $OCH_3$ | O | |
| O | $SO_2$ | H | Br | $CH_3$ | H | H | $OCH_3$ | O | |
| O | S | H | $CO_2CH_3$ | $CH_3$ | H | H | $OCH_3$ | O | |
| O | $SO_2$ | H | $SO_2CH_3$ | $CH_3$ | H | H | $OCH_3$ | O | |
| O | S | H | $OSO_2CH_3$ | $CH_3$ | H | H | $OCH_3$ | O | |
| O | $SO_2$ | H | $SO_2(CH_3)_2$ | $CH_3$ | H | H | $OCH_3$ | O | |
| 1 | $SO_2$ | H | H | H | H | H | $OCH_3$ | O | |

TABLE VIc

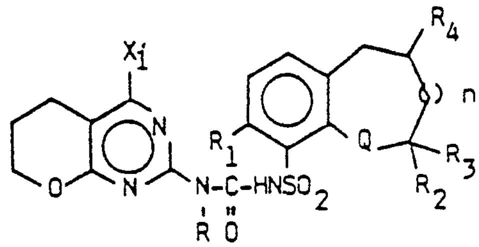

| n | Q | R | $R_1$ | $R_2$ | $R_3$ | $R_4$ | $X_1$ | m.p.(°) |
|---|---|---|---|---|---|---|---|---|
| O | $SO_2$ | H | H | H | H | H | $CH_3$ | |
| O | $SO_2$ | H | H | H | H | H | $OCH_3$ | |
| O | $SO_2$ | H | H | H | H | H | $OCH_2CH_3$ | |
| O | $SO_2$ | H | H | H | H | $CH_3$ | $OCH_3$ | |
| O | O | H | H | H | $CH_3$ | H | $OCH_3$ | |
| O | S | H | H | $CH_3$ | H | H | $OCH_3$ | |
| O | $SO_2$ | H | H | H | $CH_3$ | H | $OCH_3$ | |
| O | S | H | $CH_3$ | $CH_3$ | H | H | $OCH_3$ | |
| O | $SO_2$ | H | $OCH_3$ | $CH_3$ | H | H | $OCH_3$ | |
| O | S | H | Cl | $CH_3$ | H | H | $OCH_3$ | |
| O | $SO_2$ | H | Br | $CH_3$ | H | H | $OCH_3$ | |
| O | S | H | $CO_2CH_3$ | $CH_3$ | H | H | $OCH_3$ | |
| O | $SO_2$ | H | $SO_2CH_3$ | $CH_3$ | H | H | $OCH_3$ | |
| O | S | H | $OSO_2CH_3$ | $CH_3$ | H | H | $OCH_3$ | |
| O | $SO_2$ | H | $SO_2N(CH_3)_2$ | $CH_3$ | H | H | $OCH_3$ | |
| 1 | $SO_2$ | H | H | H | H | H | $OCH_3$ | |

TABLE VId

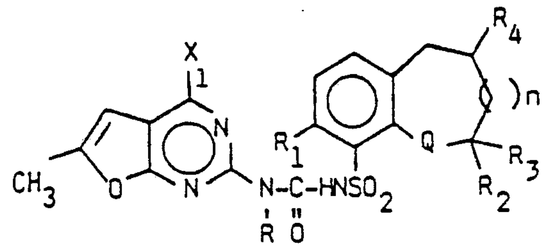

| n | Q | R | $R_1$ | $R_2$ | $R_3$ | $R_4$ | $X_1$ | m.p.(°) |
|---|---|---|---|---|---|---|---|---|
| 0 | $SO_2$ | H | H | H | H | H | $CH_3$ | |
| 0 | $SO_2$ | H | H | H | H | H | $OCH_3$ | |
| 0 | $SO_2$ | H | H | H | H | H | $OCH_2CH_3$ | |
| 0 | $SO_2$ | H. | H | H | H | $CH_3$ | $OCH_3$ | |
| 0 | O | H | H | H | $CH_3$ | H | $OCH_3$ | |
| 0 | S | H | H | $CH_3$ | H | H | $OCH_3$ | |
| 0 | $SO_2$ | H | H | H | $CH_3$ | H | $OCH_3$ | |
| 0 | S | H | $CH_3$ | $CH_3$ | H | H | $OCH_3$ | |
| 0 | $SO_2$ | H | $OCH_3$ | $CH_3$ | H | H | $OCH_3$ | |
| 0 | S | H | Cl | $CH_3$ | H | H | $OCH_3$ | |
| 0 | $SO_2$ | H | Br | $CH_3$ | H | H | $OCH_3$ | |
| 0 | S | H | $CO_2CH_3$ | $CH_3$ | H | H | $OCH_3$ | |
| 0 | $SO_2$ | H | $SO_2CH_3$ | $CH_3$ | H | H | $OCH_3$ | |
| 0 | S | H | $OSO_2CH_3$ | $CH_3$ | H | H | $OCH_3$ | |
| 0 | $SO_2$ | H | $SO_2N(CH_3)_2$ | $CH_3$ | H | H | $OCH_3$ | |
| 1 | $SO_2$ | H | H | H | H | H | $OCH_3$ | |

TABLE VIIa

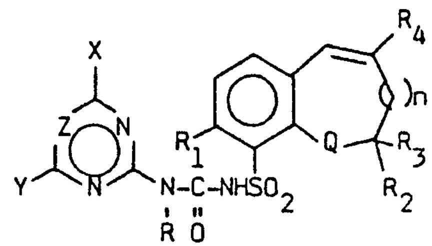

| n | Q | R | $R_1$ | $R_2$ | $R_3$ | $R_4$ | X | Y | Z | m.p.(°) |
|---|---|---|---|---|---|---|---|---|---|---|
| O | O | H | H | H | H | H | $CH_3$ | $CH_3$ | CH | |
| O | O | H | H | H | H | H | $CH_3$ | $OCH_3$ | CH | |
| O | O | H | H | H | H | H | $OCH_3$ | $OCH_3$ | CH | |
| O | O | H | H | H | H | H | $CH_3$ | $CH_3$ | N | |
| O | O | H | H | H | H | H | $CH_3$ | $OCH_3$ | N | |
| O | O | H | H | H | H | H | $OCH_3$ | $OCH_3$ | N | |
| O | S | H | H | H | H | H | $CH_3$ | $CH_3$ | CH | |
| O | S | H | H | H | H | H | $CH_3$ | $OCH_3$ | CH | |
| O | S | H | H | H | H | H | $OCH_3$ | $OCH_3$ | CH | |
| O | S | H | H | H | H | H | $CH_3$ | $CH_3$ | N | |
| O | S | H | H | H | H | H | $CH_3$ | $OCH_3$ | N | |
| O | S | H | H | H | H | H | $OCH_3$ | $OCH_3$ | N | |
| O | $SO_2$ | H | H | H | H | H | $CH_3$ | $CH_3$ | CH | |
| O | $SO_2$ | H | H | H | H | H | $CH_3$ | $OCH_3$ | CH | |
| O | $SO_2$ | H | H | H | H | H | $OCH_3$ | $OCH_3$ | CH | |
| O | $SO_2$ | H | H | H | H | H | $CH_3$ | $CH_3$ | N | |

TABLE VIIa (continued)

| n | Q | R | $R_1$ | $R_2$ | $R_3$ | $R_4$ | X | Y | Z | m.p.(°) |
|---|---|---|---|---|---|---|---|---|---|---|
| O | $SO_2$ | H | H | H | H | H | $CH_3$ | $OCH_3$ | N | |
| O | $SO_2$ | H | H | H | H | H | $OCH_3$ | $OCH_3$ | N | |
| O | O | H | H | $CH_3$ | H | H | $CH_3$ | $CH_3$ | CH | |
| O | O | H | H | $CH_3$ | H | H | $CH_3$ | $OCH_3$ | CH | |
| O | O | H | H | $CH_3$ | H | H | $OCH_3$ | $OCH_3$ | CH | |
| O | O | H | H | $CH_3$ | H | H | $CH_3$ | $CH_3$ | N | |
| O | O | H | H | $CH_3$ | H | H | $CH_3$ | $OCH_3$ | N | |
| O | O | H | H | $CH_3$ | H | H | $OCH_3$ | $OCH_3$ | N | |
| O | S | H | H | $CH_3$ | H | H | $CH_3$ | $CH_3$ | CH | |
| O | S | H | H | $CH_3$ | H | H | $CH_3$ | $OCH_3$ | CH | |
| O | S | H | H | $CH_3$ | H | H | $OCH_3$ | $OCH_3$ | CH | |
| O | S | H | H | $CH_3$ | H | H | $CH_3$ | $CH_3$ | N | |
| O | S | H | H | $CH_3$ | H | H | $CH_3$ | $OCH_3$ | N | |
| O | S | H | H | $CH_3$ | H | H | $OCH_3$ | $OCH_3$ | N | |
| O | $SO_2$ | H | H | $CH_3$ | H | H | $CH_3$ | $CH_3$ | CH | |
| O | $SO_2$ | H | H | $CH_3$ | H | H | $CH_3$ | $OCH_3$ | CH | |
| O | $SO_2$ | H | H | $CH_3$ | H | H | $OCH_3$ | $OCH_3$ | CH | |
| O | $SO_2$ | H | H | $CH_3$ | H | H | $CH_3$ | $CH_3$ | N | |
| O | $SO_2$ | H | H | $CH_3$ | H | H | $CH_3$ | $OCH_3$ | N | |
| O | $SO_2$ | H | H | $CH_3$ | H | H | $OCH_3$ | $OCH_3$ | N | |

TABLE VIIa (continued)

| n | Q | R | $R_1$ | $R_2$ | $R_3$ | $R_4$ | X | Y | Z | m.p.(°) |
|---|---|---|---|---|---|---|---|---|---|---|
| 0 | O | H | H | $CH_3$ | $CH_3$ | H | $CH_3$ | $CH_3$ | CH | |
| 0 | O | H | H | $CH_3$ | $CH_3$ | H | $CH_3$ | $OCH_3$ | CH | |
| 0 | O | H | H | $CH_3$ | $CH_3$ | H | $OCH_3$ | $OCH_3$ | CH | |
| 0 | O | H | H | $CH_3$ | $CH_3$ | H | $CH_3$ | $CH_3$ | N | |
| 0 | O | H | H | $CH_3$ | $CH_3$ | H | $CH_3$ | $OCH_3$ | N | |
| 0 | O | H | H | $CH_3$ | $CH_3$ | H | $OCH_3$ | $OCH_3$ | N | |
| 0 | S | H | H | $CH_3$ | $CH_3$ | H | $CH_3$ | $CH_3$ | CH | |
| 0 | S | H | H | $CH_3$ | $CH_3$ | H | $CH_3$ | $OCH_3$ | CH | |
| 0 | S | H | H | $CH_3$ | $CH_3$ | H | $OCH_3$ | $OCH_3$ | CH | |
| 0 | S | H | H | $CH_3$ | $CH_3$ | H | $CH_3$ | $CH_3$ | N | |
| 0 | S | H | H | $CH_3$ | $CH_3$ | H | $CH_3$ | $OCH_3$ | N | |
| 0 | S | H | H | $CH_3$ | $CH_3$ | H | $OCH_3$ | $OCH_3$ | N | |
| 0 | $SO_2$ | H | H | $CH_3$ | $CH_3$ | H | $CH_3$ | $CH_3$ | CH | |
| 0 | $SO_2$ | H | H | $CH_3$ | $CH_3$ | H | $CH_3$ | $OCH_3$ | CH | |
| 0 | $SO_2$ | H | H | $CH_3$ | $CH_3$ | H | $OCH_3$ | $OCH_3$ | CH | |
| 0 | $SO_2$ | H | H | $CH_3$ | $CH_3$ | H | $CH_3$ | $CH_3$ | N | |
| 0 | $SO_2$ | H | H | $CH_3$ | $CH_3$ | H | $CH_3$ | $OCH_3$ | N | |
| 0 | $SO_2$ | H | H | $CH_3$ | $CH_3$ | H | $OCH_3$ | $OCH_3$ | N | |
| 1 | O | H | H | H | H | H | $CH_3$ | $CH_3$ | CH | |
| 1 | O | H | H | H | H | H | $CH_3$ | $OCH_3$ | CH | |
| 1 | O | H | H | H | H | H | $OCH_3$ | $OCH_3$ | CH | |

TABLE VIIa (continued)

| n | Q | R | $R_1$ | $R_2$ | $R_3$ | $R_4$ | X | Y | Z | m.p.(°) |
|---|---|---|---|---|---|---|---|---|---|---|
| 1 | O | H | H | H | H | H | $CH_3$ | $CH_3$ | N | |
| 1 | O | H | H | H | H | H | $CH_3$ | $OCH_3$ | N | |
| 1 | O | H | H | H | H | H | $OCH_3$ | $OCH_3$ | N | |
| 1 | S | H | H | H | H | H | $CH_3$ | $CH_3$ | CH | |
| 1 | S | H | H | H | H | H | $CH_3$ | $OCH_3$ | CH | |
| 1 | S | H | H | H | H | H | $OCH_3$ | $OCH_3$ | CH | |
| 1 | S | H | H | H | H | H | $CH_3$ | $CH_3$ | N | |
| 1 | S | H | H | H | H | H | $CH_3$ | $OCH_3$ | N | |
| 1 | S | H | H | H | H | H | $OCH_3$ | $OCH_3$ | N | |
| 1 | $SO_2$ | H | H | H | H | H | $CH_3$ | $CH_3$ | CH | |
| 1 | $SO_2$ | H | H | H | H | H | $CH_3$ | $OCH_3$ | CH | |
| 1 | $SO_2$ | H | H | H | H | H | $OCH_3$ | $OCH_3$ | CH | |
| 1 | $SO_2$ | H | H | H | H | H | $CH_3$ | $CH_3$ | N | |
| 1 | $SO_2$ | H | H | H | H | H | $CH_3$ | $OCH_3$ | N | |
| 1 | $SO_2$ | H | H | H | H | H | $OCH_3$ | $OCH_3$ | N | |
| 1 | O | H | H | $CH_3$ | H | H | $CH_3$ | $CH_3$ | CH | |
| 1 | O | H | H | $CH_3$ | H | H | $CH_3$ | $OCH_3$ | CH | |
| 1 | O | H | H | $CH_3$ | H | H | $OCH_3$ | $OCH_3$ | CH | |
| 1 | O | H | H | $CH_3$ | H | H | $CH_3$ | $CH_3$ | N | |
| 1 | O | H | H | $CH_3$ | H | H | $CH_3$ | $OCH_3$ | N | |

TABLE VIIa (continued)

| n | Q | R | $R_1$ | $R_2$ | $R_3$ | $R_4$ | X | Y | Z | m.p.(°) |
|---|---|---|---|---|---|---|---|---|---|---|
| 1 | O | H | H | $CH_3$ | H | H | $OCH_3$ | $OCH_3$ | N | |
| 1 | S | H | H | $CH_3$ | H | H | $CH_3$ | $CH_3$ | CH | |
| 1 | S | H | H | $CH_3$ | H | H | $CH_3$ | $OCH_3$ | CH | |
| 1 | S | H | H | $CH_3$ | H | H | $OCH_3$ | $OCH_3$ | CH | |
| 1 | S | H | H | $CH_3$ | H | H | $CH_3$ | $CH_3$ | N | |
| 1 | S | H | H | $CH_3$ | H | H | $CH_3$ | $OCH_3$ | N | |
| 1 | S | H | H | $CH_3$ | H | H | $OCH_3$ | $OCH_3$ | N | |
| 1 | $SO_2$ | H | H | $CH_3$ | H | H | $CH_3$ | $CH_3$ | CH | |
| 1 | $SO_2$ | H | H | $CH_3$ | H | H | $CH_3$ | $OCH_3$ | CH | |
| 1 | $SO_2$ | H | H | $CH_3$ | H | H | $OCH_3$ | $OCH_3$ | CH | |
| 1 | $SO_2$ | H | H | $CH_3$ | H | H | $CH_3$ | $CH_3$ | N | |
| 1 | $SO_2$ | H | H | $CH_3$ | H | H | $CH_3$ | $OCH_3$ | N | |
| 1 | $SO_2$ | H | H | $CH_3$ | H | H | $OCH_3$ | $OCH_3$ | N | |
| 1 | O | H | H | $CH_3$ | $CH_3$ | H | $CH_3$ | $CH_3$ | CH | |
| 1 | O | H | H | $CH_3$ | $CH_3$ | H | $CH_3$ | $OCH_3$ | CH | |
| 1 | O | H | H | $CH_3$ | $CH_3$ | H | $OCH_3$ | $OCH_3$ | CH | |
| 1 | O | H | H | $CH_3$ | $CH_3$ | H | $CH_3$ | $CH_3$ | N | |
| 1 | O | H | H | $CH_3$ | $CH_3$ | H | $CH_3$ | $OCH_3$ | N | |
| 1 | O | H | H | $CH_3$ | $CH_3$ | H | $OCH_3$ | $OCH_3$ | N | |
| 1 | S | H | H | $CH_3$ | $CH_3$ | H | $CH_3$ | $CH_3$ | CH | |

TABLE VIIa (continued)

| n | Q | R | $R_1$ | $R_2$ | $R_3$ | $R_4$ | X | Y | Z | m.p.(°) |
|---|---|---|---|---|---|---|---|---|---|---|
| 1 | S | H | H | $CH_3$ | $CH_3$ | H | $CH_3$ | $OCH_3$ | CH | |
| 1 | S | H | H | $CH_3$ | $CH_3$ | H | $OCH_3$ | $OCH_3$ | CH | |
| 1 | S | H | H | $CH_3$ | $CH_3$ | H | $CH_3$ | $CH_3$ | N | |
| 1 | S | H | H | $CH_3$ | $CH_3$ | H | $CH_3$ | $OCH_3$ | N | |
| 1 | S | H | H | $CH_3$ | $CH_3$ | H | $OCH_3$ | $OCH_3$ | N | |
| 1 | $SO_2$ | H | H | $CH_3$ | $CH_3$ | H | $CH_3$ | $CH_3$ | CH | |
| 1 | $SO_2$ | H | H | $CH_3$ | $CH_3$ | H | $CH_3$ | $OCH_3$ | CH | |
| 1 | $SO_2$ | H | H | $CH_3$ | $CH_3$ | H | $OCH_3$ | $OCH_3$ | CH | |
| 1 | $SO_2$ | H | H | $CH_3$ | $CH_3$ | H | $CH_3$ | $CH_3$ | N | |
| 1 | $SO_2$ | H | H | $CH_3$ | $CH_3$ | H | $CH_3$ | $OCH_3$ | N | |
| 1 | $SO_2$ | H | H | $CH_3$ | $CH_3$ | H | $OCH_3$ | $OCH_3$ | N | |
| 0 | $SO_2$ | $CH_3$ | H | $CH_3$ | H | H | $OCH_3$ | $OCH_3$ | N | |
| 0 | $SO_2$ | H | H | H | H | $CH_3$ | $CH_3$ | $CH_3$ | CH | |
| 0 | $SO_2$ | H | H | H | H | $CH_3$ | $CH_3$ | $OCH_3$ | CH | |
| 0 | $SO_2$ | H | H | H | H | $CH_3$ | $OCH_3$ | $OCH_3$ | CH | |
| 0 | $SO_2$ | H | H | H | H | $CH_3$ | $CH_3$ | $CH_3$ | N | |
| 0 | $SO_2$ | H | H | H | H | $CH_3$ | $CH_3$ | $OCH_3$ | N | |
| 0 | $SO_2$ | H | H | H | H | $CH_3$ | $OCH_3$ | $OCH_3$ | N | |
| 0 | O | H | $OCH_3$ | $CH_3$ | $CH_3$ | H | $CH_3$ | $CH_3$ | CH | |
| 0 | O | H | $OCH_3$ | $CH_3$ | $CH_3$ | H | $CH_3$ | $OCH_3$ | CH | |
| 0 | O | H | $OCH_3$ | $CH_3$ | $CH_3$ | H | $OCH_3$ | $OCH_3$ | CH | |

TABLE VIIa (continued)

| n | Q | R | $R_1$ | $R_2$ | $R_3$ | $R_4$ | X | Y | Z | m.p.(°) |
|---|---|---|---|---|---|---|---|---|---|---|
| O | O | H | $OCH_3$ | $CH_3$ | $CH_3$ | H | $CH_3$ | $CH_3$ | N | |
| O | O | H | $OCH_3$ | $CH_3$ | $CH_3$ | H | $CH_3$ | $OCH_3$ | N | |
| O | O | H | $OCH_3$ | $CH_3$ | $CH_3$ | H | $OCH_3$ | $OCH_3$ | N | |
| O | O | H | $CH_3$ | H | H | H | $OCH_3$ | $OCH_3$ | CH | |
| O | S | H | Cl | $CH_3$ | H | H | $OCH_3$ | $OCH_3$ | CH | |
| O | $SO_2$ | H | Br | H | $CH_3$ | H | $OCH_3$ | $OCH_3$ | CH | |
| O | O | H | $CO_2CH_3$ | $CH_3$ | H | H | $CH_3$ | $OCH_3$ | CH | |
| O | S | H | $CO_2CH_2CH_3$ | H | $CH_3$ | H | $OCH_3$ | $OCH_3$ | CH | |
| O | $SO_2$ | H | $CO_2CH_2CH_2CH_3$ | $CH_3$ | H | H | $CH_3$ | $OCH_3$ | N | |
| O | O | H | $CO_2CH(CH_3)_2$ | H | $CH_3$ | H | $OCH_3$ | $OCH_3$ | N | |
| O | S | H | $CO_2CH_2CH{=}CH_2$ | $CH_3$ | H | H | $CH_3$ | $OCH_3$ | CH | |
| O | $SO_2$ | H | $CO_2CH_2CH_2OCH_3$ | H | $CH_3$ | H | $OCH_3$ | $OCH_3$ | CH | |
| O | O | H | $CO_2CH_2CH_2Cl$ | $CH_3$ | H | H | $CH_3$ | $OCH_3$ | N | |
| O | S | H | $SO_2CH_3$ | H | $CH_3$ | H | $OCH_3$ | $OCH_3$ | N | |
| O | $SO_2$ | H | $SO_2CH_2CH_2CH_3$ | $CH_3$ | H | H | $CH_3$ | $OCH_3$ | CH | |
| O | O | H | $SO_2CH(CH_3)_2$ | H | $CH_3$ | H | $OCH_3$ | $OCH_3$ | CH | |
| O | S | H | $OSO_2CH_3$ | $CH_3$ | H | H | $CH_3$ | $OCH_3$ | N | |
| O | $SO_2$ | H | $OSO_2CF_3$ | H | $CH_3$ | H | $OCH_3$ | $OCH_3$ | N | |
| O | O | H | $OSO_2CH(CH_3)_2$ | H | $CH_3$ | H | $CH_3$ | $OCH_3$ | CH | |
| O | $SO_2$ | H | $SO_2N(CH_3)_2$ | $CH_3$ | H | H | $OCH_3$ | $OCH_3$ | CH | |

TABLE VIIa (continued)

| n | Q | R | $R_1$ | $R_2$ | $R_3$ | $R_4$ | X | Y | Z | m.p.(°) |
|---|---|---|---|---|---|---|---|---|---|---|
| O | $SO_2$ | H | $SO_2N(CH_2CH_3)_2$ | H | $CH_3$ | H | $CH_3$ | $OCH_3$ | N | |
| O | O | H | $SO_2N(CH_3)CH_2CH_3$ | $CH_3$ | H | H | $OCH_3$ | $OCH_3$ | N | |
| O | S | H | H | $CH_3$ | $CH_2CH_3$ | H | $CH_3$ | $OCH_3$ | CH | |
| O | $SO_2$ | H | H | H | $CH_2CH_2CH_3$ | H | $OCH_3$ | $OCH_3$ | CH | |
| O | O | H | H | H | $CH(CH_3)_2$ | H | $CH_3$ | $OCH_3$ | N | |
| O | S | H | H | $CH_2CH_3$ | H | H | $OCH_3$ | $OCH_3$ | N | |
| O | $SO_2$ | H | H | $CH_2CH_2CH_3$ | H | H | $CH_3$ | $OCH_3$ | CH | |
| O | O | H | H | $CH(CH_3)_2$ | H | H | $OCH_3$ | $OCH_3$ | CH | |
| O | S | H | H | H | $CH_3$ | H | Cl | $OCH_3$ | CH | |
| O | $SO_2$ | H | H | $CH_3$ | H | H | $CH_3$ | $NH_2$ | CH | |
| O | O | H | H | H | $CH_3$ | H | $CH_3$ | $NHCH_3$ | CH | |
| O | S | H | H | $CH_3$ | H | H | $CH_3$ | $N(CH_3)_2$ | CH | |
| O | $SO_2$ | H | H | H | $CH_3$ | H | $CH_3$ | $OC_2H_5$ | CH | |
| O | O | H | H | $CH_3$ | H | H | $CH_3$ | $OCH_2CH_3$ | N | |
| O | S | H | H | H | $CH_3$ | H | $OCH_3$ | $CH_2O_5CH_3$ | CH | |

TABLE VIIb

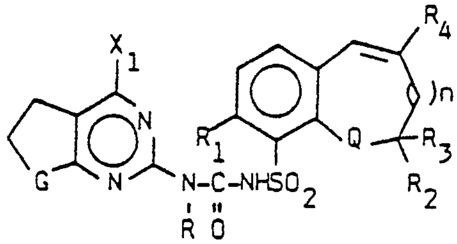

| n | Q | R | $R_1$ | $R_2$ | $R_3$ | $R_4$ | $X_1$ | G | m.p.(°) |
|---|---|---|---|---|---|---|---|---|---|
| O | $SO_2$ | H | H | H | H | H | $CH_3$ | $CH_2$ | |
| O | $SO_2$ | H | H | H | H | H | $OCH_3$ | $CH_2$ | |
| O | $SO_2$ | H | H | H | H | H | $OCH_2CH_3$ | $CH_2$ | |
| O | $SO_2$ | H | H | H | H | H | $CH_3$ | O | |
| O | $SO_2$ | H | H | H | H | H | $OCH_3$ | O | |
| O | $SO_2$ | H | H | H | H | H | $OCH_2CH_3$ | O | |
| O | $SO_2$ | H | H | H | H | $CH_3$ | $OCH_3$ | O | |
| O | O | H | H | H | $CH_3$ | H | $OCH_3$ | O | |
| O | S | H | H | $CH_3$ | H | H | $OCH_3$ | O | |
| O | $SO_2$ | H | H | H | $CH_3$ | H | $OCH_3$ | O | |
| O | S | H | $CH_3$ | $CH_3$ | H | H | $OCH_3$ | O | |
| O | $SO_2$ | H | $OCH_3$ | $CH_3$ | H | H | $OCH_3$ | O | |
| O | S | H | Cl | $CH_3$ | H | H | $OCH_3$ | O | |
| O | $SO_2$ | H | Br | $CH_3$ | H | H | $OCH_3$ | O | |
| O | S | H | $CO_2CH_3$ | $CH_3$ | H | H | $OCH_3$ | O | |
| O | $SO_2$ | H | $SO_2CH_3$ | $CH_3$ | H | H | $OCH_3$ | O | |
| O | S | H | $OSO_2CH_3$ | $CH_3$ | H | H | $OCH_3$ | O | |
| O | $SO_2$ | H | $SO_2N(CH_3)_2$ | $CH_3$ | H | H | $OCH_3$ | O | |
| 1 | $SO_2$ | H | H | H | H | H | $OCH_3$ | O | |

TABLE VIIc

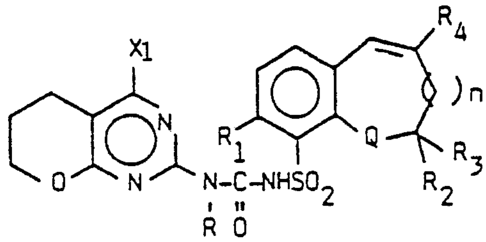

| n | Q | R | $R_1$ | $R_2$ | $R_3$ | $R_4$ | $X_1$ | m.p.(°) |
|---|---|---|---|---|---|---|---|---|
| O | $SO_2$ | H | H | H | H | H | $CH_3$ | |
| O | $SO_2$ | H | H | H | H | H | $OCH_3$ | |
| O | $SO_2$ | H | H | H | H | H | $OCH_3CH_3$ | |
| O | $SO_2$ | H | H | H | H | $CH_2OCH_3$ | | |
| O | O | H | H | H | $CH_3$ | H | $OCH_3$ | |
| O | S | H | H | $CH_3$ | H | H | $OCH_3$ | |
| O | $SO_2$ | H | H | H | $CH_3$ | H | $OCH_3$ | |
| O | S | H | $CH_3$ | $CH_3$ | H | H | $OCH_3$ | |
| O | $SO_2$ | H | $OCH_3$ | $CH_3$ | H | H | $OCH_3$ | |
| O | S | H | Cl | $CH_3$ | H | H | $OCH_3$ | |
| O | $SO_2$ | H | Br | $CH_3$ | H | H | $OCH_3$ | |
| O | S | H | $CO_2CH_3$ | $CH_3$ | H | H | $OCH_3$ | |
| O | $SO_2$ | H | $SO_2CH_3$ | $CH_3$ | H | H | $OCH_3$ | |
| O | S | H | $OSO_2CH_3$ | $CH_3$ | H | H | $OCH_3$ | |
| O | $SO_2$ | H | $SO_2N(CH_3)_2$ | $CH_3$ | H | H | $OCH_3$ | |
| 1 | $SO_2$ | H | H | H | H | H | $OCH_3$ | |

TABLE VIId

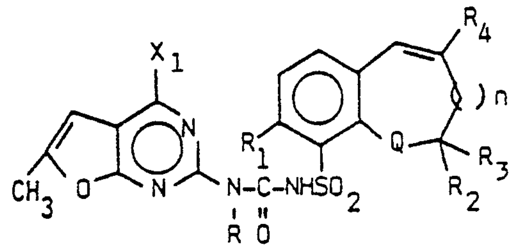

| n | Q | R | $R_1$ | $R_2$ | $R_3$ | $R_4$ | $X_1$ | m.p.(°) |
|---|---|---|---|---|---|---|---|---|
| O | $SO_2$ | H | H | H | H | H | $CH_3$ | |
| O | $SO_2$ | H | H | H | H | H | $OCH_3$ | |
| O | $SO_2$ | H | H | H | H | H | $OCH_2CH_3$ | |
| O | $SO_2$ | H | H | H | H | $CH_3$ | $OCH_3$ | |
| O | O | H | H | H | $CH_3$ | H | $OCH_3$ | |
| O | S | H | H | $CH_3$ | H | H | $OCH_3$ | |
| O | $SO_2$ | H | H | H | $CH_3$ | H | $OCH_3$ | |
| O | S | H | $CH_3$ | $CH_3$ | H | H | $OCH_3$ | |
| O | $SO_2$ | H | $OCH_3$ | $CH_3$ | H | H | $OCH_3$ | |
| O | S | H | Cl | $CH_3$ | H | H | $OCH_3$ | |
| O | $SO_2$ | H | Br | $CH_3$ | H | H | $OCH_3$ | |
| O | S | H | $CO_2CH_3$ | $CH_3$ | H | H | $OCH_3$ | |
| O | $SO_2$ | H | $SO_2CH_3$ | $CH_3$ | H | H | $OCH_3$ | |
| O | S | H | $OSO_2CH_3$ | $CH_3$ | H | H | $OCH_3$ | |
| O | $SO_2$ | H | $SO_2N(CH_3)_2$ | $CH_3$ | H | H | $OCH_3$ | |
| 1 | $SO_2$ | H | H | H | H | H | $OCH_3$ | |

TABLE VIIIa

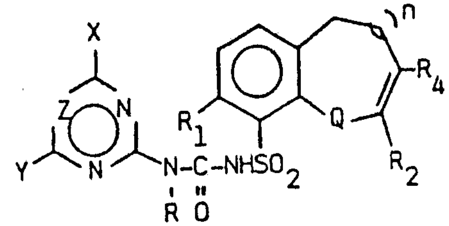

| n | Q | R | $R_1$ | $R_2$ | $R_4$ | X | Y | Z | m.p.(°) |
|---|---|---|---|---|---|---|---|---|---|
| O | S | H | H | H | H | $CH_3$ | $CH_3$ | CH | |
| O | S | H | H | H | H | $CH_3$ | $OCH_3$ | CH | |
| O | S | H | H | H | H | $OCH_3$ | $OCH_3$ | CH | |
| O | S | H | H | H | H | $CH_3$ | $CH_3$ | N | |
| O | S | H | H | H | H | $CH_3$ | $OCH_3$ | N | |
| O | S | H | H | H | H | $OCH_3$ | $OCH_3$ | N | |
| O | $SO_2$ | H | H | H | H | $CH_3$ | $CH_3$ | CH | |
| O | $SO_2$ | H | H | H | H | $CH_3$ | $OCH_3$ | CH | |
| O | $SO_2$ | H | H | H | H | $OCH_3$ | $OCH_3$ | CH | |
| O | $SO_2$ | H | H | H | H | $CH_3$ | $CH_3$ | N | |
| O | $SO_2$ | H | H | H | H | $CH_3$ | $OCH_3$ | N | |
| O | $SO_2$ | H | H | H | H | $OCH_3$ | $OCH_3$ | N | |
| O | S | H | H | $CH_3$ | H | $CH_3$ | $CH_3$ | CH | |
| O | S | H | H | $CH_3$ | H | $CH_3$ | $OCH_3$ | CH | |
| O | S | H | H | $CH_3$ | H | $OCH_3$ | $OCH_3$ | CH | |
| O | S | H | H | $CH_3$ | H | $CH_3$ | $CH_3$ | N | |

TABLE VIIIa

| n | Q | R | $R_1$ | $R_2$ | $R_4$ | X | Y | Z | m.p.(°) |
|---|---|---|---|---|---|---|---|---|---|
| 0 | S | H | H | $CH_3$ | H | $CH_3$ | $OCH_3$ | N | |
| 0 | S | H | H | $CH_3$ | H | $OCH_3$ | $OCH_3$ | N | |
| 0 | $SO_2$ | H | H | $CH_3$ | H | $CH_3$ | $CH_3$ | CH | |
| 0 | $SO_2$ | H | H | $CH_3$ | H | $CH_3$ | $OCH_3$ | CH | |
| 0 | $SO_2$ | H | H | $CH_3$ | H | $OCH_3$ | $OCH_3$ | CH | |
| 0 | $SO_2$ | H | H | $CH_3$ | H | $CH_3$ | $CH_3$ | N | |
| 0 | $SO_2$ | H | H | $CH_3$ | H | $CH_3$ | $OCH_3$ | N | |
| 0 | $SO_2$ | H | H | $CH_3$ | H | $OCH_3$ | $OCH_3$ | N | |
| 1 | S | H | H | H | H | $CH_3$ | $CH_3$ | CH | |
| 1 | S | H | H | H | H | $CH_3$ | $OCH_3$ | CH | |
| 1 | S | H | H | H | H | $OCH_3$ | $OCH_3$ | CH | |
| 1 | S | H | H | H | H | $CH_3$ | $CH_3$ | N | |
| 1 | S | H | H | H | H | $CH_3$ | $OCH_3$ | N | |
| 1 | S | H | H | H | H | $OCH_3$ | $OCH_3$ | N | |
| 1 | $SO_2$ | H | H | H | H | $CH_3$ | $CH_3$ | CH | |
| 1 | $SO_2$ | H | H | H | H | $CH_3$ | $OCH_3$ | CH | |
| 1 | $SO_2$ | H | H | H | H | $OCH_3$ | $OCH_3$ | CH | |
| 1 | $SO_2$ | H | H | H | H | $CH_3$ | $CH_3$ | N | |
| 1 | $SO_2$ | H | H | H | H | $CH_3$ | $OCH_3$ | N | |
| 1 | $SO_2$ | H | H | H | H | $OCH_3$ | $OCH_3$ | N | |

TABLE VIIIa (continued)

| n | Q | R | $R_1$ | $R_2$ | $R_4$ | X | Y | Z | m.p.(°) |
|---|---|---|---|---|---|---|---|---|---|
| 1 | S | H | H | $CH_3$ | H | $CH_3$ | $CH_3$ | CH | |
| 1 | S | H | H | $CH_3$ | H | $CH_3$ | $OCH_3$ | CH | |
| 1 | S | H | H | $CH_3$ | H | $OCH_3$ | $OCH_3$ | CH | |
| 1 | S | H | H | $CH_3$ | H | $CH_3$ | $CH_3$ | N | |
| 1 | S | H | H | $CH_3$ | H | $CH_3$ | $OCH_3$ | N | |
| 1 | S | H | H | $CH_3$ | H | $OCH_3$ | $OCH_3$ | N | |
| 1 | $SO_2$ | H | H | $CH_3$ | H | $CH_3$ | $CH_3$ | CH | |
| 1 | $SO_2$ | H | H | $CH_3$ | H | $CH_3$ | $OCH_3$ | CH | |
| 1 | $SO_2$ | H | H | $CH_3$ | H | $OCH_3$ | $OCH_3$ | CH | |
| 1 | $SO_2$ | H | H | $CH_3$ | H | $CH_3$ | $CH_3$ | N | |
| 1 | $SO_2$ | H | H | $CH_3$ | H | $CH_3$ | $OCH_3$ | N | |
| 1 | $SO_2$ | H | H | $CH_3$ | H | $OCH_3$ | $OCH_3$ | N | |
| O | $SO_2$ | $CH_3$ | H | $CH_3$ | H | $OCH_3$ | $OCH_3$ | N | |
| O | S | H | $CH_3$ | H | H | $OCH_3$ | $OCH_3$ | CH | |
| O | S | H | Cl | $CH_3$ | H | $OCH_3$ | $OCH_3$ | CH | |
| O | $SO_2$ | H | Br | H | H | $OCH_3$ | $OCH_3$ | CH | |
| O | S | H | $CO_2CH_3$ | $CH_3$ | H | $CH_3$ | $OCH_3$ | CH | |
| O | S | H | $CO_2CH_2CH_3$ | H | H | $OCH_3$ | $OCH_3$ | CH | |
| O | $SO_2$ | H | $CO_2CH_2CH_2CH_3$ | $CH_3$ | H | $CH_3$ | $OCH_3$ | N | |
| O | S | H | $CO_2CH(CH_3)_2$ | H | H | $OCH_3$ | $OCH_3$ | N | |
| O | S | H | $CO_2CH_2CH{=}CH_2$ | $CH_3$ | H | $CH_3$ | $OCH_3$ | CH | |

TABLE VIIIa (continued)

| n | Q | R | $R_1$ | $R_2$ | $R_4$ | X | Y | Z | m.p.(°) |
|---|---|---|---|---|---|---|---|---|---|
| O | $SO_2$ | H | $CO_2CH_2CH_2OCH_3$ | H | H | $OCH_3$ | $OCH_3$ | CH | |
| O | S | H | $CO_2CH_2CH_2Cl$ | $CH_3$ | H | $CH_3$ | $OCH_3$ | N | |
| O | S | H | $SO_2CH_3$ | H | H | $OCH_3$ | $OCH_3$ | N | |
| O | $SO_2$ | H | $SO_2CH_2CH_2CH_3$ | $CH_3$ | H | $CH_3$ | $OCH_3$ | CH | |
| O | S | H | $SO_2CH(CH_3)_2$ | H | H | $OCH_3$ | $OCH_3$ | CH | |
| O | S | H | $OSO_2CH_3$ | $CH_3$ | H | $CH_3$ | $OCH_3$ | N | |
| O | $SO_2$ | H | $OSO_2CF_3$ | H | H | $OCH_3$ | $OCH_3$ | N | |
| O | S | H | $OSO_2CH(CH_3)_2$ | H | H | $CH_3$ | $OCH_3$ | CH | |
| O | $SO_2$ | H | $SO_2N(CH_3)_2$ | $CH_3$ | H | $OCH_3$ | $OCH_3$ | CH | |
| O | $SO_2$ | H | $SO_2N(CH_2CH_3)_2$ | H | H | $CH_3$ | $OCH_3$ | N | |
| O | $SO_2$ | H | $SO_2N(CH_3)CH_2CH_3$ | $CH_3$ | H | $OCH_3$ | $OCH_3$ | N | |
| O | S | H | H | $CH_2CH_3$ | H | $OCH_3$ | $OCH_3$ | N | |
| O | $SO_2$ | H | H | $CH_2CH_2CH_3$ | H | $CH_3$ | $OCH_3$ | CH | |
| O | S | H | H | $CH(CH_3)_2$ | H | $OCH_3$ | $OCH_3$ | CH | |
| O | S | H | H | H | H | Cl | $OCH_3$ | CH | |
| O | $SO_2$ | H | H | $CH_3$ | H | $CH_3$ | $NH_2$ | CH | |
| O | S | H | H | H | H | $CH_3$ | $NHCH_3$ | CH | |
| O | S | H | H | $CH_3$ | H | $CH_3$ | $N(CH_3)_2$ | CH | |
| O | $SO_2$ | H | H | H | H | $CH_3$ | $OC_2H_5$ | CH | |
| O | S | H | H | $CH_3$ | H | $CH_3$ | $OCH_2CH_3$ | N | |
| O | S | H | H | H | H | $OCH_3$ | $CH_2OCH_3$ | CH | |

TABLE VIIIb

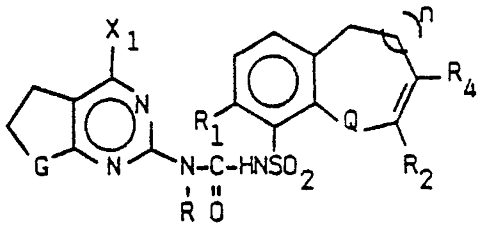

| n | Q | R | $R_1$ | $R_2$ | $R_4$ | $X_1$ | G | m.p.(°) |
|---|---|---|---|---|---|---|---|---|
| O | $SO_2$ | H | H | H | H | $CH_3$ | $CH_2$ | |
| O | $SO_2$ | H | H | H | H | $OCH_3$ | $CH_2$ | |
| O | $SO_2$ | H | H | H | H | $OCH_2CH_3$ | $CH_2$ | |
| O | $SO_2$ | H | H | H | H | $CH_3$ | O | |
| O | $SO_2$ | H | H | H | H | $OCH_3$ | O | |
| O | $SO_2$ | H | H | H | H | $OCH_2CH_3$ | O | |
| O | $SO_2$ | H | H | H | $CH_3$ | $OCH_3$ | O | |
| O | S | H | H | $CH_3$ | H | $OCH_3$ | O | |
| O | $SO_2$ | H | H | H | H | $OCH_3$ | O | |
| O | S | H | $CH_3$ | $CH_3$ | H | $OCH_3$ | O | |
| O | $SO_2$ | H | $OCH_3$ | $CH_3$ | H | $OCH_3$ | O | |
| O | S | H | Cl | $CH_3$ | H | $OCH_3$ | O | |
| O | $SO_2$ | H | Br | $CH_3$ | H | $OCH_3$ | O | |
| O | S | H | $CO_2CH_3$ | $CH_3$ | H | $OCH_3$ | O | |
| O | $SO_2$ | H | $SO_2CH_3$ | $CH_3$ | H | $OCH_3$ | O | |
| O | S | H | $OSO_2CH_3$ | $CH_3$ | H | $OCH_3$ | O | |
| O | $SO_2$ | H | $SO_2N(CH_3)_2$ | $CH_3$ | H | $OCH_3$ | O | |
| 1 | $SO_2$ | H | H | H | H | $OCH_3$ | O | |

TABLE VIIIc

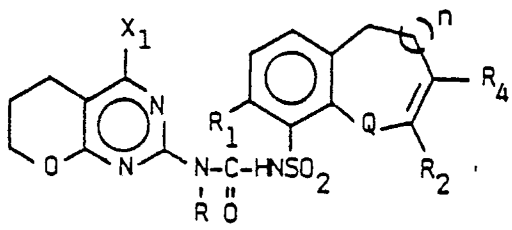

| n | Q | R | $R_1$ | $R_2$ | $R_4$ | $X_1$ | m.p.(°) |
|---|---|---|---|---|---|---|---|
| O | $SO_2$ | H | H | H | H | $CH_3$ | |
| O | $SO_2$ | H | H | H | H | $OCH_3$ | |
| O | $SO_2$ | H | H | H | H | $OCH_2CH_3$ | |
| O | $SO_2$ | H | H | H | $CH_3$ | $OCH_3$ | |
| O | S | H | H | $CH_3$ | H | $OCH_3$ | |
| O | $SO_2$ | H | H | H | H | $OCH_3$ | |
| O | S | H | $CH_3$ | $CH_3$ | H | $OCH_3$ | |
| O | $SO_2$ | H | $OCH_3$ | $CH_3$ | H | $OCH_3$ | |
| O | S | H | Cl | $CH_3$ | H | $OCH_3$ | |
| O | $SO_2$ | H | Br | $CH_3$ | H | $OCH_3$ | |
| O | S | H | $CO_2CH_3$ | $CH_3$ | H | $OCH_3$ | |
| O | $SO_2$ | H | $SO_2CH_3$ | $CH_3$ | H | $OCH_3$ | |
| O | S | H | $OSO_2CH_3$ | $CH_3$ | H | $OCH_3$ | |
| O | $SO_2$ | H | $SO_2N(CH_3)_2$ | $CH_3$ | H | $OCH_3$ | |
| 1 | $SO_2$ | H | H | H | H | $OCH_3$ | |

TABLE VIIId

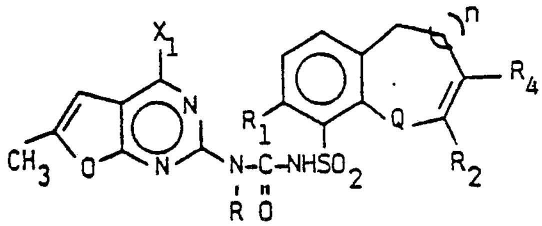

| n | Q | R | $R_1$ | $R_2$ | $R_4$ | $X_1$ | m.p.(°) |
|---|---|---|---|---|---|---|---|
| O | $SO_2$ | H | H | H | H | $CH_3$ | |
| O | $SO_2$ | H | H | H | H | $OCH_3$ | |
| O | $SO_2$ | H | H | H | H | $OCH_2CH_3$ | |
| O | $SO_2$ | H | H | H | $CH_3$ | $OCH_3$ | |
| O | S | H | H | $CH_3$ | H | $OCH_3$ | |
| O | $SO_2$ | H | H | H | H | $OCH_3$ | |
| O | S | H | $CH_3$ | $CH_3$ | H | $OCH_3$ | |
| O | $SO_2$ | H | $OCH_3$ | $CH_3$ | H | $OCH_3$ | |
| O | S | H | C | $CH_3$ | H | $OCH_3$ | |
| O | $SO_2$ | H | Br | $CH_3$ | H | $OCH_3$ | |
| O | S | H | $CO_2CH_3$ | $CH_3$ | H | $OCH_3$ | |
| O | $SO_2$ | H | $SO_2CH_3$ | $CH_3$ | H | $OCH_3$ | |
| O | S | H | $OSO_2CH_3$ | $CH_3$ | H | $OCH_3$ | |
| O | $SO_2$ | H | $SO_2N(CH_3)_2$ | $CH_3$ | H | $OCH_3$ | |
| 1 | $SO_2$ | H | H | H | H | $OCH_3$ | |

TABLE IXa

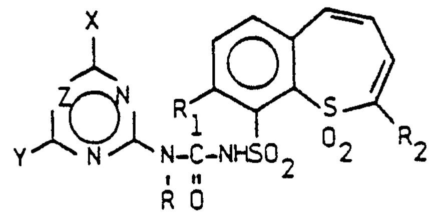

| R | $R_1$ | $R_2$ | X | Y | Z | m.p.(°) |
|---|---|---|---|---|---|---|
| H | H | H | $CH_3$ | $CH_3$ | CH | |
| H | H | H | $CH_3$ | $OCH_3$ | CH | |
| H | H | H | $OCH_3$ | $OCH_3$ | CH | |
| H | H | H | $CH_3$ | $CH_3$ | N | |
| H | H | H | $CH_3$ | $OCH_3$ | N | |
| H | H | H | $OCH_3$ | $OCH_3$ | N | |
| $CH_3$ | H | H | $OCH_3$ | $OCH_3$ | N | |
| H | H | $CH_3$ | $CH_3$ | $CH_3$ | CH | |
| H | H | $CH_3$ | $CH_3$ | $OCH_3$ | CH | |
| H | H | $CH_3$ | $OCH_3$ | $OCH_3$ | CH | |
| H | H | $CH_3$ | $CH_3$ | $CH_3$ | N | |
| H | H | $CH_3$ | $CH_3$ | $OCH_3$ | N | |
| H | H | $CH_3$ | $OCH_3$ | $OCH_3$ | N | |
| H | H | $CH_2CH_3$ | $CH_3$ | $OCH_3$ | CH | |
| H | H | $CH_2CH_2CH_3$ | $OCH_3$ | $OCH_3$ | CH | |
| H | H | $CH(CH_3)_2$ | $CH_3$ | $OCH_3$ | CH | |
| H | $OCH_3$ | H | $CH_3$ | $OCH_3$ | N | |
| H | Cl | H | $OCH_3$ | $OCH_3$ | N | |
| H | Br | H | $CH_3$ | $OCH_3$ | CH | |
| H | $CO_2CH_3$ | H | $OCH_3$ | $OCH_3$ | CH | |
| H | $SO_2CH_3$ | H | $CH_3$ | $OCH_3$ | N | |
| H | $OSO_2CH_3$ | H | $OCH_3$ | $OCH_3$ | N | |
| H | $SO_2N(CH_3)_2$ | H | $CH_3$ | $OCH_3$ | CH | |
| H | H | H | Cl | $OCH_3$ | CH | |
| H | H | H | $OCH_3$ | $OCH_2CH_3$ | CH | |
| H | H | H | $OCH_3$ | $CH_2OCH_3$ | CH | |

TABLE IXa (continued)

| R | $R_1$ | $R_2$ | X | Y | Z | m.p.(°) |
|---|---|---|---|---|---|---|
| H | H | H | $OCH_3$ | $NH_2$ | CH | |
| H | H | H | $OCH_3$ | $NHCH_3$ | CH | |
| H | H | H | $OCH_3$ | $N(CH_3)_2$ | CH | |

TABLE IXb

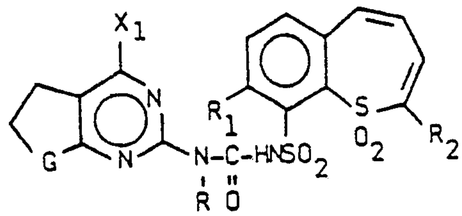

| R | $R_1$ | $R_2$ | $X_1$ | G | m.p.(°) |
|---|---|---|---|---|---|
| H | H | H | $CH_3$ | O | |
| H | H | H | $OCH_3$ | O | |
| H | H | H | $OCH_2CH_3$ | O | |
| H | H | $CH_3$ | $OCH_3$ | O | |
| $CH_3$ | H | H | $OCH_3$ | O | |
| H | $OCH_3$ | H | $OCH_3$ | O | |
| H | Cl | H | $OCH_3$ | O | |
| H | Br | H | $OCH_3$ | O | |
| H | $CO_2CH_3$ | H | $OCH_3$ | O | |
| H | $SO_2CH_3$ | H | $OCH_3$ | O | |
| H | $OSO_2CH_3$ | H | $OCH_3$ | O | |
| H | $SO_2N(CH_3)_2$ | H | $OCH_3$ | O | |
| H | H | H | $OCH_3$ | $CH_2$ | |

TABLE IXc

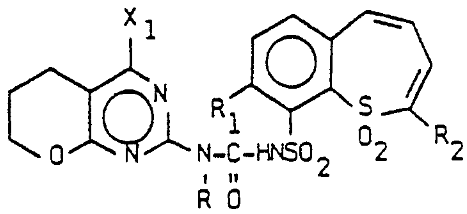

| R | $R_1$ | $R_2$ | $X_1$ | m.p.(°) |
|---|---|---|---|---|
| H | H | H | $CH_3$ | |
| H | H | H | $OCH_3$ | |
| H | H | H | $OCH_2CH_3$ | |
| H | H | $CH_3$ | $OCH_3$ | |
| $CH_3$ | H | H | $OCH_3$ | |
| H | $OCH_3$ | H | $OCH_3$ | |
| H | Cl | H | $OCH_3$ | |
| H | Br | H | $OCH_3$ | |
| H | $CO_2CH_3$ | H | $OCH_3$ | |
| H | $SO_2CH_3$ | H | $OCH_3$ | |
| H | $OSO_2CH_3$ | H | $OCH_3$ | |
| H | $SO_2N(CH_3)_2$ | H | $OCH_3$ | |

TABLE IXd

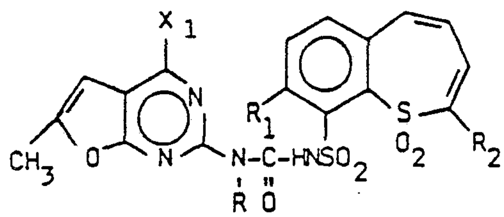

| R | $R_1$ | $R_2$ | $X_1$ | m.p.(°) |
|---|---|---|---|---|
| H | H | H | $CH_3$ | |
| H | H | H | $OCH_3$ | |
| H | H | H | $OCH_2CH_3$ | |
| H | H | $CH_3$ | $OCH_3$ | |
| $CH_3$ | H | H | $OCH_3$ | |
| H | $OCH_3$ | H | $OCH_3$ | |
| H | Cl | H | $OCH_3$ | |
| H | Br | H | $OCH_3$ | |
| H | $CO_2CH_3$ | H | $OCH_3$ | |
| H | $SO_2CH_3$ | H | $OCH_3$ | |
| H | $OSO_2CH_3$ | H | $OCH_3$ | |
| H | $SO_2N(CH_3)_2$ | H | $OCH_3$ | |

*Formulations*

Useful formulations of the compounds of Formulae I and I′ can be prepared in conventinal ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediate for further formulation. The formulations, broadly, contain about 0.1% to 99% by weight of active ingredient(s) and at least one of (a) about 0.1% to 20% surfactant(s) and (b) about 1% to 99.9% solid or liquid diluent(s). More specifically, they will contain these ingredients in the following approximate proportions:

TABLE X

| | Active Ingredient | Weight Percent* Diluent(s) | Surfactant(s) |
|---|---|---|---|
| Wettable Powders | 20—90 | 0—74 | 1—10 |
| Oil Suspensions, Emulsions, Solutions, (including Emulsifiable Concentrates) | 3—50 | 40—95 | 0—15 |
| Aqueous Suspension | 10—50 | 40—84 | 1—20 |
| Dusts | 1—25 | 70—99 | 0—5 |
| Granules and Pellets | 0.1—95 | 5—99.9 | 0—15 |
| High Strength Compositions | 90—99 | 0—10 | 0—2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Siseley and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060, 084). Granules and pellets may be made by spraying the active material upon preformed granular carriers or by agglomeration techniques. See J. E. Browning, "Agglomeration", *Chemical Engineering*, December 4, 1967, pp. 147ff. and "Perry's Chemical Engineer's Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182.

H. Gysin and E. Knusli, U.S. Patent 2,891,855, June 23, 1959, Col. 3, line 66 through Col. 5, line 17 and Examples 1—4;

G. C. Klingman, "Weed Control as a Science", John Wiley & Sons, Inc., New York, 1961, pp. 81—96; and

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example 28

*Wettable Powder*

| | |
|---|---|
| N-[(4,6-dimethylpyrimidin-2-yl)aminocarbonyl]-2,3-dihydro-2-methyl-7-benzothiophenesulfonamide, 1,1- dioxide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, re-blended, and packaged.

Example 29

*Oil Suspension*

| | |
|---|---|
| N-[(4-methoxy-6-methylpyrimidin-2-yl)aminocarbonyl]-2,3-dihydro-2-methyl-7-benzothiophenesulfonamide, 1,1-dioxide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 30

*Wettable Powder*

| | |
|---|---|
| N-[(4,6-dimethoxypyrimidin-2-yl)aminocarbonyl]-2,3-dihydro-2-methyl-7-benzothiophenesulfonamide, 1,1-dioxide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is re-blended and sifted through a U.S.S. No. 50 sieve (0.3 mm opening) and packaged.

Example 31

*Low Strength Granule*

| | |
|---|---|
| N-[(4,6-dimethyl-1,3,5-triazin-2-yl)aminocarbonyl]-2,3-dihydro-2-methyl-7-benzothiophenesulfonamide, 1,1-dioxide | 0.1% |
| attapulgite granules (U.S.S. 20—40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 32

*Granule*

| | |
|---|---|
| N-[(4-methoxy-6-methyl-1,3,5-triazin-2-yl)aminocarbonyl]-2,3-dihydro-2-methyl-7-benzofuransulfonamide 1,1-dioxide | 80% |
| wetting agent | 1% |
| crude ligninsulfonate salt (containing 5—20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1410—149 microns), and packaged for use.

Example 33

*High Strength Concentrate*

| | |
|---|---|
| N-[(4,6-dimethoxy-1,3,5-triazin-2-yl)aminocarbonyl]-2,3-dihydro-2-methyl-7-benzothiophenesulfonamide 1,1-dioxide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 34

*Wettable Powder*

| | |
|---|---|
| N-[(4,6-dimethoxypyrimidin-2-yl)aminocarbonyl]-3,4-dihydro-2H-1-benzothiopyran-8-sulfonamide, 1,1-dioxide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 35

*Granule*

| | |
|---|---|
| Wettable Powder of Example 34 | 5% |
| attapulgite granules (U.S.S. 20—40 mesh; 0.84—0.42 mm) | 95% |

A slurry of wettable powder containing ≈ 25% solids is sprayed on the surface of attapulgite granules in a double-cone blender. The granules are dried and packaged.

Example 36

*Extruded Pellet*

| | |
|---|---|
| N-[(4-methoxy-6-methyl-1,3,5-triazin-2-yl)aminocarbonyl]-3,4-dihydro-2H-1-benzothiopyran-8-sulfonamide, 1,1-dioxide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 37

*Aqueous Suspension*

| | |
|---|---|
| N-[(4-methoxy-6-methyl-1,3,5-triazin-2-yl)aminocarbonyl]-3,4-dihydro-2H-1-benzothiopyran-8-sulfonamide, 1,1-dioxide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 38

*Solution*

| | |
|---|---|
| N-[(4,6-dimethoxypyrimidin-2-yl)aminocarbonyl]-3,4-dihydro-2H-1-benzothiopyran-8-sulfonamide, 1,1-dioxide, sodium salt | 5% |
| water | 95% |

The salt is added directly to the water with stirring to produce the solution, which may then be packaged for use.

Example 39

*Wettable Powder*

| | |
|---|---|
| N-[(4,6-dimethoxypyrimidin-2-yl)aminocarbonyl]-3,4-dihydro-2H-1-benzothiopyran-8-sulfonamide, 1,1-dioxide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 40

*Wettable Powder*

| | |
|---|---|
| N-[(4-methoxy-6-methyl-1,3,5-triazin-2-yl)aminocarbonyl]-3,4-dihydro-2H-l-benzothiopyran-8-sulfonamide, 1,1-dioxide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 41

*Oil Suspension*

| | |
|---|---|
| N-[(4,6-dimethoxypyrimidin-2-yl)aminocarbonyl]-3,4-dihydro-2H-l-benzothiopyran-8-sulfonamide, 1,1-dioxide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 42

*Dust*

N-[(4-methoxy-6-methylpyrimidin-2-yl)aminocarbonyl]-3,4-dihydro-2H-l-benzothiopyran-8-sulfonamide,

| | |
|---|---|
| 1,1-dioxide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

*Utility*

The compounds of the present invention are active herbicides. They have utility for broad-spectrum pre- and/or post-emergence weed control in areas where complete control of all vegetation is desired, such as around fuel storage tanks, ammunition depots, industrial storage areas, parking lots, drive-in theaters, around billboards, highway and railroad structures. Alternatively, the compounds may be used to modify plant growth.

The rates of application for the compounds of the invention are determined by a number of factors, including their use as growth modifiers, the crop species involved, the types of weeds to be controlled, weather and climate, formulations selected, mode of application, amount of foliage present, etc. In general terms, the subject compounds should be applied at levels of around 0.05 to 10 kg/ha, the lower rates being suggested for use on lighter soils and/or those having a low organic matter content, for growth modification or for situations where only short-term persistence is required.

The compounds of the invention may be used in combination with any other commercial herbicide examples of which are those of the triazine, triazole, uracil, urea, amide, diphenylether, carbamate and bipyridylium types.

The herbicidal properties of the subject compounds were discovered in a number of greenhouse tests. The test procedures and results follow.

*Test A*

Seeds of crabgrass (*Digitaria* sp.), barnyardgrass (*Echinochloa crusgalli*), wild oats (*Avena fatua*), sicklepod (*Cassia obtusifolia*), morningglory (*Ipomoea* sp.), cocklebur (*Xanthium pensylvanicum*), sorghum, corn, soybean, sugar beet, rice, wheat and purple nutsedge (*Cyperus rotundus*) tubers were planted and treated pre-emergence with the chemicals dissolved in a non-phytotoxic solvent. At the same time, these crop and weed species, along with cotton and bush bean, were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

A = growth acceleration;
C = chlorosis/necrosis;
D = defoliation;
E = emergence inhibition;
G = growth retardation;
H = formative effects;
U = unusual pigmentation;
X = axillary stimulation; and
6Y = abscised buds or flowers.

It will be seen that the compounds have high pre- and post-emergence activity at the low rates of application selected for this evaluation. A few compounds, e.g., Compounds 17, 46 and 53, have low activity at the very low rates of application selected for this test. It is reasonable to assume that these compounds would exhibit higher activity at higher use rates.

Table A Structures

Compound 1

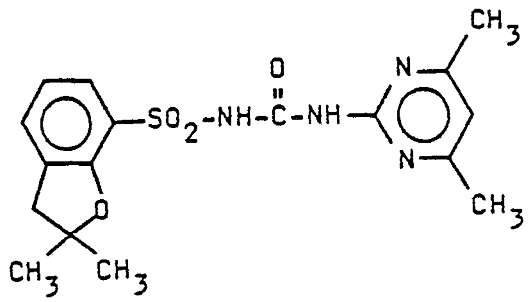

Compound 2

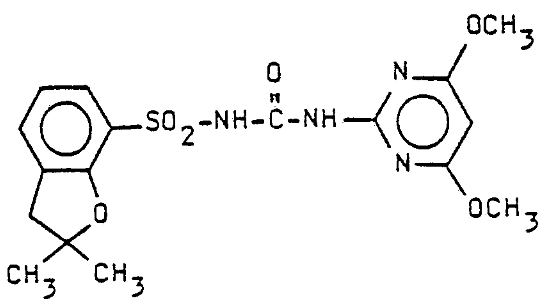

Compound 3

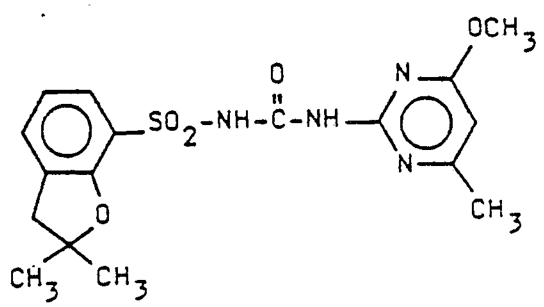

Compound 4

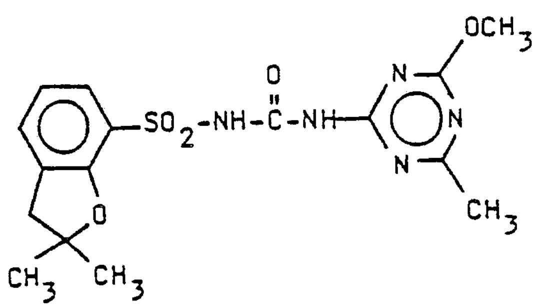

Table A Structures (Continued)

Compound 5

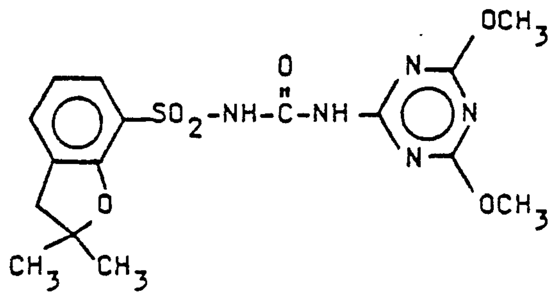

Compound 6

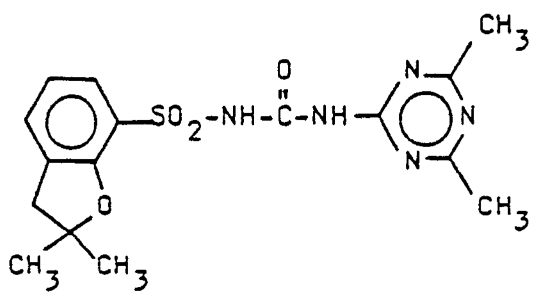

Compound 7

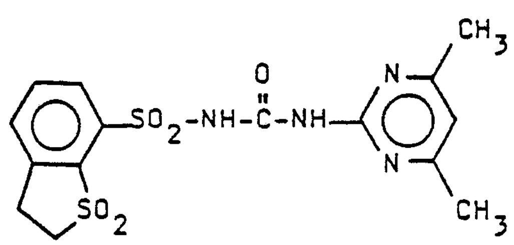

Compound 8

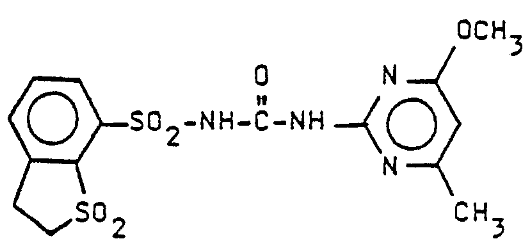

Table A Structures (Continued)

Compound 9

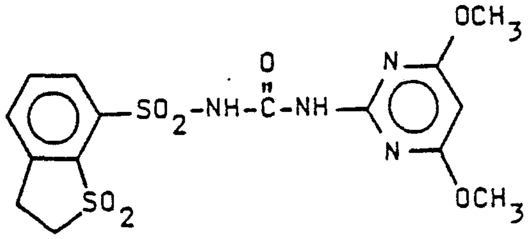

Compound 10

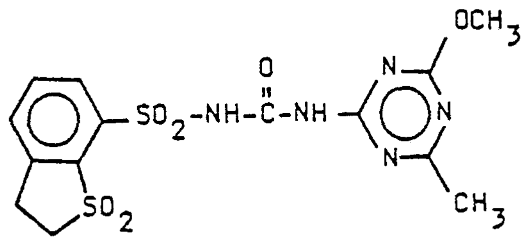

Compound 11

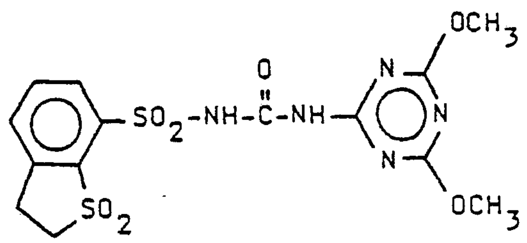

Compound 12

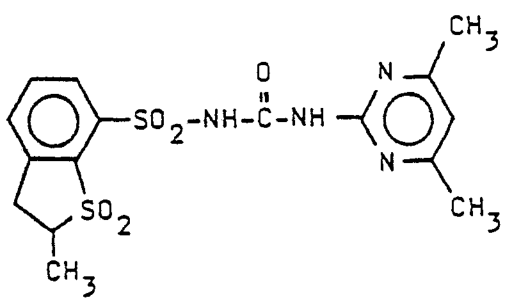

Table A Structures (Continued)

Compound 13

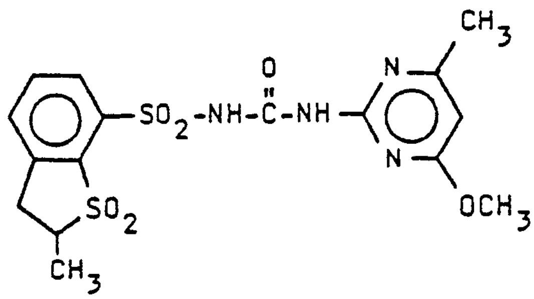

Compound 14

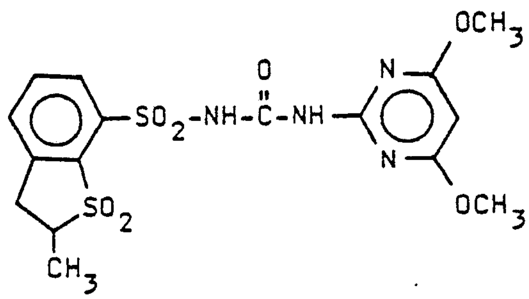

Compound 15

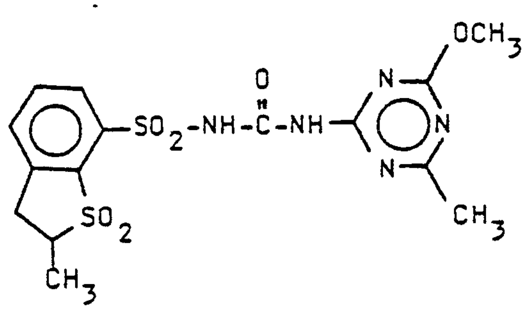

Compound 16

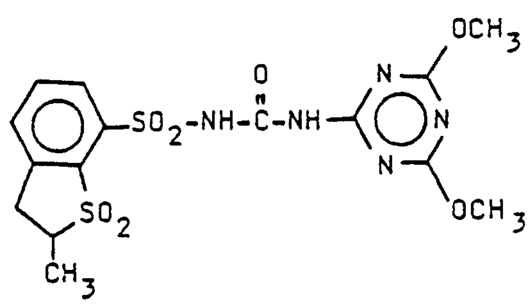

Table A Structures (Continued)

Compound 17

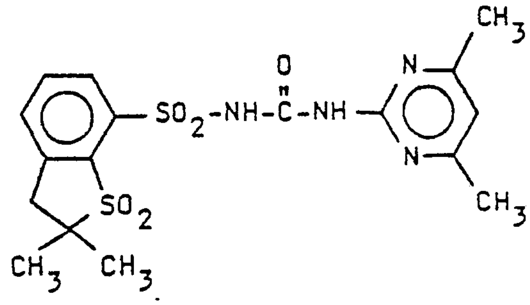

Compound 18

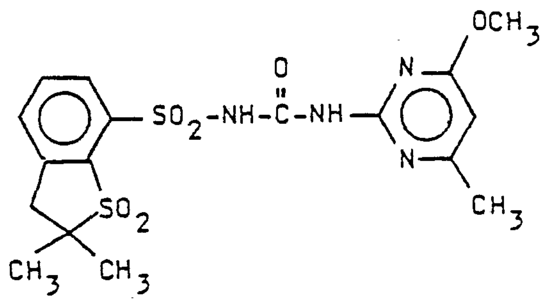

Compound 19

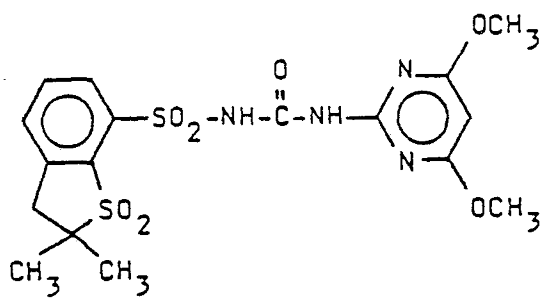

Compound 20

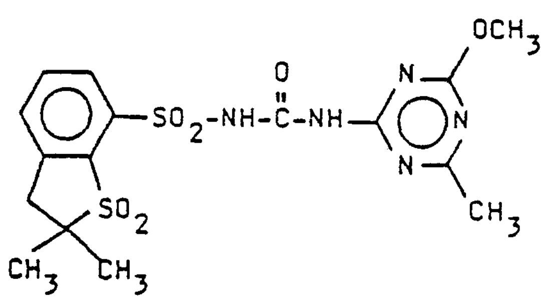

Table A Structures (Continued)

Compound 21

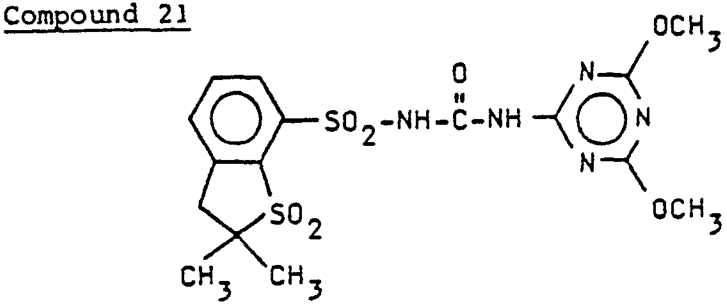

Compound 22

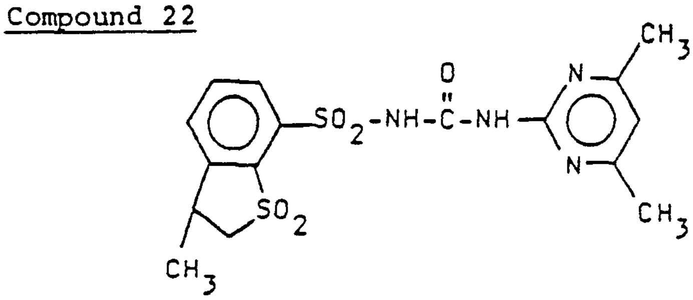

Compound 23

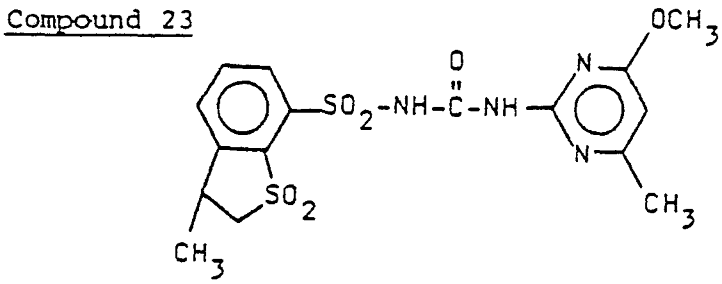

Compound 24

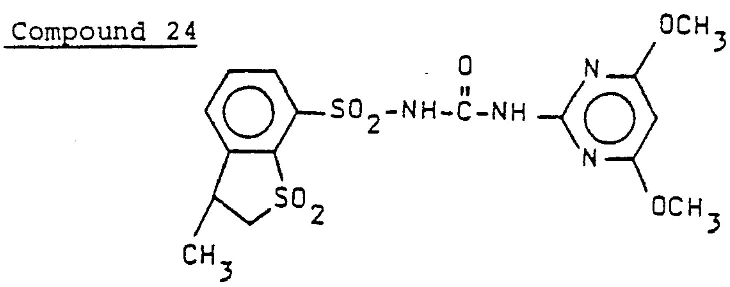

Compound 25

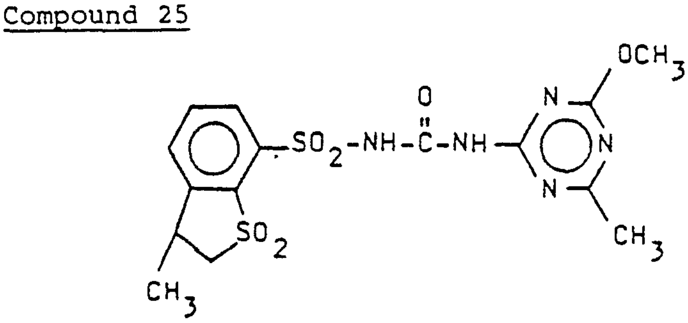

Table A Structures (Continued)

Compound 26

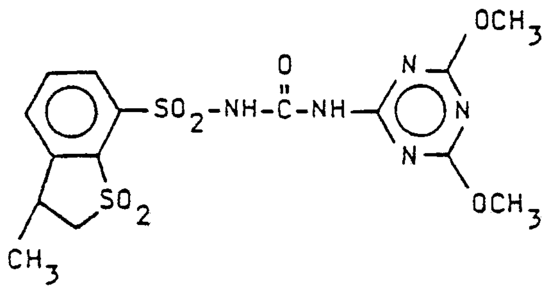

Compound 27

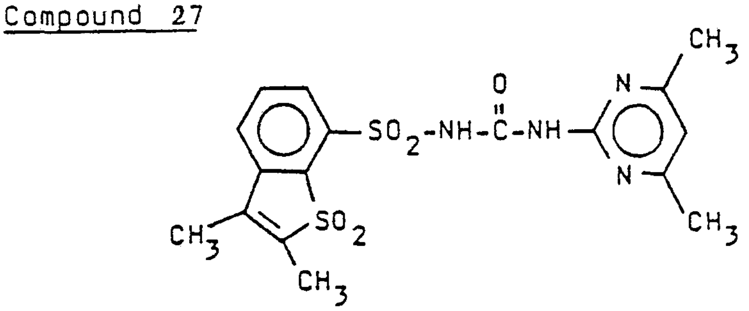

Compound 28

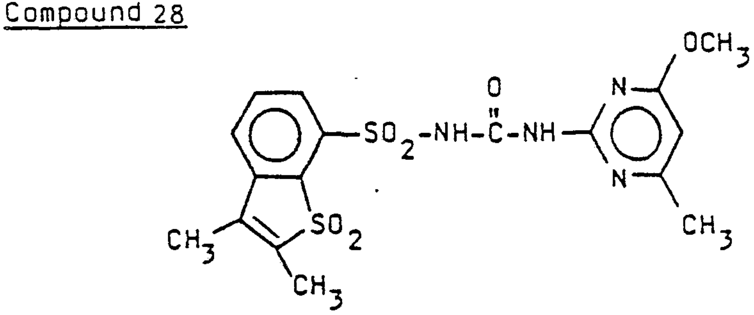

Compound 29

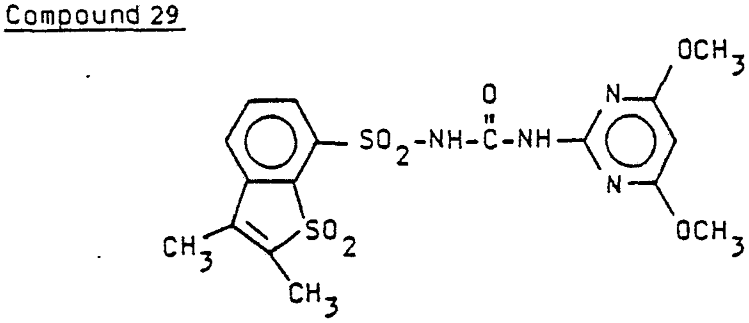

Table A Structures (Continued)

Compound 30

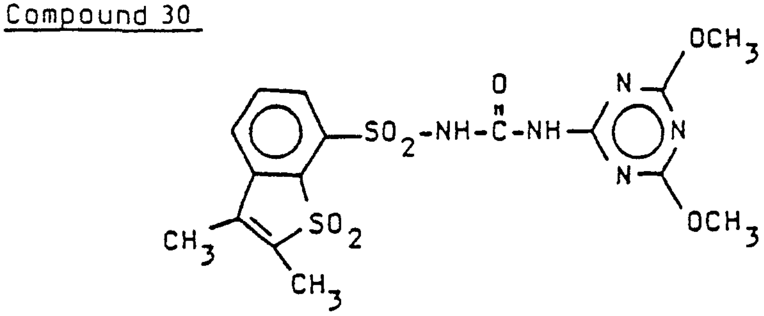

Compound 31

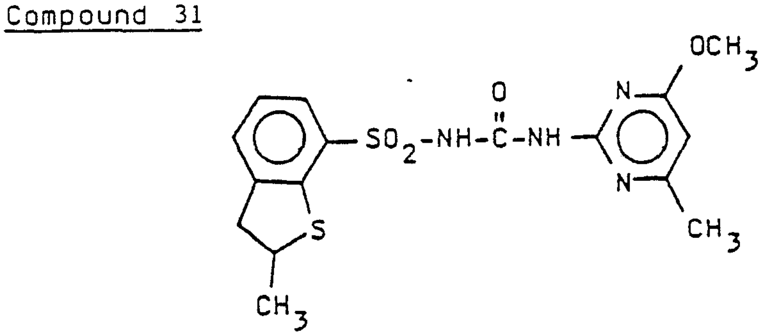

Compound 32

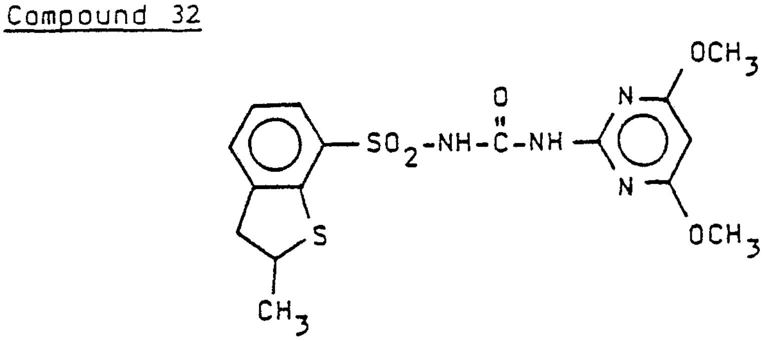

Compound 33

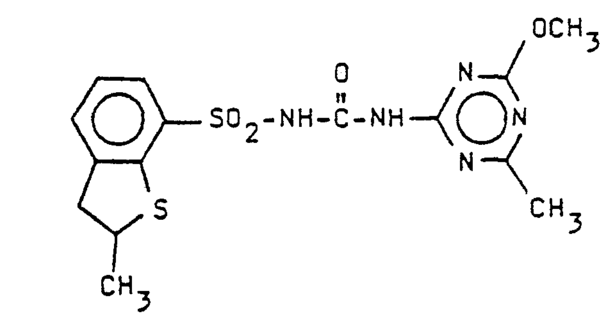

Table A Structures (Continued)

Compound 34

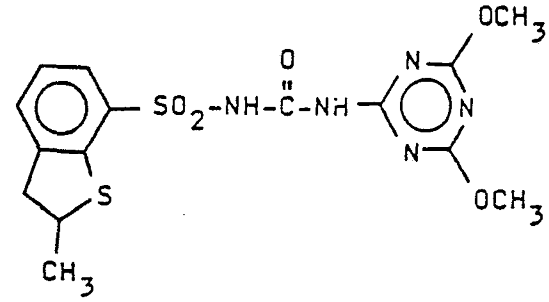

Compound 35

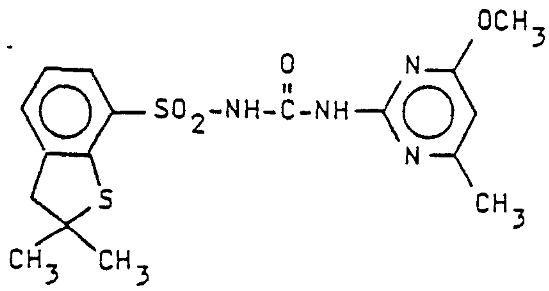

Compound 36

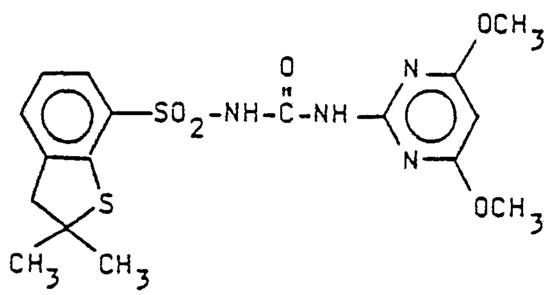

Compound 37

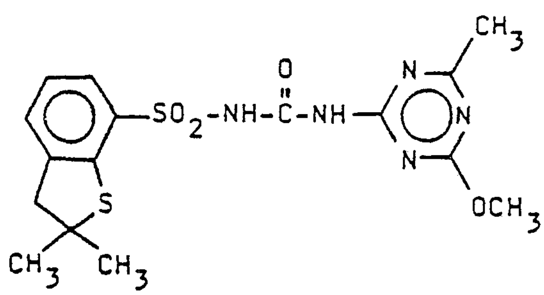

Table A Structures (Continued)

Compound 38

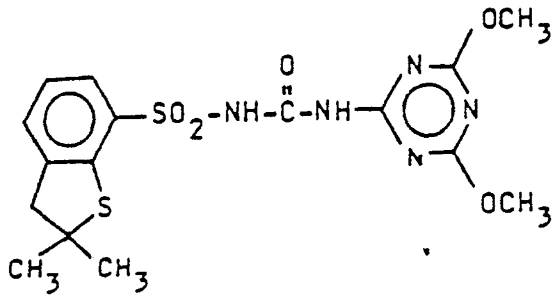

Compound 39

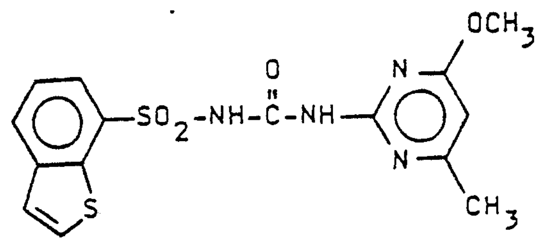

Compound 40

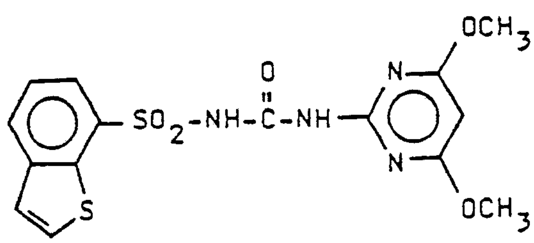

Compound 41

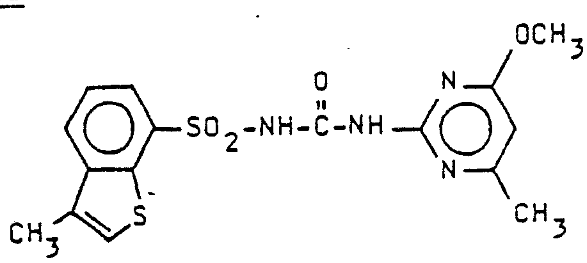

Table A Structures (Continued)

Compound 42

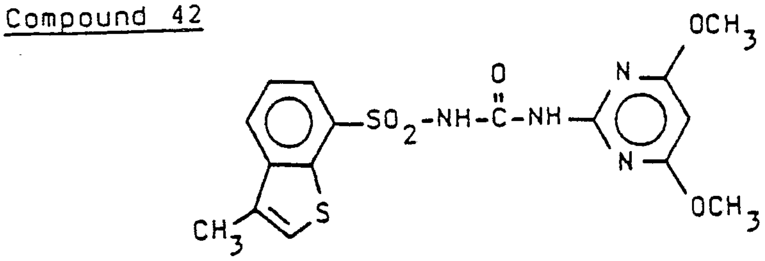

Compound 43

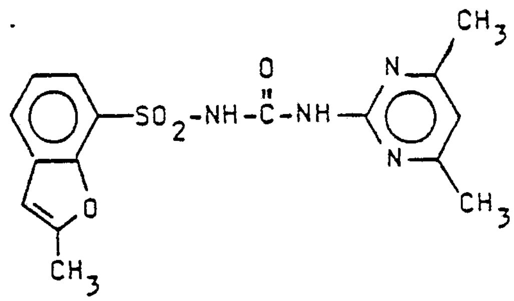

Compound 44

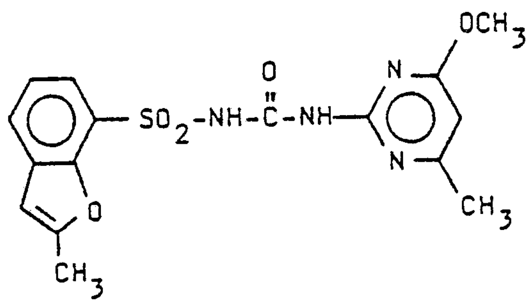

Compound 45

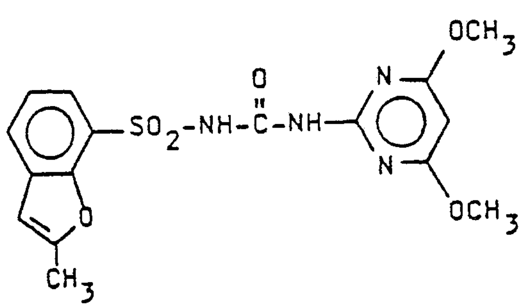

Table A Structures (Continued)

Compound 46

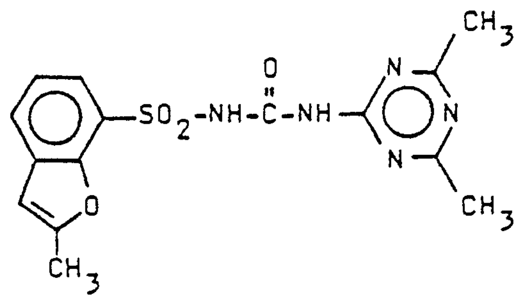

Compound 47

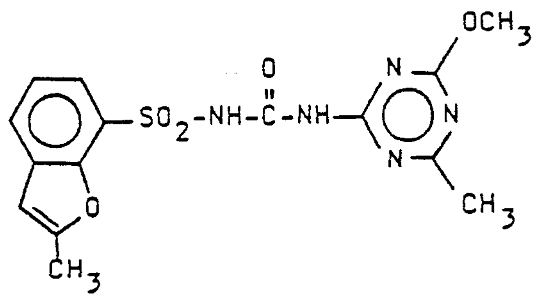

Compound 48

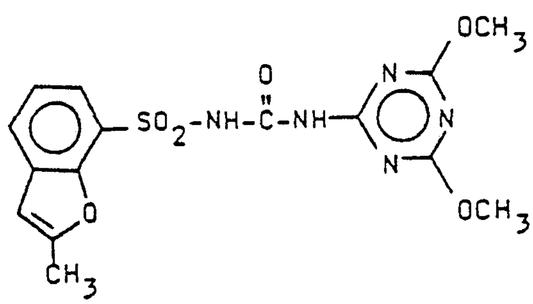

Compound 49

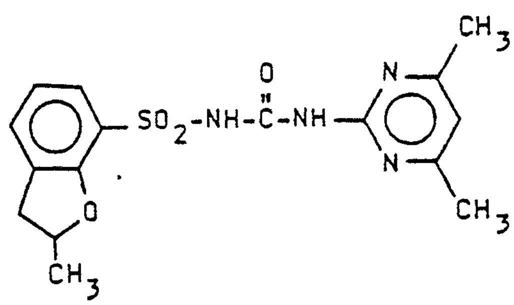

Table A Structures (Continued)

Compound 50

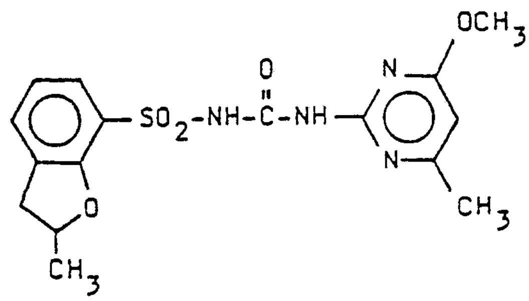

Compound 51

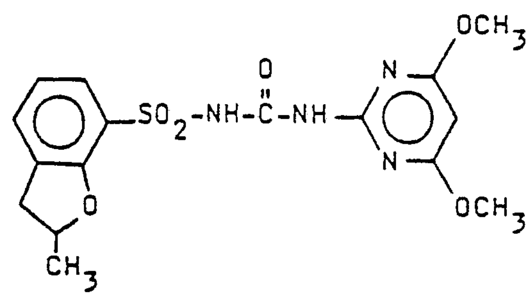

Compound 52

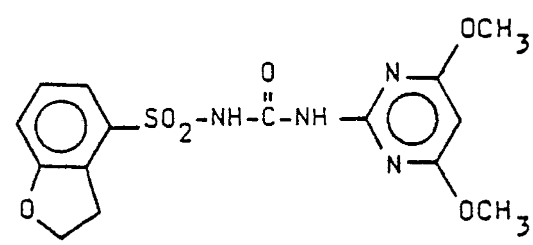

Compound 53

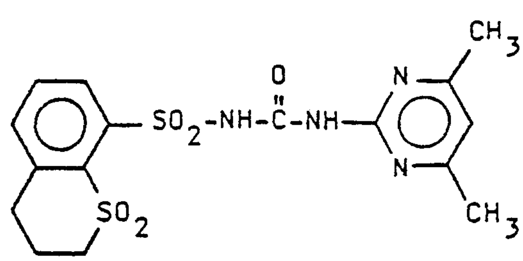

Table A Structures (Continued)

Compound 54

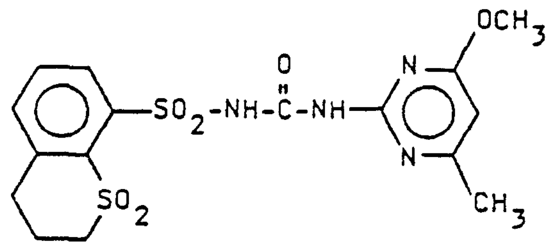

Compound 55

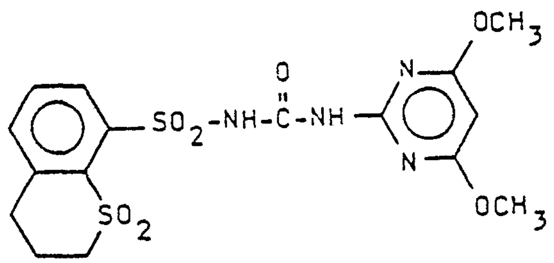

Compound 56

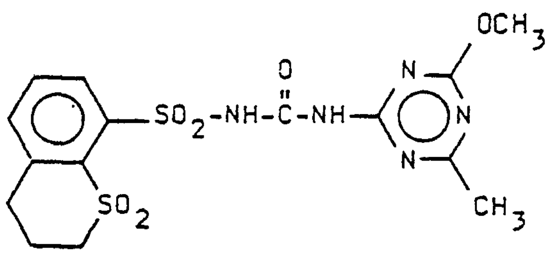

Compound 57

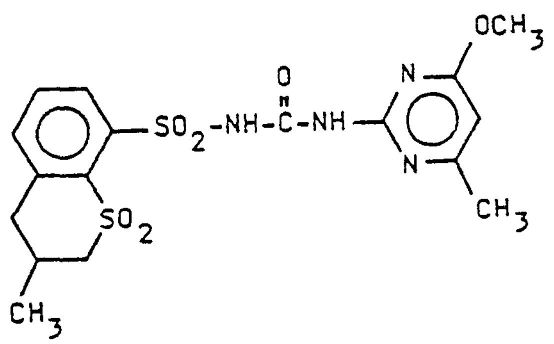

Table A Structures (Continued)

Compound 58

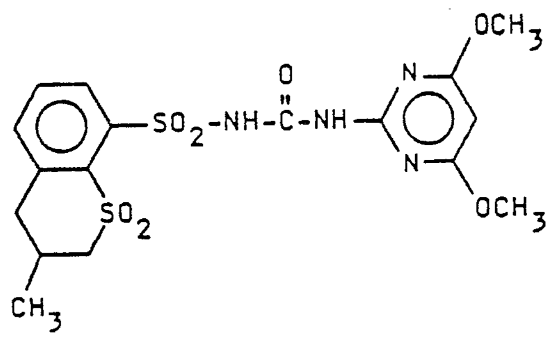

Compound 59

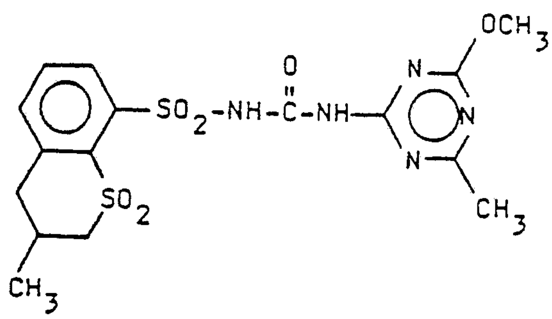

Compound 60

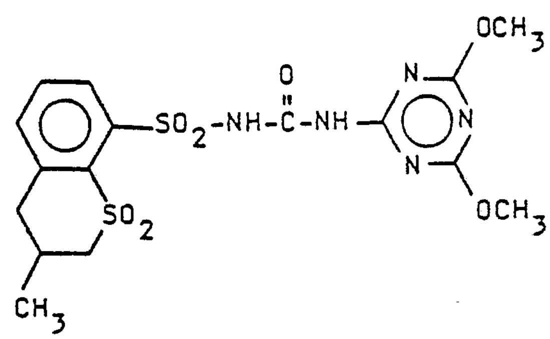

Table A Structures (Continued)

Compound 61

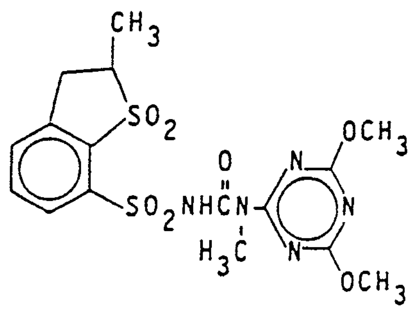

Compound 62

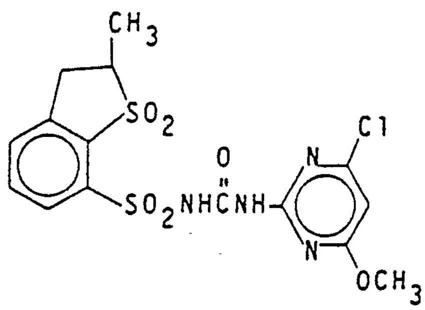

Compound 63

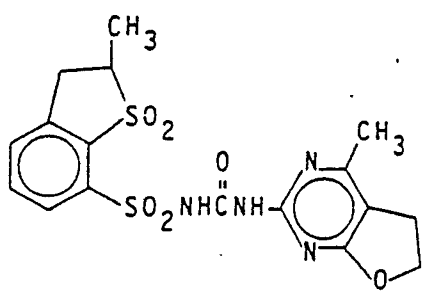

Table A Structures (Continued)

Compound 64

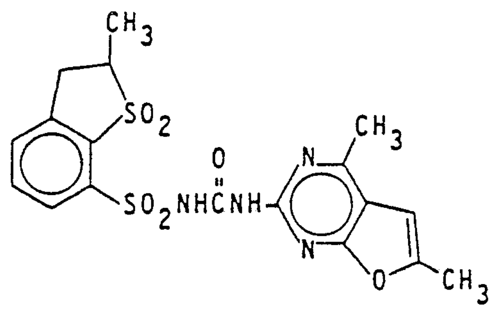

Compound 65

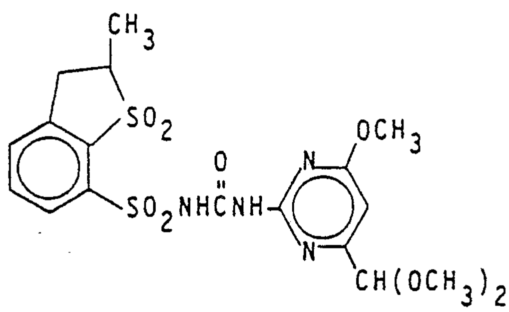

Compound 66

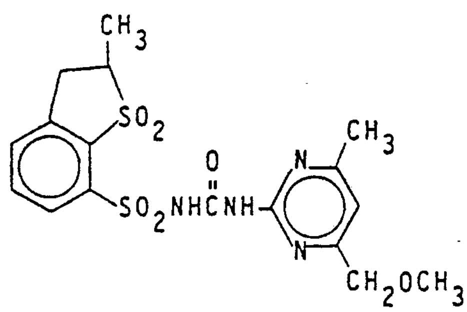

TABLE A

| | Structure No. 1 | Structure No. 2 | Structure No. 3 |
|---|---|---|---|
| Rate kg/ha | 0.05 | 0.05 | 0.05 |
| POST-EMERGENCE | | | |
| Bush bean | 3C,8G,6Y | 6C,9G | 6C,9G,6Y |
| Cotton | 3C,3H,9G | 6C,9G | 6C,9H |
| Morningglory | 2C,9G | 3C,9H | 6C,9G |
| Cocklebur | 5G | 10C | 9C |
| Sicklepod | 1C,5G | 6C,9G | 4C,8H |
| Nutsedge | 4G | 6C,9G | 2C,8G |
| Crabgrass | 4G | 6C,9G | 2C,7G |
| Barnyardgrass | 2C,9H | 10C | 10C |
| Wild Oats | 2C,6G | 7C,9G | 5C,9G |
| Wheat | 2C,6G | 2C,9G | 6C,9G |
| Corn | 2C,8H | 10C | 3U,9G |
| Soybean | 3C,9G,5X | 5C,9G | 4C,9G |
| Rice | 2C,9H | 5C,9G | 5C,9G |
| Sorghum | 3C,9H | 5C,9G | 9G |
| Sugar beet | | | |
| PRE-EMERGENCE | | | |
| Morningglory | 2C,6H | 8G | 9G |
| Cocklebur | 8H | 9H | 9H |
| Sicklepod | 6G | 7G | 2C,7G |
| Nutsedge | 3G | 10E | 2C,9G |
| Crabgrass | 1C | 2C,6G | 2C,8G |
| Barnyardgrass | 4C,9H | 5C,9H | 6C,9H |
| Wild Oats | 2C,9H | 4C,9H | 3C,9H |
| Wheat | 1C,9G | 9H | 9G |
| Corn | 2C,7G | 10H | 5C,9H |
| Soybean | 2C | 9H | 9H |
| Rice | 2C | 10E | 5C,9H |
| Sorghum | 2C,8H | 5C,9G | 4C,9H |
| Sugar beet | | | |

TABLE A (continued)

| | Structure No. 4 | 5 | 6 |
|---|---|---|---|
| Rate kg/ha | 0.05 | 0.05 | 0.05 |
| POST-EMERGENCE | | | |
| Bush bean | 9C | 9C | 3C,9G,6Y |
| Cotton | 6C,9G | 9C | 2C,5G |
| Morningglory | 10C | 9C | 2C |
| Cocklebur | 9C | 10C | 2C |
| Sicklepod | 4C,7H | 2C,8H | 2A |
| Nutsedge | 7X | 3C,6G | 0 |
| Crabgrass | 3C,8G | 2C,8G | 0 |
| Barnyardgrass | 9C | 9C | 2C,7H |
| Wild Oats | 6C,9G | 9C | 1C |
| Wheat | 9C | 10C | 0 |
| Corn | 10C | 10C | 2C,8H |
| Soybean | 9C | 9C | 2C,5H |
| Rice | 6C,9G | 6C,9G | 6G |
| Sorghum | 9C | 10C | 2C,8H |
| Sugar beet | | | |
| PRE-EMERGENCE | | | |
| Morningglory | 9G | 9G | 2C,5H |
| Cocklebur | 9H | 9H | 2C,9H |
| Sicklepod | 5C,9G | 5C,9G | 1C |
| Nutsedge | 5G | 2C,7G | 0 |
| Crabgrass | 3C,6G | 3C,7H | 0 |
| Barnyardgrass | 5C,9H | 5C,9H | 2C,7H |
| Wild Oats | 4C,9H | 4C,9H | 2C,8G |
| Wheat | 2C,9G | 2C,9G | 1C |
| Corn | 5C,9G | 10H | 4C,7H |
| Soybean | 9H | 9H | 1H,1A |
| Rice | 10E | 10E | 4C,7H |
| Sorghum | 7C,9H | 6C,9H | 4C,8H |
| Sugar beet | | | |

TABLE A (continued)

| Rate kg/ha | Compound 7 | Compound 8 | |
|---|---|---|---|
| | 0.05 | 0.4 | 0.05 |
| POST-EMERGENCE | | | |
| Bush bean | 5C,9G,6Y | 9C | 9C |
| Cotton | 5C,9G | 9C | 9C |
| Morningglory | 4C,9G | 10C | 9C |
| Cocklebur | 3C,9G | 9C | 10C |
| Sicklepod | 3C,8G | 10C | 9C |
| Nutsedge | 9G | 5C,9G | 9G |
| Crabgrass | 4C,9G | 6C,9G | 5C |
| Barnyardgrass | 9C | 9C | 5C |
| Wild Oats | 9C | 6C,9G | 6C |
| Wheat | 9C | 9C | 6C |
| Corn | 4U,9G | 3U,9C | 5U |
| Soybean | 4C,9G | 9C | 9C |
| Rice | 9C | 5C,9G | 5C |
| Sorghum | 5C,9G | 5C,9G | 4C |
| Sugar beet | 9C | — | — |
| PRE-EMERGENCE | | | |
| Morningglory | 9G | 9C | 9G |
| Cocklebur | 3C,8H | 9H | 9H |
| Sicklepod | 3C,7G | 9G | 9G |
| Nutsedge | 3C,8G | 10E | 10E |
| Crabgrass | 3C,7G | 10E | 5C,9G |
| Barnyardgrass | 3C,8H | 9H,7C | 6C,9H |
| Wild Oats | 5C,9G | 6C,9H | 6C,9H |
| Wheat | 3C,9G | 10E | 10E |
| Corn | 2C,9G | 10H | 10E |
| Soybean | 3C,6H | 9H | 9H |
| Rice | 10E | 10E | 10E |
| Sorghum | 3C,9G | 10E | 6C,9H |
| Sugar beet | 4C,9G | 10E | 10E |

TABLE A (continued)

| | Compound 9 | | Compound 10 | |
|---|---|---|---|---|
| Rate kg/ha | 0.4 | 0.05 | 0.4 | 0.05 |
| POST-EMERGENCE | | | | |
| Bush bean | 9C | 6C,9G,6Y | 9C | 6C,9G,6Y |
| Cotton | 9C | 10C | 4C,9G | 5C,9G |
| Morningglory | 10C | 10C | 4C,9G | 3C,7H |
| Cocklebur | 9C | 9C | 5C,9G | 4C,8G |
| Sicklepod | 9C | 9C | 5C,9G | 3C,7G |
| Nutsedge | 9C | 9G | 2C,8G | 0 |
| Crabgrass | 7C,9G | 7C,9G | 4C,9G | 2C,8G |
| Barnyardgrass | 9C | 7C,9G | 9C | 4C,9H |
| Wild Oats | 6C,9G | 6C,9G | 9C | 6C,9G |
| Wheat | 7C,9G | 6C,9G | 6C,9G | 9C |
| Corn | 9C | 9C | 7C,9G | 4U,9G |
| Soybean | 9C | 9C | 9C | 3C,7G |
| Rice | 6C,9G | 5C,9G | 5C,9G | 4C,9G |
| Sorghum | 9C | 5C,9G | 2C,9G | 2C,9G |
| Sugar beet | — | — | — | — |
| PRE-EMERGENCE | | | | |
| Morningglory | 9H | 5C,9G | 9G | 2C,5G |
| Cocklebur | 9H | 9H | 9H | 5H |
| Sicklepod | 9G,4C | 5C,9G | 9G | 7G |
| Nutsedge | 10E | 10E | 2G | 1C,3G |
| Crabgrass | 6C,9G | 5C,9G | 2C,6G | 2G |
| Barnyardgrass | 6C,9H | 5C,8H | 3C,9H | 4G |
| Wild Oats | 6C,9H | 5C,9H | 3C,8H | 2C,6G |
| Wheat | 6C,9H | 5C,9H | 5C,9H | 9G |
| Corn | 10E | 5C,9G | 5C,9H | 2C,8G |
| Soybean | 9H | 9H | 2C,8H | 1C,1H |
| Rice | 10E | 10E | 10E | 10E |
| Sorghum | 10E | 6C,9H | 10H | 9H |
| Sugar beet | 10E | 10E | 10E | 6G |

TABLE A (continued)

| Rate kg/ha | Compound 11 | Compound 12 | Compound 13 | Compound 14 |
|---|---|---|---|---|
| | 0.4 | 0.05 | 0.05 | 0.05 |
| POST-EMERGENCE | | | | |
| Bush bean | 9C | 9C | 9C | 9C |
| Cotton | 4C,8G | 5C,9G | 6C,9G | 5C,9G |
| Morningglory | 9C | 5C,9G | 9C | 9C |
| Cocklebur | 4C,9G | 9C | 9C | 10C |
| Sicklepod | 5C,9G | 4C,9G | 9C | 9C |
| Nutsedge | 7G | 4C,8G | 10C | 6C,9G |
| Crabgrass | 4C,9G | 5C,9G | 9C | 7C,9G |
| Barnyardgrass | 4C,9H | 5C,9H | 6C,9H | 9C |
| Wild Oats | 6C,9G | 9C | 9C | 9C |
| Wheat | 4C,9G | 9C | 9C | 9C |
| Corn | 3C,9G | 5U,9C | 10C | 9C |
| Soybean | 9C | 9C | 9C | 9C |
| Rice | 5C,9G | 5C,9G | 6C,9G | 6C,9G |
| Sorghum | 2C,9G | 5U,9C | 9C | 9C |
| Sugar beet | — | 9C | 9C | 9C |
| PRE-EMERGENCE | | | | |
| Morningglory | 9G | 9G | 9C | 9C |
| Cocklebur | 9H | 9H | 9H | 9H |
| Sicklepod | 9G | 5C,9G | 2C,9G | 9G |
| Nutsedge | 5G | 10E | 10E | 10E |
| Crabgrass | 2C,6G | 5C,9G | 6C,9H | 6C,9G |
| Barnyardgrass | 3C,9H | 4C,9H | 6C,9H | 6C,9H |
| Wild Oats | 2C,8H | 4C,8G | 6C,9H | 6C,9H |
| Wheat | 9G | 9H | 9H | 9H |
| Corn | 2U,9G | 5C,9H | 9H | 9H |
| Soybean | 2C,8H | 8H | 9H | 9H |
| Rice | 10E | 10E | 10E | 10E |
| Sorghum | 4C,9H | 5C,9H | 10H | 6C,9H |
| Sugar beet | 10E | 10E | 10E | 10E |

TABLE A (continued)

| | Compound 15 | | Compound 16 | |
|---|---|---|---|---|
| Rate kg/ha | 0.4 | 0.05 | 0.4 | 0.05 |
| POST-EMERGENCE | | | | |
| Bush bean | 9C | 9C | 9C | 6C,9G,6Y |
| Cotton | 4C,9G | 5C,9G | 4C,9G | 4C,9G |
| Morningglory | 9C | 4C,8H | 5C,9G | 4C,9G |
| Cocklebur | 9C | 4C,9H | 9C | 5C,9G |
| Sicklepod | 9C | 5C,9G | 9C | 5C,9G |
| Nutsedge | 3C,9G | 3C,9G | 9G | 2C,8G |
| Crabgrass | 9C | 4C,8G | 7C,9G | 2C,8G |
| Barnyardgrass | 9C | 9C | 9C | 9C |
| Wild Oats | 9C | 9C | 9C | 9C |
| Wheat | 9C | 8U,9G | 9C | 9C |
| Corn | 10C | 9C | 9C | 7U, 9C |
| Soybean | 6C,9G | 5C,9G | 6C,9G | 5C,9G |
| Rice | 9C | 9C | 9C | 9C |
| Sorghum | 9C | 6C,9G | 9C | 6C,9G |
| Sugar beet | 9C | 9C | 9C | 9C |
| PRE-EMERGENCE | | | | |
| Morningglory | 9C | 9G | 9C | 9G |
| Cocklebur | 9H | 9H | 9H | 2C,8H |
| Sicklepod | 9G | 9G | 9C | 9G |
| Nutsedge | 10E | 2G | 10E | 5C,9G |
| Crabgrass | 5C,9G | 7G | 7C,9G | 2C,6G |
| Barnyardgrass | 6C,9H | 3C,8G | 6C,9H | 5C,8G |
| Wild Oats | 6C,9H | 4C,9H | 6C,9H | 5C,9H |
| Wheat | 10E | 5C,9H | 6C,9H | 9H |
| Corn | 10E | 5C,9H | 10H | 5C,9H |
| Soybean | 8H | 8H | 9H | 8H |
| Rice | 10E | 10E | 10E | 10E |
| Sorghum | 7C,9H | 5C,9H | 7C,9H | 5C,9H |
| Sugar beet | 10E | 10E | 10E | 10E |

TABLE A (continued)

| Rate kg/ha | Compound 17<br>0.05 | Compound 18<br>0.05 | Compound 19<br>0.05 |
|---|---|---|---|
| POST-EMERGENCE | | | |
| Bush bean | 1C | 10D,9G,6Y | 9D,9G,6Y |
| Cotton | 1C,2H | 4C,9G | 4C,9G |
| Morningglory | 2C,3G | 2C,8H | 3C,9G |
| Cocklebur | 2G | 2C,9H | 5C,9G |
| Sicklepod | 0 | 2C,8H | 6C,9G |
| Nutsedge | 0 | 2C,8G | 9C |
| Crabgrass | 0 | 3C,9G | 9C |
| Barnyardgrass | 3G | 9C | 9C |
| Wild Oats | 0 | 2C,9G | 6C,9G |
| Wheat | 0 | 10C | 9C |
| Corn | 0 | 5U,9G | 10C |
| Soybean | 0 | 3C,9G | 5C,9G |
| Rice | 5G | 5C,9G | 9C |
| Sorghum | 1C | 5C,9G | 10C |
| Sugar beet | 2G | 9C | 9C |
| PRE-EMERGENCE | | | |
| Morningglory | 0 | 9H | 5C,9H |
| Cocklebur | 0 | 9H | 9H |
| Sicklepod | 0 | 8G | 9G |
| Nutsedge | 0 | 2C | 10E |
| Crabgrass | 0 | 2C,3H | 2C,6G |
| Barnyardgrass | 0 | 5C,9H | 5C,9H |
| Wild Oats | 0 | 5C,9G | 5C,9G |
| Wheat | 0 | 10C | 6C,9H |
| Corn | 0 | 2C,9H | 9C |
| Soybean | 0 | 3C,8H | 9H |
| Rice | 0 | 5C,9H | 10E |
| Sorghum | 0 | 5C,9H | 5C,9H |
| Sugar beet | 2C | 5C,9G | 9C |

TABLE A (continued)

| Rate kg/ha | Compound 20 | Compound 21 | Compound 22 |
|---|---|---|---|
| | 0.05 | 0.05 | 2.0 |
| POST-EMERGENCE | | | |
| Bush bean | 4C,9G,6Y | 6C,8G,6Y | 3C,5G,6Y |
| Cotton | 3C,4G | 4C,8G | 4C,8G |
| Morningglory | 5C,8H | 4C,9H | 4C,8G |
| Cocklebur | 5C,9H | 4C,8H | 4C,9G |
| Sicklepod | 2C,4H | 3C,3H | 3G |
| Nutsedge | 2C | 0 | 2G |
| Crabgrass | 2C,7G | 2C,8G | 2G |
| Barnyardgrass | 9C | 9C | 0 |
| Wild Oats | 8C | 9C | 0 |
| Wheat | 9C | 10C | 5G,5X |
| Corn | 7U,9C | 9C | 3H |
| Soybean | 4C,8G,5X | 2C,9G | 1C,5G |
| Rice | 6C,9G | 5C,9G | 2C,5G |
| Sorghum | 5U,9C | 9C | 2C,7H |
| Sugar beet | 5C,9G | 9C | — |
| PRE-EMERGENCE | | | |
| Morningglory | 3C,8H | 9G | 2H |
| Cocklebur | 9H | 5C,9G | 8H |
| Sicklepod | 2C,7G | 2C,3H | 1C |
| Nutsedge | 0 | 0 | 0 |
| Crabgrass | 1H | 0 | 0 |
| Barnyardgrass | 2C,5G | 6H | 4G |
| Wild Oats | 2C,9G | 2C,9G | 5G |
| Wheat | 2C,9G | 2C,9G | 4G |
| Corn | 3C,9H | 5C,9H | 5C,8H |
| Soybean | 2C,3H | 1C,1H | 2G |
| Rice | 10E | 10E | 0 |
| Sorghum | 5C,9G | 5C,9H | 2C,7G |
| Sugar beet | 1C | 4C,8G | — |

TABLE A (continued)

| Rate kg/ha | Compound 23<br>0.05 | Compound 24<br>0.05 |
|---|---|---|
| POST-EMERGENCE | | |
| Bush bean | 3C,9G,6Y | 7C,9G,6Y |
| Cotton | 3C,4H,9G | 4C,4H,9G |
| Morningglory | 3C,9G | 3C,8G |
| Cocklebur | 3C,9G | 3C,9G |
| Sicklepod | 2C,5G | 3C,8H |
| Nutsedge | 3G | 3G |
| Crabgrass | 0 | 2H |
| Barnyardgrass | 2C,5H | 6H |
| Wild Oats | 2G | 0 |
| Wheat | 8G | 4G |
| Corn | 3C,9G | 2C,9G |
| Soybean | 3C,9G | 3H,9G |
| Rice | 3C,9G | 6C,9G |
| Sorghum | 3C,9G | 9G |
| Sugar beet | — | — |
| PRE-EMERGENCE | | |
| Morningglory | 2C,8H | 2C,8G |
| Cocklebur | 8H | 9H |
| Sicklepod | 0 | 3C,8H |
| Nutsedge | 0 | 2C,6G |
| Crabgrass | 2C,4G | 1C |
| Barnyardgrass | 6H | 2C,7H |
| Wild Oats | 2G | 2C,8H |
| Wheat | 7G | 2C,9G |
| Corn | 2C,9H | 2C,8G |
| Soybean | 2C,6G | 2C,6H |
| Rice | 3C,7H | 9H |
| Sorghum | 4C,8H | 3C,9G |
| Sugar beet | — | — |

TABLE A (continued)

| | Compound 25 | | Compound 26 | |
|---|---|---|---|---|
| Rate kg/ha | 0.4 | 0.05 | 2.0 | 0.05 |
| POST-EMERGENCE | | | | |
| Bush bean | 5C,9G,6Y | 2C,2H | 6C,9G,6Y | 1C,3G |
| Cotton | 4C,8G | 1C | 2C,3H,8G | 1C |
| Morningglory | 3C | 1C | 5C,9H | 1C |
| Cocklebur | 2C,5H | 1C | 2C,5H | 0 |
| Sicklepod | 3C | 1C | 3C,4H | 1C |
| Nutsedge | 2G | 0 | 0 | 0 |
| Crabgrass | 2G | 0 | 1C,3G | 0 |
| Barnyardgrass | 2C,7H | 0 | 5C,9H | 0 |
| Wild Oats | 9G,7X | 4G,5X | 5C,9G | 0 |
| Wheat | 10C | 1C,5G | 9C | 0 |
| Corn | 9C | 2C,7H | 6U,9C | 0 |
| Soybean | 2H,5G | 1C,1H | 3C,5G | 0 |
| Rice | 9C | 2U,9G | 9C | 0 |
| Sorghum | 9C | 2C,9H | 2U,9G | 0 |
| Sugar beet | — | — | — | — |
| PRE-EMERGENCE | | | | |
| Morningglory | 2C,4G | 0 | 8G | 0 |
| Cocklebur | 1H | 0 | 8H | 2G |
| Sicklepod | 0 | 0 | 8G | 2G |
| Nutsedge | 0 | 0 | 8G | 2G |
| Crabgrass | 0 | 0 | 2G | 2G |
| Barnyardgrass | 2G | 0 | 2C,8G | 2G |
| Wild Oats | 5H | 0 | 2C,8G | 0 |
| Wheat | 8G | 0 | 2C,9G | 0 |
| Corn | 3C,9G | 2C,5G | 2C,9G | 0 |
| Soybean | 0 | 1H | 1C,2H | 2G |
| Rice | 9G,9E | 2G | 5C,7G | 0 |
| Sorghum | 2C,9G | 1C,4G | 3C,9G | 0 |
| Sugar beet | — | — | — | — |

TABLE A (continued)

| | Compound 27 | Compound 28 | Compound 29 |
|---|---|---|---|
| Rate kg/ha | 0.4 | 0.4 | 0.4 |
| POST-EMERGENCE | | | |
| Bush bean | 1C | 4C,9G,6Y | 9D,9G,6Y |
| Cotton | 1C | 5C,9G | 5C,9G |
| Morningglory | 0 | 4C,8H | 4C,6H |
| Cocklebur | 1C | 5C,9G | 3C,9G |
| Sicklepod | 0 | 2C,3H | 3C,8G |
| Nutsedge | 0 | 2G | 2G |
| Crabgrass | 2G | 4G | 1C,3G |
| Barnyardgrass | 1C | 2C,8H | 10C |
| Wild Oats | 0 | 0 | 3G |
| Wheat | 0 | 3G | 5G |
| Corn | 0 | 3C,8G | 2C,9H |
| Soybean | 0 | 4C,8G | 5C,9G |
| Rice | 0 | 4C,9G | 6C,9G |
| Sorghum | 2C | 5C,9G | 9G |
| Sugar beet | — | — | — |
| PRE-EMERGENCE | | | |
| Morningglory | 0 | 9G | 9G |
| Cocklebur | — | 9H | 8H |
| Sicklepod | 0 | 2C,8G | 9G |
| Nutsedge | 10E | 10E | 10E |
| Crabgrass | 1C | 2C | 4G |
| Barnyardgrass | 1C | 2C,9H | 5C,9H |
| Wild Oats | 2G | 2C,6G | 2C,6G |
| Wheat | 0 | 9G | 9H |
| Corn | 2C,5G | 2C,9G | 2C,9G |
| Soybean | 0 | 2C,6H | 9H |
| Rice | 1C | 10E | 10E |
| Sorghum | 2C,3G | 5C,9H | 10H |
| Sugar beet | — | — | — |

TABLE A (continued)

| | Compound 30 | Compound 31 | Compound 32 | Compound 33 |
|---|---|---|---|---|
| Rate kg/ha | 0.4 | 0.05 | 0.05 | 0.05 |
| POST-EMERGENCE | | | | |
| Bush bean | 4C,9G,6Y | 9C | 9C | 5C,9G,6Y |
| Cotton | 5C,9G | 4C,9G | 4C,9G | 4C,8G |
| Morningglory | 2C | 5C,9G | 9C | 4C,7G |
| Cocklebur | 2C | 9C | 10C | 10C |
| Sicklepod | 2C | 5C,9G | 9C | 4C,8G |
| Nutsedge | 0 | 5C,9G | 9C | 2G |
| Crabgrass | 2G | 9C | 6C,9G | 1C,3G |
| Barnyardgrass | 2H | 9C | 9C | 4C,9H |
| Wild Oats | 3G | 9C | 9C | 2C,8G |
| Wheat | 2G | 9C | 9C | 8G |
| Corn | 4C,8H | 9C | 9C | 5U,9G |
| Soybean | 5C,6G | 5C,9G | 9C | 5C,9G |
| Rice | 3C,9G | 9C | 9C | 6C,9G |
| Sorghum | 2C,9G | 9C | 9C | 3C,9G |
| Sugar beet | — | 9C | 5C,9G | 5C,9G |
| PRE-EMERGENCE | | | | |
| Morningglory | 2G | 9G | 10C | 9C |
| Cocklebur | 9H | 8H | 9H | 9H |
| Sicklepod | 2C,6G | 9C | 3C,9G | 8G |
| Nutsedge | 0 | 9G | 10E | 2C |
| Crabgrass | 2G | 4C,8G | 5C,9G | 2C,4G |
| Barnyardgrass | 2C,7G | 9C | 9H | 3C,6H |
| Wild Oats | 3C | 4C,9G | 5C,9G | 2C,9G |
| Wheat | 2C,9H | 9C | 10C | 2C,8G |
| Corn | 2C,9H | 9C | 5C,9G | 3C,9H |
| Soybean | 2C,3H | 4C,8H | 3C,8H | 2C,7H |
| Rice | 10E | 10E | 10E | 10E |
| Sorghum | 5C,9G | 9H | 6C,9H | 5C,9H |
| Sugar beet | — | 5C,9G | 5C,9G | 10C |

TABLE A (continued)

| Rate kg/ha | Compound 34<br>0.05 | Compound 35<br>0.05 | Compound 36<br>0.05 | Compound 37<br>0.05 |
|---|---|---|---|---|
| POST-EMERGENCE | | | | |
| Bush bean | 5C,9G,6Y | 4C,6G,6Y | 5C,9G,6Y | 4C,8G,6Y |
| Cotton | 4C,8H | 4C,7H | 4C,9G | 4C,7H |
| Morningglory | 3C,7G | 4C | 3C,8G | 4C,9G |
| Cocklebur | 9C | 3C,8H | 9C | 3C,9H |
| Sicklepod | 3C,8G | 2C | 4C,6H | 2C |
| Nutsedge | 3G | 2G | 4C,9G | 0 |
| Crabgrass | 3G | 1C | 2C,8G | 1C |
| Barnyardgrass | 3C,9H | 4C,9H | 9C | 3C,9H |
| Wild Oats | 3C,9G | 2C,6G | 9C | 1C,3G |
| Wheat | 2C,9G,5X | 2C,8G | 9C | 1C,5G |
| Corn | 3U,9G | 2C,8H | 4U,9C | 2C,9G |
| Soybean | 5C,9G | 2C,8G | 4C,9G | 4C,9G |
| Rice | 5C,9G | 5C,9G | 6C,9G | 6C,9G |
| Sorghum | 2C,9G | 4C,9H | 9C | 2C,9G |
| Sugar beet | 9C | 4C,7G | 9C | 4C,8G |
| PRE-EMERGENCE | | | | |
| Morningglory | 9G | 2C,8H | 9G | 3C,8G |
| Cocklebur | 9H | — | 9H | 9H |
| Sicklepod | 9G | 4G | 2C,4G | 2C,5G |
| Nutsedge | 2C | 4G | 10E | 0 |
| Crabgrass | 1C,2G | 2G | 2G | 1C |
| Barnyardgrass | 3C,8H | 3C,9H | 4C,9H | 5C,8H |
| Wild Oats | 2C,6G | 2C,8G | 2C,9G | 3C,7G |
| Wheat | 1C,5G | 2C,9G | 2C,9H | 7G |
| Corn | 2C,9H | 2C,9H | 2C,9H | 2C,9G |
| Soybean | 2C,6G | 2C,3H | 2C,5H | 3C,6H |
| Rice | 10E | 5C,9H | 10E | 10E |
| Sorghum | 4C,9G | 3C,9H | 2C,9H | 2C,9H |
| Sugar beet | 10E | 9G | 4C,9G | 4C,9G |

TABLE A (continued)

| | Compound 38 | Compound 39 | Compound 40 | Compound 41 |
|---|---|---|---|---|
| Rate kg/ha | 0.05 | 0.4 | 0.4 | 0.4 |
| POST-EMERGENCE | | | | |
| Bush bean | 4C,9G,6Y | 5C,9G,6Y | 9C | 2C |
| Cotton | 2C,2H | 5C,9G | 5C,9G | 2C,2H |
| Morningglory | 4C,8H | 10C | 4C,8G | 1C |
| Cocklebur | 4C,9H | 10C | 9C | 3C,7H |
| Sicklepod | 2C,3H | 9C | 9C | 2C |
| Nutsedge | 0 | 9G | 4C,9G | 2G |
| Crabgrass | 2C | 5C,9G | 5C,9G | 0 |
| Barnyardgrass | 4C,8H | 5C,9H | 9C | 0 |
| Wild Oats | 3C,9G | 5C,9H | 4C,9G | 0 |
| Wheat | 2C,5G | 4C,9G | 1C,7G | 0 |
| Corn | 1U,9G | 3U,9G | 4U,9G | 0 |
| Soybean | 3C,9G | 9C | 9C | 2G |
| Rice | 5C,9G | 5C,9G | 5C,9G | 0 |
| Sorghum | 2C,9G | 2U,9G | 3C,9G | 0 |
| Sugar beet | 3C,5H | 9C | 5C,9G | — |
| PRE-EMERGENCE | | | | |
| Morningglory | 8G | 9G | 9G | 8G |
| Cocklebur | 8H | 9H | 9H | 8H |
| Sicklepod | 7G | 9G | 9G | 5G |
| Nutsedge | 2G | 10E | 10E | 5G |
| Crabgrass | 1C | 10E | 5C,9G | 0 |
| Barnyardgrass | 4C,8H | 5C,9H | 5C,9H | 3C |
| Wild Oats | 2C,8G | 5C,9H | 4C,8G | 0 |
| Wheat | 2C,7G | 9H | 8G | 0 |
| Corn | 2C,9G | 9H | 3C,9G | 3C |
| Soybean | 2C,4H | 9H | 9H | 2C |
| Rice | 10E | 10E | 10E | 2C,5G |
| Sorghum | 2C,9H | 10E | 5C,9G | 1C |
| Sugar beet | 3C,9G | 10E | 10E | — |

TABLE A (continued)

| Rate kg/ha | Compound 42<br>0.4 |
|---|---|
| POST-EMERGENCE | |
| Bush bean | 4C,4G,6Y |
| Cotton | 2C |
| Morningglory | 0 |
| Cocklebur | 3C,8G |
| Sicklepod | 2C |
| Nutsedge | 0 |
| Crabgrass | 0 |
| Barnyardgrass | 0 |
| Wild Oats | 0 |
| Wheat | 0 |
| Corn | 0 |
| Soybean | 2C,4G |
| Rice | 0 |
| Sorghum | 0 |
| Sugar beet | — |
| PRE-EMERGENCE | |
| Morningglory | 2G |
| Cocklebur | 8H |
| Sicklepod | 5G |
| Nutsedge | 0 |
| Crabgrass | 0 |
| Barnyardgrass | 2C |
| Wild Oats | 0 |
| Wheat | 1C |
| Corn | 2C |
| Soybean | 0 |
| Rice | 3C,8H |
| Sorghum | 3C,5H |
| Sugar beet | — |

TABLE A (continued)

| Rate kg/ha | Compound 43<br>0.05 | Compound 44<br>0.05 | Compound 45<br>0.05 | Compound 46<br>0.05 |
|---|---|---|---|---|
| POST-EMERGENCE | | | | |
| Bush bean | 5C,8G,6Y | 9C | 9C | 4C,7G,6Y |
| Cotton | 2C,5G | 9C | 9C | 1C |
| Morningglory | 1C,4G | 1C,4G | 4C,8H | 1C |
| Cocklebur | 1C | 2C,8H | 9C | 1C |
| Sicklepod | 0 | 4C,9G | 9C | 0 |
| Nutsedge | 1C,4G | 3C,7G | 10C | 2G |
| Crabgrass | 2G | 3C,7G | 2C,6G | 0 |
| Barnyardgrass | 2C,6H | 5C,9H | 10C | 0 |
| Wild Oats | 0 | 2C,9H | 5G | 0 |
| Wheat | 0 | 1C,9G | 5G | 0 |
| Corn | 1C,4H | 2C,8G | 2C,9G | 0 |
| Soybean | 2C,4H | 9C | 9C | 3C,3H |
| Rice | 2C,4G | 3C,9G | 9G | 1C,4G |
| Sorghum | 3C,8H | 3C,9G | 2C,9G | 1C,3G |
| Sugar beet | 1C,5G | 4C,7G | — | 4C,9G |
| PRE-EMERGENCE | | | | |
| Morningglory | 0 | 9G | 9G | 1C |
| Cocklebur | — | — | — | 0 |
| Sicklepod | 3H | 9G | 9G | 1C |
| Nutsedge | 9G | 10E | 10E | 0 |
| Crabgrass | 2G | 5G | 8G | 2G |
| Barnyardgrass | 1C | 5C,9H | 9H | 1C |
| Wild Oats | 1C | 3C,8H | 3C,8G | 0 |
| Wheat | 1C,5G | 2C,9H | 8G | 0 |
| Corn | 1C,5G | 9H | 2C,9G | 0 |
| Soybean | 0 | 8H | 7H | 0 |
| Rice | 2G | 10E | 9H | 0 |
| Sorghum | 3C,5G | 10H | 5C,9H | 2G |
| Sugar beet | 2G | 10E | — | 1C |

TABLE A (continued)

| | Compound 47 | Compound 48 | Compound 49 | Compound 50 |
|---|---|---|---|---|
| Rate kg/ha | 0.05 | 0.05 | 0.05 | 0.05 |
| POST-EMERGENCE | | | | |
| Bush bean | 9C | 9C | 9C | 9C |
| Cotton | 2C,8G | 9C | 4C,8G | 5C,9G |
| Morningglory | 3C,6G | 5C,9G | 2C,8H | 5C,9G |
| Cocklebur | 3C,6G | 9C | 9C | 10C |
| Sicklepod | 5C,9G | 5C,9G | 5C,9G | 9C |
| Nutsedge | 0 | 1C,5G | 4C,8G | 9C |
| Crabgrass | 2C,5G | 1C,4G | 4C,9G | 9C |
| Barnyardgrass | 2C,9H | 3C,9H | 9C | 9C |
| Wild Oats | 0 | 1C,5G | 2C,9G | 9C |
| Wheat | 2G | 4G | 2C,9G | 9C |
| Corn | 1U,9H | 1C,9G | 10C | 10C |
| Soybean | 9C | 9C | 5C,9G | 9C |
| Rice | 5C,9G | 5C,9G | 5C,9G | 9C |
| Sorghum | 1C,9G | 1C,9G | 9C | 10C |
| Sugar beet | 2C,9G | 3C,9G | 2C,8H | 9C |
| PRE-EMERGENCE | | | | |
| Morningglory | 9G | 9G | 10C | 9H |
| Cocklebur | 9H | 9H | 9H | 9H |
| Sicklepod | 8G | 9G | 2C,9G | 9G |
| Nutsedge | 9G | 8G | 10E | 10E |
| Crabgrass | 2G | 0 | 1C | 8G |
| Barnyardgrass | 3C | 2C,7H | 2C,9H | 9H |
| Wild Oats | 2C,4G | 2C,6G | 2C,9H | 6C,9G |
| Wheat | 6G | 6G | 5C,9H | 10C |
| Corn | 1C,9G | 2U,9G | 5C,9H | 10H |
| Soybean | 7H | 9H | 2C,8H | 9H |
| Rice | 10E | 10E | 10E | 10E |
| Sorghum | 2C,8H | 2C,9H | 3C,9H | 6C,9H |
| Sugar beet | 10E | 10E | 9G | 10E |

TABLE A (continued)

| | Compound 51 | Compound 52 |
|---|---|---|
| Rate kg/ha | 0.05 | 0.4 |
| POST-EMERGENCE | | |
| Bush bean | 9C | 5C,8G,6Y |
| Cotton | 5C,9G | 4C,3H,9G |
| Morningglory | 4C,5G | 1C,2H |
| Cocklebur | 10C | 5G |
| Sicklepod | 4C,8H | 2C,8H |
| Nutsedge | 9C | 9G |
| Crabgrass | 5C,9G | 0 |
| Barnyardgrass | 9C | 2C,8H |
| Wild Oats | 9C | 0 |
| Wheat | 9C | 0 |
| Corn | 10C | 2C,6G |
| Soybean | 9C | 2C,9G |
| Rice | 9C | 6G |
| Sorghum | 10C | 2C,9H |
| Sugar beet | 9C | — |
| PRE-EMERGENCE | | |
| Morningglory | 8H | 6G |
| Cocklebur | 9H | 8H |
| Sicklepod | 9G | 5G |
| Nutsedge | 10E | 2C,8G |
| Crabgrass | 8G | 1C |
| Barnyardgrass | 9H | 3C,6H |
| Wild Oats | 5C,9G | 2C,6C |
| Wheat | 10H | 0 |
| Corn | 5C,9H | 2C,7G |
| Soybean | 2C,8H | 2H |
| Rice | 10E | 5G |
| Sorghum | 9H | 2C,5G |
| Sugar beet | 10E | — |

TABLE A (continued)

| | Compound 53 | Compound 54 | Compound 55 |
|---|---|---|---|
| Rate, kg/ha | 0.05 | 0.05 | 0.05 |
| POST-EMERGENCE | | | |
| Bush bean | 3c,6Y | 5C,9G,6Y | 5C,9G,6Y |
| Cotton | 1C | 2C,5G | 2C,4G |
| Morningglory | 2C | 5C,9G | 5C,9G |
| Cocklebur | 1C | 5C,9G | 5C,9G |
| Sicklepod | 1C | 4C,8G | 5C,9G |
| Nutsedge | 0 | 1C | 1C,5G |
| Crabgrass | 0 | 4C,6G | 5C,9H |
| Barnyardgrass | 1C,3H | 5C,9H | 5C,9H |
| Wild Oats | 0 | 5C,9G | 5C,9G |
| Wheat | 1C | 2C,9G | 4C,9G |
| Corn | 0 | 3C,9G | 4C,9G |
| Soybean | 1C | 4C,9G,5X | 3C,8G |
| Rice | 2G | 5C,9G | 6C,9G |
| Sorghum | 1C,4G | 4C,9G | 1C,9G |
| Sugar beet | 2C,4G | 4C,8G | 9C |
| PRE-EMERGENCE | | | |
| Morningglory | 0 | 4C,6H | 9G |
| Cocklebur | 0 | 9H | 9H |
| Sicklepod | 0 | 3C | 2C,8G |
| Nutsedge | 0 | 1C | 2C,3G |
| Crabgrass | 0 | 2C,4G | 2C,5G |
| Barnyardgrass | 0 | 2C,5G | 3C,9H |
| Wild Oats | 0 | 4C,6G | 5C,9G |
| Wheat | 0 | 3C,9G | 2C,9H |
| Corn | 0 | 4C,8G | 3C,9G |
| Soybean | 0 | 3C,2H | 3C,3H |
| Rice | 0 | 2C,3G | 10E |
| Sorghum | 0 | 5C,9H | 5C,9G |
| Sugar beet | 0 | 2C,4G | 10E |

TABLE A (continued)

| | Compound 56 | Compound 56 | Compound 57 |
|---|---|---|---|
| Rate, kg/ha | 0.05 | 0.4 | 2 |
| POST-EMERGENCE | | | |
| Bush bean | 5C,8C,6Y | 4C,8G,6Y | 1C |
| Cotton | 2C,6G | 4C,8G | 2C |
| Morningglory | 2C,5G | 4C,5H | 3C |
| Cocklebur | 1H | 4C,8H | 2C |
| Sicklepod | 0 | 1C | 0 |
| Nutsedge | 0 | 0 | 0 |
| Crabgrass | 2G | 3C,8G | 2C,3G |
| Barnyardgrass | 8H | 5C,8H | 2C |
| Wild Oats | 2C,8G | 4C,8G | 0 |
| Wheat | 2C,8G | 4U,9G | 0 |
| Corn | 3C,9G | 3U,9G | 3C,4G |
| Soybean | 2C,3H | 2C,7G | 2C |
| Rice | 5C,9G | 5C,9G | 3C |
| Sorghum | 3C,9G | 2C,9G | 3C,5G |
| Sugar beet | 2C,3H | 3C,3H | 3C |
| PRE-EMERGENCE | | | |
| Morningglory | 2C | 4C,6H | 2C,7H |
| Cocklebur | — | 9H | 8H |
| Sicklepod | 0 | 3C,6H | 3C |
| Nutsedge | 0 | 0 | 2C,7G |
| Crabgrass | 0 | 1C,5G | 2C,4G |
| Barnyardgrass | 1C | 2C,8H | 3C,8H |
| Wild Oats | 1C,4G | 4C,9G | 4C,8G |
| Wheat | 1C,5G | 3C,9G | 2C,8G |
| Corn | 2C,4G | 2C,9G | 3C,9H |
| Soybean | 2C | 2C,3H | 2C,2H |
| Rice | 2C,4G | 10E | 4C,9H |
| Sorghum | 2C,8G | 9H | 4C,9G |
| Sugar beet | 3C,8G | 7G | 5C,9G |

TABLE A (continued)

| Rate, kg/ha | Compound 58<br>2 | Compound 59<br>2 |
|---|---|---|
| POST-EMERGENCE | | |
| Bush bean | 0 | 1C |
| Cotton | 1C | 4C,4H |
| Morningglory | 1C | 2C,2H |
| Cocklebur | 1C | 1C,5G |
| Sicklepod | 0 | 1C |
| Nutsedge | 0 | 0 |
| Crabgrass | 2C,4G | 3C,6G |
| Barnyardgrass | 1C | 3C,9H |
| Wild Oats | 0 | 2C,8G |
| Wheat | 0 | 2C,7G |
| Corn | 0 | 2C,8H |
| Soybean | 1C | 0 |
| Rice | 2C,5G | 2C,8G |
| Sorghum | 2C,5H | 2C,9G |
| Sugar beet | 1C | 1C |
| PRE-EMERGENCE | | |
| Morningglory | 8G | 8H |
| Cocklebur | 8H | 9H |
| Sicklepod | 2C,6G | 0 |
| Nutsedge | 8G | 3G |
| Crabgrass | 2G | 0 |
| Barnyardgrass | 2C,8H | 3C,9H |
| Wild Oats | 2C,9G | 2C,5G |
| Wheat | 2C,9G | 2C,9G |
| Corn | 2C,8G | 2C,8G |
| Soybean | 2C,2H | 1C |
| Rice | 5C,9H | 9H |
| Sorghum | 2C,9G | 3C,9G |
| Sugar beet | 4C,8G | 5G |

TABLE A (continued)

| | Compound 60 |
|---|---|
| Rate, kg/ha | 2 |
| POST-EMERGENCE | |
| Bush bean | 0 |
| Sorghum | 1C |
| Corn | 0 |
| Soybean | 2G |
| Wheat | 0 |
| Wild Oats | 0 |
| Rice | 0 |
| Barnyardgrass | 0 |
| Crabgrass | 0 |
| Morningglory | 0 |
| Cocklebur | 2G |
| Sicklepod | 0 |
| Nutsedge | 0 |
| Sugar beet | 1C |
| | 0 |
| PRE-EMERGENCE | |
| Sorghum | 2C,7H |
| Corn | 9H |
| Soybean | 1C |
| Wheat | 10E |
| Wild Oats | 1C |
| Rice | 2C,3H |
| Barnyardgrass | 0 |
| Crabgrass | 3G |
| Morningglory | 3C,6H |
| Cocklebur | 0 |
| Sicklepod | 8H |
| Nutsedge | 2C,9G |
| Sugar beet | 4G |

TABLE A (continued)

| | Compound 61 | Compound 62 |
|---|---|---|
| Rate (kg/ha) | 0.05 | 0.05 |
| POST-EMERGENCE | | |
| Bush bean | 5C,9G,6Y | 9C |
| Cotton | 5C,9G | 5C,9G |
| Sorghum | 6C,9G | 9C |
| Corn | 3U,9G | 10C |
| Soybean | 5C,9G | 5C,9G |
| Wheat | 2C,9G | 9C |
| Wild Oats | 2C,9G | 3C,9G |
| Rice | 4C,9G | 9C |
| Barnyardgrass | 3C,8H | 9C |
| Crabgrass | 2C,9G | 9C |
| Morningglory | 5C,9G | 5C,9G |
| Cocklebur | 9C | 10C |
| Sicklepod | 3C,8G | 5C,9G |
| Nutsedge | 9C | 9C |
| Sugar beet | 9C | 9C |
| PRE-EMERGENCE | | |
| Sorghum | 2C,9H | 5C,9H |
| Corn | 2C,6H | 5C,9G |
| Soybean | 1C | 2C,8H |
| Wheat | 0 | 5C,9G |
| Wild Oats | 0 | 2C,8G |
| Rice | 8H | 10E |
| Barnyardgrass | 1C | 5C,9G |
| Crabgrass | 0 | 2C,9G |
| Morningglory | 2C,8H | 8G |
| Cocklebur | 8H | 8H |
| Sicklepod | 4G | 5C,9G |
| Nutsedge | 0 | 10E |
| Sugar Beet | 9G | 10C |

TABLE A (continued)

| | Compound 63 | Compound 64 |
|---|---|---|
| Rate (kg/ha) | 0.05 | 0.05 |
| POST-EMERGENCE | | |
| Bush bean | 9D,9G,6Y | 5C,9G,6Y |
| Cotton | 4C,7G | 2G |
| Sorghum | 3C,9G | 5C,9G |
| Corn | 9C | 9C |
| Soybean | 4C,9G | 5C,9G |
| Wheat | 9C | 9C |
| Wild Oats | 9C | 9C |
| Rice | 4C,9G | 5C,9G |
| Barnyardgrass | 9C | 9C |
| Crabgrass | 2C,9G | 5C,9G |
| Morningglory | 1C,3G | 2C,7G |
| Cocklebur | 2C,7H | 5C,9G |
| Sicklepod | 2C,5H | 3C,8G |
| Nutsedge | 2G | 2C,9G |
| Sugar beet | 1C | 5G |
| PRE-EMERGENCE | | |
| Sorghum | 5H | 1C,9G |
| Corn | 2C,9H | 2C,9G |
| Soybean | 2G | 2C,6H |
| Wheat | 2C,8G | 9H |
| Wild Oats | 2G | 2C,9G |
| Rice | 9H | 10E |
| Barnyardgrass | 0 | 5C,9H |
| Crabgrass | 0 | 5G |
| Morningglory | 0 | 7G |
| Cocklebur | 8G | 9H |
| Sicklepod | 1C,5G | 8G |
| Nutsedge | 0 | 10E |
| Sugar Beet | 7G | 9G |

TABLE A (continued)

| Rate (kg/ha) | Compound 65<br>0.05 | Compound 66<br>0.05 |
|---|---|---|
| POST-EMERGENCE | | |
| Bush bean | 5C,9G,6Y | 9C |
| Cotton | — | 4C,9G |
| Sorghum | 6C,9G | 5C,9G |
| Corn | 6C,9H | 9C |
| Soybean | 5C,9H | 4C,9G |
| Wheat | 9C | 9C |
| Wild Oats | 6C,9H | 5C,9G |
| Rice | 6C,9G | 6C,9G |
| Barnyardgrass | 5C,9H | 9C |
| Crabgrass | 2C | 9C |
| Morningglory | 4C,9H | 4C,9G |
| Cocklebur | 4C,9H | 10C |
| Sicklepod | 1C | 3C,9G |
| Nutsedge | 2C,9G | 6C,9G |
| Sugar beet | 4C,9H | 5C,9G |
| PRE-EMERGENCE | | |
| Sorghum | 3C,9G | 5C,9H |
| Corn | 3C,7H | 3C,9G |
| Soybean | 3C,3H | 3C,7G |
| Wheat | 3C,9H | 3C,9H |
| Wild Oats | 2C,9G | 3C,9H |
| Rice | 5C,9H | 10E |
| Barnyardgrass | 1C | 2C,9H |
| Crabgrass | 2G | 5C,9G |
| Morningglory | 9G | 9G |
| Cocklebur | 3C,7H | 9H |
| Sicklepod | 2C | 1C,9G |
| Nutsedge | 0 | 10E |
| Sugar Beet | 3C,7H | 5C,9G |

**Claims for the Contracting States: BE CH DE FR GB IT LI LU NL SE**

1. A compound of the formula:

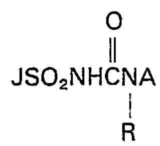

I

wherein
J is

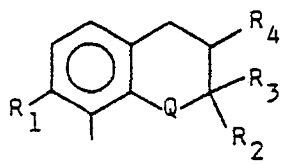
,
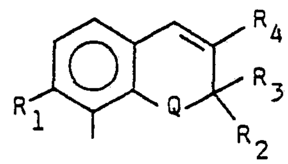
,

$J_1$ $J_2$

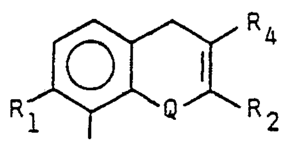
,
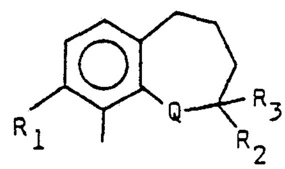
,

$J_3$ $J_4$

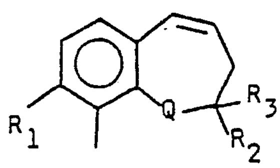
,
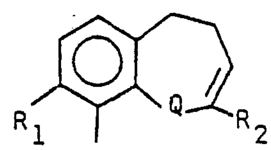

$J_5$ $J_6$

or

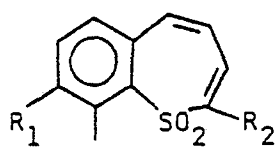
;

$J_7$

and
Q is O, S or $SO_2$;
R is H or $CH_3$;
$R_1$ is H, $CH_3$, $OCH_3$, Cl, Br, $CO_2R_5$, $SO_2R_6$, $OSO_2R_7$ or $SO_2NR_8R_9$;
$R_2$ and $R_3$ are independently H or $C_1$—$C_3$ alkyl;
$R_4$ is H or $CH_3$;
$R_5$ is $C_1$—$C_3$ alkyl, $CH_2CH{=}CH_2$, $CH_2CH_2OCH_3$ or $CH_2CH_2Cl$;
$R_6$ is $C_1$—$C_3$ alkyl;
$R_7$ is $C_1$—$C_3$ alkyl or $CF_3$;
$R_8$ and $R_9$ are independently $C_1$—$C_2$ alkyl;

A is

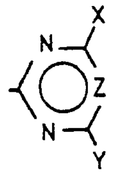 , 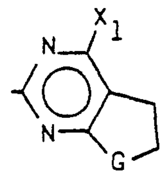 ,

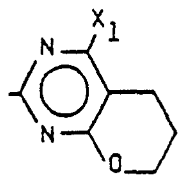 or 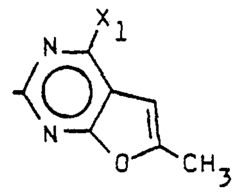 ;

G is O or $CH_2$;
X is $CH_3$, $OCH_3$ or Cl;
$X_1$ is $CH_3$, $OCH_3$ or $OC_2H_5$;
Y is $CH_3$, $OCH_3$, $OC_2H_5$, $CH_2OCH_3$, $C_2H_5$, $NH_2$, $NHCH_3$ or $N(CH_3)_2$;
Z is CH or N;

and their agriculturally suitable salts provided that

1) when X is Cl, then Z is CH and Y is $OCH_3$, $NH_2$, $NHCH_3$, $N(CH_3)_2$ or $OC_2H_5$;
2) the total number of carbon atoms in $R_2$ and $R_3$ are less than or equal to 3;
3) In Structure $J_2$ when $R_2$ and $R_3$ is other than H, then $R_4$ is H;
4) In Structure $J_3$ when $R_2$ is other than H, then $R_4$ is H;
5) when Q is S, then $R_1$ is not $SO_2NR_8R_9$;
6) In Structures $J_3$ and $J_6$, Q may not be O;
7) In Formula $J_7$, $R_1$ is not $CH_3$;
8) when J is $J_1$ wherein Q is O and $R_1$, $R_2$, $R_3$ and $R_4$ are H and A is

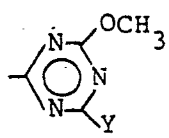 ,

then Y is $OC_2H_5$, $CH_2OCH_3$, $C_2H_5$, $NH_2$, or $NHCH_3$; and

9) when J is $J_1$ wherein Q is O and $R_1$, $R_2$, $R_3$ and $R_4$ are H and A is

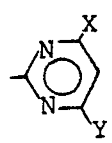 ,

wherein X is $CH_3$ or $OCH_3$, then Y is $OC_2H_5$, $CH_2OCH_3$, $C_2H_5$, $NH_2$, $NHCH_3$ or $N(CH_3)_2$.

2. Compounds of Claim 1 wherein J is $J_1$.
3. Compounds of Claim 1 wherein J is $J_2$.
4. Compounds of Claim 1 wherein J is $J_3$.
5. Compounds of Claim 1 wherein J is $J_4$.
6. Compounds of Claim 1 wherein J is $J_5$.
7. Compounds of Claim 1 wherein J is $J_6$.
8. Compounds of Claim 1 wherein J is $J_7$.
9. Compounds of any of Claims 2 to 8 where R is H.
10. Compounds of Claim 9 where $R_1$ is H.
11. Compounds of Claim 10 where A is

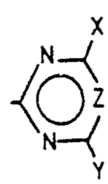

and X is $OCH_3$ or Cl.

12. Compounds of Claim 11 where Y is $CH_3$ or $OCH_3$.

13. The compound of Claim 1 which is N-[(4-methoxy-6-methylpyrimidin-2-yl)aminocarbonyl]-3,4-dihydro-2H-1-benzothiopyran-8-sulfonamide, 1,1-dioxide.

14. The compound of Claim 1 which is N[(4,6-dimethoxypyrimidin-2-yl)aminocarbonyl]3,4-dihydro-2H-1-benzothiopyran-8-sulfonamide, 1,1-dioxide.

15. The compound of Claim 1 which is N-[(4-methoxy-6-methyl-1,3,5-triazin-2-yl)aminocarbonyl]-3,4-dihydro-2H-1-benzothiopyran-8-sulfonamide, 1,1-dioxide.

16. A compound of the formula:

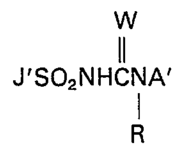

I'

wherein J' is

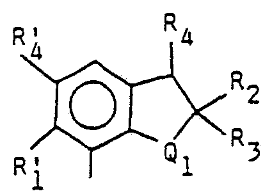
,
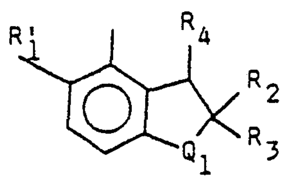
,
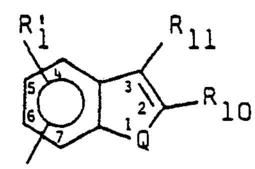

J'1 J'2 J'3

and

Q is O, S or $SO_2$;
$Q_1$ is O, SO or $SO_2$;
R is H or $CH_3$;
$R_1'$ is H, $CH_3$, $OCH_3$, Cl, Br, $NO_2$, $CO_2R_5$, $SO_2R_6$, $OSO_2R_7$ or $SO_2NR_8R_9$;
$R_2$ is H or $C_1$—$C_3$ alkyl;
$R_3$ is H or $CH_3$;
$R_4$ is H or $CH_3$;
$R_4'$ is H, Cl, $CH_3$, $CF_3$, $OCH_3$ or Br;
$R_5$ is $C_1$—$C_3$ alkyl, $CH_2CH{=}CH_2$, $CH_2CH_2OCH_3$ or $CH_2CH_2Cl$;
$R_6$ is $C_1$—$C_3$ alkyl;
$R_7$ is $C_1$—$C_3$ alkyl or $CF_3$;
$R_8$ and $R_9$ are independently $C_1$—$C_2$ alkyl;
$R_{10}$ is H, Cl, Br or $C_1$—$C_3$ alkyl;
$R_{11}$ is H, $CH_3$, Cl or Br;
W is O or S;
A' is

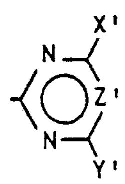
,
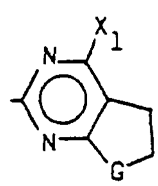
,
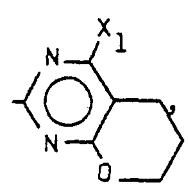
.

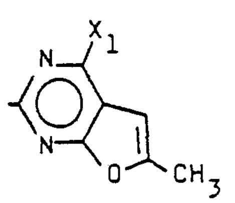
or
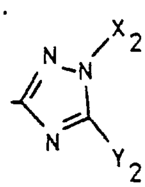
;

G is O or $CH_2$;
X' is H, $CH_3$, $OCH_3$ or Cl;
$X_1$ is $CH_3$, $OCH_3$ or $OC_2H_5$;

$X_2$ is $CH_3$, $C_2H_5$ or $CH_2CF_3$;
Y' is $CH_3$, $OCH_3$, $OC_2H_5$, $CH_2OCH_3$, $NH_2$, $NHCH_3$, $N(CH_3)_2$, $CH(OCH_3)_2$,

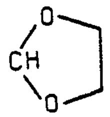 ,

$C_2H_5$, $CF_3$, $CH_2{=}CHCH_2O$, $SCH_3$, $HC{\equiv}CCH_2O$ or $CF_3CH_2O$;
$Y_2$ is $C_2H_5$, $CH_3$, $OCH_3$, $OC_2H_5$, $SCH_3$ or $SC_2H_5$; and
Z' is CH, N, $CCH_3$, $CC_2H_5$, CCl or CBr;
and their agriculturally suitable salts provided that
1) in Structures $J_1'$ and $J_2'$, when $Q_1$ is O and $R_1'$ is other than H, Cl, Br or $NO_2$, then at least one of $R_2$ and $R_3$ is alkyl, and when Q is S, then $R_1'$ cannot be $NO_2$;
2) in Structure $J_3'$, when $R_1'$ is $NO_2$ or $SO_2NR_8R_9$, then $R_{10}$ is $C_1$—$C_3$ alkyl and $R_{11}$ is $CH_3$;
3) when X' is Cl, then Z' is CH and Y' is $OCH_3$, $NH_2$, $NHCH_3$ or $N(CH_3)_2$;
4) when $Q_1$ is SO, then W is O; and
5) when $R_4'$ is other than H, then $R_1'$ is H.

17. Compounds of Claim 16 where J' is $J_1'$ and $R_4$ is H.
18. Compounds of Claim 16 where J' is $J_2'$.
19. Compounds of Claim 17 or 18 where W is O and R is H.
20. Compounds of Claim 19 where $R_1'$ is H, Cl, $CH_3$, $OCH_3$, $CO_2R_5$ or $SO_2R_6$.
21. Compounds of Claim 20 where $R_4'$ (if present) and $R_1'$ are H and $R_2$ and $R_3$ are independently H or $CH_3$.
22. Compounds of Claim 16 where J' is $J_3'$, W is O and R is H.
23. Compounds of Claim 22 where $R_{11}$ is H and $R_1'$ is H, Cl, $CH_3$, $OCH_3$, $CO_2R_5$ or $SO_2R_6$ and $SO_2NHC(W)NRA'$ is bonded to the 7-position.
24. Compounds of Claim 23 where $R_1'$ is H and $R_{10}$ is H or $CH_3$.
25. Compounds of Claims 21 or 24 where A' is

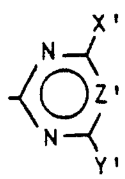 ;

Z' is CH or N; and X' is $CH_3$, $OCH_3$ or CL.
26. Compounds of Claim 25 where Y' is $CH_3$, $OCH_3$, $CH_2OCH_3$ or $N(CH_3)_2$.
27. The compound of Claim 16 which is N-[(4,6-dimethylpyrimidin-2-yl)aminocarbonyl]-2,3-dihydro-2-methyl-7-benzothiophenesulfonamide, 1,1-dioxide.
28. The compound of Claim 16 which is N-[(4-methoxy-6-methylpyrimidin-2-yl)aminocarbonyl]-2,3-dihydro-2-methyl-7-benzothiophenesulfonamide, 1,1-dioxide.
29. The compound of Claim 16 which is N-[(4,6-dimethoxypyrimidin-2-yl)aminocarbonyl]-2,3-dihydro-2-methyl-7-benzothiophenesulfonamide, 1,1-dioxide.
30. The compound of Claim 16 which is N-[(4,6-dimethyl-1,3,5-triazin-2-yl)aminocarbonyl]-2,3-dihydro-2-methyl-7-benzothiophenesulfonamide, 1,1-dioxide.
31. The compound of Claim 16 which is N-[(4-methoxy-6-methyl-1,3,5-triazin-2-yl)aminocarbonyl]-2,3-dihydro-2-methyl-7-benzothiophenesulfonamide, 1,1-dioxide.
32. The compound of Claim 16 which is N-[(4,6-dimethoxy-1,3,5-triazin-2-yl)aminocarbonyl]-2,3-dihydro-2-methyl-7-benzothiophenesulfonamide, 1,1-dioxide.
33. The compound of Claim 16 which is 2,3-dihydro-N-[(4-methoxy-6-methylpyrimidin-2-yl)aminocarbonyl]-2-methylbenzofuran-7-sulfonamide.
34. The compound of Claim 16 which is N-[(4,6-dimethoxypyrimidin-2-yl)aminocarbonyl]-2,3-dihydro-2-methylbenzofuran-7-sulfonamide.
35. The compound of Claim 16 which is N-[(4,6-dimethoxypyrimidin-2-yl)aminocarbonyl]-2,3-dihydrobenzo[b]thiophene-7-sulfonamide, 1,1-dioxide.
36. The compound of Claim 16 which is 2,3-dihydro-N-[(4-methoxy-6-methylpyrimidin-2-yl)aminocarbonyl]benzo[b]thiophene-7-sulfonamide, 1,1-dioxide.
37. A compound of Claim 16 wherein J' is $J_1'$ or $J_3'$; $R_1'$, $R_2$, $R_3$, $R_4$, $R_5$, $R_6$, $R_7$, $R_8$ and $R_9$ are as defined in Claim 16; $R_4'$ is H, $R_{10}$ is H, $CH_3$, Cl or Br, W is O, Q is O, S or $SO_2$, A' is as defined in Claim 16 excepting

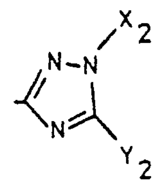

X', $X_1$, G and Z' are as defined in Claim 16, Y' is $CH_3$, $OCH_3$, $OC_2H_5$, $CH_2OCH_3$, $NH_2$, $NHCH_3$ or $N(CH_3)_2$.

38. A composition suitable for controlling the growth of undesired vegetation which comprises an effective amount of a compound of any of Claims 1 to 37 and at least one of the following: surfactant, solid or liquid diluent.

39. A method for controlling the growth of undesired vegetation which comprises applying to the locus to be protected an effective amount of a compound of any of Claims 1 to 37.

40. A method for regulating the growth of plants which comprises applying to the locus of such plants an effective but substantially non-phytotoxic amount of a compound according to any of claims 1 to 37.

41. A method for synthesizing compounds of the formula

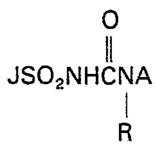 or 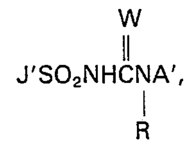,

wherein J, J', A, A', R and W are as defined in Claims 1 or 16, which comprises the steps of:

(I) (a) contacting and reacting an appropriate sulfonamide compound of general formula

$JSO_2NH_2$ (IV) or $J'SO_2NH_2$ (IV')

with diphenylcarbonate in the presence of a strong base;

(b) isolating the resulting sulfonylcarbamate of formula

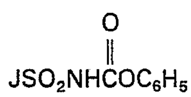 or 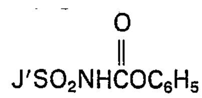

and contacting and reacting said sulfonylcarbamate with an appropriate amine

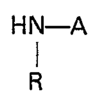 or 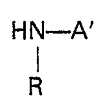

(III) (III')

to produce compounds of the desired formula or

(II) (a) contacting and reacting said sulfonamide (IV) or (IV') with an appropriate methyl carbamate compound of formula

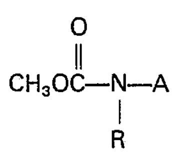 or 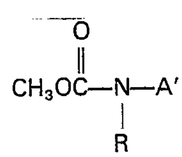

(V) (V')

in the presence of trimethylaluminum; and

(b) isolating the resulting product of the desired formula; or

(III) (a) contacting and reacting an appropriate sulfonyl isocyanate compound of formula

$JSO_2NCO$ or $J'SO_2NCW$

(VI) (VI')

with an appropriate amine of said formula (III) or (III') in an inert aprotic solvent; and
(b) isolating the resulting product of the desired formula.

**Claims for the Contracting State: AT**

1. A process for the preparation of a compound of the formula:

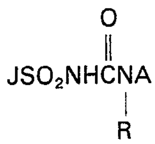

I

wherein
J is

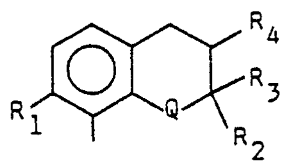

,

J1

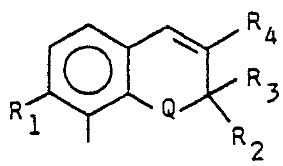

,

J2

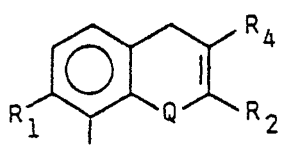

,

J3

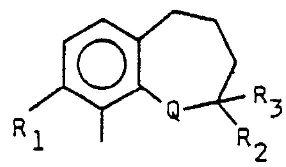

,

J4

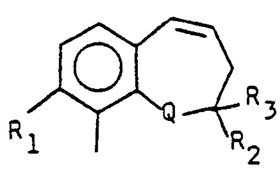

,

J5

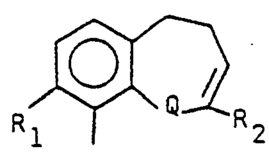

J6

or

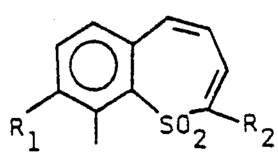

;

J7

and
Q is O, S or $SO_2$;
R is H or $CH_3$;
$R_1$ is H, $CH_3$, $OCH_3$, Cl, Br, $CO_2R_5$, $SO_2R_6$, $OSO_2R_7$ or $SO_2NR_8R_9$;
$R_2$ and $R_3$ are independently H or $C_1$—$C_3$ alkyl;

$R_4$ is H or $CH_3$;
$R_5$ is $C_1$—$C_3$ alkyl, $CH_2CH{=}CH_2$, $CH_2CH_2OCH_3$ or $CH_2CH_2Cl$;
$R_6$ is $C_1$—$C_3$ alkyl;
$R_7$ is $C_1$—$C_3$ alkyl or $CF_3$;
$R_8$ and $R_9$ are independently $C_1$—$C_2$ alkyl;
A is

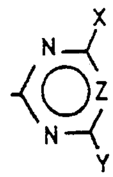 , 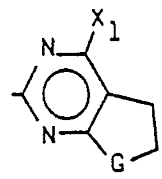 ,

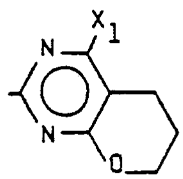 or 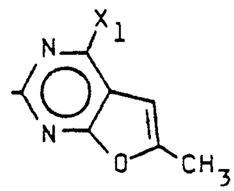 ;

G is O or $CH_2$;
X is $CH_3$, $OCH_3$ or Cl;
$X_1$ is $CH_3$, $OCH_3$ or $OC_2H_5$;
Y is $CH_3$, $OCH_3$, $OC_2H_5$, $CH_2OCH_3$, $C_2H_5$, $NH_2$, $NHCH_3$ or $N(CH_3)_2$;
Z is CH or N;

and their agriculturally suitable salts provided that

1) when X is Cl, then Z is CH and Y is $OCH_3$, $NH_2$, $NHCH_3$, $N(CH_3)_2$ or $OC_2H_5$;
2) the total number of carbon atoms in $R_2$ and $R_3$ are less than or equal to 3;
3) In Structure $J_2$ when $R_2$ and $R_3$ is other than H, then $R_4$ is H;
4) In Structure $J_3$ when $R_2$ is other than H, then $R_4$ is H;
5) when Q is S, then $R_1$ is not $SO_2NR_8R_9$;
6) In Structures $J_3$ and $J_6$, Q may not be O;
7) In Formula $J_7$, $R_1$ is not $CH_3$;
8) when J is $J_1$ wherein Q is O and $R_1$, $R_2$, $R_3$ and $R_4$ are H and A is

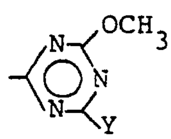 ,

then Y is $OC_2H_5$, $CH_2OCH_3$, $C_2H_5$, $NH_2$, or $NHCH_3$; and

9) when J is $J_1$ wherein Q is O and $R_1$, $R_2$, $R_3$ and $R_4$ are H and A is

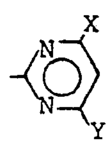 ,

wherein X is $CH_3$ or $OCH_3$, then Y is $OC_2H_5$, $CH_2OCH_3$, $C_2H_5$, $NH_2$, $NHCH_3$ or $N(CH_3)_2$, which comprises the steps of:

(I) (a) contacting and reacting an appropriate sulfonamide compound of general formula

$$JSO_2NH_2$$

(IV)

with diphenylcarbonate in the presence of a strong base;

(b) isolating the resulting sulfonylcarbamate of formula

$$JSO_2NH\overset{O}{\overset{\|}{C}}OC_6H_5$$

and contacting and reacting said sulfonylcarbamate with an appropriate amine

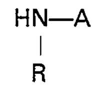 (III)

to produce compounds of the desired formula or

(II) (a) contacting and reacting said sulfonamide (IV) with an appropriate methyl carbamate compound of formula

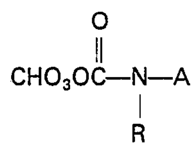

(V)

in the presence of trimethylaluminum; and

(b) isolating the resulting product of the desired formula; or

(III) (a) contacting and reacting an appropriate sulfonyl isocyanate compound of formula

$JSO_2NCO$

(VI)

with an appropriate amine of said formula (III) in an inert aprotic solvent; and

(b) isolating the resulting product of the desired formula.

2. A process of Claim 1 wherein J is $J_1$.

3. A process of Claim 1 wherein J is $J_2$.

4. A process of Claim 1 wherein J is $J_3$.

5. A process of Claim 1 wherein J is $J_4$.

6. A process of Claim 1 wherein J is $J_5$.

7. A process of Claim 1 wherein J is $J_6$.

8. A process of Claim 1 wherein J is $J_7$.

9. A process of any of Claims 2 to 8 where R is H.

10. A process of Claim 9 where $R_1$ is H.

11. A process of Claim 10 where A is

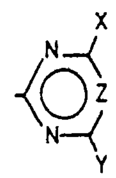

and X is $OCH_3$ or Cl.

12. A process of Claim 11 where Y is $CH_3$ or $OCH_3$.

13. A process of Claim 1 wherein the product is N-[(4-methoxy-6-methylpyrimidin-2-yl)aminocarbonyl]-3,4-dihydro-2H-1-benzothiopyran-8-sulfonamide, 1,1-dioxide or an agriculturally suitable salt thereof.

14. A process of Claim 1 wherein the product is N[(4,6-dimethoxypyrimidin-2-yl)aminocarbonyl]3,4-dihydro-2H-1-benzothiopyran-8-sulfonamide, 1,1-dioxide or an agriculturally suitable salt thereof.

15. A process of Claim 1 wherein the product is N-(4-methoxy-6-methyl-1,3,5-triazin-2-yl)aminocarbonyl]-3,4-dihydro-2H-1-benzothiopyran-8-sulfonamide, 1,1-dioxide or an agriculturally suitable salt thereof.

16. A process for the preparation of a compound of the formula:

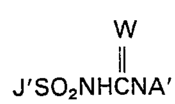

I'

wherein J′ is

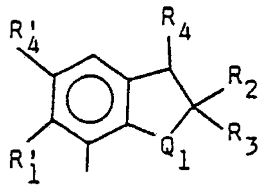 , 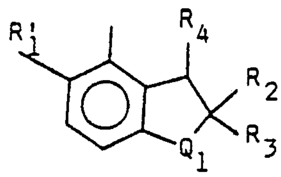 , 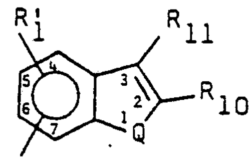

$\underline{J_1'}$ $\underline{J_2'}$ $\underline{J_3'}$

and

Q is O, S or $SO_2$;
$Q_1$ is O, S, SO or $SO_2$;
R is H or $CH_3$;
$R_1'$ is H, $CH_3$, $OCH_3$, Cl, Br, $NO_2$, $CO_2R_5$, $SO_2R_6$, $OSO_2R_7$ or $SO_2NR_8R_9$;
$R_2$ is H or $C_1$—$C_3$ alkyl;
$R_3$ is H or $CH_3$;
$R_4$ is H or $CH_3$;
$R_4'$ is H, Cl, $CH_3$, $CF_3$, $OCH_3$ or Br;
$R_5$ is $C_1$—$C_3$ alkyl, $CH_2CH{=}CH_2$, $CH_2CH_2OCH_3$ or $CH_2CH_2Cl$;
$R_6$ is $C_1$—$C_3$ alkyl;
$R_7$ is $C_1$—$C_3$ alkyl or $CF_3$;
$R_8$ and $R_9$ are independently $C_1$—$C_2$ alkyl;
$R_{10}$ is H, Cl, Br or $C_1$—$C_3$ alkyl;
$R_{11}$ is H, $CH_3$, Cl or Br;
W is O or S;
A′ is

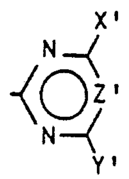 , 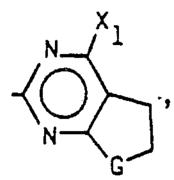 , 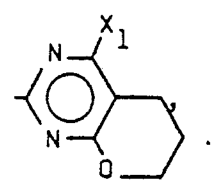

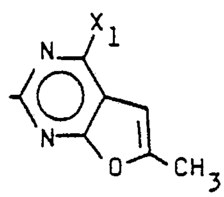 or 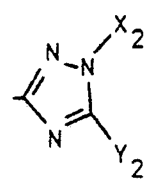 ;

G is O or $CH_2$;
X′ is H, $CH_3$, $OCH_3$ or Cl;
$X_1$ is $CH_3$, $OCH_3$ or $OC_2H_5$;
$X_2$ is $CH_3$, $C_2H_5$ or $CH_2CF_3$;
Y′ is $CH_3$, $OCH_3$, $OC_2H_5$, $CH_2OCH_3$, $NH_2$, $NHCH_3$, $N(CH_3)_2$, $CH(OCH_3)_2$,

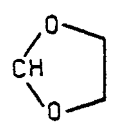 ,

$C_2H_5$, $CF_3$, $CH_2{=}CHCH_2O$, $SCH_3$, $HC{\equiv}CCH_2O$ or $CF_3CH_2O$;
$Y_2$ is $C_2H_5$, $CH_3$, $OCH_3$, $OC_2H_5$, $SCH_3$ or $SC_2H_5$; and
Z′ is CH, N, $CCH_3$, $CC_2H_5$, CCl or CBr;

and their agriculturally suitable salts provided that

1) in Structures $J_1'$ and $J_2'$, when $Q_1$ is O and $R_1'$ is other than H, Cl, Br or $NO_2$, then at least one of $R_2$ and $R_3$ is alkyl, and when Q is S, then $R_1'$ cannot be $NO_2$;

2) in Structure $J_3'$, when $R_1'$ is $NO_2$ or $SO_2NR_8R_9$, then $R_{10}$ is $C_1$—$C_3$ alkyl and $R_{11}$ is $CH_3$;

3) when X′ is Cl, then Z′ is CH and Y′ is $OCH_3$, $NH_2$, $NHCH_3$ or $N(CH_3)_2$;

4) when $Q_1$ is SO, then W is O; and

5) when $R_4'$ is other than H, then $R_1'$ is H,
which comprises the steps of:

(I) (a) contacting and reacting an appropriate sulfonamide compound of general formula

$$J'SO_2NH_2$$

(IV')

with diphenylcarbonate in the presence of a strong base;

(b) isolating the resulting sulfonylcarbamate of formula

$$J'SO_2NH\overset{\overset{O}{\parallel}}{C}OC_6H_5$$

and contacting and reacting said sulfonylcarbamate with an appropriate amine

$$\underset{\underset{R}{|}}{HN}\text{—}A' \qquad (III')$$

to produce compounds of the desired formula or

(II) (a) contacting and reacting said sulfonamide (IV') with an appropriate methyl carbamate compound of formula

$$CH_3O\overset{\overset{O}{\parallel}}{C}\text{—}\underset{\underset{R}{|}}{N}\text{—}A'$$

(V')

in the presence of trimethylaluminum; and

(b) isolating the resulting product of the desired formula; or

(III) (a) contacting and reacting an appropriate sulfonyl isocyanate compound of formula

$$J'SO_2NCW$$

(VI')

with an appropriate amine of said formula (III') in an inert aprotic solvent; and

(b) isolating the resulting product of the desired formula.

17. A process of Claim 16 where J' is $J_1'$ and $R_4$ is H.

18. A process of Claim 16 where J' is $J_2'$.

19. A process of Claim 17 or 18 where W is O and R is H.

20. A process of Claim 19 where $R_1'$ is H, Cl, $CH_3$, $OCH_3$, $CO_2R_5$ or $SO_2R_6$.

21. A process of Claim 20 where $R_4'$ (if present) and $R_1'$ are H and $R_2$ and $R_3$ are independently H or $CH_3$.

22. A process of Claim 16 where J' is $J_3'$, W is O and R is H.

23. A process of Claim 22 where $R_{11}$ is H and $R_1'$ is H, Cl, $CH_3$, $OCH_3$, $CO_2R_5$ or $SO_2R_6$ and $SO_2NHC(W)NRA'$ is bonded to the 7-position.

24. A process of Claim 23 where $R_1'$ is H and $R_{10}$ is H or $CH_3$.

25. A process of Claims 21 or 24 where A' is

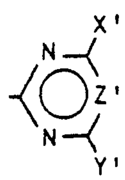 ;

Z' is CH or N; and X' is $CH_3$, $OCH_3$ or CL.

26. A process of Claim 25 where Y' is $CH_3$, $OCH_3$, $CH_2OCH_3$ or $N(CH_3)_2$.

27. A process of Claim 16 which is N-[(4,6-dimethylpyrimidin-2-yl)aminocarbonyl]-2,3-dihydro-2-methyl-7-benzothiophenesulfonamide, 1,1-dioxide, or an agriculturally suitable salt thereof.

28. A process of Claim 16 wherein the product is N-[(4-methoxy-6-methylpyrimidin-2-yl)aminocarbonyl]-2,3-dihydro-2-methyl-7-benzothiophenesulfonamide, 1,1-dioxide, or an agriculturally suitable salt thereof.

29. A process of Claim 16 wherein the product is N-[(4,6-dimethoxypyrimidin-2-yl)aminocarbonyl]-2,3-dihydro-2-methyl-7-benzothiophenesulfonamide, 1,1-dioxide, or an agriculturally suitable salt thereof.

30. A process of Claim 16 wherein the product is N-[(4,6-dimethyl-1,3,5-triazin-2-yl)aminocarbonyl]-2,3-dihydro-2-methyl-7-benzothiophenesulfonamide, 1,1-dioxide, or an agriculturally suitable salt thereof.

31. A process of Claim 16 wherein the product is N-[(4-methoxy-6-methyl-1,3,5-triazin-2-yl)aminocarbonyl]-2,3-dihydro-2-methyl-7-benzothiophenesulfonamide, 1,1-dioxide, or an agriculturally suitable salt thereof.

32. A process of Claim 16 wherein the product is N-[(4,6-dimethoxy-1,3,5-triazin-2-yl)aminocarbonyl]-2,3-dihydro-2-methyl-7-benzothiophenesulfonamide, 1,1-dioxide, or an agriculturally suitable salt thereof.

33. A process of Claim 16 wherein the product is 2,3-dihydro-N-[(4-methoxy-6-methylpyrimidin-2-yl)aminocarbonyl]-2-methylbenzofuran-7-sulfonamide or an agriculturally suitable salt thereof.

34. A process of Claim 16 wherein the product is N-[(4,6-dimethoxypyrimidin-2-yl)aminocarbonyl]-2,3-dihydro-2-methylbenzofuran-7-sulfonamide or an agriculturally suitable salt thereof.

35. A process of Claim 16 wherein the product is N-[(4,6-dimethoxypyrimidin-2-yl)aminocarbonyl]-2,3-dihydrobenzo[b]thiophene-7-sulfonamide, 1,1-dioxide or an agriculturally suitable salt thereof.

36. A process of Claim 16 wherein the product is 2,3-dihydro-N-[(4-methoxy-6-methylpyrimidin-2-yl)-aminocarbonyl]benzo[b]thiophene-7-sulfonamide, 1,1-dioxide or an agriculturally suitable salt thereof.

37. A process of Claim 16 wherein J' is $J_1'$ or $J_3'$; $R_1'$, $R_2$, $R_3$, $R_4$, $R_5$, $R_6$, $R_7$, $R_8$ and $R_9$ are as defined in Claim 16; $R_4'$ is H, $R_{10}$ is H, $CH_3$, Cl or Br, W is O, Q is O, S or $SO_2$, A' is as defined in Claim 16 excepting

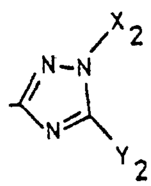

X', $X_1$, G and Z' are as defined in Claim 16, Y' is $CH_3$, $OCH_3$, $OC_2H_5$, $CH_2OCH_3$, $NH_2$, $NHCH_3$ or $N(CH_3)_2$.

38. A composition suitable for controlling the growth of undesired vegetation which comprises an effective amount of a compound of formula (I) or (I') as defined in any of Claims 1 to 37 and at least one of the following: surfactant, solid or liquid diluent.

39. A method for controlling the growth of undesired vegetation which comprises applying to the locus to be protected an effective amount of a compound of formula (I) or (I') as defined in any of Claims 1 to 37.

40. A method for regulating the growth of plants which comprises applying to the locus of such plants an effective but substantially non-phytotoxic amount of a compound of formula (I) or (I') as defined in any of claims 1 to 37.

**Patentansprüche für die Vertragsstaaten: BE CH DE FR GB IT LI LU NL SE**

1. Verbindung der Formel

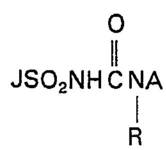

I

worin J

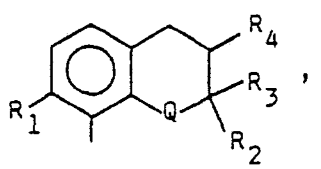

$J_1$

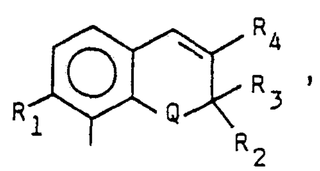

$J_2$

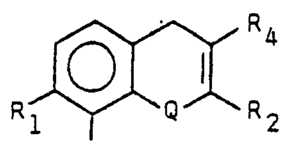

,

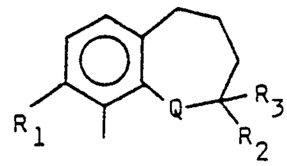

,

$\underline{J_3}$ $\underline{J_4}$

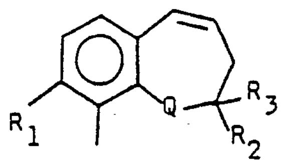

,

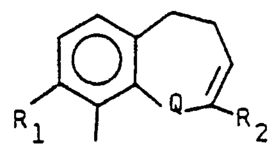

$\underline{J_5}$ $\underline{J_6}$

oder

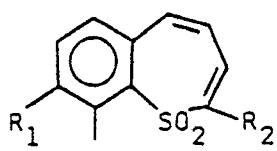

; ist;

$\underline{J_7}$

und Q O, S oder $SO_2$ ist;

R H oder $CH_3$ ist;

$R_1$ H, $CH_3$, $OCH_3$, Cl, Br, $CO_2R_5$, $SO_2R_6$, $OSO_2R_7$ oder $SO_2NR_8R_9$ ist;

$R_2$ und $R_3$ unabhängig voneinander H oder $C_1$—$C_3$-Alkyl sind;

$R_4$ H oder $CH_3$ ist;

$R_6$ $C_1$—$C_3$-Alkyl, $CH_2CH{=}CH_2$, $CH_2CH_2OCH_3$ oder $CH_2CH_2Cl$ ist;

$R_6$ $C_1$—$C_3$-Alkyl ist;

$R_7$ $C_1$—$C_3$-Alkyl oder $CF_3$ ist;

$R_8$ und $R_9$ unabhängig voneinander $C_1$—$C_2$-Alkyl sind;

A

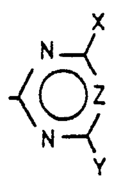

,

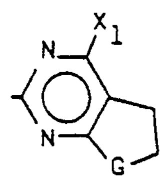

,

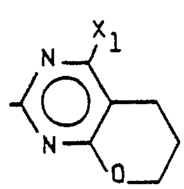

oder

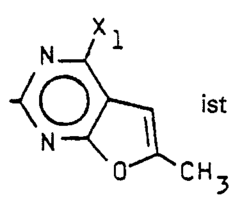

ist ;

G O oder $CH_2$ ist;

X $CH_3$, $OCH_3$ oder Cl ist;

$X_1$ $CH_3$, $OCH_3$ oder $OC_2H_5$ ist;

Y $CH_3$, $OCH_3$, $OC_2H_5$, $CH_2OCH_3$, $C_2H_5$, $NH_2$, $NHCH_3$ oder $N(CH_3)_2$ ist;

Z CH oder N ist; und ihre landwirtschaftlich geeigneten Salze, mit der Massgabe, dass

1) wenn X Cl ist, dann Z CH ist und Y $OCH_3$, $NH_2$, $NHCH_3$, $N(CH_3)_2$ oder $OC_2H_5$ ist;

2) die Gesamtzahl der Kohlenstoffatome in $R_2$ und $R_3$ weniger als oder gleich 3 ist;

3) wenn in Struktur $J_2$ $R_2$ und $R_3$ von H verschieden sind, dann $R_4$ H ist;

4) wenn $R_2$ in Struktur $J_3$ verschieden von H ist, dann $R_4$ H ist;

5) wenn Q S ist, dann $R_1$ nicht $SO_2NR_8R_9$ ist;

6) in Strukturen $J_3$ und $J_6$ Q nicht O sein darf;

7) in Formel $J_7$ $R_1$ nicht $CH_3$ ist;

8) wenn J $J_1$ ist, worin Q O ist und $R_1$, $R_2$, $R_3$ und $R_4$ H sind und A

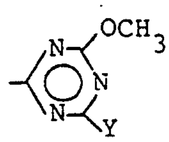,

ist, dann Y $OC_2H_5$, $CH_2OCH_3$, $C_2H_5$, $NH_2$ oder $NHCH_3$ ist; und

9) wenn J $J_2$ ist, worin Q O ist und $R_1$, $R_2$, $R_3$ und $R_4$ H sind und A

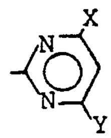,

ist, worin X $CH_3$ oder $OCH_3$ ist, dann Y $OC_2H_5$, $CH_2OCH_3$, $C_2H_5$, $NH_2$, $NHCH_3$ oder $N(CH_3)_2$ ist.

2. Verbindungen nach Anspruch 1, worin J $J_1$ ist.

3. Verbindungen nach Anspruch 1, worin J $J_2$ ist.

4. Verbindungen nach Anspruch 1, worin J $J_3$ ist.

5. Verbindungen nach Anspruch 1, worin J $J_4$ ist.

6. Verbindungen nach Anspruch 1, worin J $J_5$ ist.

7. Verbindungen nach Anspruch 1, worin J $J_6$ ist.

8. Verbindungen nach Anspruch 1, worin J $J_7$ ist.

9. Verbindungen nach einem der Ansprüche 2 bis 8, worin R H ist.

10. Verbindungen nach Anspruch 9, worin $R_1$ H ist.

11. Verbindungen nach Anspruch 10, worin A

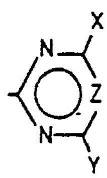

ist und X $OCH_3$ oder Cl ist.

12. Verbindungen nach Anspruch 11, worin Y $CH_3$ oder $OCH_3$ ist.

13. Verbindung nach Anspruch 1, welche N-[(4 - Methoxy - 6 - methylpyrimidin - 2 - yl)aminocarbonyl] - 3,4 - dihydro - 2H - 1 - benzothiopyran - 8 - sulfonamid - 1,1 - dioxid ist.

14. Verbindung nach Anspruch 1, welche N - [(4,6 - Dimethoxy - pyrimidin - 2 - yl)aminocarbonyl] - 3,4 - dihydro - 2H - 1 - benzothiopyran - 8 - sulfonamid - 1,1 - dioxid ist.

15. Verbindung nach Anspruch 1, welche N - [(4 - Methoxy - 6 - methyl - 1,3,5-triazin - 2 - yl)aminocarbonyl] - 3,4 - dihydro - 2H - 1 - benzothiopyran - 8 - sulfonamid - 1,1 - dioxid ist.

16. Verbindung der Formel

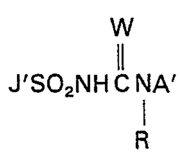 .  I'

worin J'

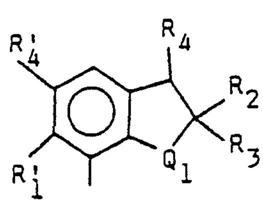,

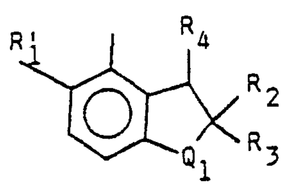,

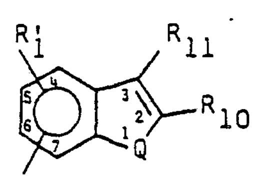

J'1    J'2    J'3

ist und

Q O, S oder $SO_2$ ist;

$Q_1$ O, S, SO oder $SO_2$ ist;

R H oder $CH_3$ ist;

$R'_1$ H, $CH_3$, $OCH_3$, Cl, Br, $NO_2$, $CO_2R_5$, $SO_2R_6$, $OSO_2R_7$ oder $SO_2NR_8R_9$ ist;

$R_2$ H oder $C_1$—$C_3$-Alkyl ist;

$R_3$ H oder $CH_3$ ist;

$R_4$ H oder $CH_3$ ist;

$R'_4$ H, Cl, $CH_3$, $CF_3$, $OCH_3$ oder Br ist;

$R_5$ $C_1$—$C_3$-Alkyl, $CH_2CH{=}CH_2$, $CH_2CH_2OCH_3$ oder $CH_2CH_2Cl$ ist;

$R_6$ $C_1$—$C_3$-Alkyl ist;

$R_7$ $C_1$—$C_3$-Alkyl oder $CF_3$ ist;

$R_8$ und $R_9$ unabhängig voneinander $C_1$—$C_2$-Alkyl sind;

$R_{10}$ H, Cl, Br oder $C_1$—$C_3$-Alkyl ist;

$R_{11}$ H, $CH_3$, Cl oder Br ist;

W O oder S ist;

A'

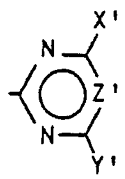 , 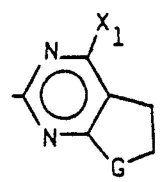 , 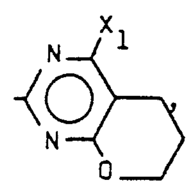 ,

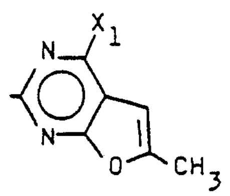 oder 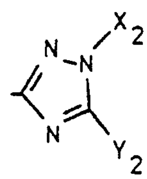 ist ;

ist;

G O oder $CH_2$ ist;

X' H, $CH_3$, $OCH_3$ oder Cl ist;

$X_1$ $CH_3$, $OCH_3$ oder $OC_2H_5$ ist;

$X_2$ $CH_3$, $C_2H_5$ oder $CH_2CF_3$ ist;

Y' $CH_3$, $OCH_3$, $OC_2H_5$, $CH_2OCH_3$, $NH_2$, $NHCH_3$, $N(CH_3)_2$; $CH(OCH_3)_2$,

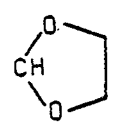 ,

$C_2H_5$, $CF_3$, $CH_2{=}CHCH_2O$, $SCH_3$, $HC{\equiv}CCH_2O$ oder $CF_3CH_2O$ ist;

$Y_2$ $C_2H_5$, $CH_3$, $OCH_3$, $OC_2H_5$, $SCH_3$ oder $SC_2H_5$ ist; und

Z' CH, N, $CCH_3$, $CC_2H_5$, CCl oder CBr ist;

und ihre landwirtschaftlich geeigneten Salze, mit der Massgabe, dass

1) wenn $Q_1$ in den Strukturen $J'_1$ und $J'_2$ O ist und $R'_1$ von H, Cl, Br oder $NO_2$ verschieden ist, dann wenigstens eines aus $R_2$ und $R_3$ Alkyl ist; und wenn Q S ist, dann $R'_1$ nicht $NO_2$ sein kann;

2) wenn $R'_1$ in Struktur $J'_3$ $NO_2$ oder $SO_2NR_8R_9$ ist, dann $R_{10}$ $C_1$—$C_3$-Alkyl ist und $R_{11}$ $CH_3$ ist;

3) wenn X' Cl ist, dann Z' CH ist und Y' $OCH_3$, $NH_2$, $NHCH_3$ oder $N(CH_3)_2$ ist;

4) wenn $Q_1$ SO ist, dann W O ist; und

5) wenn $R'_4$ von H verschieden ist, dann $R'_1$ H ist.

17. Verbindungen nach Anspruch 16, worin J' $J'_1$ ist und $R_4$ H ist.

18. Verbindungen nach Anspruch 16, worin J' $J'_2$ ist.

19. Verbindungen nach Anspruch 17 oder 18, worin W O ist und R H ist.

20. Verbindungen nach Anspruch 19, worin $R'_1$ H, Cl, $CH_3$, $OCH_3$, $CO_2R_5$ oder $SO_2R_6$ ist.

21. Verbindungen nach Anspruch 20, worin $R'_4$ (falls vorhanden) und $R'_1$ H sind und $R_2$ und $R_3$ unabhängig voneinander H oder $CH_3$ sind.

22. Verbindungen nach Anspruch 16, worin J' $J'_3$ ist, W O ist und R H ist.

23. Verbindungen nach Anspruch 22, worin $R_{11}$ H ist und $R'_1$ H, Cl, $CH_3$, $OCH_3$, $CO_2R_5$ oder $SO_2R_6$ ist und $SO_2NHC(W)NRA'$ an die 7-Stellung gebunden ist.

24. Verbindungen nach Anspruch 23, worin $R'_1$ H ist und $R_{10}$ H oder $CH_3$ ist.

25. Verbindungen nach den Ansprüchen 21 oder 24, worin A'

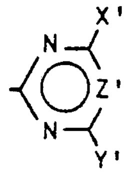 ;

ist; Z' CH oder N ist; und X' $CH_3$, $OCH_3$ oder Cl ist.

26. Verbindungen nach Anspruch 25, worin Y' $CH_3$, $OCH_3$, $CH_2OCH_3$ oder $N(CH_3)_2$ ist.

27. Verbindung nach Anspruch 16, welche N - [(4,6 - Dimethyl - pyrimidin - 2 -yl)aminocarbonyl] - 2,3 - dihydro - 2 - methyl - 7 - benzothiophensulfonamid - 1,1 - dioxid ist.

28. Verbindung nach Anspruch 16, welche N - [(4 - Methoxy - 6 - methylpyrimidin - 2 - yl)aminocarbonyl] - 2,3 - dihydro - 2 - methyl - 7 - benzothiophensulfonamid - 1,1 - dioxid ist.

29. Verbindung nach Anspruch 16, welche N - [(4,6 - Dimethoxy - pyrimidin - 2 - yl)aminocarbonyl] - 2,3 - dihydro - 2 - methyl - 7 - enzothiophensulfonamid - 1,1 - dioxid ist.

30. Verbindung nach Anspruch 16, welche N - [(4,6 - Dimethyl - 1,3,5-triazin - 2 - yl)aminocarbonyl] - 2,3 - dihydro - 2 - methyl - 7 - benzothiophensulfonamid - 1,1 - dioxid ist.

31. Verbindung nach Anspruch 16, welche N - [(4 - Methoxy - 6 - methyl - 1,3,5 - triazin - 2 - yl)aminocarbonyl] - 2,3 - dihydro - 2 - methyl - 7 - benzothiophensulfonamid - 1,1 - dioxid ist.

32. Verbindung nach Anspruch 16, welche N - [(4,6 - Dimethoxy - 1,3,5-triazin - 2 - yl)aminocarbonyl] - 2,3 - dihydro - 2 - methyl - 7 -benzothiophensulfonamid - 1,1 - dioxid ist.

33. Verbindung nach Anspruch 16, welche 2,3 - Dihydro - N - [(4 - methoxy - 6 - methylpyrimidin - 2 - yl)aminocarbonyl] - 2 - methylbenzofuran - 7 - sulfoamid ist.

34. Verbindung nach Anspruch 16, welche N - [(4,6 - Dimethoxy - pyrimidin - 2 - yl)aminocarbonyl] - 2,3 - dihydro - 2 - methyl - benzofuran - 7 - sulfoamid ist.

35. Verbindung nach Anspruch 16, welche N - [(4,6 - Dimethoxy - pyrimidin - 2 - yl)aminocarbonyl] - 2,3 - dihydrobenzo[b] - thiophen - 7 - sulfoamid - 1,1 - dioxid ist.

36. Verbindung nach Anspruch 16, welche 2,3 - Dihydro - N - [(4 - methoxy - 6 - methylpyrimidin - 2 - yl)aminocarbonyl]benzo[b]thiopen - 7 - sulfonamid - 1,1 - dioxid ist.

37. Verbindung nach Anspruch 16, worin J' $J'_1$ oder $J'_3$ ist;
$R'_1$, $R_2$, $R_3$, $R_4$, $R_5$, $R_6$, $R_7$, $R_8$ und $R_9$ wie in Anspruch 16 definiert sind;
$R'_4$ H ist,
$R_{10}$ H, $CH_3$, Cl oder Br ist,
W O ist
Q O, S oder $SO_2$ ist,
A' wie in Anspruch 16 definiert ist, ausgenommen

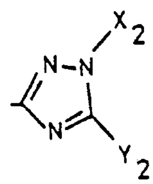

X', $X_1$, G und Z' wie in Anspruch 16 definiert sind,
Y' $CH_3$, $OCH_3$, $OC_2H_5$, $CH_2OCH_3$, $NH_2$, $NHCH_3$ oder $N(CH_3)_2$ ist.

38. Zusammensetzung, geeignet zur Bekämpfung des Wachstums unerwünschter Vegetation, welche eine wirksame Menge einer Verbindung nach einem der Ansprüche 1 bis 37 und wenigstens eines der folgenden: oberflächenaktives Mittel, festes oder flüssiges Verdünnungsmittel umfasst.

39. Verfahren zur Bekämpfung des Wachstums unerwünschter Vegetation, welches die Anwendung einer wirksamen Menge einer Verbindung nach einem der Ansprüche 1 bis 37 auf den zu schützenden Ort umfasst.

40. Verfahren zur Regulierung des Wachstums von Pflanzen, welches die Anwendung einer wirksamen, jedoch im wesentlichen nicht-phytotoxischen Menge einer Verbindung nach einem der Ansprüche 1 bis 37 auf den Ort solcher Pflanzen umfasst.

41. Verfahren zur Herstellung von Verbindungen der Formel

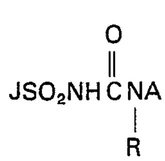 oder 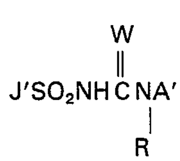

worin J, J', A, A', R und W wie in den Ansprüchen 1 oder 16 definiert sind, welches die Schritte umfasst:

(I) (a) Inberührungbringen und Reagieren einer geeigneten Sulfonamidverbindung der allgemeinen Formel

$JSO_2NH_2$ oder $J'SO_2NH_2$

(IV) (IV')

mit Diphenylcarbonat in Gegenwart einer starken Base;

(b) Isolierung der resultierenden Sulfonylcarbamats der Formel

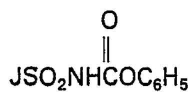 oder 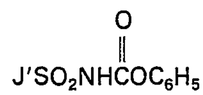

und Inberührungbringen und Reagieren des Sulfonylcarbamats mit einem geeigneten Amin

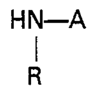 oder 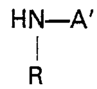

(III) (III')

unter Bildung von Verbindungen der erwünschten Formel oder

(II) (a) Inberührungbringen und Reagieren des Sulfonamids (IV) oder (IV') mit einer geeigneten Methylcarbamatverbindung der Formel

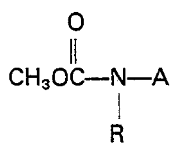 oder 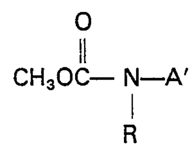

in Gegenwart von Trimethylaluminium; und

(b) Isolieren des resultierenden Produkts der erwünschten Formel; oder

(III) (a) Inberührungbringen und Reagieren einer geeigneten Sulfonylisocyanatverbindung der Formel

$JSO_2NCO$ oder $J'SO_2NCW$

(VI) (VI')

mit einem geeigneten Amin der Formel (III) oder (III') in einem inerten aprotischen Lösungsmittel; und

(b) Isolieren des resultierenden Produkts der erwünschten Formel.

**Patentansprüche für den Vertragsstaat: AT**

1. Verfahren zur Herstellung einer Verbindung der Formel

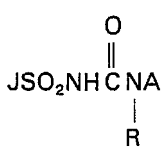 I

worin J

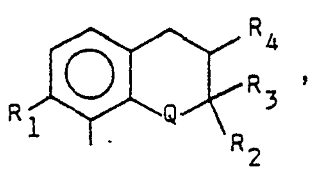 , 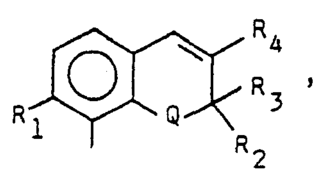 ,

J1 J2

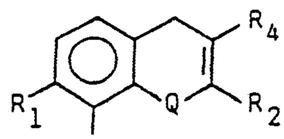 , 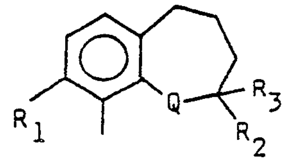 ,

$\underline{J_3}$ $\underline{J_4}$

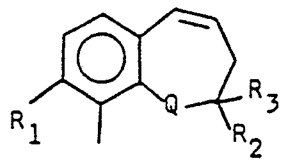 , 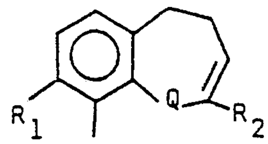

$\underline{J_5}$ $\underline{J_6}$

oder 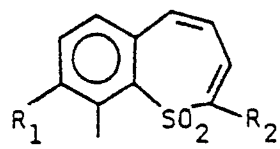 ist;

$\underline{J_7}$

und Q O, S oder $SO_2$ ist;

R H oder $CH_3$ ist;

$R_1$ H, $CH_3$, $OCH_3$, Cl, Br, $CO_2R_5$, $SO_2R_6$, $OSO_2R_7$ oder $SO_2NR_8R_9$ ist;

$R_2$ und $R_3$ unabhängig voneinander H oder $C_1$—$C_3$-Alkyl sind;

$R_4$ H oder $CH_3$ ist;

$R_5$ $C_1$—$C_3$-Alkyl, $CH_2CH{=}CH_2$, $CH_2CH_2OCH_3$ oder $CH_2CH_2Cl$ ist;

$R_6$ $C_1$—$C_3$-Alkyl ist;

$R_7$ $C_1$—$C_3$-Alkyl oder $CF_3$ ist;

$R_8$ und $R_9$ unabhängig voneinander $C_1$—$C_2$-Alkyl sind;

A

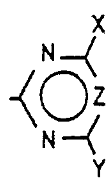 , 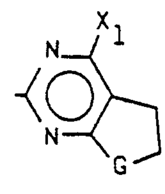 ,

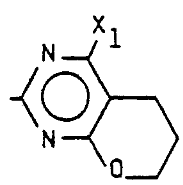 oder 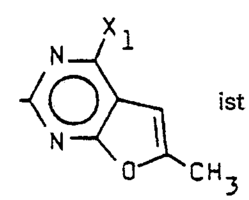 ist ;

ist;

G O oder $CH_2$ ist;

X $CH_3$, $OCH_3$ oder Cl ist;

$X_1$ $CH_3$, $OCH_3$ oder $OC_2H_5$ ist;

Y $CH_3$, $OCH_3$, $OC_2H_5$, $CH_2OCH_3$, $C_2H_5$, $NH_2$, $NHCH_3$ oder $N(CH_3)_2$ ist;

Z CH oder N ist; und ihre landwirtschaftlich geeigneten Salze, mit der Massgabe, dass

1) wenn X Cl ist, dann Z CH ist und Y $OCH_3$, $NH_2$, $NHCH_3$, $N(CH_3)_2$ oder $OC_2H_5$ ist;

2) die Gesamtzahl der Kohlenstoffatome in $R_2$ und $R_3$ weniger als oder gleich 3 ist;

3) wenn in Struktur $J_2$ $R_2$ und $R_3$ von H verschieden sind, dann $R_4$ H ist;

4) wenn $R_2$ in Struktur $J_3$ verschieden von H ist, dann $R_4$ H ist;

5) wenn Q S ist, dann $R_1$ nicht $SO_2NR_8R_9$ ist;

6) in Strukturen $J_3$ und $J_6$ Q nicht O sein darf;

7) in Formel $J_7$ $R_1$ nicht $CH_3$ ist;

8) wenn J $J_1$ ist, worin Q O ist und $R_1$, $R_2$, $R_3$ und $R_4$ H sind und A

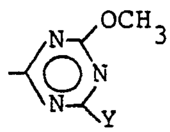

,

ist, dann Y $OC_2H_5$, $CH_2OCH_3$, $C_2H_5$, $NH_2$ oder $NHCH_3$ ist; und

9) wenn J $J_1$ ist, worin Q O ist und $R_1$, $R_2$, $R_3$ und $R_4$ H sind und A

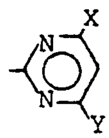

,

ist, worin X $CH_3$ oder $OCH_3$ ist, dann Y $OC_2H_5$, $CH_2OCH_3$, $C_2H_5$, $NH_2$, $NHCH_3$ oder $N(CH_3)_2$ ist, welches die Schritte umfasst:

(I) (a) Inberührungbringen und Reagieren einer geeigneten Sulfonamidverbindung der allgemeinen Formel

$$JSO_2NH_2 \qquad \text{(IV)}$$

mit Diphenylcarbonat in Gegenwart einer starken Base;

(b) Isolierung der resultierenden Sulfonylcarbamats der Formel

$$JSO_2NH\overset{\overset{\displaystyle O}{\|}}{C}OC_6H_5$$

und Inberührungbringen und Reagieren des Sulfonylcarbamats mit einem geeigneten Amin

$$\underset{\underset{\displaystyle R}{|}}{HN}—A \qquad \text{(III)}$$

unter Bildung von Verbindungen der erwünschten Formel oder

(II) (a) Inberührungbringen und Reagieren des Sulfonamids (IV) mit einer geeigneten Methylcarbamatverbindung der Formel

$$CH_3O\overset{\overset{\displaystyle O}{\|}}{C}—\underset{\underset{\displaystyle R}{|}}{N}—A \qquad \text{(V)}$$

in Gegenwart von Trimethylaluminium; und

(b) Isolieren des resultierenden Produkts der erwünschten Formel; oder

(III) (a) Inberührungbringen und Reagieren einer geeigneten Sulfonylisocyanatverbindung der Formel

$$JSO_2NCO \qquad \text{(VI)}$$

mit einem geeigneten Amin der Formel (III) in einem inerten aprotischen Lösungsmittel; und

(b) Isolieren des resultierenden Produkts der erwünschten Formel.

2. Verfahren nach Anspruch 1, worin J $J_1$ ist.

3. Verfahren nach Anspruch 1, worin J $J_2$ ist.

4. Verfahren nach Anspruch 1, worin J $J_3$ ist.

5. Verfahren nach Anspruch 1, worin J $J_4$ ist.

6. Verfahren nach Anspruch 1, worin J $J_5$ ist.

7. Verfahren nach Anspruch 1, worin J $J_6$ ist.

8. Verfahren nach Anspruch 1, worin J $J_7$ ist.

9. Verfahren nach einem der Ansprüche 2 bis 8, worin R H ist.

10. Verfahren nach Anspruch 9, worin $R_1$ H ist.

11. Verfahren nach Anspruch 10, worin A

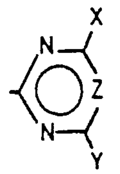

ist und X $OCH_3$ oder Cl ist.

12. Verfahren nach Anspruch 11, worin Y $CH_3$ oder $OCH_3$ ist.

13. Verfahren nach Anspruch 1, worin das Produkt N-[(4 - Methoxy - 6 - methylpyrimidin - 2 - yl)aminocarbonyl] - 3,4 - dihydro - 2H - 1 - benzothiopyran - 8 - sulfonamid - 1,1 - dioxid oder ein landwirtschaftlich geeignetes Salz davon ist.

14. Verfahren nach Anspruch 1, worin das Produkt N - [(4,6 - Dimethoxy - pyrimidin - 2 - yl)aminocarbonyl] - 3,4 - dihydro - 2H - 1 - benzo - thiopyran - 8 - sulfonamid - 1,1 - dioxid oder ein landwirtschaftlich geeignetes Salz davon ist.

15. Verfahren nach Anspruch 1, worin das Produkt N - [(4 - Methoxy - 6 - methyl - 1,3,5-triazin - 2 - yl)aminocarbonyl] - 3,4 - dihydro - 2H - 1 - benzothiopyran - 8 - sulfonamid - 1,1 - dioxid oder ein landwirtschaftlich geeignetes Salz davon ist.

16. Verfahren zur Herstellung einer Verbindung der Formel

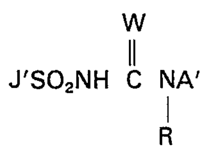

I'

worin J'

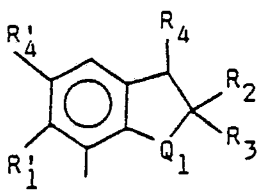

,

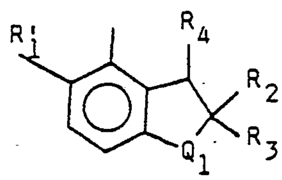

,

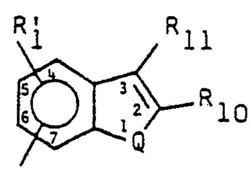

J'1 J'2 J'3

ist und

Q O, S oder $SO_2$ ist;
$Q_1$ O, S, SO oder $SO_2$ ist;
R H oder $CH_3$ ist;
$R'_1$ H, $CH_3$, $OCH_3$, Cl, Br, $NO_2$, $CO_2R_5$, $SO_2R_6$, $OSO_2R_7$ oder $SO_2NR_8R_9$ ist;
$R_2$ H oder $C_1$—$C_3$-Alkyl ist;
$R_3$ H oder $CH_3$ ist;
$R_4$ H oder $CH_3$ ist;
$R'_4$ H, Cl, $CH_3$, $CF_3$, $OCH_3$ oder Br ist;
$R_5$ $C_1$—$C_3$-Alkyl, $CH_2CH{=}CH_2$, $CH_2CH_2OCH_3$ oder $CH_2CH_2Cl$ ist;
$R_6$ $C_1$—$C_3$-Alkyl ist;
$R_7$ $C_1$—$C_3$-Alkyl oder $CF_3$ ist;
$R_8$ und $R_9$ unabhängig voneinander $C_1$—$C_2$-Alkyl sind;
$R_{10}$ H, Cl, Br oder $C_1$—$C_3$-Alkyl ist;
$R_{11}$ H, $CH_3$, Cl oder Br ist;
W O oder S ist;
A'

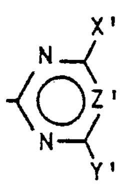

,

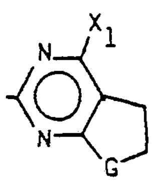

,

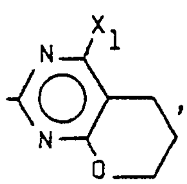

,

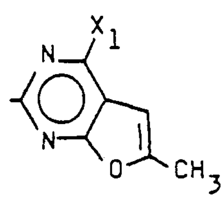

oder

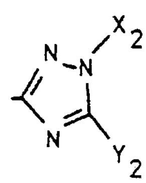

;

ist;

G O oder $CH_2$ ist;

X' H, $CH_3$, $OCH_3$ oder Cl ist;

$X_1$ $CH_3$, $OCH_3$ oder $OC_2H_5$ ist;

$X_2$ $CH_3$, $C_2H_5$ oder $CH_2CF_3$ ist;

Y' $CH_3$, $OCH_3$, $OC_2H_5$, $CH_2OCH_3$, $NH_2$, $NHCH_3$, $N(CH_3)_2$; $CH(OCH_3)_2$,

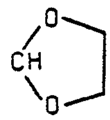 ,

$C_2H_5$, $CF_3$, $CH_2{=}CHCH_2O$, $SCH_3$, $HC{\equiv}CCH_2O$ oder $CF_3CH_2O$ ist;

$Y_2$ $C_2H_5$, $CH_3$, $OCH_3$, $OC_2H_5$, $SCH_3$ oder $SC_2H_5$ ist; und

Z' CH, N, $CCH_3$, $CC_2H_5$, CCl oder CBr ist;

und ihre landwirtschaftlich geeigneten Salze, mit der Massgabe, dass

1) wenn $Q_1$ in den Strukturen $J'_1$ und $J'_2$ O ist und $R'_1$ von H, Cl, Br oder $NO_2$ verschieden ist, dann wenigstens eines von $R_2$ und $R_3$ Alkyl ist und wenn Q S ist, dann $R'_1$ nicht $NO_2$ sein kann;

2) wenn $R'_1$ in Struktur $J'_3$ $NO_2$ oder $SO_2NR_8R_9$ ist, dann $R_{10}$ $C_1$—$C_3$-Alkyl ist und $R_{11}$ $CH_3$ ist;

3) wenn X' Cl ist, dann Z' CH ist und Y' $OCH_3$, $NH_2$, $NHCH_3$ oder $N(CH_3)_2$ ist;

4) wenn $Q_1$ SO ist, dann W O ist; und

5) wenn $R'_4$ von H verschieden ist, dann $R'_1$ H ist.

welches die Schritte umfasst:

(I) (a) Inberührungbringen und Reagieren einer geeigneten Sulfonamidverbindung der allgemeinen Formel

$$J'SO_2NH_2 \qquad (IV')$$

mit Diphenylcarbonat in Gegenwart einer starken Base;

(b) Isolieren des resultierenden Sulfonylcarbamats der Formel

$$J'SO_2NH\overset{O}{\overset{\|}{C}}OC_6H_5$$

und Inberührungbringen und Reagieren dieses Sulfonylcarbamats mit einem geeigneten Amin

$$\underset{\substack{| \\ R}}{HN}—A' \qquad (III')$$

unter Bildung von Verbindungen der erwünschten Formel oder

(II) (a) Inberührungbringen und Reagieren des Sulfonamids (IV') mit einer geeigneten Methylcarbamatverbindung der Formel

$$CH_3O\overset{O}{\overset{\|}{C}}—\underset{\substack{| \\ R}}{N}—A' \qquad (V')$$

in Gegenwart von Trimethylaluminium; und

(b) Isolieren des resultierenden Produkts der erwünschten Formel; oder

(III) (a) Inberührungbringen und Reagieren einer geeigneten Sulfonylisocyanatverbindung der Formel

$$J'SO_2NCW \qquad (VI')$$

mit einem geeigneten Amin der Formel (III') in einem inerten aprotischen Lösungsmittel; und

(b) Isolieren des resultierenden Produkts der erwünschten Formel.

17. Verfahren nach Anspruch 16, worin J' $J'_1$ ist und $R_4$ H ist.

18. Verfahren nach Anspruch 16, worin J' $J'_2$ ist.

19. Verfahren nach Anspruch 17 oder 18, worin W O ist und R H ist.

20. Verfahren nach Anspruch 19, worin $R'_1$ H, Cl, $CH_3$, $OCH_3$, $CO_2R_5$ oder $SO_2R_6$ ist.

21. Verfahren nach Anspruch 20, worin $R'_4$ (falls vorhanden) und $R'_1$ H sind und $R_2$ und $R_3$ unabhängig voneinander H oder $CH_3$ sind.

22. Verfahren nach Anspruch 16, worin J' $J'_3$ ist, W O ist und R H ist.

23. Verfahren nach Anspruch 22, worin $R_{11}$ H ist und $R'_1$ H, Cl, $CH_3$, $OCH_3$, $CO_2R_5$ oder $SO_2R_6$ ist und $SO_2NHC(W)NRA'$ an die 7-Stellung gebunden ist.

24. Verfahren nach Anspruch 23, worin $R'_1$ H ist und $R_{10}$ H oder $CH_3$ ist.

25. Verfahren nach den Ansprüchen 21 oder 24, worin A'

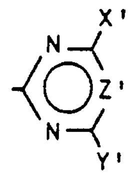 ;

ist; Z' CH oder N ist; und X' $CH_3$, $OCH_3$ oder Cl ist.

26. Verfahren nach Anspruch 25, worin Y' $CH_3$, $OCH_3$, $CH_2OCH_3$ oder $N(CH_3)_2$ ist.

27. Verfahren nach Anspruch 16, worin das Produkt N - [(4,6 - Dimethyl - pyrimidin - 2 - yl)aminocarbonyl] - 2,3 - dihydro - 2 - methyl - 7 - benzothiophensulfonamid - 1,1 - dioxid oder ein landwirtschaftlich geeignetes Salz davon ist.

28. Verfahren nach Anspruch 16, worin das Produkt N - [(4 - Methoxy - 6 - methylpyrimidin - 2 - yl)amino-carbonyl] - 2,3 - dihydro - 2 - methyl - 7 - benzothiophen - sulfonamid - 1,1 - dioxid oder ein landwirtschaftlich geeignetes Salz davon ist.

29. Verfahren nach Anspruch 16, worin das Produkt N - [(4,6 - Dimethoxy - pyrimidin - 2 - yl)aminocarbonyl] - 2,3 - dihydro - 2 - methyl - 7 - benzothiophensulfonamid - 1,1 - dioxid oder ein landwirtschaftlich geeignetes Salz davon ist.

30. Verfahren nach Anspruch 16, worin das Produkt N - [(4,6 - Dimethyl - 1,3,5-triazin - 2 - yl)aminocarbonyl] - 2,3 - dihydro - 2 - methyl - 7 - benzothiophensulfonamid - 1,1 - dioxid oder ein landwirtschaftlich geeignetes Salz davon ist.

31. Verfahren nach Anspruch 16, worin das Produkt N - [(4 - Methoxy - 6 - methyl - 1,3,5 - triazin - 2 - yl)aminocarbonyl] - 2,3 - dihydro - 2 - methyl - 7 - benzothiophensulfonamid - 1,1 - dioxid oder ein landwirtschaftlich geeignetes Salz davon ist.

32. Verfahren nach Anspruch 16, worin das Produkt N - [(4,6 - Dimethoxy - 1,3,5-triazin - 2 - yl)aminocarbonyl] - 2,3 - dihydro - 2 - methyl - 7 - benzothiophensulfonamid - 1,1 - dioxid oder ein landwirtschaftlich geeignetes Salz davon ist.

33. Verfahren nach Anspruch 16, worin das Produkt 2,3 - Dihydro - N - [(4 - methoxy 6 methylpyrimidin - 2 - yl)aminocarbonyl] - 2 - methylbenzofuran - 7 - sulfoamid oder ein landwirtschaftlich geeignetes Salz davon ist.

34. Verfahren nach Anspruch 16, worin das Produkt N - [(4,6 - Dimethoxypyrimidin - 2 - yl)aminocarbonyl] - 2,3 - dihydro - 2 - methyl - benzofuran - 7 - sulfonamid oder ein landwirtschaftlich geeignetes Salz davon ist.

35. Verfahren nach Anspruch 16, worin das Produkt N - [(4,6 - Dimethoxy - pyrimidin - 2 - yl)aminocarbonyl] - 2,3 - dihydrobenzo[b] - thiophen - 7 - sulfoamid-1,1-dioxid oder ein landwirtschaftlich geeignetes Salz davon ist.

36. Verfahren nach Anspruch 16, worin das Produkt 2,3 - Dihydro - N - [(4 - methoxy - 6 - methylpyrimidin - 2 - yl)aminocarbonyl]benzo[b]thiophen - 7 - sulfonamid - 1,1 - dioxid oder ein landwirtschaftlich geeignetes Salz davon ist.

37. Verfahren nach Anspruch 16, worin J' $J'_1$ oder $J'_3$ ist;
$R'_1$, $R_2$, $R_3$, $R_4$, $R_5$, $R_6$, $R_7$, $R_8$ und $R_9$ wie in Anspruch 16 definiert sind;
$R'_4$ H ist,
$R_{10}$ H, $CH_3$, Cl oder Br ist,
W O ist
Q O, S oder $SO_2$ ist,
A' wie in Anspruch 16 definiert ist, ausgenommen

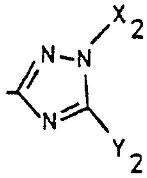

X', $X_1$, G und Z' wie in Anspruch 16 definiert sind,
Y' $CH_3$, $OCH_3$, $OC_2H_5$, $CH_2OCH_3$, $NH_2$, $NHCH_3$ oder $N(CH_3)_2$ ist.

38. Zusammensetzung, geeignet zur Bekämpfung des Wachstums unerwünschter Vegetation, welche eine wirksame Menge einer Verbindung der Formel (I) oder (I'), wie in einem der Ansprüche 1 bis 37

definiert, und wenigstens eines der folgenden: oberflächenaktives Mittel, festes oder flüssiges Verdünnungsmittel umfasst.

39. Verfahren zur Bekämpfung des Wachstums unerwünschter Vegetation, welches die Anwendung einer wirksamen Menge einer Verbindung der Formel (I) oder (I'), wie in einem der Ansprüche 1 bis 37 definiert, auf den zu schützenden Ort umfasst.

40. Verfahren zur Regulierung des Wachstums von Pflanzen, welches die Anwendung einer wirksamen, jedoch im wesentlichen nicht-phytotoxischen Menge einer Verbindung der Formel (I) oder (I'), wie in einem der Ansprüche 1 bis 37 definiert, auf den Ort solcher Pflanzen umfasst.

**Revendications pour les Etats contractants: BE CH DE FR GB IT LI LU NL SE**

1. Un composé de la formule

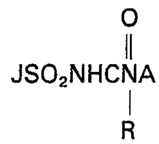

I

où

J est

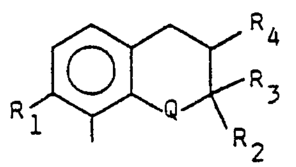 ,

J1

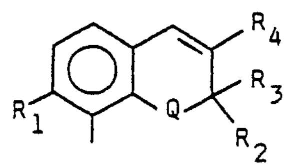 ,

J2

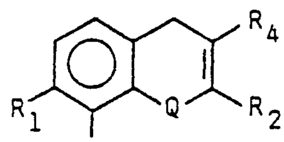 ,

J3

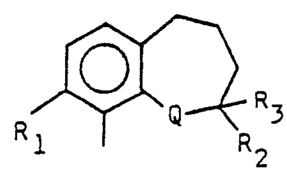 ,

J4

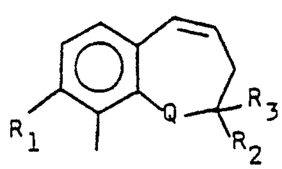 ,

J5

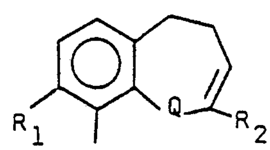

J6

ou 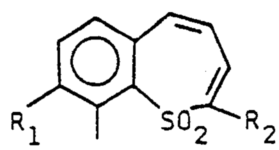 ;

J7

et Q est O, S ou $SO_2$;
R est H ou $CH_3$;
$R_1$ est H, $CH_3$, $OCH_3$, Cl, Br, $CO_2R_5$, $SO_2R_6$, $OSO_2R_7$ ou $SO_2NR_8R_9$.
$R_2$ et $R_3$ sont indépendamment, H ou un groupe alcoyle en $C_1$—$C_3$;
$R_4$ est H ou $CH_3$;

$R_5$ est un groupe alcoyle en $C_1$—$C_3$, $CH_2CH{=}CH_2$, $CH_2CH_2OCH_3$ ou $CH_2CH_2Cl$;
$R_6$ est un groupe alcoyle en $C_1$—$C_3$;
$R_7$ est un groupe alcoyle en $C_1$—$C_3$ ou $CF_3$;
$R_8$ et $R_0$ sont, indépendamment, des groupes alcoyle en $C_1$—$C_2$;
A est

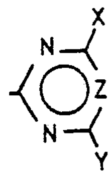 , 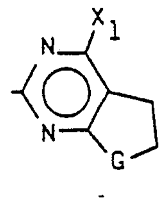 ,

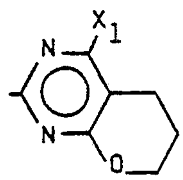 ou 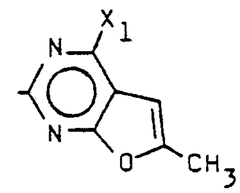 ;

G est O ou $CH_2$;
X est $CH_3$, $OCH_3$ ou Cl;
$X_1$ est $CH_3$, $OCH_3$ ou $OC_2H_5$;
Y est $CH_3$, $OCH_3$, $OC_2H_5$, $CH_2OCH_3$, $C_2H_5$, $NH_2$, $NHCH_3$ ou $N(CH_3)_2$;
Z est CH ou N; et leurs sels convenant pour l'agriculture; avec les conditions suivantes:
1) quand X est Cl, alors Z est CH et Y est $OCH_3$, $NH_2$, $NHCH_3$, $N(CH_3)_2$ ou $OC_2H_5$;
2) le nombre total d'atomes de carbone dans $R_2$ et $R_3$ est inférieur ou égal à 3;
3) dans la structure $J_2$, quand $R_2$ et $R_3$ sont autres que H, alors $R_4$ est H;
4) dans la structure $J_3$, quand $R_2$ est autre que H, alors $R_4$ est H;
5) quand Q est S, alors $R_1$ n'est pas $SO_2NR_8R_9$;
6) dans les structures $J_3$ et $J_6$, Q ne peut pas être O;
7) dans la formule $J_7$, $R_1$ n'est pas $CH_3$;
8) quand J est $J_1$ où Q est O et $R_1$, $R_2$, $R_3$ et $R_4$ sont H et A est

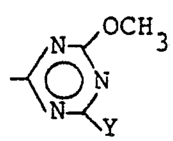 ,

alors Y est $OC_2H_5$, $CH_2OCH_3$, $C_2H_5$, $NH_2$ ou $NHCH_3$; et
9) quand J est $J_1$ où Q est O et $R_1$, $R_2$, $R_3$ et $R_4$ sont H et A est

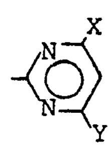 ,

où X est $CH_3$ ou $OCH_3$, alors Y est $OC_2H_5$, $CH_2OCH_3$, $C_2H_5$, $NH_2$, $NHCH_3$ ou $N(CH_3)_2$.

2. Composés selon la revendication 1, dans lesquels J est $J_1$.
3. Composés selon la revendication 1, dans lesquels J est $J_2$.
4. Composés selon la revendication 1, dans lesquels J est $J_3$.
5. Composés selon la revendication 1, dans lesquels J est $J_4$.
6. Composés selon la revendication 1, dans lesquels J est $J_5$.
7. Composés selon la revendication 1, dans lesquels J est $J_6$.
8. Composés selon la revendication 1, dans lesquels J est $J_7$.
9. Composés selon l'une quelconque des revendications 2 à 8, dans lesquels R est H.
10. Composés selon la revendication 9, dans lesquels $R_1$ est H.
11. Composés selon la revendication 10, dans lesquels A est

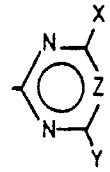

et X est $OCH_3$ ou Cl.

12. Composés selon la revendication 11, dans lesquels Y est $CH_3$ ou $OCH_3$.

13. Le composé selon la revendication 1 qui est le 1,1-dioxyde de N - [(4 - méthoxy - 6 - méthylpyrimidin - 2 - yl)amino - carbonyl] - 3,4 - dihydro - 2H - 1 - benzothiopyran - 8 - sulfonamide.

14. Le composé selon la revendication 1 qui est le 1,1 - dioxyde de N - [(4,6 - diméthoxypyrimidin - 2 - yl)amino - carbonyl] - 3,4 - dihydro - 2H - 1 - benzothiopyran - 8 - sulfonamide.

15. Le composé selon la revendication 1 qui est le 1,1-dioxyde de N - [(4 - méthoxy - 6 - méthyl - 1,3,5 - triazin - 2 - yl)aminocarbonyl] - 3,4 - dihydro - 2H - 1 - benzothiopyran - 8 - sulfonamide.

16. Un composé de la formule:

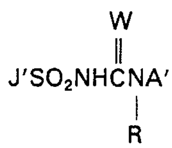

I'

où

J' est

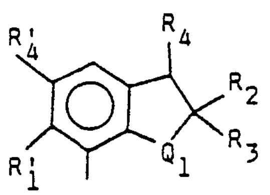 , 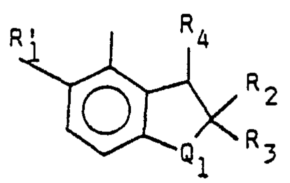 , 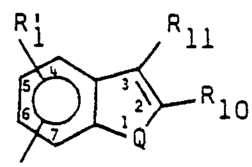

J'1 J'2 J'3

et

Q est O, S ou $SO_2$;

$Q_1$ est O, S, SO ou $SO_2$;

R est H ou $CH_3$;

$R'_1$ est H, $CH_3$, $OCH_3$, Cl, Br, $NO_2$, $CO_2R_5$, $SO_2R_6$, $OSO_2R_7$ ou $SO_2NR_8R_9$;

$R_2$ est H ou un groupe alcoyle en $C_1$—$C_3$;

$R_3$ est H ou $CH_3$;

$R_4$ est H ou $CH_3$;

$R'_4$ est H, Cl, $CH_3$, $CF_3$, $OCH_3$ ou Br;

$R_5$ est un groupe alcoyle en $C_1$—$C_3$, $CH_2CH{=}CH_2$, $CH_2CH_2OCH_3$ ou $CH_2CH_2Cl$;

$R_6$ est un groupe alcoyle en $C_1$—$C_3$;

$R_7$ est un groupe alcoyle en $C_1$—$C_3$ ou $CF_3$;

$R_8$ et $R_9$ sont, indépendamment, des groupes alcoyle en $C_1$—$C_2$;

$R_{10}$ est H, Cl, Br ou un groupe alcoyle en $C_1$—$C_3$;

$R_{11}$ est H, $CH_3$, Cl ou Br;

W est O ou S;

A' est

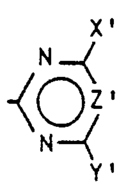 , 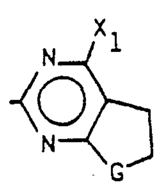 , 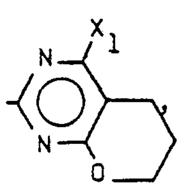 .

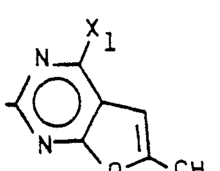 ou 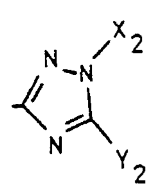 ;

G est O ou $CH_2$;
X′ est H, $CH_3$, $OCH_3$ ou Cl;
$X_1$ est $CH_3$, $OCH_3$ ou $OC_2H_5$;
$X_2$ est $CH_3$, $C_2H_5$ ou $CH_2CF_3$;
Y′ est $CH_3$, $OCH_3$, $OC_2H_5$, $CH_2OCH_3$, $NH_2$, $NHCH_3$, $N(CH_3)_2$, $CH(OCH_3)_2$,

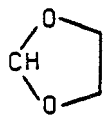 ,

$C_2H_5$, $CF_3$, $CH_2{=}CHCH_2O$, $SCH_3$, $HC{\equiv}CCH_2O$ ou $CF_3CH_2O$;
$Y_2$ est $C_2H_5$, $CH_3$, $OCH_3$, $OC_2H_5$, $SCH_3$ ou $SC_2H_5$; et
Z′ est CH, N, $CCH_3$, $CC_2H_5$, CCl ou CBr;
et leurs sels convenant pour l'agriculture; avec les conditions suivantes;

1) dans les structures $J_1'$ et $J_2'$, quand $Q_1$ est O et $R_1'$ est autre que H, Cl, Br ou $NO_2$, alors au moins un de $R_2$ et $R_3$ est un groupe alcoyle, et quand Q est S, alors $R_1'$ ne peut pas être $NO_2$;

2) dans la structure $J_3'$, quand $R_1'$ est $NO_2$ ou $SO_2NR_8R_9$, alors $R_{10}$ est un groupe alcoyle en $C_1$—$C_3$ et $R_{11}$ est $CH_3$;

3) quand X′ est Cl, alors Z′ est CH et Y′ est $OCH_3$, $NH_2$, $NHCH_3$ ou $N(CH_3)_2$;

4) quand Q est SO, alors W est O; et

5) quand $R_4'$ est autre que H, alors $R_1'$ est H.

17. Composés selon la revendication 16 dans lesquels J′ est $J_1'$ et $R_4$ est H.

18. Composés selon la revendication 16 dans lesquels J′ est $J_2'$.

19. Composés selon la revendication 17 ou 18 dans lesquels W est O et R est H.

20. Composés selon la revendication 19 dans lesquels $R_1'$ est H, Cl, $CH_3$, $OCH_3$, $CO_2R_5$ ou $SO_2R_6$.

21. Composés selon la revendication 20 dans lesquels $R_4'$ (s'il est présent) et $R_1'$ sont H et $R_2$ et $R_3$ sont indépendamment H ou $CH_3$.

22. Composés selon la revendication 16 dans lesquels J′ est $J_3'$, W est O et R est H.

23. Composés selon la revendication 22 dans lesquels $R_{11}$ est H et $R_1'$ est H, Cl, $CH_3$, $OCH_3$, $CO_2R_5$ ou $SO_2R_6$ et $SO_2NHC(W)NRA'$ est lié à la position 7.

24. Composés selon la revendication 23 dans lesquels $R_1'$ est H et $R_{10}$ est H ou $CH_3$.

25. Composés selon les revendications 21 ou 24 dans lesquels A′ est

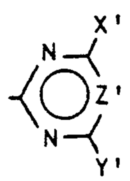 ;

Z′ est CH ou N; et
X′ est $CH_3$, $OCH_3$ ou Cl.

26. Composés selon la revendication 25 dans lesquels Y′ est $CH_3$, $OCH_3$, $CH_2OCH_3$ ou $N(CH_3)_2$.

27. Le composé selon la revendication 16 qui est le 1,1-dioxyde de N - [(4,6 - diméthylpyrimidin - 2 - yl) - aminocarbonyl] - 2,3 - dihydro - 2 - méthyl - 7 - benzothiophènesulfonamide.

28. Le composé selon la revendication 16 qui est le 1,1-dioxyde de N - [(4 - méthoxy - 6 - méthylpyrimidin - 2 - yl) - aminocarbonyl] - 2,3 - dihydro - 2 - méthyl - 7 - benzothiophènesulfonamide.

29. Le composé selon la revendication 16 qui est le 1,1-dioxyde de N - [(4,6 - diméthoxypyrimidin - 2 - yl) - aminocarbonyl] - 2,3 - dihydro - 2 - méthyl - 7 - benzothiophènesulfonamide.

30. Le composé selon la revendication 16 qui est le 1,1-dioxyde de N - [(4,6 - diméthyl - 1,3,5 - triazin - 2 - yl) - aminocarbonyl] - 2,3 - dihydro - 2 - méthyl - 7 - benzothiophènesulfonamide.

31. Le composé selon la revendication 16 qui est le 1,1-dioxyde de N - [(4 - méthoxy - 6 - méthyl - 1,3,5 - triazin - 2 - yl) - aminocarbonyl] - 2,3 - dihydro - 2 - méthyl - 7 - benzothiophènesulfonamide.

32. Le composé selon la revendication 16 qui est le 1,1-dioxyde de N - [(4,6 - diméthoxy - 1,3,5 - triazin - 2 - yl) - aminocarbonyl] - 2,3 - dihydro - 2 - méthyl - 7 - benzothiophènesulfonamide.

33. Le composé selon la revendication 16 qui est le 2,3 - dihydro - N - [(4 - méthoxy - 6 - méthylpyrimidin - 2 - yl) - aminocarbonyl] - 2 - méthylbenzofuran - 7 - sulfonamide.

34. Le composé selon la revendication 16 qui est le N - [(4,6 - diméthoxypyrimidin - 2 - yl) - aminocarbonyl] - 2,3 - dihydro - 2 - méthylbenzofuran - 7 - sulfonamide.

35. Le composé selon la revendication 16 qui est le 1,1-dioxyde de N - [(4,6 - diméthoxypyrimidin - 2 - yl) - aminocarbonyl] - 2,3 - dihydrobenzo(b)thiophène - 7 - sulfonamide.

36. Le composé selon la revendication 16 qui est le 1,1-dioxyde de 2,3 - dihydro - N - [(4 - méthoxy - 6 - méthylpyrimidin - 2 - yl) - aminocarbonyl]benzo(b)thiophène - 7 - sulfonamide.

37. Un composé selon la revendication 16 dans lequel

J′ est $J'_1$ ou $J'_3$;

$R'_1$, $R_2$, $R_3$, $R_4$, $R_5$, $R_6$, $R_7$, $R_8$ et $R_9$ sont tels que définis dans la revendication 16;

$R'_4$ et H,

$R_{10}$ est H, $CH_3$, Cl ou Br,

W est O,

Q est O, S ou $SO_2$,

A′ est tel que défini dans la revendication 16 à l'exception de

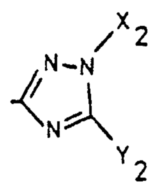

X′, $X_1$, G et Z′ sont tels que définis dans la revendication 16,

Y′ est $CH_3$, $OCH_3$, $OC_2H_5$, $CH_2OCH_3$, $NH_2$, $NHCH_3$, ou $N(CH_3)_2$.

38. Une composition utile pour lutter contre la croissance de végétation indésirable, qui comprend une quantité efficace d'un composé selon l'une quelconque des revendications 1 à 37 et au moins un des ingrédients suivants: agent tensio-actif, diluant solide ou liquide.

39. Un procédé de lutte contre le croissance de végétation indésirable, qui comprend l'application au lieu à protéger d'une quantité efficace d'un composé selon l'une quelconque des revendications 1 à 37.

40. Un procédé pour régler la croissance de plantes qui comprend l'application au site de ces plantes d'une quantité efficace, mais substantiellement non-phytoxique, d'un composé selon l'une quelconque des revendications 1 à 37.

41. Un procédé de préparation de composés de la formule

```
        O                    W
        ‖                    ‖
JSO2NHCNA       ou    J'SO2NHCNA'
         |                    |
         R                    R
```

où J, J′, A, A′, R et W sont tels que définis dans les revendications 1 ou 16, qui comprend les étapes consistant à

(I) (a) mettre en contact et faire réagir un sulfonamide approprié de formule générale

$JSO_2NH_2$ ou $J'SO_2NH_2$

(IV) (IV′)

avec du carbonate de diphényle en présence d'une base forte;

(b) isoler le sulfonylcarbamate résultant de formule

```
       O                        O
       ‖                        ‖
JSO2NHCOC6H5       ou    J'SO2NHCOC6H5
```

et mettre en contact et faire réagir ce sulfonylcarbamate avec une amine appropriée

```
HN—A      ou     HN—A'
|                |
R                R

(III)            (III')
```

de manière à produire des composés de la formule désirée, ou

(II) (a) mettre en contact et faire réagir le sulfonamide (IV) ou (IV') avec un méthylcarbamate approprié de formule

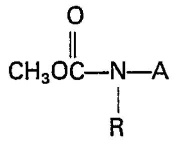 ou 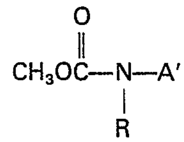

(V) (V')

en présence de triméthylaluminium; et

(b) isoler le produit résultant de la formule désirée; ou

(III) (a) mettre en contact et faire réagir un sulfonyl isocyanate approprié de formule

$JSO_2NCO$ ou $J'SO_2NCW$

(VI) (VI')

avec un amine appropriée de la formule (III) ou (III') dans un solvant aprotique inerte; et

(b) isoler le produit résultant de la formule désirée.

**Revendications pour l'Etat contractant: AT**

1. Un procédé de préparation d'un composé de la formule

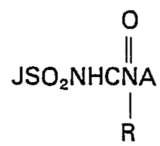

I

où

J est

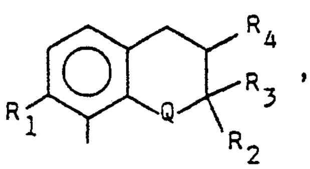, 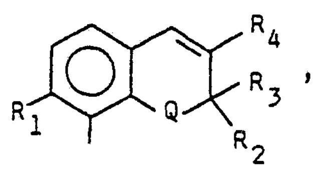,

J1 J2

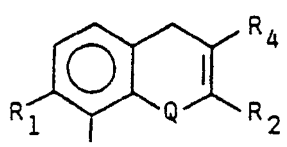, 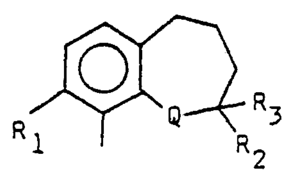,

J3 J4

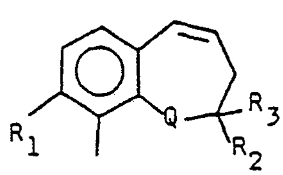, 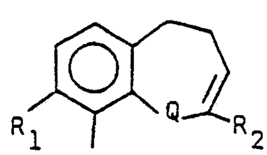 ou

J5 J6

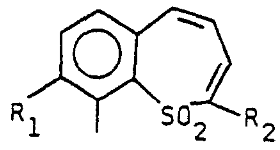 ;

$J_7$

et Q est O, S ou $SO_2$;
R est H ou $CH_3$;
$R_1$ est H, $CH_3$, $OCH_3$, Cl, Br, $CO_2R_5$, $SO_2R_6$, $OSO_2R_7$ ou $SO_2NR_8R_9$.
$R_2$ et $R_3$ sont indépendamment, H ou un groupe alcoyle en $C_1$—$C_3$;
$R_4$ est H ou $CH_3$;
$R_5$ est un groupe alcoyle en $C_1$—$C_3$, $CH_2CH{=}CH_2$, $CH_2CH_2OCH_3$ ou $CH_2CH_2Cl$;
$R_6$ est un groupe alcoyle en $C_1$—$C_3$;
$R_7$ est un groupe alcoyle en $C_1$—$C_3$ ou $CF_3$;
$R_8$ et $R_9$ sont, indépendamment, des groupes alcoyle en $C_1$—$C_2$;
A est

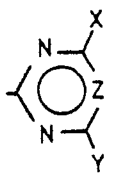 , 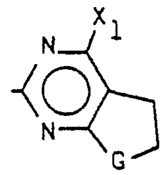 ,

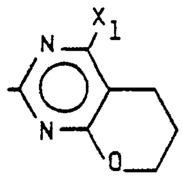 ou 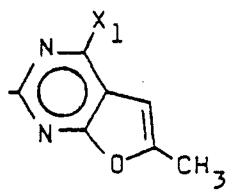 ;

G est O ou $CH_2$;
X est $CH_3$, $OCH_3$ ou Cl;
$X_1$ est $CH_3$, $OCH_3$ ou $OC_2H_5$;
Y est $CH_3$, $OCH_3$, $OC_2H_5$, $CH_2OCH_3$, $C_2H_5$, $NH_2$, $NHCH_3$ ou $N(CH_3)_2$;

et leurs sels convenant pour l'agriculture; avec les conditions suivantes:

1) quand X est Cl, alors Z est CH et Y est $OCH_3$, $NH_2$, $NHCH_3$, $N(CH_3)_2$ ou $OC_2H_5$;
2) le nombre total d'atomes de carbone dans $R_2$ et $R_3$ est inférieur ou égal à 3;
3) dans la structure $J_2$, quand $R_2$ et $R_3$ sont autres que H, alors $R_4$ est H;
4) dans la structure $J_3$, quand $R_2$ est autre que H, alors $R_4$ est H;
5) quand Q est S, alors $R_1$ n'est pas $SO_2NR_8R_9$;
6) dans les structures $J_3$ et $J_6$, Q ne peut pas être O;
7) dans la formule $J_7$, $R_1$ n'est pas $CH_3$;
8) quand J est $J_1$ où Q est O et $R_1$, $R_2$, $R_3$ et $R_4$ sont H et A est

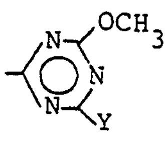 ,

alors Y est $OC_2H_5$, $CH_2OCH_3$, $C_2H_5$, $NH_2$ ou $NHCH_3$; et

9) quand J est $J_1$ où Q est O et $R_1$, $R_2$, $R_3$ et $R_4$ sont H et A est

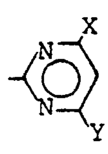 ,

où X est $CH_3$ ou $OCH_3$, alors Y est $OC_2H_5$, $CH_2OCH_3$, $C_2H_5$, $NH_2$, $NHCH_3$ ou $N(CH_3)_2$ qui comprend les étapes consistant à:

(I) (a) mettre en contact et faire réagir un sulfonamide approprié de formule générale

$$JSO_2NH_2 \qquad (IV)$$

avec du carbonate de diphényle en présence d'une base forte;

(b) isoler le sulfonylcarbamate résultant de formule

O
||
$JSO_2NHCOC_6H_5$

et mettre en contact et faire réagir ce sulfonylcarbamate avec une amine appropriée

HN—A (III)
|
H

de manière à produire des composés de la formule désirée, ou

(II) (a) mettre en contact et faire réagir le sulfonamide (IV) avec un méthylcarbamate approprié de formule

O
||
$CH_3OC$—N—A (V)
|
R

en présence de triméthylaluminium; et

(b) isoler le produit résultant de la formule désirée; ou

(III) (a) mettre en contact et faire réagir un sulfonyl isocyanate approprié de formule

$$JSO_2NHO \qquad (VI)$$

avec une amine appropriée de la formule (III) dans un solvant aprotique inerte et

(b) isoler le produit résultant de la formule désirée.

2. Un procédé selon la revendication 1, dans lequel J est $J_1$.

3. Un procédé selon la revendication 1, dans lequel J est $J_2$.

4. Un procédé selon la revendication 1, dans lequel J est $J_3$.

5. Un procédé selon la revendication 1, dans lequel J est $J_4$.

6. Un procédé selon la revendication 1, dans lequel J est $J_5$.

7. Un procédé selon la revendication 1, dans lequel J est $J_6$.

8. Un procédé selon la revendication 1, dans lequel J est $J_7$.

9. Un procédé selon l'une quelconque des revendications 2 à 8, dans lequel R est H.

10. Un procédé selon la revendication 9, dans lequel $R_1$ est H.

11. Un procédé selon la revendication 10, dans lequel A est

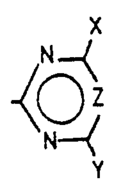

et X est $OCH_3$ ou Cl.

12. Un procédé selon la revendication 11, dans lequel Y est $CH_3$ ou $OCH_3$.

13. Un procédé selon la revendication 1, dans lequel le produit est le 1,1-dioxyde de N - [(4 - méthoxy - 6 - méthylpyrimidin - 2 - yl)aminocarbonyl] - 3,4 - dihydro - 2H - 1 - benzothiopyran - 8 - sulfonamide ou un de ses sels convenant pour l'agriculture.

14. Un procédé selon la revendication 1, dans lequel le produit est le 1,1 - dioxyde de N - [(4,6 - diméthoxypyrimidin - 2 - yl)aminocarbonyl] - 3,4 - dihydro - 2H - 1 - benzothiopyran - 8 - sulfonamide ou un de ses sels convenant pour l'agriculture.

15. Un procédé selon la revendication 1, dans lequel le produit est le 1,1-dioxyde de N - [(4 - méthoxy - 6 - méthyl - 1,3,5 - triazin - 2 - yl)aminocarbonyl] - 3,4 - dihydro - 2H - 1 - benzothiopyran - 8 - sulfonamide ou un de ses sels convenant pour l'agriculture.

16. Un procédé de préparation d'un composé de la formule:

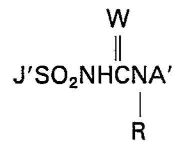 I'

où

J' est

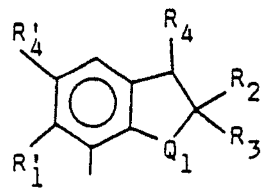 , 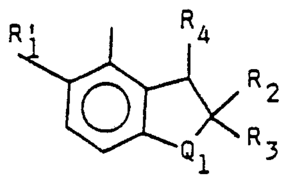 , 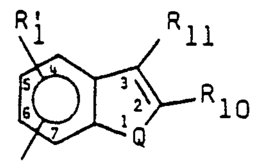

J'1 J'2 J'3

et

Q est O, S ou $SO_2$;
$Q_1$ est O, S, SO ou $SO_2$;
R est H ou $CH_3$;
$R_1'$ est H, $CH_3$, $OCH_3$, Cl, Br, $NO_2$, $CO_2R_5$, $SO_2R_6$, $OSO_2R_7$ ou $SO_2NR_8R_9$;
$R_2$ est H ou un groupe alcoyle en $C_1$—$C_3$;
$R_3$ est H ou $CH_3$;
$R_4$ est H ou $CH_3$;
$R_4'$ est H, Cl, $CH_3$, $CF_3$, $OCH_3$ ou Br;
$R_5$ est un groupe alcoyle en $C_1$—$C_3$, $CH_2CH{=}CH_2$, $CH_2CH_2OCH_3$ ou $CH_2CH_2Cl$;
$R_6$ est un groupe alcoyle en $C_1$—$C_3$;
$R_7$ est un groupe alcoyle en $C_1$—$C_3$ ou $CF_3$;
$R_8$ et $R_9$ sont, indépendamment, des groupes alcoyle en $C_1$—$C_3$;
$R_{10}$ est H, Cl, Br ou un groupe alcoyle en $C_1$—$C_3$;
$R_{11}$ est H, $CH_3$, Cl ou Br;
W est O ou S;
A' est

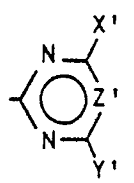 , 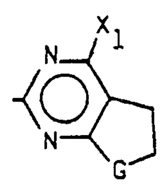 , 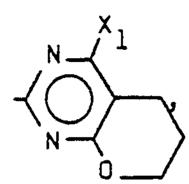 .

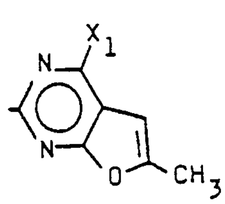 ou 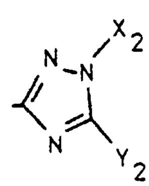 ;

G est O ou $CH_2$;
X' est H, $CH_3$, $OCH_3$ ou Cl;
$X_1$ est $CH_3$, $OCH_3$ ou $OC_2H_5$;
$X_2$ est $CH_3$, $C_2H_5$ ou $CH_2CF_3$;
Y' est $CH_3$, $OCH_3$, $OC_2H_5$, $CH_2OCH_3$, $NH_2$, $NHCH_3$, $N(CH_3)_2$, $CH(OCH_3)_2$,

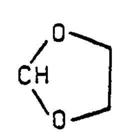 ,

$C_2H_5$, $CF_3$, $CH_2{=}CHCH_2O$, $SCH_3$, $HC{\equiv}CCH_2O$ ou $CF_3CH_2O$;

$Y_2$ est $C_2H_5$, $CH_3$, $OCH_3$, $OC_2H_5$, $SCH_3$ ou $SC_2H_5$; et

Z′ est CH, N, $CCH_3$, $CC_2H_5$, CCl ou CBr;

et leurs sels convenant pour l'agriculture; avec les conditions suivantes;

1) dans les structures $J'_1$ et $J'_2$, quand $Q_1$ est O et $R'_1$ est autre que H, Cl, Br ou $NO_2$, alors au moins un de $R_2$ et $R_3$ est un groupe alcoyle, et quand Q est S, alors $R'_1$ ne peut pas être $NO_2$;

2) dans la structure $J'_3$, quand $R'_1$ est $NO_2$ ou $SO_2NR_8R_9$, alors $R_{10}$ est un groupe alcoyle en $C_1$—$C_3$ et $R_{11}$ est $CH_3$;

3) quand X′ est Cl, alors Z′ est CH et Y′ est $OCH_3$, $NH_2$, $NHCH_3$ ou $N(CH_3)_2$;

4) quand Q est SO, alors W est O; et

5) quand $R'_4$ est autre que H, alors $R'_1$ est H.

qui comprend les étapes consistant à:

(I) (a) mettre en contact et faire réagir un sulfonamide approprié de la formule générale

$$J'SO_2NH_2 \qquad (IV')$$

avec du carbonate de diphényle en présence d'une base forte;

(b) isoler le sulfonylcarbamate résultant de formule

O
||
$J'SO_2NHCOC_6H_5$

et mettre en contact et faire réagir ce sulfonylcarbamate avec une amine appropriée

HN—A′ (III′)
|
H

de manière à produire des composés de la formule désirée, ou

(II) (a) mettre en contact et faire réagir le sulfonamide (IV′) avec un méthylcarbamate approprié de formule

O
||
$CH_3OC$—N—A′ (V′)
|
R

en présence de triméthylaluminium; et

(b) isoler le produit résultant de la formule désirée; ou

(III) (a) mettre en contact et faire réagir un sulfonyl isocyanate de formule

$$J'SO_2NCW \qquad (VI')$$

avec une amine appropriée de la formule (III′) dans un solvant aprotique inerte et

(b) isoler le produit résultant de la formule désirée.

17. Un procédé selon la revendication 16, dans lequel J′ est $J'_1$ et $R_4$ est H.

18. Un procédé selon la revendication 16, dans lequel J′ est $J'_2$.

19. Un procédé selon la revendication 17 ou 18, dans lequel W est O et R est H.

20. Un procédé selon la revendication 19, dans lequel $R'_1$ est H, Cl, $CH_3$, $OCH_3$, $CO_2R_5$ ou $SO_2R_6$.

21. Un procédé selon la revendication 20, dans lequel $R'_4$ (s'il est présent) et $R'_1$ sont H et $R_2$ et $R_3$ sont, indépendamment H ou $CH_3$.

22. Un procédé selon la revendication 16, dans lequel J′ est $J'_3$, W est O et R est H.

23. Un procédé selon la revendication 22, dans lequel $R_{11}$ est H et $R'_1$ est H, Cl, $CH_3$, $OCH_3$, $CO_2R_5$ ou $SO_2R_6$ et $SO_2NHC(W)NRA'$ est lié à la position 7.

24. Un procédé selon la revendication 23, dans lequel $R'_1$ est H et $R_{10}$ est H ou $CH_3$.

25. Un procédé selon les revendications 21 ou 24, dans lequel A′ est

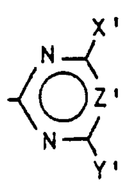 ;

Z' est CH ou N; et
X' est $CH_3$, $OCH_3$ ou Cl.

26. Un procédé selon la revendication 25, dans lequel Y' est $CH_3$, $OCH_3$, $CH_2OCH_3$ ou $N(CH_3)_2$.

27. Un procédé selon la revendication 16, dans lequel le produit est le N,N-dioxyde de N - [(4,6 - diméthylpyrimidin - 2 - yl) - aminocarbonyl] - 2,3 - dihydro - 2 - méthyl - 7 - benzothiophènesulfonamide ou un de ses sels convenant pour l'agriculture.

28. Un procédé selon la revendication 16, dans lequel le produit est le 1,1-dioxyde de N - [(4 - méthoxy - 6 - méthylpyrimidin - 2 - yl) - aminocarbonyl] - 2,3 - dihydro - 2 - méthyl - 7 - benzothiophènesulfonamide ou un de ses sels convenant pour l'agriculture.

29. Un procédé selon la revendication 16, dans lequel le produit est le 1,1-dioxyde de N - [(4,6 - diméthoxypyrimidin - 2 - yl) - aminocarbonyl] - 2,3 - dihydro - 2 - méthyl - 7 - benzothiophènesulfonamide ou un de ses sels convenant pour l'agriculture.

30. Un procédé selon la revendication 16, dans lequel le produit est le 1,1-dioxyde de N - [(4,6 - diméthyl - 1,3,5 - triazin - 2 - yl - aminocarbonyl] - 2,3 - dihydro - 2 - méthyl - 7 - benzothiophènesulfonamide ou un de ses sels convenant pour l'agriculture.

31. Un procédé selon la revendication 16, dans lequel le produit est le 1,1-dioxyde de N - [(4 - méthoxy - 6 - méthyl - 1,3,5 - triazin - 2 - yl) - aminocarbonyl] - 2,3 - dihydro - 2 - méthyl - 7 - benzothiophènesulfonamide ou un de ses sels convenant pour l'agriculture.

32. Un procédé selon la revendication 16 , dans lequel le produit est le 1,1-dioxyde de N - [(4,6 - diméthoxy - 1,3,5 - triazin - 2 - yl) - aminocarbonyl] - 2,3 - dihydro - 2 - méthyl - benzothiophènesulfonamide ou un de ses sels convenant pour l'agriculture.

33. Un procédé selon la revendication 16, dans lequel le produit est le 2,3 - dihydro - N - [(4 - méthoxy - 6 - méthylpyrimidin - 2 - yl)aminocarbonyl] - 2 - méthylbenzofuran - 7 - sulfonamide ou un de ses sels convenant pour l'agriculture.

34. Un procédé selon la revendication 16, dans lequel le produit est le N - [(4,6 - diméthoxypyrimidin - 2 - yl) - aminocarbonyl] - 2,3 - dihydro - 2 - méthylbenzofuran - 7 - sulfonamide ou un de ses sels convenant pour l'agriculture.

35. Un procédé selon la revendication 16, dans lequel le produit est le 1,1-dioxyde de N - [(4,6 - diméthoxypyrimidin - 2 - yl)aminocarbonyl] - 2,3 - dihydrobenzo(b)thiophène - 7 - sulfonamide ou un de ses sels convenant pour l'agriculture.

36. Un procédé selon la revendication 16, dans lequel le produit est le 1,1-dioxyde de 2,3 - dihydro - N - [(4 - méthoxy - 6 - méthylpyrimidin - 2 - yl)aminocarbonyl]benzo(b)thiophène - 7 - sulfonamide ou un de ses sels convenant pour l'agriculture.

37. Un procédé selon la revendication 16 dans lequel
J' est $J_1'$ ou $J_3'$;
$R_1'$, $R_2$, $R_3$, $R_4$, $R_5$, $R_6$, $R_7$, $R_8$ et $R_9$ sont tels que définis dans la revendication 16;
$R_1'$ est H,
$R_{10}$ est H, $CH_3$, Cl ou Br,
W est O,
Q est O, S ou $SO_2$,
A' est tel que défini dans la revendication 16 à l'exception de

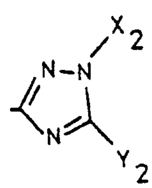

X', $X_1$, G et Z' sont tels que définis dans la revendication 16,
Y' est $CH_3$, $OCH_3$, $OC_2H_5$, $CH_2OCH_3$, $NH_2$, $NHCH_3$, ou $N(CH_3)_2$.

38. Une composition utile pour lutter contre la croissance de végétation indésirable, qui comprend une quantité efficace d'un composé de formule (I) ou (I') tel que défini dans l'une quelconque des revendications 1 à 37 et au moins un des ingrédients suivants: agent tensio-actif, diluant solide ou liquide.

39. Un procédé de lutte contre le croissance de végétation indésirable, qui comprend l'application au lieu à protéger d'une quantité efficace d'un composé de formule (I) ou (I') tel que défini dans l'une quelconque des revendications 1 à 37.

40. Un procédé pour régler la croissance de plantes, qui comprend l'application au site de ces plantes d'une quantité efficace, mais substantiellement non-phytotoxique, d'un composé d'un composé de formule (I) ou (I') tel que défini dans l'une quelconque des revendications 1 à 37.